(19) Europäisches Patentamt / European Patent Office / Office européen des brevets

(11) EP 2 985 352 A1

(12) **EUROPÄISCHE PATENTANMELDUNG**

(43) Veröffentlichungstag: **17.02.2016 Patentblatt 2016/07**

(51) Int Cl.: ***C12Q 1/68*** *(2006.01)*

(21) Anmeldenummer: **15184023.8**

(22) Anmeldetag: **23.09.2009**

(84) Benannte Vertragsstaaten: **AT BE BG CH CY CZ DE DK EE ES FI FR GB GR HR HU IE IS IT LI LT LU LV MC MK MT NL NO PL PT RO SE SI SK SM TR**

(62) Dokumentnummer(n) der früheren Anmeldung(en) nach Art. 76 EPÜ: **09171107.7 / 2 309 001**

(71) Anmelder: **Analytik Jena AG**
**07745 Jena (DE)**

(72) Erfinder:
- **MÖLLER, Eva**
  **07751 Jena (DE)**
- **WLOTZKA, Britta**
  **99084 Erfurt (DE)**
- **RURYK, Andriy**
  **07745 Jena (DE)**

(74) Vertreter: **Winter, Brandl, Fürniss, Hübner, Röss, Kaiser, Polte - Partnerschaft mbB**
**Patent- und Rechtsanwaltskanzlei**
**Alois-Steinecker-Strasse 22**
**85354 Freising (DE)**

Bemerkungen:
Diese Anmeldung ist am 07-09-2015 als Teilanmeldung zu der unter INID-Code 62 erwähnten Anmeldung eingereicht worden.

(54) **VERFAHREN ZUR IN VITRO ERFASSUNG UND UNTERSCHEIDUNG VON PATHOPHYSIOLOGISCHEN ZUSTÄNDEN**

(57) Die vorliegende Erfindung betrifft die Verwendung von definierten Polynukleotiden zur Bildung wenigstens eines Multigenbiomarkers zur Herstellung eines Multiplex-Assays als Hilfsmittel zur in vitro Erfassung und/oder Früherkennung und/oder Unterscheidung und/oder Verlaufsbeobachtung und/oder Beurteilung von pathophysiologischen Zuständen eines Patienten, wobei der pathophysiologische Zustand ausgewählt wird aus der Gruppe bestehend aus: SIRS, Sepsis und deren Schweregraden; sepsisähnlichen Zuständen; septischem Schock; Bakteriämie, infektiösem/nichtinfektiösem Multiorganversagen; Früherkennung dieser Zustände; Fokuskontrolle; Kontrolle von chirurgischen Sanierungsmaßnahmen des Infektionsfokus; Responder/non-Responder für eine bestimmte Therapie; Therapiekontrolle; Unterscheidung zwischen infektiöser und nicht- infektiöser Genese bei systemischen Reaktionen des Organismus, wie z.B. SIRS, Sepsis, postoperative Komplikationen, chronischem und/oder akuter Organdysfunktion, Schock-Reaktion, Entzündungsreaktion und/oder Trauma.

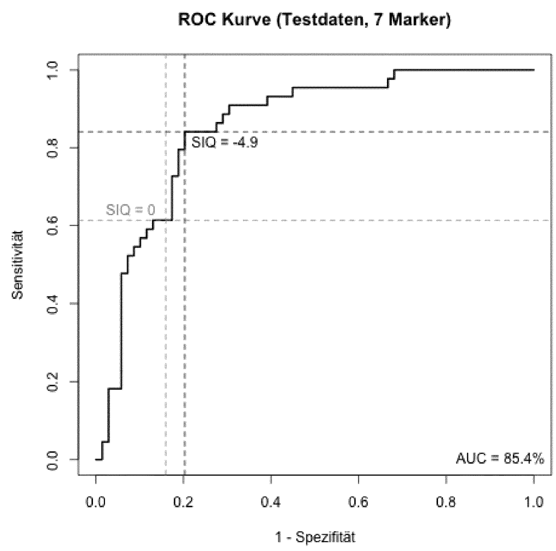


Fig. 1

EP 2 985 352 A1

## Beschreibung

Die vorliegende Erfindung betrifft ein Verfahren zur in vitro Erfassung und/oder Früherkennung und/oder Unterscheidung und/oder Verlaufsbeobachtung von pathophysiologischen Zuständen gemäß Anspruch 1 sowie die Verwendung von wenigstens drei Polynukleotiden zur Bildung wenigstens eines Multigenbiomarkers zur Herstellung eines Multiplex-Assays als Hilfsmittel zur Beurteilung, ob bei einem Patienten ein pathophysiologischer Zustand vorliegt, und/oder zur Feststellung des Schweregrades und/oder zur Früherkennung und/oder zur Unterscheidung und/oder Verlaufsbeobachtung von pathophysiologischen Zuständen gemäß Anspruch 4.

Insbesondere betrifft die vorliegende Erfindung die Verwendung von Polynukleotiden zur Erfassung von Genaktivitäten von mindestens einem Multigenbiomarker, zur Herstellung eines Hilfsmittels zur Diagnose bei Patienten mit bestimmten pathophysiologischen Zuständen, wie beispielsweise Sepsis und Sepsis-ähnliche Zuständen, mit ähnlichen Merkmalen wie ein "In Vitro Diagnostic Multivariate Index Assay" (IVDMIA).

Sepsis ("Blutvergiftung") ist eine lebensbedrohliche Infektion, die den gesamten Organismus erfasst. Sie ist mit hoher Sterblichkeit verbunden, kommt immer häufiger vor und erfasst Menschen in jedem Lebensalter. Sepsis gefährdet den medizinischen Fortschritt in vielen Bereichen der Hochleistungsmedizin und verbraucht einen Großteil der Ressourcen im Gesundheitswesen. Die Sterblichkeit der schweren Sepsis hat sich in den letzten Jahrzehnten nicht entscheidend verbessert. Die letzten beiden Innovationssprünge nach Einführung der Blutkultur (ca. 1880) waren die Einführung der Antibiotika vor über 60 und der Beginn der Intensivmedizin vor etwa 50 Jahren. Um heute ähnlich entscheidende Behandlungsfortschritte zu erzielen, müssen neuartige Diagnostika zur Verfügung gestellt werden.

Sepsis wird durch Infektionserreger verursacht. Da es bislang keine spezielle Therapie gegen die Sepsis gibt, hängt der Erfolg der Behandlung weitgehend von der erfolgreichen Bekämpfung der zugrunde liegenden Infektion und der Qualität der intensivmedizinischen Behandlung ab. Entscheidend für das Überleben ist die frühzeitige Gabe eines Antibiotikums, das außerdem den verursachenden Erreger erfolgreich bekämpft [Kumar et. al., 2006]. Defizite der Sepsis-Diagnostik verzögern jedoch den Therapiebeginn und die Wahl eines geeigneten Antibiotikums. Da die Identifizierung des Sepsiserregers mit den derzeitigen Methoden der Blutkultur nur in weniger als 25 % der Sepsisfälle gelingt und die Befunde im Fall des Erregernachweises erst nach 2-3 Tagen vorliegen, muss die initiale Wahl des Antibiotikums oder Antimykotikums (gegen Pilze gerichtete Substanzen) "kalkuliert", d.h. auf Verdacht gewählt werden. In 20 - 30 % der Fälle ist diese Wahl nicht richtig.

Weitere Ursachen für die Verzögerung der Therapie liegen in der Fehlinterpretation der Krankheitssymptome und Laborwerte. Verbesserte Diagnostika, die die Sepsisdiagnose vereinfachen und beschleunigen, können zu einer erheblichen Reduktion der Sepsissterblichkeit und Verkürzung ihrer Behandlungsdauer beitragen. Medizinische Fachgesellschaften bestätigen die Defizite der bisherigen Sepsis-Diagnostik in Umfragen unter nordamerikanischen und europäischen Intensivmedizinern [Marshall et. al., 2003]. Auch die Betroffeneninitiative "Deutsche Sepsis Hilfe e.V." und der Deutschen Sepsis-Gesellschaft beklagen die Defizite.

Im Zuge der Entwicklung marktreifer in-vitro-Diagnostika aus dem Bereich molekularer Diagnostik wurde am 26.7.2007 ein Richtlinienentwurf der Food and Drug Administration (FDA) der Vereinigten Staaten von Amerika veröffentlicht. Diese Richtlinie liefert Empfehlungen, Definitionen und Anhaltspunkte für den Entwicklungs- und Zulassungsprozess. Weiterhin werden Spezifikationen für die neue Klasse der "in vitro-diagnostischen multivariaten Index-Assays (IVDMIA)" vorgeschlagen. Kennzeichen dieser Assays sind:

1) Die Kombination von mehreren Einzelwerten mittels eines Interpretationsschrittes, um einen einzelnen patientenspezifischen Ausgabewert in Form eines Index, Score oder einer Klassifikation zu erhalten. Dieser Wert ist für diagnostische Aussagen, zur Schadensbegrenzung, Behandlung oder Vorbeugung einer Krankheit einsetzbar.
2) Das erreichte Ergebnis ist von den Messwerten in einer Weise abgeleitet, die keine Rückschlüsse auf die eigentlichen Messdaten erlaubt. Daher kann das Ergebnis vom End-Anwender nicht bestätigt bzw. nachvollzogen werden.
3) Dadurch ist es notwendig, dem Anwender alle Informationen zur Interpretation des Testergebnisses zur Verfügung zu stellen.

Eine Infektion ist mit den Merkmalen Aufnahme von Pathogenen, deren Vermehrung im Organismus und der damit verbundenen Auslösung von pathophysiologischen und symptomatischen Reaktionen verbunden. Im Unterschied dazu liegen bei einer Kolonisierung keinerlei Krankheitssymptome des Wirts-Organismus vor.
Infolge einer Infektion kommt es innerhalb des Körpers zu einer Konfrontation zwischen den Pathogenen und der Körpereigenen Abwehr. Bei der unspezifischen Abwehr handelt es sich um körpereigene, keimschädigende Substanzen, die im Blut gelöst sind (humoral) sowie um Granulocyten und Makrophagen, die begrenzt in der Lage sind, Eindringlinge, Fremdkörper und Zelltrümmer zu beseitigen. Das Wirkprinzip der spezifischen Abwehr besteht darin, Fremdkörper und Pathogene mit im Blut zirkulierenden Antikörpern zu markieren, um sie anschließend durch T-Lymphozyten vernichten zu lassen.
Bei der Ausbreitung eines Pathogens können mehrere krankheitserzeugende Prozesse initiiert werden. Zum einen werden Abwehrreaktionen wie z. B. Fieber, Gefäßerweiterungen und/oder Einkapselungen ausgelöst. Es kann zu einen Schädigung oder Zerstörung von Geweben, Organen oder Organsystemen z. B. Multiorganversagen (MOV) kommen. In Abhängigkeit vom Pathogen kann der Erreger Gifte, Exotoxine, absondern, die zu teilweise heftigen Reaktionen der Wirtsantwort führen. Eine andere Möglichkeit besteht darin, dass sich Erregerbestandteile, sogenannte Endotoxine, im Falle einer Keimabtötung wie ein Gift auswirken können.
Bei einer Beschränkung des Infektionsgeschehens auf einen Bereich des Organismus spricht man von einer lokalen Infektion, wie z. B. im Falle von Abszessen oder Wundinfektionen. Die Symptome einer lokalen Infektion sind Rötung, Schwellung, Schmerz und eingeschränkte Funktion. Wenn sich die Pathogene dagegen im ganzen Körper z. B. über die Blutbahnen oder die Lymphbahnen ausbreiten handelt es sich um eine allgemeine oder generalisierte oder systemische Infektion. Vom Beginn einer Infektion bis zur Auslösung von Reaktionen (Symptomen) ist abhängig vom Individuum eine unterschiedlich lange Zeitspanne zu beobachten, die als Inkubationszeit bezeichnet wird.

Die vielgestaltige Art, Symptomatik, Schweregrad und Verläufe von Infektionen machen einen spezifischen Nachweis oder eine Differentialdiagnose bezüglich steriler Entzündungs-erkrankungen in der klinischen Routine sehr schwierig und häufig unpräzise. Hierin ist ein Hauptgrund für häufige schwere infektiöse Komplikationen in vielen unterschiedlichen Indikationen und medizinischen Disziplinen zu sehen. Es besteht ein großer medizinischer Bedarf in einer Vielzahl medizinischer Disziplinen mit ausreichender Sensitivität und Spezifität solche infektiösen Komplikationen zu erkennen, durch adäquate klinische Interventionen zu behandeln und eine Verlaufskontrolle der individuellen klinischen Maßnahmen zur Behandlung der infektiösen Komplikationen verfügbar zu machen. Dies gilt insbesondere für den Übergang von lokalen zu generalisierten Infektionen, die in kurzer Zeit zu lebensbedrohlichen Zuständen führen.
Die Unterscheidung von systemisch-inflammatorisch und infektiös bedingten Krankheitszuständen spielt für die klinischen Entscheidungen zur Behandlung von Patienten und anschließender Verlaufsbeobachtung neben der Sepsis auch in einer Reihe von weiteren Indikationen eine wichtige Rolle. In diesem Zusammenhang kann der Behandlung von akut und chronisch kranken Patienten sowie der peri-operative Kontrolle gesehen werden. Es ist bekannt, dass im Fall einer akuten Pankreatitis die Prognose eines letalen Ausgangs durch eine Infektion von 16% auf 40% signifikant verschlechtert. Bei der Ausbildung einer komplexen Superinfektion besteht ein hohes Risiko einer Sepsis mit einer Mortalität von bis zu 90%. Des Weiteren ist die Verlaufsbeobachtung einer intra-abdominalen Inflammation und/oder Infektion bei chronisch kranken, postoperativen und Trauma-Patienten von Bedeutung. Es bestehen auch heute Schwierigkeiten einer eindeutigen klinischen Diagnose von intra-abdominalen Infektionen. Die Verlaufsbeobachtung chronisch Kranker, wie zum Beispiel Patienten mit Leberzirrhose oder Niereninsuffizienz ist von klinischer Relevanz, da diese Patientengruppe in Abhängigkeit der Organdekompensation prädestiniert sein kann inflammatorische und oder infektiöse Krankenverläufe zu nehmen. Insbesondere niereninsuffiziente Patienten mit Peritonealdialyse neigen zu chronischen Inflammationen und Infektionen [Blake 2008]. Von besonderem Interesse ist die Beobachtung von Patienten mit Leberzirrhose, da diese spontane bakterielle Peritonitiden entwickeln können, die eine hohe Mortalität aufweisen. [Koulaouzidis et al. 2009]. Die Diagnose von sekundären Peritonitiden im Rahmen einer postoperativen Nachbehandlung ist von großem klinischem Wert und kann den Operationserfolg stark beeinflussen. Postoperative Infektionen sind auch heute noch ein großes Problem in der chirurgischen Behandlung. Ein Prozent der durchgeführten Laparotomien führen zu Komplikationen nach der Operation. Dabei kann die Komplikationsrate zwischen den chirurgischen Prozeduren stark schwanken. Insbesondere Eingriffe am Magen-Darm-Trakt können durch Nahtinsuffizienzen zu einer fulminanten Ausbreitung von Bakterien in den sterilen Bauchraum führen. Infektiöse Verläufe spielen unter anderem auch in der Operationsfolgebehandlung nach Transplantationen, Thorakotomien, Extremitäten- und Gelenkkorrekturen und neurochirurgischen Eingriffen eine Rolle.
Dem Fachmann ist bekannt, dass es sich bei diesen Ausführungen um lediglich um eine beispielhafte Auswahl handelt und es zahlreiche weitere Anwendungsfelder gibt, für die die Identifizierung einer infektiösen Komplikation von großer Wichtigkeit ist. Mit der vorliegenden Erfindung wird eine Lösung für dieses diagnostische Problem bereitgestellt.

Die vorliegende Erfindung betrifft insbesondere Gene und/oder deren Fragmente und ihre Verwendung zur Erstellung von Multigenbiomarkern, welche spezifisch für einen Zustand und/oder Untersuchungsfrage sind.

Die Erfindung betrifft ferner von den Markergenen abgeleitete PCR-Primer und Sonden für Hybridisierungs- bzw. Vervielfertigungsverfahren.

Nach wie vor ist die Sepsis eines der schwierigsten Krankheitsbilder in der modernen Intensivmedizin, wobei für den klinisch tätigen Arzt nicht nur die Therapie, sondern auch die Diagnose eine Herausforderung darstellt. Trotz Fortschritten im pathophysiologischen Verständnis und der supportiven Behandlung von Intensivpatienten sind generalisierte inflammatorische Zustände wie SIRS und Sepsis bei Patienten auf Intensivstationen sehr häufig auftretende und erheblich zur Sterblichkeit beitragende Erkrankungen [Marshal et al., 2003; Alberti et al., 2003]. Die Sterblichkeit beträgt ca. 20 % bei SIRS, ca. 40 % bei Sepsis und steigt bei Entwicklung von multiplen Organdysfunktionen bis auf 70-80 % an [Brun-Buisson et al., 1995; Le-Gall et al., 1995; Brun-Buisson et al., 2003]. Der Morbiditäts- und Letalitätsbeitrag von SIRS und Sepsis ist von fachübergreifender klinischmedizinischer Bedeutung, denn dadurch werden in zunehmendem Maße die Behandlungserfolge der fortgeschrittensten Therapieverfahren zahlreicher medizinischer Fachgebiete (z.B. Traumatologie, Neurochirurgie, Herz-/Lungenchirurgie, Viszeralchirurgie, Transplantationsmedizin, Hämatologie/ Onkologie, etc.) gefährdet, denen ohne Ausnahme eine Erhöhung des Krankheitsrisikos für SIRS und Sepsis immanent ist. Dies drückt sich auch im kontinuierlichen Anstieg der Häufigkeit der Sepsis aus: zwischen 1979 und 1987 wurde ein Anstieg um 139%, nämlich von 73,6 auf 176 Krankheitsfälle je 100.000 Krankenhauspatienten verzeichnet [MMWR Morb Mortal Wkly Rep 1990]. Die Senkung der Morbidität und Letalität einer Vielzahl von schwer erkrankten Patienten ist daher an einen gleichzeitigen Fortschritt in der Vorbeugung, Behandlung und insbesondere der Erkennung und Verlaufsbeobachtung der Sepsis und schweren Sepsis gebunden.

Im Laufe der Zeit hat der Sepsisbegriff einen erheblichen Bedeutungswandel erfahren. Eine Infektion bzw. der dringliche Verdacht auf eine Infektion sind auch heute noch wesentlicher Bestandteil aktueller Sepsisdefinitionen. Besondere Berücksichtigung findet jedoch dabei die Beschreibung Infektionsort-ferner Organfehlfunktionen im Rahmen der inflammatorischen Wirtsreaktion. Im internationalen Schrifttum haben sich zwischenzeitlich die Kriterien der Konsensuskonferenz des "American College of Chest Physicians/Society of Critical Care Medicine Consensus Conference (ACCP/SCCM)" aus dem Jahr 1992 am breitesten zur Definition des Sepsis-Begriffs durchgesetzt [Bone et al., 1992]. Entsprechend dieser Kriterien werden die klinisch definierten Schweregrade "systemic inflammatory response syndrom" (SIRS), "Sepsis", "severe Sepsis" und "septic shock" unterschieden. Als SIRS wird dabei die systemische Antwort des inflammatorischen Systems auf einen nichtinfektiösen Reiz definiert. Dazu müssen mindestens zwei der folgenden klinischen Kriterien erfüllt sein: Fieber >38°C oder Hypothermie <36°C, eine Leukozytose >12g/l oder eine Leukopenie <4g/l bzw. eine Linksverschiebung im Differentialblutbild, eine Herzfrequenz von über 90/min, eine Tachypnoe >20 Atemzüge/min oder ein $PaCO_2$ (Partialdruck des Kohlendioxid im arteriellen Blut) <4,3 kPa. Diese Definition hat eine hohe Sensitivität, aber eine niedrige Spezifität. Für intensivmedizinische Belange ist sie wenig hilfreich, da in der Regel jeder Intensivpatient, zumindest für kurze Zeit, die SIRS-Kriterien erfüllt.

Als Sepsis werden solche klinischen Zustände definiert, bei denen die SIRS-Kriterien erfüllt sind und ursächlich eine Infektion nachgewiesen wird oder zumindest sehr wahrscheinlich ist. Eine Infektion wird definiert als ein pathologischer Prozess, welcher durch eine Invasion von Pathogenen beziehungsweise potentiell pathogenen Organismen in ein normalerweise steriles Gewebe hervorgerufen wird. Wenn es dem Körper nicht gelingt, diese Infektion auf den Ursprungsort zu begrenzen, induzieren die Krankheitserreger oder deren Toxine in den vom Infektionsort entfernten Organen bzw. Geweben des Körpers eine Entzündung. Eine sofortige intensivmedizinische Behandlung, die zielgerichtete Gabe von Antibiotika und die operative Sanierung des infektiösen Herdes sind nötig, um eine Genesung zu erreichen. Eine schwere Sepsis ist vom zusätzlichen Auftreten von Organfehlfunktionen gekennzeichnet. Häufige Organfehlfunktionen sind Änderungen der Bewusstseinslage, eine Oligurie, eine Laktazidose oder eine Sepsis-induzierte Hypotension mit einem systolischen Blutdruck von weniger als 90 mmHg bzw. ein Druckabfall um mehr als 40 mmHg vom Ausgangswert. Wenn eine solche Hypotension nicht durch die Verabreichung von Kristalloiden und/oder Kolloiden zu beheben ist und es zusätzlich zu einer Katecholaminpflichtigkeit des Patienten kommt, so spricht man von einem septischen Schock. Dieser wird bei etwa 20 % aller Sepsispatienten nachgewiesen.

Es besteht unter vielen Medizinern Einigkeit darüber, dass die Konsensuskriterien nach [Bone et al., 1992] keiner spezifischen Definition von Sepsis entsprechen. So zeigte eine von der European Society of Intensive Care Medicine (ESICM) durchgeführte Umfrage, dass 71 % der befragten Ärzte trotz langjähriger klinischer Erfahrungen Unsicherheit bei der Diagnosestellung einer Sepsis hatten [Poeze et al., 2003]. Der Versuch, eine einheitliche Terminologie durchzusetzen, fand in der klinischen Umsetzung variable Akzeptanz. Insbesondere die Fortschritte im Verständnis der Pathophysiologie der Sepsis veranlasste verschiedene Experten, nach einer entsprechenden Modifikation der bisherigen Definitionen zu suchen. Die Definitionen von Sepsis, schwerer Sepsis und septischem Schock wurden bestätigt und als nützlich für Kliniker und Forscher beurteilt. Allerdings wurden die diagnostischen Kriterien der Sepsis erheblich erweitert, um dem klinischen Aspekt der Infektabwehr gerecht zu werden. Die internationale Sepsis-Konferenz 2001 schlug außerdem ein neues Konzept (PIRO genannt) zur Beschreibung der Sepsis vor, welches sich aus den Kriterien Prädisposition, Infektion, Immunantwort

(Response) und Organdysfunktion zusammensetzt [Levy et al., 2003]. Trotz einer neuen Definition der SIRS/Sepsis mit dem Akronym PIRO [Opal et al., 2005] wird in den meisten Studien immer noch die ACCP/SCCM Konsensuskonferenz aus dem Jahre 1992 benutzt [Bone et al., 1992], um ihre Patienten zu klassifizieren.

Mehrere Ansätze zur Diagnosestellung von SIRS und Sepsis wurden entwickelt. Diese Ansätze können in 3 Gruppen geteilt werden.

Der erste Gruppe enthält Score-Systeme wie beispielsweise APACHE, SAPS und SIRS welche die Patienten auf der Basis einer Vielzahl physiologischer Indices stratifizieren können. Während in einigen Studien für den APACHE II Score ein diagnostisches Potential nachgewiesen werden konnte, haben andere Studien gezeigt, dass APACHE II und SAPS II nicht zwischen Sepsis und SIRS differenzieren können [Carrigan et al., 2004].

Die zweite Gruppe enthält Proteinmarker, die aus Plasma und Serum nachgewiesen werden. Solche sind zum Beispiel CA125, S100B, Copeptin, Glycin N-acyltransferase (GNAT), Protachykinin und/oder seine Fragmente, Aldose 1-Epimerase (Mutarotase), Chp, Carbamoylphosphat Synthetase 1, LASP-1 (Brahms Diagnostika GmbH Deutschland), IL-1 Ra, MCP-1, MPIF-1, TNF-alpha, TNF-R1, MIG, BLC, HVEM, IL-10, IL-15, MCP-2, M-CSF, MIP-3b, MMP-9, PARC, ST-2; IL-6, sIL-2R, CD141, MMP-9, EGF, ENA-78, EOT, Gro-beta, IL-1b, Leptin, MIF, MIP-1a, OSM, Protein C, P-Selectin, und HCC4 (Molecular Staging, Inc., USA) oder CD 14 Antigen, Lipopolysaccharid-Bindungsstellen auf den Proteinen Alkalische Phosphatase und Inter-Alpha-Trypsin Inhibitor (Mochida Pharm Co, Ltd. Japan). Trotz der großen Menge von patentierten Biomarkern konnten sich nur wenige im klinischen Alltag durchsetzen. Von diesen scheinen Procalcitonin (PCT, BRAHMS) und das C-reaktive Protein (CRP, Eli Lilly) die Marker zu sein, die am besten zwischen infektiösen und nicht infektiösen Ursachen der SIRS unterscheiden können.

Procalcitonin ist ein 116 Aminosäuren langes Peptid das eine Rolle bei Entzündungsreaktionen spielt. Dieser Marker ist im Laufe der Zeit zunehmend als neuer Infektionsmarker auf Intensivstationen eingesetzt worden [Sponholz et al., 2006]. Dieser Marker gilt als Infektionsmarker und dient dazu, den Schweregrad der Sepsis festzulegen, wobei die Dynamik der Werte wichtiger ist als die Absolutwerte selbst, um z. B. bei Herzchirurgie-Patienten zwischen infektiöser und nicht-infektiöser Komplikation zu unterscheiden [Sponholz et al., 2006]. Trotz der weitgehenden Akzeptanz des Biomarkers PCT konnte in internationalen Studien gezeigt werden, dass die erreichten Sensitivitäten und Spezifitäten des Sepsismarkers PCT vor allem bei der Abgrenzung einer systemischen bakteriellen SIRS, also Sepsis, von einer nicht -bakteriellen SIRS noch immer unzureichend sind [Ruokonen et al., 1999; Suprin et al. 2000; Ruokonen et al.,2002; Tang et al., 2007a]. Die Meta-Analyse von Tang und Kollegen [Tang et al., 2007a], in der 18 Studien berücksichtigt wurden, zeigt, dass PCT nur schlecht geeignet ist, um SIRS von Sepsis zu diskriminieren. Darüberhinaus betonen die Autoren, dass PCT eine sehr schwache diagnostische Genauigkeit mit einem Odd Ratio (OR) von 7.79 hat. Als Regel benennen die Autoren, dass ein OR <25 nicht aussagekräftig, zwischen 25 und 100 hilfreich und im Falle von mehr als 100 hoch genau ist [Tang et al., 2007a].

C-reaktives Protein (CRP) ist ein 224 Aminosäuren langes Protein, das eine Rolle bei Entzündungsreaktionen spielt. Die Messung von CRP soll dazu dienen, um den Krankheitsverlauf sowie die Wirksamkeit der gewählten Therapie zu verfolgen.

In mehreren Berichten wurde beschrieben, dass im intensivmedizinischen Bereich PCT ein besser geeigneter diagnostischer Marker als CRP ist [Sponholz et al., 2006; Kofoed et al., 2007]. Darüber hinaus wird PCT als besser geeignet als CRP angesehen, um eine nicht infektiöse versus infektiöse SIRS sowie bakterielle versus virale Infektion zu unterscheiden [Simon et al., 2004].

Es ist für den Fachmann naheliegend das die, mit dieser Erfindung bereitgestellten, Lösung mit den vorgenannten Biomarkern wie z. B. aber nicht ausschließlich PCT oder CRP kombiniert werden kann um die diagnostische Aussage zu erweitern.

Die dritte Gruppe enthält Biomarker oder Profile, die auf Transkriptom - Ebene identifiziert wurden. Diese molekularen Parameter sollten eine bessere Korrelation der molekularen inflammatorischen/immunologischen Wirtsantwort mit dem Schweregrad der Sepsis ermöglichen, aber auch Aussagen zur individuellen Prognose liefern. Nach derartigen Biomarkern wird derzeit von verschiedenen wissenschaftlichen Gruppen und kommerziellen Organisationen intensiv gesucht, wie zum Beispiel Veränderungen der Cytokinkonzentrationen im Blut verursacht durch Bakterienzellwandbestandteile wie Lipopolysaccharide [Mathiak et al., 2003], oder Verwendung von Genexpressionsprofilen in einer Blutprobe zur Bestimmung von Unterschieden bei überlebenden und nicht-überlebenden Sepsispatienten [Pachot et al., 2006]. Genexpressionsprofile oder Klassifikatoren sind für die Bestimmung des Schwergrads von Sepsis [WO 2004/087949], die Unterscheidung zwischen einer lokalen oder systemischen Infektion [nicht veröffentlichte DE 10 2007 036 678.9], die Identifizierung der Infektionsquelle [WO 2007/124820] oder von Genexpressionssignaturen für die Unterscheidung zwischen mehreren Ätiologien und Pathogen-assoziierten Signaturen [Ramilo et al., 2007] geeignet. Allerdings besteht aufgrund der unzureichenden Spezifität und Sensitivität der Konsensuskriterien nach [Bone et al., 1992], der aktuell verfügbaren Proteinmarker sowie aufgrund des Zeitbedarfs des Nachweises der Infektionsursache durch Blutkultur ein dringender Bedarf für neue Verfahren, die die Komplexität der Erkrankung berücksichtigen. Viele Geneexpressionsstudien, die entweder einzelne Gene und/oder Kombinationen von Genen, die als Klassifkatoren benannt sind, verwenden sowie zahlreiche Beschreibungen von statistischen Verfahren zur Ableitung eines Score und/oder Index [WO03084388; US6960439] gehören zum Stand der Technik.

Es besteht heute ein Konsens dahingehend, dass komplexe Erkrankungen sinnvoll nur über mehrere Parameter beschrieben werden können.

In zunehmendem Maße finden molekulare Signaturen Eingang in die klinische Diagnostik, insbesondere bei komplexen Erkrankungen, die mit herkömmlichen Biomarkern nicht erfasst werden können, aber auch zur Beurteilung von Risiken für die Patienten und zur Identifizierung von Respondern beim Einsatz von Medikamenten und Therapien. Nachfolgende Aufzählung soll den aktuellen Stand und die Einsatzgebiete der Genexpressionsdiagnostik verdeutlichen.

1) Die Microarray-basierte, 70 Gene umfassende Signatur namens MammaPrint (Agendia, NL) erlaubt es, eine Prognose über das Rezidiv- und Metastasierungsrisiko von Frauen mit Brustkrebs zu treffen. Dabei wird untersucht, ob das Risiko, in den nächsten Jahren entfernte Metastasen zu entwickeln, als hoch oder niedrig eingestuft werden kann und sie von einer Chemotherapie profitieren würden. Die Zulassung dieses Tests durch die FDA brachte die Entwicklung von Richtlinien für eine neue Klasse von diagnostischen Tests, sog. IVDMIA (in vitro diagnostic multivariate index assay) mit sich. Die MammaPrint-Signatur wird auf einem Mikroarray in den Laboren des Herstellers gemessen und berechnet.

2) An Formalin-fixierten Gewebeproben wird mittels des Oncotype DX -Multigen-Assays (Genomic Health, USA) die Wahrscheinlichkeit des Wiederauftretens von Brustkrebs in Patientinnen beurteilt, sowie das Ansprechen der Patientinnen auf Chemotherapie geprüft. 21 Gene werden als "Recurrence-Score" zusammengefasst. Die Messung findet in den Räumen der Firma statt, es kommt ebenfalls die TaqMan-PCR Technologie zum Einsatz.

3) Der AlloMap Genexpressionstest der Firma XDx (USA) wird zur Überwachung eventueller Abstoßungsreaktionen bei Patienten mit Herztransplantation eingesetzt, die ca. 30 % der Patienten innerhalb eines Jahres zeigen. Bislang waren zur Diagnose mehrere Biopsien notwendig. Der Test basiert auf 11 quantitativen PCR-Assays (zusätzlich 9 Kontrollen und Referenzen) unter Nutzung der TaqMan Technologie (Hoffman-La Roche) in den Räumen des Herstellers. Das Probenmaterial ist Blut. Bereits zwei Monate nach der Transplantation sind die Messergebnisse zuverlässig und sagen das Ausbleiben von Abstoßungsreaktionen für die nächsten 80 Tage voraus.

Eine Gemeinsamkeit dieser Tests ist, dass die addressierte diagnostische Fragestelllung Untersuchungszeiten bis zum Vorliegen des Ergebnisses von mehreren Tagen erlaubt. Für diagnostische Tests in der Indikation Sepsis dagegen, muß die Information innerhalb eines einzigen Arbeitstages vorliegen.

Bei der Verwendung von Genexpressionsmarkern für die Bestimmung eines pathophysiologischen Zustandes werden stets die in einer Probe vorhandenen Mengen der entsprechenden mRNA, die Genexpressions-Level, quantitativ bestimmt. Die durch diese Genexpressionslevel ermittelte Information ist die jeweilige Über- oder Unterexpression dieser mRNAs die bezogen auf einen Kontroll-Zustand oder bezogen auf Kontroll-Gene experimentell ermittelt wird. Die Feststellung von Über- oder Unterexpression kann analog zur Bestimmung der Konzentration einer Protein-Biomarkers gesehen werden.

Mehrere Anwendungen von Genexpressionsprofilen sind in dem Stand der Technik bekannt.

Pachot und Kollegen demonstrieren den Nutzen von Expressionssignaturen für die Verlaufsbeurteilung von Patienten mit septischem Schock. Hier finden sich molekulare Unterschiede, die die Wiederherstellung eines funktionierenden Immunsystems in den Überlebenden wiederspiegeln. 28 Markergene mit Funktionen im innaten Immunsystem zeigen innerhalb des ersten Tages nach Diagnose des septischen Schocks mit hoher Sensitivität (100%) und Spezifität (88%) an, ob die Immunparalyse reversibel ist und damit das Überleben des Patienten ermöglicht. Allerdings war in der Untersuchung das Patientenkollektiv zu klein (38) um ein robustes Profil zu erstellen und eine Validierung dieses Datensatzes durch einen unabähngigen Datenatz ist bislang nicht erfolgt. Der Stand der Technik enthält zahlreiche Studien zur Identifikation von Genexpressionsmarkern [Tang et al., 2007b] oder Genexpressionsprofilen für die Feststellung einer systemischen Infektion [Johnson et al., 2007].

Tang und Kollegen [Tang et al., 2007b] suchten in einer bestimmten Blutzellpopulation, den Neutrophilen, nach einer Signatur, welche eine Unterscheidung von Patienten mit SIRS und Sepsis ermöglicht. 50 Marker aus dieser Zellpopulation genügen um die Immunantwort auf eine systemische Infektion wiederzugeben und neue Erkenntnisse zur Pathophysiologie und den beteiligten Signalwegen zu ermöglichen.

Die Klassifikation von Patienten mit und ohne Sepsis gelingt mit hoher Sicherheit (PPV 88% bzw. 91% in Trainings- und Testdatensatz). Die Anwendbarkeit für die klinische Diagnose ist allerdings dadurch begrenzt, dass im Blut diese Signatur von Signalen aus anderen Blutzelltypen überlagert werden kann. Bezüglich der Anwendbarkeit ist die Präparation dieser Blutzellpopulation mit erheblich erhöhtem Aufwand verbunden. Die Aussagekraft der in dieser Studie publizierten Ergebnisse ist für praktische Anwendungen jedoch limitiert, weil die Patientenauswahl sehr stark heterogen war. Es wurden Patienten in die Studie eingeschlossen, die stark unterschiedliche Begleiterkrankungen wie z. B zu 11% bis 16% Tumorerkrankungen aufwiesen oder sehr stark unterschiedlichen therapeutischen Maßnahmen unterlagen (z.B. 27% bis 64% Vasopressor-Therapie), wodurch die Genexpressionsprofile stark beeinflusst wurden.

Johnson und Kollegen [Johnson et al., 2007] beschreiben an einem Kollektiv von Traumapatienten, dass sich die Ausprägung einer Sepsis bereits bis zu 48 Stunden vor der klinischen Diagnose anhand molekularer Veränderungen messen lässt. Die Traumapatienten wurden über mehrere Tage untersucht. Ein Teil der Patienten entwickelte eine Sepsis. Nichtinfektiöse SIRS-Patienten wurden mit präseptischen Patienten verglichen. Die identifizierte Signatur aus 459 Transkripten setzt sich aus Markern der Immunantwort und Entzündungsmarkern zusammen. Probenmaterial war Vollblut, die Analysen wurden auf einem Mikroarray durchgeführt. Unklar ist, ob sich diese Signatur auch auf andere Kollektive septischer bzw. präseptischer Patienten ausdehnen lässt. Eine Klassifikation und der diagnostische Nutzen dieser Signatur wurde nicht gezeigt.

Weiterhin werden im Stand der Technik andere Signaturen beschrieben, beispielsweise die Antwort des Wirtes auf eine Infektion.

Die Spezifität der Wirtsantwort auf unterschiedliche Erreger ist bisher in mehreren experimentellen Systemen untersucht worden. In keiner Studie jedoch wurden Genexpressionsprofile und/oder Signaturen durch Test aus Vollblut von Sepsis-Patienten beschrieben.

Das Ziel von Feezor und Kollegen [Feezor et al., 2003] war es, Unterschiede zwischen Infektionen mit gram-negativen und gram-positiven Erregern zu identifizieren. Blutproben von drei verschiedenen Spendern wurden *ex vivo* mit *E.coli-LPS* und Hitze-inaktiviertem *S.aureus* stimuliert. Mittels Microarraytechnologie wurden Genexpressionsuntersuchungen durchgeführt. Die Arbeitsgruppe fand sowohl Gene, die nach *S.aureus*-Stimulation hochreguliert und nach LPS-Stimulation herunterreguliert waren, als auch Gene die nach LPS-Behandlung stärker exprimiert wurden als nach der Zugabe von Hitze-inaktivierten *S.aureus-Keimen.* Gleichzeitig wurden viele Gene durch gram-positive und gram-negative Stimulation in gleichem Maße hochreguliert. Dies betrifft zum Beispiel die Zytokine TNF-$\alpha$, IL-1$\beta$ und IL-6. Die differentiell exprimierten Gene wurden leider nicht namentlich veröffentlicht, so dass lediglich ein indirekter Vergleich anderen Ergebnissen möglich ist. Neben der Genexpression wurden von Feezor et al. auch die Plasmakonzentrationen einiger Zytokine untersucht. Dabei korrelierten die Genexpressionsdaten nicht zwangsweise mit den Plasmakonzentrationen. Bei der Genexpression wird die Menge an mRNA vermessen. Diese ist jedoch vor der Proteinsynthese posttranskriptionaler Regulation unterworfen, woraus die beobachteten Unterschiede resultieren können.

Die interessanteste Publikation zu diesem Thema wurde von einer texanischen Forschungsgruppe um Ramilo [Ramilo et al., 2007], veröffentlicht. Hier wurden ebenfalls Genexpressionsuntersuchungen an humanen Blutzellen durchgeführt, die Unterschiede in der molekularen Wirtsreaktion auf verschiedene Pathogene aufdeckten. Dazu wurden pediatrische Patienten mit akuten Infektionen, wie z.B. akuten Atemwegserkrankungen, Harnwegsinfektionen, Bakteriämien, lokalen Abszessen, Knochen- und Gelenkinfektionen sowie Meningitis untersucht. Microarrayexperimente wurden mit RNA-Proben durchgeführt, die aus peripheren mononuklearen Blutzellen von jeweils zehn Patienten mit *E.coli*- bzw. *S.aureus*-Infektion isoliert wurden. Die Identifizierung der Erreger erfolgte mittels Blutkultur. Anhand des Trainingsdatensatzes wurden 30 Gene identifiziert, durch deren Verwendung die verursachenden pathogenen Keime mit hoher Genauigkeit diagnostiziert werden konnten.

Trotz der zahlreichen publizierten Studien und der darin beschriebenen individuellen Signaturen, die den Stand der Technik begründen, erlaubt keine davon eine diagnostische Ausage über Sepsis und/oder Sepsis-ähnliche Zustände. Keine dieser Publikationen bietet die Zuverlässigkeit, Genauigkeit und Robustheit der hier offenbarten Erfindung. Diese Studien sind darauf konzentriert, den aus wissenschaftlicher Sicht "besten" Multigenbiomarker (Klassifikator) zu identifizieren, jedoch nicht, wie in der vorliegenden Erfindung, den für eine spezifische klinische Fragestellung optimalen Multigenbiomarker [Simon et al., 2005].

Somit ist es Aufgabe der vorliegenden Erfindung, ein Testsystem zur Verfügung zu stellen, mit dem eine schnelle und zuverlässige Aussage über einen pathophysiologischen Zustand, beispielsweise Sepsis und generalisierte Infektion, möglich ist.

Die Lösung dieser Aufgabe erfolgt verfahrenstechnisch durch die Merkmale des Anspruchs 1.

Bezüglich einer Verwendung wird die Aufgabe durch die Merkmale der Anspruch 4 gelöst.

In allgemeiner Form betrifft die vorliegende Erfindung ein System, das folgende Elemente umfaßt:

- Set von Genaktivitätsmarkern
- Referenzgene als interne Kontrolle der Genaktivitätsmarkersignale in Vollblut
- Detektion hauptsächlich über Real-Time-PCR oder andere Amplifikationsverfahren oder Hybridisierungsverfahren
- Verwendung eines Algorithmus, um die Einzelergebnisse der Genaktivitätsmarker zu einem gemeinsamen numerischen Wert, Index oder auch Score, umzuwandeln
- Darstellung dieses numerischen Wertes auf einer entsprechend eingeteilten Skala
- Kalibrierung, d.h. Einteilung der Skala entsprechend der intendierten Anwendung durch vorherige Validierungsexperimente.

Das System liefert eine Lösung für das Problem der Feststellung von Krankheitszuständen wie z. B. die Unterscheidung von infektiösem und nichtinfektiösem Multiorganversagen aber auch für andere in diesem Kontext relevante Anwendungen und Fragestellungen.

Insbesondere betrifft die vorliegende Erfindung ein Verfahren zur in vitro Erfassung und/oder Früherkennung und/oder Unterscheidung und/oder Verlaufsbeobachtung und/oder Beurteilung von pathophysiologischen Zuständen, ausgewählt aus der Gruppe bestehend aus: SIRS, Sepsis und deren Schweregraden; sepsisähnlichen Zuständen; septischem Schock; Bakteriämie, infektiösem/nicht-infektiösem Multiorganversagen; Früherkennung dieser Zustände; Fokuskontrolle; Kontrolle von chirurgischen Sanierungsmaßnahmen des Infektionsfokus; Responder/non-Responder für eine bestimmte Therapie; Therapiekontrolle; Unterscheidung zwischen infektiöser und nicht- infektiöser Genese bei systemischen Reaktionen des Organismus, wie z.B. SIRS, Sepsis, postoperative Komplikationen, chronischem und/oder akuter Organdysfunktion, Schock-Reaktion, Entzündungsreaktion und/oder Trauma; wobei das Verfahren folgende Schritte umfasst:

a) Isolierung von Probennukleinsäuren aus einer von einem Patienten stammenden Probe;

b) Bestimmung von Genaktivitäten mittels einer Mehrzahl von wenigstens drei Polynukleotiden, ausgewählt aus der Gruppe bestehend aus M2, M3, M4, M6, M7, M8, M9, M10, M12, M13, M15, M16 und M17; und/oder deren Isoformen und/oder deren Genloci und/oder deren Transkripten und/oder Fragmenten mit einer Länge von mindestens 5 Nucleotiden davon, zur Bildung wenigstens eines für die Erfassung und/oder Unterscheidung und/oder den Verlauf von pathophysiologischen Zuständen eines Patienten charakteristischen Multigenbiomarkers; wobei die Polynucleotide gemäß folgender Tabelle definiert sind:

| Polynukleotid | Transkriptvariante | Accession Number | Seq ID |
|---|---|---|---|
| M2 | M2_1 | NM_001031700 | 4 |
| | M2_2 | NM_016613 | 25 |
| | M2_3 | NM_001128424 | 15 |
| M4 | M4_1 | NM_203330 | 35 |
| | M4_2 | NM_000611 | 3 |
| | M4_3 | NM_203329 | 34 |
| | M4_4 | NM_203331 | 36 |
| | M4_5 | NM_001127223 | 11 |
| | M4_6 | NM_001127225 | 12 |
| | M4_7 | NM_001127226 | 13 |
| | M4_8 | NM_001127227 | 14 |
| M6 | M6_1 | NM_001831 | 18 |
| | M6_2 | NM_203339 | 37 |
| M7 | M7_1 | NM_031311 | 28 |
| | M7_2 | NM_019049 | 26 |
| M9 | M9 | NM_006682 | 24 |
| M10 | M10 | NM_033554 | 32 |
| M15 | M15_1 | NM_003580 | 23 |
| | M15_2 | NM_001144772 | 16 |
| M3 | M3_A | NM_001123041 | 9 |
| | M3_B | NM_001123396 | 10 |
| M8 | M8 | NM_025209 | 27 |
| M12 | M12 | NM_002185 | 19 |
| M13 | M13 | NM_001080394 | 7 |
| M16 | M16 | NM_003268 | 22 |
| M17 | M17 | NM_182491 | 33 |

c) Bestimmung von Genaktivitäten wenigstens eines internen Referenzgens, auf die die unter b) bestimmten Genaktivitäten bezogen, insbesondere normalisiert, werden;

d) Bilden eines Wertes aus den einzelnen bestimmten Genaktivitäten des Multigenbiomarkers, der den pathophysiologischen Zustand anzeigt.

Ein bevorzugtes Verfahren ist dadurch gekennzeichnet, dass das Referenzgen ausgewählt wird aus Polynukleotiden der Gruppe bestehend aus R1, R2 und R3 und/oder deren Isoformen und/oder deren Genloci und/oder deren Transkripten und/oder Fragmenten mit einer Länge von mindestens 5 Nucleotiden davon, wobei die Referenzgene gemäß folgender Tabelle definiert sind:

| Polynukleotid | Transkriptvariante | Accession Number | Seq ID |
|---|---|---|---|
| R1 | R1_A | NM_001228 | 17 |
| | R1_B | NM_033355 | 29 |
| | R1_C | NM_033356 | 30 |
| | R1_E | NM_033358 | 31 |
| | R1_F | NM_001080124 | 5 |
| | R1_G | NM_001080125 | 6 |
| R2 | R2_1 | NM_002209 | 20 |
| | R2_2 | NM_001114380 | 8 |
| R3 | R3 | NM_003082 | 21 |

Ein weiter bevorzugtes Verfahren ist dadurch gekennzeichnet, dass als Polynukleotidsequenzen Genloci, sense und/oder antisense Stränge von prä-mRNA und/oder mRNA, small RNA, insbesondere scRNA, snoRNA, micro RNA, siRNA, dsRNA , ncRNA oder transposable Elemente zur Erfassung der Genexpressionsprofile verwendet werden.

Eine weitere bevorzugte Ausführungsform liegt in einem Verfahren, das dadurch gekennzeichnet ist, dass in Schritt b) die Genaktivität von 4, 5, 6, 7, 8, 9, 10, 11, oder 12 Polynucleotiden, oder von sämtlichen 13 Polynucleotiden bestimmt wird, wobei die Polynucleotide ausgewählt werden aus der Gruppe, bestehend aus: M2, M3, M4, M6, M7, M8, M9, M10, M12, M13, M15, M16 und M17 und/oder deren Isoformen und/oder deren Genloci und/oder deren Transkripten und/oder Fragmenten mit einer Länge von mindestens 5 Nucleotiden davon, wobei die Polynucleotide gemäß folgender Tabelle definiert sind:

| Polynukleotid | Transkriptvariante | Accession Number | Seq ID |
|---|---|---|---|
| M2 | M2_1 | NM_001031700 | 4 |
| | M2_2 | NM_016613 | 25 |
| | M2_3 | NM_001128424 | 15 |
| M4 | M4_1 | NM_203330 | 35 |
| | M4_2 | NM_000611 | 3 |
| | M4_3 | NM_203329 | 34 |
| | M4_4 | NM_203331 | 36 |
| | M4_5 | NM_001127223 | 11 |
| | M4_6 | NM_001127225 | 12 |
| | M4_7 | NM_001127226 | 13 |
| | M4_8 | NM_001127227 | 14 |
| M6 | M6_1 | NM_001831 | 18 |
| | M6_2 | NM_203339 | 37 |
| M7 | M7_1 | NM_031311 | 28 |
| | M7_2 | NM_019049 | 26 |
| M9 | M9 | NM_006682 | 24 |
| M10 | M10 | NM_033554 | 32 |
| M15 | M15_1 | NM_003580 | 23 |
| | M15_2 | NM_001144772 | 16 |

(fortgesetzt)

| Polynukleotid | Transkriptvariante | Accession Number | Seq ID |
|---|---|---|---|
| M3 | M3_A | NM_001123041 | 9 |
| | M3_B | NM_001123396 | 10 |
| M8 | M8 | NM_025209 | 27 |
| M12 | M12 | NM_002185 | 19 |
| M13 | M13 | NM_001080394 | 7 |
| M16 | M16 | NM_003268 | 22 |
| M17 | M17 | NM_182491 | 33 |

Hierbei hat sich herausgestellt, dass die Verwendung von 7 Polynucleotiden häufig optimal ist.

Die Erfindung betrifft in einer weiteren Ausführungsform die Verwendung von wenigstens drei Polynukleotiden, ausgewählt aus der Gruppe, bestehend aus: M2, M3, M4, M6, M7, M8, M9, M10, M12, M13, M15, M16 und M17 und/oder deren Isoformen und/oder deren Genloci und/oder deren Transkripten und/oder Fragmenten mit einer Länge von mindestens 5 Nucleotiden davon, zur Bildung wenigstens eines Multigenbiomarkers zur Herstellung eines Multiplex-Assays als Hilfsmittel zur in vitro Erfassung und/oder Früherkennung und/oder Unterscheidung und/oder Verlaufsbeobachtung und/oder Beurteilung von pathophysiologischen Zuständen eines Patienten, wobei der pathophysiologische Zustand ausgewählt wird aus der Gruppe bestehend aus: SIRS, Sepsis und deren Schweregraden; sepsisähnlichen Zuständen; septischem Schock; Bakteriämie, infektiösem/nicht-infektiösem Multiorganversagen; Früherkennung dieser Zustände; Fokuskontrolle; Kontrolle von chirurgischen Sanierungsmaßnahmen des Infektionsfokus; Responder/non-Responder für eine bestimmte Therapie; Therapiekontrolle; Unterscheidung zwischen infektiöser und nicht- infektiöser Genese bei systemischen Reaktionen des Organismus, wie z.B. SIRS, Sepsis, postoperative Komplikationen, chronischem und/oder akuter Organdysfunktion, Schock-Reaktion, Entzündungsreaktion und/oder Trauma; wobei die Polynucleotide gemäß folgender Tabelle definiert sind:

| Polynukleotid | Transkriptvariante | Accession Number | Seq ID |
|---|---|---|---|
| M2 | M2_1 | NM_001031700 | 4 |
| | M2_2 | NM_016613 | 25 |
| | M2_3 | NM_001128424 | 15 |
| M4 | M4_1 | NM_203330 | 35 |
| | M4_2 | NM_000611 | 3 |
| | M4_3 | NM_203329 | 34 |
| | M4_4 | NM_203331 | 36 |
| | M4_5 | NM_001127223 | 11 |
| | M4_6 | NM_001127225 | 12 |
| | M4_7 | NM_001127226 | 13 |
| | M4_8 | NM_001127227 | 14 |
| M6 | M6_1 | NM_001831 | 18 |
| | M6_2 | NM_203339 | 37 |
| M7 | M7_1 | NM_031311 | 28 |
| | M7_2 | NM_019049 | 26 |
| M9 | M9 | NM_006682 | 24 |
| M10 | M10 | NM_033554 | 32 |

(fortgesetzt)

| Polynukleotid | Transkriptvariante | Accession Number | Seq ID |
|---|---|---|---|
| M15 | M15_1 | NM_003580 | 23 |
| | M15_2 | NM_001144772 | 16 |
| M3 | M3_A | NM_001123041 | 9 |
| | M3_B | NM_001123396 | 10 |
| M8 | M8 | NM_025209 | 27 |
| M12 | M12 | NM_002185 | 19 |
| M13 | M13 | NM_001080394 | 7 |
| M16 | M16 | NM_003268 | 22 |
| M17 | M17 | NM_182491 | 33 |

Eine bevorzugte Ausführungsform der vorliegenden Erfindung ist eine Verwendung, bei der der Multigenbiomarker eine Kombination von mehreren Polynukleotid-, insbesondere Gensequenzen ist, anhand deren Genaktivitäten mittels einer Interpretationsfunktion eine Klassifikation durchgeführt und/oder ein Index gebildet wird.

In der Praxis der Anmelderin hat sich herausgestellt, dass eine solche Verwendung besonders geeignet ist, welche dadurch gekennzeichnet, dass die Genaktivitäten mittels enzymatischer Verfahren, insbesondere Amplifikationsverfahren, bevorzugt Polymereasekettenreaktion (PCR), vorzugsweise Real-Time-PCR, insbesondere sondenbasierte Verfahren wie Taq-Man, Scorpions, Molecular Beacons; und/oder mittels Hybridisierungs-verfahren, insbesondere solchen auf Microarrays; und/oder direkter mRNA-Nachweis, insbesondere Sequenzierung oder Massenspektro-metrie; und/oder isothermale Amplifikation, erfasst werden.

Eine weitere bevorzugte Ausführungsform der vorliegenden Erfindung liegt in einer Verwendung, die dadurch gekennzeichnet, dass aus den einzelnen bestimmten Genaktivitäten ein Index gebildet wird, der nach entsprechender Kalibrierung ein Maß für den Schweregrad und/oder den Verlauf des pathophysiologischen Zustands ist, wobei vorzugsweise der Index auf einer leicht interpretierbaren Skala angezeigt wird.

Es ist ferner bevorzugt, dass man die erhaltenen Genaktivitätsdaten zur Herstellung von Software für die Beschreibung mindestens eines pathophysiologischen Zustands und/oder einer Untersuchungsfrage und/oder als Hilfsmittel für Diagnosezwecke und/oder für Patientendatenmangement-Systeme, insbesondere für die Verwendung zur Patientenstratifikation und als Einschlusskriterium für klinische Studien, einsetzt.

Darüber hinaus ist eine Verwendung bevorzugt, bei welcher zur Erstellung der Genaktivitätsdaten solche spezifischen Genloci, sense und/oder antisense Stränge von prä-mRNA und/oder mRNA, small RNA, insbesondere scRNA, snoRNA, micro RNA, siRNA, dsRNA, ncRNA oder transposable Elemente, Gene und/oder Genfragmente mit einer Länge von mindestens 5 Nucleotiden verwendet werden, welche eine Sequenzhomologie von mindestens ca. 10%, insbesondere ca. 20%, vorzugsweise ca. 50%, besonders bevorzugt ca. 80% zu den Polynukleotid-sequenzen M2, M3, M4, M6, M7, M8, M9, M10, M12, M13, M15, M16 und M17aufweisen.

Eine weitere bevorzugte Ausführungsform der vorliegenden Erfindung liegt in einer Verwendung die dadurch gekennzeichnet, dass 4, 5, 6, 7, 8, 9, 10, 11 oder 12 Polynucleotide, oder sämtliche 13 Polynucleotide verwendet werden, wobei die Polynucleotide ausgewählt werden aus der Gruppe, bestehend aus: M2, M3, M4, M6, M7, M8, M9, M10, M12, M13, M15, M16 und M17 und/oder deren Isoformen und/oder deren Genloci und/oder deren Transkripten und/oder Fragmenten mit einer Länge von mindestens 5 Nucleotiden davon, wobei die Polynucleotide gemäß folgender Tabelle definiert sind:

| Polynukleotid | Transkriptvariante | Accession Number | Seq ID |
|---|---|---|---|
| M2 | M2_1 | NM_001031700 | 4 |
| | M2_2 | NM_016613 | 25 |
| | M2_3 | NM_001128424 | 15 |

(fortgesetzt)

| Polynukleotid | Transkriptvariante | Accession Number | Seq ID |
|---|---|---|---|
| M4 | M4_1 | NM_203330 | 35 |
| | M4_2 | NM_000611 | 3 |
| | M4_3 | NM_203329 | 34 |
| | M4_4 | NM_203331 | 36 |
| | M4_5 | NM_001127223 | 11 |
| | M4_6 | NM_001127225 | 12 |
| | M4_7 | NM_001127226 | 13 |
| | M4_8 | NM_001127227 | 14 |
| M6 | M6_1 | NM_001831 | 18 |
| | M6_2 | NM_203339 | 37 |
| M7 | M7_1 | NM_031311 | 28 |
| | M7_2 | NM_019049 | 26 |
| M9 | M9 | NM_006682 | 24 |
| M10 | M10 | NM_033554 | 32 |
| M15 | M15_1 | NM_003580 | 23 |
| | M15_2 | NM_001144772 | 16 |
| M3 | M3_A | NM_001123041 | 9 |
| | M3_B | NM_001123396 | 10 |
| M8 | M8 | NM_025209 | 27 |
| M12 | M12 | NM_002185 | 19 |
| M13 | M13 | NM_001080394 | 7 |
| M16 | M16 | NM_003268 | 22 |
| M17 | M17 | NM_182491 | 33 |

und/oder wobei eine Anzahl von 7 Polynucleotiden bevorzugt ist.

Grundsätzlich kann die Erfindung gemäß einer alternativen Ausführungsform auch ausgeführt werden unter Verwendung mindestens eines Polynukleotids, ausgewählt aus der Gruppe, bestehend aus: M2, M3, M4, M6, M7, M8, M9, M10, M12, M13, M15, M16 und M17 und/oder deren Isoformen und/oder deren Genloci und/oder deren Transkripten und/oder Fragmenten mit einer Länge von mindestens 5 Nucleotiden davon, wobei die Polynucleotide gemäß folgender Tabelle definiert sind:

| Polynukleotid | Transkriptvariante | Accession Number | Seq ID |
|---|---|---|---|
| M2 | M2_1 | NM_001031700 | 4 |
| | M2_2 | NM_016613 | 25 |
| | M2_3 | NM_001128424 | 15 |

(fortgesetzt)

| Polynukleotid | Transkriptvariante | Accession Number | Seq ID |
|---|---|---|---|
| M4 | M4_1 | NM_203330 | 35 |
| | M4_2 | NM_000611 | 3 |
| | M4_3 | NM_203329 | 34 |
| | M4_4 | NM_203331 | 36 |
| | M4_5 | NM_001127223 | 11 |
| | M4_6 | NM_001127225 | 12 |
| | M4_7 | NM_001127226 | 13 |
| | M4_8 | NM_001127227 | 14 |
| M6 | M6_1 | NM_001831 | 18 |
| | M6_2 | NM_203339 | 37 |
| M7 | M7_1 | NM_031311 | 28 |
| | M7_2 | NM_019049 | 26 |
| M9 | M9 | NM_006682 | 24 |
| M10 | M10 | NM_033554 | 32 |
| M15 | M15_1 | NM_003580 | 23 |
| | M15_2 | NM_001144772 | 16 |
| M3 | M3_A | NM_001123041 | 9 |
| | M3_B | NM_001123396 | 10 |
| M8 | M8 | NM_025209 | 27 |
| M12 | M12 | NM_002185 | 19 |
| M13 | M13 | NM_001080394 | 7 |
| M16 | M16 | NM_003268 | 22 |
| M17 | M17 | NM_182491 | 33 |

zur Herstellung eines Assays zur Beurteilung, ob bei einem Patienten ein pathophysiologischer Zustand vorliegt, und/oder zur Feststellung des Schweregrades und/oder des Verlaufs eines pathophysiologischen Zustands.

Hier bei wird der pathophysiologische Zustand ausgewählt aus der Gruppe, bestehend aus: SIRS, Sepsis und deren Schweregraden; sepsisähnlichen Zuständen; septischem Schock; Bakteriämie, infektiösem/nicht-infektiösem Multiorganversagen; Früherkennung dieser Zustände; Fokuskontrolle; Kontrolle von chirurgischen Sanierungsmaßnahmen des Infektionsfokus; Responder/non-Responder für eine bestimmte Therapie; Therapiekontrolle; Unterscheidung zwischen infektiöser und nicht- infektiöser Genese bei systemischen Reaktionen des Organismus, wie z.B. SIRS, Sepsis, postoperative Komplikationen, chronischem und/oder akuter Organdysfunktion, Schock-Reaktion, Entzündungsreaktion und/oder Trauma.

Vorzugsweise ist die Probennukleinsäure RNA, insbesondere Gesamt-RNA oder mRNA, oder DNA, insbesondere cDNA.

Für eine weiter verfeinerte diagnostische Aussage kann es zur Beurteilung des pathophysiologischen Zustands von Vorteil sein, neben wenigstens einem der Polynucleotide, ausgewählt aus der Gruppe bestehend aus: M2, M3, M4, M6, M7, M8, M9, M10, M12, M13, M15, M16 und M17 und/oder deren Isoformen und/oder deren Genloci und/oder deren Transkripten und/oder Fragmenten mit einer Länge von mindestens 5 Nucleotiden davon, wobei die Polynucleotide gemäß folgender Tabelle definiert sind:

| Polynukleotid | Transkriptvariante | Accession Number | Seq ID |
|---|---|---|---|
| M2 | M2_1 | NM_001031700 | 4 |
| | M2_2 | NM_016613 | 25 |
| | M2_3 | NM_001128424 | 15 |
| M4 | M4_1 | NM_203330 | 35 |
| | M4_2 | NM_000611 | 3 |
| | M4_3 | NM_203329 | 34 |
| | M4_4 | NM_203331 | 36 |
| | M4_5 | NM_001127223 | 11 |
| | M4_6 | NM_001127225 | 12 |
| | M4_7 | NM_001127226 | 13 |
| | M4_8 | NM_001127227 | 14 |
| M6 | M6_1 | NM_001831 | 18 |
| | M6_2 | NM_203339 | 37 |
| M7 | M7_1 | NM_031311 | 28 |
| | M7_2 | NM_019049 | 26 |
| M9 | M9 | NM_006682 | 24 |
| M10 | M10 | NM_033554 | 32 |
| M15 | M15_1 | NM_003580 | 23 |
| | M15_2 | NM_001144772 | 16 |
| M3 | M3_A | NM_001123041 | 9 |
| | M3_B | NM_001123396 | 10 |
| M8 | M8 | NM_025209 | 27 |
| M12 | M12 | NM_002185 | 19 |
| M13 | M13 | NM_001080394 | 7 |
| M16 | M16 | NM_003268 | 22 |
| M17 | M17 | NM_182491 | 33 |

noch wenigstens einen weiteren Marker zu verwenden, welcher ausgewählt wird aus der Gruppe bestehend aus: Procalcitonin (PCT), C-reaktives Protein (CRP); Leukocytenzahl; Cytokinen; Interleukinen sowie weiteren bislang im Stand der Technik bekannten, dem Fachmann wohlbekannten klinischen Laborparametern und genetischen, transkriptomischen und proteomischen Markern .

Um die vorliegende Erfindung auszuführen, ist es erforderlich, geeignete Primer-Paare (forward und reverse) einzusetzen. Deratige besonders geeignete Primer sind solche, die in folgender Tabelle aufgezählt sind:

| Marker und Referenzgene | Primer für quantitative PCR/resultierende Amplikon | SEQ ID |
|---|---|---|
| M2 | M2-fw | 38 |
| | M2-rev | 39 |
| | M2-Amplikon | 40 |

(fortgesetzt)

| Marker und Referenzgene | Primer für quantitative PCR/resultierende Amplikon | SEQ ID |
|---|---|---|
| M4 | M4-fw | 41 |
| | M4-rev | 42 |
| | M4-Amplikon | 43 |
| M6 | M6-fw | 44 |
| | M6-rev | 45 |
| | M6-Amplikon | 46 |
| M7 | M7-fw | 47 |
| | M7-rev | 48 |
| | M7-Amplikon | 49 |
| M9 | M9-fw | 50 |
| | M9-rev | 51 |
| | M9-Amplikon | 52 |
| M10 | M10-fw | 53 |
| | M10-rev | 54 |
| | M10-Amplikon | 55 |
| M15 | M15-fw | 56 |
| | M15-rev | 57 |
| | M15-Amplikon | 58 |
| M3 | M3-fw | 59 |
| | M3-rev | 60 |
| | M3-Amplikon | 61 |
| M8 | M8-fw | 62 |
| | M8-rev | 63 |
| | M8-Amplikon | 64 |
| M12 | M12-fw | 65 |
| | M12-rev | 66 |
| | M12-Amplikon | 67 |
| M13 | M13-fw | 68 |
| | M13-rev | 69 |
| | M13-Amplikon | 70 |
| M16 | M16-fw | 71 |
| | M16-rev | 72 |
| | M16-Amplikon | 73 |
| M17 | M17-fw | 74 |
| | M17-rev | 75 |
| | M17-Amplikon | 76 |

(fortgesetzt)

| Marker und Referenzgene | Primer für quantitative PCR/resultierende Amplikon | SEQ ID |
|---|---|---|
| R1 | R1-fw | 77 |
| | R1-rev | 78 |
| | R1-Amplikon | 79 |
| R2 | R2-fw | 80 |
| | R2-rev | 81 |
| | R2-Amplikon | 82 |
| R3 | R3-fw | 83 |
| | R3-rev | 84 |
| | R3-Amplikon | 85 |

Es muß jedoch betont werden, dass die genannten Primer lediglich beispielhaft sind.

Die genannten Amplikons können beispielsweise als Sonden für Hybridisierungsverfahren verwendet werden.

Die Erfindung betrifft ferner einen Kit zur Durchführung des erfindungsgemäßen Verfahrens, enthaltend mindestens einen Multigenbiomarker, welcher eine Mehrzahl von Polynukleotidsequenzen umfasst, die ausgewählt werden aus der Gruppe, bestehend aus: M2, M3, M4, M6, M7, M8, M9, M10, M12, M13, M15, M16 und M17 und/oder deren Isoformen und/oder deren Genloci und/oder deren Transkripten und/oder Fragmenten mit einer Länge von mindestens 5 Nucleotiden davon, wobei die Polynucleotide gemäß folgender Tabelle definiert sind:

| Polynukleotid | Transkriptvariante | Accession Number | Seq ID |
|---|---|---|---|
| M2 | M2_1 | NM_001031700 | 4 |
| | M2_2 | NM_016613 | 25 |
| | M2_3 | NM_001128424 | 15 |
| M4 | M4_1 | NM_203330 | 35 |
| | M4_2 | NM_000611 | 3 |
| | M4_3 | NM_203329 | 34 |
| | M4_4 | NM_203331 | 36 |
| | M4_5 | NM_001127223 | 11 |
| | M4_6 | NM_001127225 | 12 |
| | M4_7 | NM_001127226 | 13 |
| | M4_8 | NM_001127227 | 14 |
| M6 | M6_1 | NM_001831 | 18 |
| | M6_2 | NM_203339 | 37 |
| M7 | M7_1 | NM_031311 | 28 |
| | M7_2 | NM_019049 | 26 |
| M9 | M9 | NM_006682 | 24 |
| M10 | M10 | NM_033554 | 32 |
| M15 | M15_1 | NM_003580 | 23 |
| | M15_2 | NM_001144772 | 16 |

(fortgesetzt)

| Polynukleotid | Transkriptvariante | Accession Number | Seq ID |
|---|---|---|---|
| M3 | M3_A | NM_001123041 | 9 |
| | M3_B | NM_001123396 | 10 |
| M8 | M8 | NM_025209 | 27 |
| M12 | M12 | NM_002185 | 19 |
| M13 | M13 | NM_001080394 | 7 |
| M16 | M16 | NM_003268 | 22 |
| M17 | M17 | NM_182491 | 33 |

wobei der Multigenbiomarker spezifisch für einen pathophysiologischen Zustand eines Patienten ist und solche Zustände einschließt, die ausgewählt sind aus der Gruppe bestehend aus: SIRS, Sepsis und deren Schweregraden; sepsisähnlichen Zuständen; septischem Schock; Bakteriämie, infektiösem/nicht-infektiösem Multiorganversagen; Früherkennung dieser Zustände; Fokuskontrolle; Kontrolle von chirurgischen Sanierungsmaßnahmen des Infektionsfokus; Responder/non-Responder für eine bestimmte Therapie; Therapiekontrolle; Unterscheidung zwischen infektiöser und nicht-infektiöser Genese bei systemischen Reaktionen des Organismus, wie z.B. SIRS, Sepsis, postoperative Komplikationen, chronischem und/oder akuter Organdysfunktion, Schock-Reaktion, Entzündungsreaktion und/oder Trauma.

Ein bevorzugter Kit ist dadurch gekennzeichnet, dass die Polynukleotidsequenzen auch Genloci, sense und/oder antisense Stränge von prä-mRNA und/oder mRNA, small RNA, insbesondere scRNA, snoRNA, micro RNA, siRNA, dsRNA , ncRNA oder transposable Elemente umfassen.

Ein weiter bevorzugter Kit ist dadurch gekennzeichnet, dass der er wenigstens ein Referenzgen enthält, welches ausgewählt ist, aus der Gruppe bestehend aus: R1, R2 und R3 und/oder deren Isoformen und/oder deren Genloci und/oder deren Transkripten und/oder Fragmenten mit einer Länge von mindestens 5 Nucleotiden davon, wobei die Referenzgene gemäß folgender Tabelle definiert sind:

| Polynukleotid | Transkriptvariante | Accession Number | Seq ID |
|---|---|---|---|
| R1 | R1_A | NM_001228 | 17 |
| | R1_B | NM_033355 | 29 |
| | R1_C | NM_033356 | 30 |
| | R1_E | NM_033358 | 31 |
| | R1_F | NM_001080124 | 5 |
| | R1_G | NM_001080125 | 6 |
| R2 | R2_1 | NM_002209 | 20 |
| | R2_2 | NM_001114380 | 8 |
| R3 | R3 | NM_003082 | 21 |

Eine ebenfalls bevorzugte Verwendung ist dadurch gekennzeichnet, dass aus den einzelnen bestimmten Genaktivitäten ein Index (Score) gebildet wird, der nach entsprechender Kalibrierung ein Maß für den Schweregrad und/oder den Verlauf des pathophysiologischen Zustands, insbesondere der Sepsis oder des sepsisähnlichen Zustandes, ist.

Es ist ferner bevorzugt, den Index (Score) auf einer leicht interpretierbaren Skala anzuzeigen.

In der Praxis der Anmelderin hat sich herausgestellt, daß hier eine dimensionslose Skala von -5 bis +5 oder zur Verstärkung der Unterschiede ein entsprechendes Vielfaches, z.B. von -50 bis +50 besonders geeignet ist, um pathophysiologische Zustände zu klassifizieren. Dieser Score wird "SIQ-Score" genannt.

Im Rahmen eines optimierten EDV-gestützten Krankenhausmanagements wie auch zur weiteren Forschung auf dem Gebiet der Sepsis hat es sich als vorteilhaft herausgestellt, dass man die erhaltenen Genaktivitätsdaten zur Herstellung von Software für die Beschreibung mindestens eines pathophysiologischen Zustands und/oder einer Untersuchungsfrage und/oder als Hilfsmittel für Diagnosezwecke und/oder für Patientendatenmangement-Systeme einsetzt.

Der Index wird vorzugsweise mittels statistischer Verfahren wie überwachte Klassifikationsverfahren aus dem Bereich des maschinellen und statischen Lernens wie z.B. (diagonale, lineare, quadratische) Diskriminanzanalyse, Super vector machines, verallgemeinerte partielle kleinste Quadrate, k-nächste Nachbarn, random forests, k-nächste Nachbar ermittelt. Für eine lineare Diskriminanzanalyse kann beispielsweise folgende Formel verwendet werden:

$$f_{LD}\left(x_1, \ldots, x_p\right) = \sum_{i=1}^{p} w_i x_i - w_0$$

Bevorzugt ist der Multigenbiomarker eine Kombination von mehreren Polynukleotid-, insbesondere Gensequenzen, anhand deren Genaktivitäten mittels einer Interpretationsfunktion eine Klassifikation durchgeführt und/oder ein Index oder Score gebildet wird.

Für die Zwecke der vorliegenden Erfindung hat es sich ferner als vorteilhaft herausgestellt, dass die Genaktivitäten mittels enzymatischer Verfahren, insbesondere Amplifikationsverfahren, bevorzugt Polymereasekettenreaktion (PCR), vorzugsweise Real-Time-PCR; und/oder mittels Hybridisierungsverfahren, insbesondere solchen auf Microarrays, erfasst werden.

Bei der Erfassung der Genaktivitäten auftretende differentielle Expressionssignale der in dem Multigenbiomarker enthaltenen Polynukleotidsequenzen können vorteilhaft und eindeutig einem pathophysiologischen Zustand, einem Verlauf und/oder Therapiemonitoring zugeordnet werden.

Typischerweise wird aus den einzelnen bestimmten Genaktivitäten ein Index (Score, SIQ-Score) gebildet wird, der nach entsprechender Kalibrierung ein Maß für den Schweregrad und/oder den Verlauf des pathophysiologischen Zustands, insbesondere der Sepsis oder des sepsisähnlichen Zustandes, ist.

Dieser Score kann dem behandelnden Arzt ein schnelles diagnostisches Hilfsmittel in die Hand geben.

Die vorliegende Erfindung ermöglicht es, als Teil eines integrierten Systems (In Vitro Diagnostic Multivariate Index Assay, IVDMIA) eine potenzielle infektiöse Komplikation in Patienten mit SIRS oder möglichen Sepsis einzuschätzen. Dieses System umfasst die Wahl der Patienten und die Bestimmung von deren Genexpressionssignalen in einem interpretierbaren Index, welchen der Arzt als Hilfsmittel zur Diagnose verwenden kann.

Die Anmelderin hat mehrere Verfahren entwickelt, das unterschiedliche Sequenzpools benutzt, um Zustände festzustellen und/oder zu unterscheiden oder definierte Untersuchungsfragen zu beantworten. Beispiele sind in folgenden Patentschriften zu finden: Unterscheidung zwischen SIRS, Sepsis und sepsisähnlichen Zuständen [WO 2004/087949; WO 2005/083115], Erstellung von Kriterien zur Vorhersage des Krankheitsverlaufs bei Sepsis [WO 05/106020], Unterscheidung zwischen nichtinfektiösen und infektiösen Ursachen eines Multiorganversagens [WO 2006/042581], *in vitro* Klassifizierung von Genexpressionsprofilen von Patienten mit infektiösem/nichtinfektiösem Multiorganversagen [WO 2006/100203], Feststellung der lokalen Ursachen eines Fiebers unklarer Genese [WO 2007/144105], Polynukleotide zur Erfassung von Genaktivitäten für die Unterscheidung zwischen lokaler und systemischer Infektion [DE 10 2007 036 678.9].

Die Erfindung betrifft Polynukleotidsequenzen, ein Verfahren und ferner Kits zur Erstellung von Multigenbiomarkern, die in einem und/oder mehreren Modulen Merkmale eines *"In Vitro* Diagnostic Multivariate Index Assay" (IVDMIA) aufweisen.

Bezüglich der in der vorliegenden Anmeldung verwendeten Nucleotidsequenzen ist Folgendes zu bemerken:

RefSeq ist eine öffentliche Datenbank, die von Nukleotid- und Proteinsequenzen mit ihren Eigenschaften sowie bibliographische Informationen beinhaltet.

Die RefSeq Datenbank wurde durch dasNational Center for Biotechnology Information (NCBI), ein Abteilung von National Library of Medicine, die zum US National Institute of Health gehört erstellt und wird fortlaufend gepflegt und aktualisiert (1).

NCBI erstellt RefSeq aus den Sequenzdaten der Archiv-Datenbank "GenBank"(2), einer umfangreichen öffentlichen Datenbank von Sequenzen, die bei GenBank in den USA, der EMBL Datenbibliothek in Großbritannien und der DNS Datenbank von Japan eingestellt und auch zwischen diesen Datenbanken ausgetauscht werden.

Die RefSeq Sammlung ist einzigartig, wenn es um die Bereitstellung von fehlerkorrigierten, nichtredundanten, explizitverlinkten Nukleotid- und Proteindatenbanken geht. Die Einträge sind nichtredundant mit dem Ziel, die verschiedenen biologischen Moleküle zu repräsentieren, die für Organismus, Stamm oder Haplotyp charakteristisch sind.

Wenn bestimmte Einträge mehrfach in der Sammlung auftreten, kann es mehrere Gründe dafür geben:

- es kodieren alternative gespleißte Transkripte für das gleiche Proteinprodukt (sog. Transkriptvarianten),
- es existieren mehrere genomische Bereiche innerhalb einer Spezies oder zwischen Spezies, welche für das gleiche Proteinprodukt kodieren,

- wenn RefSeqs erstellt werden, die alternative Haplotypen darstellen und manche mRNA- und Proteinsequenzen sind dabei identisch in allen Haplotypen.

RefSeq Datenbank liefert das kritische Fundament für Sequenzintegration, genetische und funktionelle Information und gilt international als der Standard für Genomannotation. Bei der Sequenzsuche durch BLAST sind RefSeq Angaben in mehreren NCBI-Resourcen erhältlich einschließlich Entrez Nucleotide, Entrez Protein, Entrez Gene, Map Viewer, beim FTP-Download; oder durch die Vernetzung mit PubMed (Pruitt et al. 2007; The NCBI handbook 2002). RefSeq Angaben können durch das eindeutige Accession-Format, welches den Unterstrich beinhaltet (_) identifiziert werden.
Arbeitsgruppen nutzen verschiedene Methoden und Protokolle und kompilieren die RefSeq Kollektion für verschiedene Organismen. RefSeq Unterlagen werden durch mehrere unterschiedliche Verfahren erstellt (The NCBI handbook 2002):

1. wissenschaftliche Kooperation
2. Computer-unterstützte Genomannotationsverfahren
3. Fehlerkorrektur durch die NCBI-Mitarbeiter
4. Auszüge aus GenBank

Jede Angabe hat einen Kommentar, der den Stand der jeweiligen Fehlerkorrektur aufweist sowie die Zuordnung der kooperierenden Arbeitsgruppe. Dadurch beeinhaltet die RefSeq Angabe entweder die essentiell unveränderte, initial valide Kopie der originellen GenBank Einträge oder korrigierte sowie zusätzliche Informationen, die durch Kooperationspartner oder Experten hinzu gefügt wurden (The NCBI handbook 2002).
Falls ein Molekül in GenBank durch mehrere Sequenzen repräsentiert wird, wird durch die NCBI Mitarbeiter eine Entscheidung für die "beste" Sequenz getroffen, die dann als RefSeq präsentiert wird.

Hauptziel ist es, bekannte Mutationen, Sequenzierungsfehler, Klonierungsartefakte und fehlerhafte Annotationen zu vermeiden. RefSeq Sequenzen, die von solchen Fehlertypen behaftet sind, werden korrigiert. Sequenzen werden validiert, indem geprüft wird, ob die genomische Sequenz, die zur annotierte mRNS korrespondiert, tatsächlich zur mRNA-Sequenzangabe passt, und ob kodierende Regionen tatsächlich in die korrespondierende Proteinsequenz translatiert werden. Eine weitere wichtige Aufgabe ist es, die Kollektion durch das Hinzufügen vorher nicht bekannter unterrepräsentierter Gene und/oder alternative Spliceprodukte zu erweitern sowie zusätzliche Annotation von Sequenzeigenschaften bereit zustellen, welche reife Peptidprodukte und ihre funktionellen Domänen repräsentieren und/oder seltene biologische Phänomene, wie z.B. nicht-AUG-Initiationsorte der Transkription oder Selenoproteine, hervorheben (The NCBI handbook 2002).

Die Überprüfung der Qualität erfolgt auf regelmäßer Basis, um fragwürdige Sequenzen aufzufinden und zu überprüfen. Diese Qualitätstests kontrollieren die Fehler und Konflikte in Nomenklatur, Sequenzähnlichkeiten und genomische Lokalisierung, potenzielle Klonierungsfehler (wie z.B. Chimären) und gleichen die Daten mit anderen NCBI Resourcen, inklusive HomoloGene-, Map Viewer- und GenBank verwandten Sequenzen, ab (The NCBI handbook 2002). Bei den vorliegenden hoch-qualitativen genomischen Sequenzen von Human und Maus stand die Prüfung von cDNA-basierten RefSeqs in Bezug zum Genom im Hauptfokus. Die CCDS-Kooperation (The NCBI handbook 2002) hat auch geholfen, die Aufmerksamkeit auf die Bereiche zu fokussieren, wo es Diskrepanzen zwischen mRNA und Protein-Menge gibt.
Die Qualitätssicherungsprozesse umfassen die Registrierung der Datenbankattribute, um zu dokumentieren, dass

- die Kategorie des Qualitätstests aktualisiert wurde,
- keine Probleme mit RefSeq-Transkript und -Protein gefunden wurden und deshalb der gemeldete Fehler ignoriert werden soll,
- bedingt durch Genom-Assemblierung ein Problem an dieser Position entstanden ist

  - Probleme der Genom-Assemblierung können sein:
  - Es entstehen Lücken bei der Zusammensetzung der Einzelsequenzen
  - in manchen Fällen beinhaltet die aufgestellte Sequenz eine bekannte Mutation oder seltenen Polymorphismus und ist somit keine ideale repräsentative Sequenz (Pruitt et al. 2007).

Die Entscheidung, die in der vorliegenden Anmeldung benannte Markerpopulation auf der Basis ihrer RefSeq-Identität für die Zwecke der vorliegenden Erfindung zu verwenden, wurde aufgrund der oben beschriebenen Eigenschaften der RefSeq - Datenbank gefällt. Die Eigenschaften dieser Datenbank, die Erstellung, Qualität, Pflege und Aktualisierungen der biologischen Sequenzen betreffend, sowie das Vorliegen funktioneller Informationen auf Nukleinsäureebene, gleichermaßen für alternative Spleißvarianten, gaben dafür den Ausschlag.

Wie bereits erläutert, bietet der biologische Mechanismus des alternativen Spleißing Raum für dem Fachmann wohl bekannte Erstreckungen des Schutzumfangs. So ist denkbar, dass mit neuen Transkriptvarianten völlig neue Primärstrukturen identifiziert werden, oder dass sich Sequenzänderungen der bekannten Transkriptvarianten ergeben. Andererseits, werden die genomischen Regionen beansprucht, die für alle diese bekannten und unbekannten Varianten der kodierenden Transkripte, mitsamt ihren cis-regulatorischen Sequenzen als vollkommende genomische funktionelle Einheiten umfasst und somit unter den Schutzumfang der vorliegenden Erfindung fallen oder zumindest dem Fachmann leicht auffindbare Äquivalente zu den in den Ansprüchen, Beschreibung und im Sequenzprotokoll genannten Sequenzen zur Verfügung stellen.

**Definitionen:**

Für die Zwecke der vorliegenden Erfindung werden folgende Definitionen verwendet:

**Zustand:** die klinisch definierten Schweregrade "systemic inflammatory response syndrom" (SIRS), "sepsis", "severe sepsis" und "septic shock" wie definiert in [Bone et al., 1992] und das PIRO Konzept [Levy et al., 2003].

**Multiorganversagen:** Als Multiorganversagen bezeichnet man das gleichzeitig oder in rascher zeitlicher Abfolge auftretende Versagen von zwei oder mehr vitalen Organsystemen. Das Multiorgandysfunktionssyndrom (MODS) geht als initiale Organinsuffizienz dem MOV voraus [Zeni et al., 1997]. Man spricht heute vom Multiorganversagen wenn zwei oder mehr Organe gleichzeitig oder nacheinander Funktionstörungen aufweisen, wobei ein chronisch persistierendes Organversagen auszuschließen ist. Die Prognose des MOV hängt eng mit der Anzahl der beteiligten Organsysteme zusammen. Die Mortalität beträgt bei Versagen eines Organs innerhalb der ersten 24 Stunden 22%, nach 7 Tagen 41 %. Beim Versagen von drei Organsystemen steigt die Mortalität am ersten Tag auf 80 % und nach 4 Tagen auf 100 % an [Knaus et al., 1985].

Ein wichtiger Pathomechanismus für die Entstehung von MODS und MOV ist die Entwicklung eines systemischen Inflammationssyndromes (SIRS, [Bone et al., 1992]. MODS und MOV können sowohl infektiologischer als auch nichtinfektiologischer Genese sein.

**Fieber unklarer Genese:** Fieber unklarer Genese (Fever of unknow origin, FUO) ist klinisch definiert als ein Fieber, bei dem die Temperatur über einen Zeitraum von mehr als 3 Wochen höher als 38,8°C ist, ohne dass nach einer einwöchigen Untersuchungszeit eine eindeutige Diagnose der Ursache vorliegt. In Abhängigkeit des Ursprungs wurden vier Klassen des FUO beschrieben: FUO klassischen, nosokomialen, immunschwachen oder HIV-bezogenen Ursprungs [Roth und Basello, 2003]. FUO wurde auch als "eine eher bekannte Krankheit mit einem ungewöhnlichen Erscheinungsbild als eine seltene Störung" geschildert [Amin und Kauffman, 2003].

Nur in 10% der Patienten mit postoperativem Fieber wird eine Infektion dokumentiert [Pile et al., 2006]. In den meisten Fälle geht die Temperatur des Patienten innerhalb von vier Tagen nach dem Eingriff zurück auf Normaltemperatur. Trotzdem entwickeln einige Patienten am oder nach dem fünften postoperativem Tag eine Infektion, wobei es sich in 12% der Fälle um eine Lungenentzündung handelt. Ebenso wird von Pile und Kollegen berichtet, dass es sich bei Fieber, welches zwei Tage nach dem Eingriff auftritt, mit hoher Wahrscheinlichkeit um eine Infektion handelt, wie z.B. eine Infektion der Harnwege und/ oder des inneren Unterleibs (Peritonitis), Lungenentzündung oder eine durch einen intravenösen Katheder ausgelöste Infektion.

**Untersuchungsfrage:** Eine klinische relevante Frage, die für die Behandlung eines Patientes wichtig ist, beispielsweise: Vorhersage des Krankheitsverlaufs, Therapieüberwachung, Fokus der Infektion, Überlebenschancen, Prädisposition, etc.

Eine **systemische Infektion** ist eine Infektion, bei der sich die Erreger über die Blutbahn im gesamten Organismus ausgebreitet haben.

**SIRS:** Systemic Inflammatory Response Syndrome, nach Bone [Bone et al., 1992] und Levy [Levy et al., 2003] ein generalisierter, inflammatorischer, nichtinfektiöser Zustand eines Patienten.

**Sepsis:** Nach Bone [Bone et al., 1992] und Levy [Levy et al., 2003] ein generalisierter, inflammatorischer infektiöser Zustand eines Patienten.

**Biologische Flüssigkeit:** Als biologische Flüssigkeiten im Sinne der Erfindung werden alle Körperflüssigkeiten der Säugetiere, einschließlich des Menschen, verstanden.

**Gen:** Ein Gen ist ein Abschnitt auf der Desoxyribonukleinsäure (DNA), der die Grundinformationen zur Herstellung einer biologisch aktiven Ribonukleinsäure (RNA) sowie regulatorische Elemente, welche diese Herstellung aktivieren oder inaktivieren, enthält. Als Gene im Sinne der Erfindung werden auch alle abgeleiteten DNA-Sequenzen, Partialsequenzen und synthetische Analoga (beispielsweise Peptido-Nukleinsäuren (PNA)) verstanden. Die auf Bestimmung der Genexpression auf RNA-Ebene bezogene Beschreibung der Erfindung stellt somit ausdrücklich keine Einschränkung sondern nur eine beispielhafte Anwendung dar.

**Genlocus:** Genlocus (*Genort*) ist die Position eines Gens im Genom. Besteht das Genom aus mehreren Chromosomen, ist die Position innerhalb des Chromosoms gemeint, auf dem sich das Gen befindet. Verschiedene Ausprägungen oder Varianten dieses Gens werden als Allele bezeichnet, die sich alle an derselben Stelle auf dem Chromosom, nämlich dem Genlocus befinden. Somit beinhaltet der Begriff "Genlocus" einerseits die reine genetische Information für ein spezifisches Genprodukt und andererseits sämtliche regulatorische DNA-Abschnitte sowie sämtliche zusätzliche DNA-Sequenzen, welche mit dem Gen am Genlocus in irgendeinem funktionellen Zusammenhang stehen. Die letzteren schließen an Sequenzregionen an, die in der unmittelbar Nähe (1 Kb) aber außerhalb des 5-' und/oder 3'-Endes eines Genlocus liegen. Die Spezifizierung des Genlocus erfolgt durch die Accession-Nummer und/oder RefSeq ID des RNA-Hauptproduktes, welches von diesem Locus abstammt.

**Genaktivität:** Unter Genaktivität wird das Maß der Fähigkeit eines Gens verstanden, transkribiert zu werden und/oder Translationsprodukte zu bilden.

**Genexpression:** Der Vorgang, ein Genprodukt zu bilden und/oder Ausprägung eines Genotyps zu einem Phänotyp.

**Multigenbiomarker:** Kombination von mehreren Gen-Sequenzen deren Genaktivitäten mittels einer Interpretationsfunktion ein kombiniertes Gesamtergebnis (z.B. eine Klassifikation und/oder ein Index) bilden. Dieses Ergebnis ist spezifisch für einen Zustand und/oder eine Untersuchungsfrage.

**Hybridisierungsbedingungen:** Dem Fachmann wohl bekannte physikalische und chemische Parameter, welche die Etablierung eines thermodynamischen Gleichgewichtes aus freien und gebundenen Molekülen beeinflussen können. Im Interesse optimaler Hybridisierungsbedingungen müssen Zeitdauer des Kontaktes der Sonden- und Probenmoleküle, Kationenkonzentration im Hybridisierungspuffer, Temperatur, Volumen sowie Konzentrationen und -verhältnisse der hybridisierenden Moleküle aufeinander abgestimmt werden.

**Amplifikationsbedingungen:** Konstante oder sich zyklisch verändernde Reaktionsbedingungen, welche die Vervielfältigung des Ausgangsmateriales in Form von Nukleinsäuren ermöglichen. Im Reaktionsgemisch liegen die Einzelbausteine (Desoxyribonukleotide) für die entstehenden Nukleinsäuren vor, ebenso wie kurze Oligonukleotide, welche sich an komplementäre Bereiche im Ausgangsmaterial anlagern können, sowie ein Nukleinsäure-Synthese-Enzym, Polymerase genannt. Dem Fachmann wohl bekannte Kationenkonzentrationen, pH-Wert, Volumen und die Dauer und Temperatur der einzelnen Reaktionsschritte sind von Bedeutung für den Ablauf der Amplifikation.

**Primer:** Als Primer wird in der vorliegenden Erfindung ein Oligonukleotid bezeichnet, das als Startpunkt für Nukleinsäure-replizierende Enzyme wie z. B. die DNA-Polymerase dient. Primer können sowohl aus DNA als auch aus RNA bestehen (Primer3, siehe z.B. http://frodo.wi.mit.edu/cgi-bin/primer3/primer3-www.cgi des MIT)

**Sonde:** In der vorliegenden Anmeldung ist eine Sonde ein Nukleinsäurefragment (DNA oder RNA), das mit einer molekularen Markierung (z.B. Fluoreszenzlabel, insbesondere Scorpion®, molecular beacons, Minor Groove Binding- Sonden,TaqMan®-Sonden, Isotopenmarkierung, usw.) versehen werden kann und zur sequenzspezifischen Detektion von Ziel-DNA- und/oder Ziel-RNA-Molekülen eingesetzt wird.

**PCR:** ist die Abkürzung für die englische Bezeichnung "Polymerase Chain Reaction" (Polymerase-Kettenreaktion). Die Polymerase-Kettenreaktion ist eine Methode, um DNA *in vitro* außerhalb eines lebenden Organismus mit Hilfe einer DNA-abhängigen DNA Polymerase zu vervielfältigen. PCR wird insbesondere gemäß der vorliegenden Erfindung eingesetzt, um kurze Teile - bis zu etwa 3.000 Basenpaare - eines interessierenden DNA-Strangs zu vervielfältigen. Dabei kann es sich um ein Gen oder auch nur um einen Teil eines Gens oder auch um nicht kodierende DNA-Sequenzen handeln. Es ist dem Fachmann wohl bekannt, dass eine Reihe von PCR-Verfahren im Stand der Technik bekannt sind, welche alle durch den Begriff "PCR" mit umfasst sind. Dies gilt insbesondere für die "Real-Time-PCR" (vgl. auch die Erläuterungen weiter unten).

**PCR-Primer:** Typischerweise benötigt eine PCR zwei Primer, um auf den beiden Einzelsträngen der DNA jeweils den Startpunkt der DNA-Synthese festzulegen, wodurch der zu vervielfältigende Bereich von beiden Seiten begrenzt wird. Derartige Primer sind dem Fachmann wohlbekannt, beispielsweise aus der Website "Primer3", siehe z.B. http://frodo.wi.mit.edu/cgi-bin/primer3/primer3_www.cgi des MIT.

**Transkript:** Für die Zwecke der vorliegenden Anmeldung wird unter einem Transkript jegliches RNA-Produkt verstanden, welches anhand einer DNA-Vorlage hergestellt wird.

**Small RNA:** Kleine RNAs im Allgemeinen. Vertreter dieser Gruppe sind insbesondere, jedoch nicht ausschließlich:

a) scRNA (small cytoplasmatic RNA), welche eines von mehreren kleinen RNA-Molekülen im Cytoplasma eines Eukaryonten ist.

b) snRNA (small nuclear RNA), eine der vielen kleinen RNA-Formen, die nur im Zellkern vorkommen. Einige der snRNAs spielen beim Spleißen oder bei anderen RNA-verarbeitenden Reaktionen eine Rolle.

c) small non-protein-coding RNAs, welche die sogenannten small nucleolar RNAs (snoRNAs), microRNAs (miRNAs), short interfering RNAs (siRNAs) und small double-stranded RNAs (dsRNAs) einschließen, welche die Genexpression auf vielen Ebenen, einschließlich der Chromatin-Architektur, RNA-Editierung, RNA- Stabilität, Translation und möglicherweise auch Transkription und Spleißen. Im Allgemeinen werden diese RNAs auf mehrfachem Wege prozessiert aus den Introns und Exons längerer Primärtranskripte, einschließlich proteincodierender Transkripte. Obwohl etwa nur 1,2% des Humangenoms Proteine codiert, wird ein großer Teil dennoch transkribiert. Tatsächlich bestehen ca. 98% der in Säugern und Menschen gefundenen Transkripte aus non-protein-coding RNAs (ncRNA) aus Introns von proteincodierenden Genen und den Exons und Introns von nicht proteincodierenden Genen, einschließlich vieler, welche anti-sense zu proteincodierenden Genen sind oder mit diesen überlappen. Small nucleolar RNAs (snoRNAs) regulieren die sequenzspezifische Modifikation von Nukleotiden in Target-RNAs. Hierbei treten zwei Typen von Modifikationen auf, nämlich 2'-O-Ribosemethylierung und Pseudouridylierung, welche durch zwei große snoRNA-Familien reguliert werden, die einerseits box C/D-snoRNAs und andererseits box H/ACA snoRNAs genannt werden. Derartige snoRNAs weisen eine Länge von etwa 60 bis 300 Nukleotiden auf. miRNAs (microRNAs) und siRNAs (short interfering RNAs) sind noch kleinere RNAs mit im Allgemeinen 21 bis 25 Nukleotiden. miRNAs stammen aus endogenen kurzen Hairpin-Vorläuferstrukturen und benutzen gewöhnlich andere Loci mit ähnlichen - jedoch nicht identischen Sequenzen als Ziel translationaler Repression. siRNAs entstehen aus längeren doppelsträngigen RNAs oder langen Hairpins, oftmals exogenen Ursprungs. Sie haben gewöhnlich homologe Sequenzen an demselben Locus oder woanders im Genom zum Ziel, wo sie am sogenannten gene silencing, ein Phänomen, welches auch RNAi genannt wird, beteiligt sind. Die Grenzen zwischen miRNAs und siRNAs sind jedoch fließend.

d) Zusätzlich kann der Begriff "small RNA" auch sogenannte transposable Elemente (TEs) und insbesondere Retroelemente umfassen, welche ebenfalls für die Zwecke der vorliegenden Erfindung unter dem Begriff "small RNA" verstanden werden.

**RefSeq ID:** Diese Bezeichnung bezieht sich auf Einträge in der NCBI Datenbank (www.ncbi.nlm.nih.gov). Diese Datenbank liefert nicht-redundante Referenz-Standards zu genomischer Information. Diese genomischen Informationen schließen unter anderem Chromosomen, mRNAs, RNAs und Proteine ein. Jede RefSeq ID stellt ein einzelnes, natürlich vorkommendes Molekül eines Organismus dar. Die biologischen Sequenzen, die eine RefSeq repräsentieren, sind von GenBank Einträgen (ebenfalls NCBI) abgeleitet, sind aber eine Zusammenstellung von Informationselementen. Diese Informationselemente stammen aus Primär-Forschung auf DNA-, RNA- und Proteinebene.

**Accession-Nummer:** Eine Accession-Nummer stellt die Eintragsnummer eines Polynukleotides in der dem Fachmann bekannten NCBI-GenBank dar. In dieser Datenbank werden sowohl RefSeq ID's als auch weniger gut charakterisierte und redundante Sequenzen als Einträge verwaltet und der Öffentlichkeit zugänglich gemacht (www.ncbi.nlm.nih.gov/genbank/index.html).

**Lokale Infektion:** Die Infektion beschränkt sich auf die Eintrittspforte des Erregers (z.B. Wundinfektion)

**Generalisierte Infektion:** Pathogene dringen ins Gefäßsystem vor und ziehen den gesamten Organismus in Mitleidenschaft. Generalisierte Infektionen können zu einer Sepsis führen.

**Kolonisierung:** Die Gegenwart von Mikroorganismen löst im Organismus keinerlei Krankheitssymptome aus.

**Schwere Infektion:** Infektiöser Herd mit der Gefahr der zunehmenden Ausbreitung mit den Symptomen Fieber ab 39°C und/oder Bakteriämie.

**Bakteriämie:** Ein Zustand, in dem vorübergehend und kurzfristig Bakterien im Blut anwesend sind, ohne dass dies mit dem Auftreten von bakteriell bedingten klinischen Symptomen verbunden sein muss.

**Alternatives Splicing:** ein Prozess, bei dem die Exons des primären Gentranskripts (pre-mRNA) nach Ausschneiden der Introns in unterschiedlichen Kombinationen wiederverbunden werden.

**BLAST:** Basic Local Alignment Search Tool (nach Altschul et al., J Mol Biol 215:403-410; 1990). Sequenzvergleichalgorithmus, geschwindigkeitsoptimiert, wird für die Suche in Sequenzdatenbanken für eine optimale lokale Anpassung an die Anfragesequenz genutzt.

**cDNA:** Komplementäre DNA. DNA-Sequenz, Produkt der Reversen Transkription von mRNA.

**Codierende Sequenz:** Protein-kodierender Abschnitt eines Gens bzw. einer mRNA in Abgrenzung zu Introns (nicht kodierende Sequenzen) und 5-' oder 3'-nichttranslatierten Abschnitten. Kodierende Sequenzen der cDNA oder reifen mRNA umfassen den Bereich zwischen Start- (AUG oder ATG) und Stopcodon.

**EST:** Expressed Sequence Tag. Kurze ssDNA-Abschnitte der cDNA (normalerweise ~300-500 bp), üblicherweise in großen Mengen produziert. Repräsentieren die Gene, die in bestimmten Geweben und/oder während bestimmter Entwicklungsphasen exprimiert werden. Teilweise codierend bzw. nicht codierende Kennzeichnungen der Expression für cDNA-Bibliotheken. Wertvoll für die Größenbestimmung vollständiger Gene und im Rahmen von Kartierungen (Mapping).

**Exon:** Kodierender, der mRNA entsprechender Sequenzbereich typischer eukaryotischer Gene. Exons können die kodierenden Sequenzen, den 5'-nichttranslatierten Bereich oder den 3'-nichttranslatierten Bereich umfassen. Exons kodieren spezifische Abschnitte des vollständigen Proteins und sind normalerweise durch lange Abschnitte (Introns) getrennt, die bisweilen als "junk DNA" bezeichnet werden und deren Funktion nicht genau bekannt ist aber wohl kurze, nichttranslatierte RNAs (snRNA) oder regulatorische Informationen kodieren.

**GenBank** Nukleotidsequenz-Datenbank mit Sequenzen aus mehr als 100.000 Organismen. Einträge, die mit Eigenschaften der kodierende Bereiche annotiert sind, umfassen auch die Translationsprodukte. GenBank ist Teil der internationalen Kooperation der Sequenzdatenbanken, die auch EMBL und DDBJ umfasst.

**Intron:** Nicht-codierender Sequenzbereich eines typischen eukaryotischen Gens, wird während des RNA-Splicings aus dem primären Transkript herausgeschnitten und befindet sich somit nicht mehr in der reifen, funktionellen mRNA, rRNA oder tRNA.

**mRNA:** Messenger RNA oder manchmal nur "message". RNA, die die für Proteinkodierung notwendigen Sequenzen enthält. Der Begriff mRNA wird in Abgrenzung zum (ungesplicten) Primärtranskript nur für das reife Transkript mit PolyA-Schwanz (exklusive der über das Splicing entfernten Introns) benutzt. Weist 5'-nichttranslatierte, Aminosäuren-kodierende, 3'- nichttranslatierte Bereiche und (fast immer) einen Poly(A)-Schwanz auf. Stellt typischerweise ca. 2% der gesamten zellulären RNA.

**Poly(A)-Schwanz:** ssAdenosin-Verlängerung (~ 50-200 Monomere), die während des Splicings an das 3'-Ende der mRNA gehangen wird. Der PolyA-Tail erhöht vermutlich die Stabilität der mRNA (möglicherweise Protektion gegen Nukleasen). Nicht alle mRNA weisen das Konstrukt auf, so z.B. die Histon-mRNA.

**RefSeq** NCBI-Datenbank der Rereferenzsequenzen. Fehler-korrigierte, nicht-redundante Sequenzsammlung genomischer DNA-Contigs, mRNA- und Protein-Sequenzen bzw. von bekannten Genen und vollständiger Chromosomen.

**SNPs:** Single Nucleotide Polymorphisms. Auf einzelnen Nukleotidabweichungen beruhende genetische Unterschiede zwischen Allelen des gleiches Gens. Entstehen an spezifischen individuellen Positionen innerhalb eines Gens. **Transkriptvarianten:** Alternative Splicing-Produkte. Die Exons des primären Gentranskripts (prä-mRNA) wurden auf unterschiedliche Weise wiederverbunden und werden nachfolgend translatiert.

**3'-nichttranslatiertes Bereich:** Transkribierter 3'-terminaler mRNA-Bereich ohne proteinkodierende Information (Bereich zwischen Stopkodon und PolyA-Schwanz). Kann die Translationseffizienz oder die Stabilität der mRNA beeinflussen.

**5'- nichttranslatiertes Bereich:** Transkribierter 5'-terminaler mRNA-Bereich ohne proteinkodierende Information (Bereich zwischen initialem 7-Methylguanosin und der Base unmittelbar vor dem ATG-Startcodon). Kann die Translationseffizienz oder die Stabilität der mRNA beeinflussen.

**Polynucleotid-Isoformen:** Polynucleotide mit gleicher Funktion, jedoch unterschiedlicher Sequenz.

**Abkürzungen**

| | |
|---|---|
| AUC (area under curve) | Fläche unter der Kurve |
| CRP | C-reaktives Protein |
| CV (cross validation) | Kreuzvalidierung |
| DLDA (diagonal linear discriminant analysis) | diagonale lineare Diskriminanzanalyse Klasssifikationsverfahren |
| GPLS (generalized partial least squares) | verallgemeinerte partielle kleinste Quadrate (Klassifikationsverfahren) |
| IQR (inter quartile range) | Abstand zwischen dem 75% und 25% Perzentil |
| kNN (k nearest neighbours) | k-nächste Nachbarn (Klassifikationsverfahren) |
| LDA (linear discriminant analysis) | lineare Diskriminanzanalyse (Klassifikationsverfahren) |
| NPV (negative predictive value) | negativer prädikativer Wert (Anteil der korrekt negativen Tests) |
| OR | Odd Ratio |
| PCT | Procalcitonin |
| PPV (positive predictive value) | positiver prädikativer Wert (Anteil der korrekt positiven Tests) |
| RF (random forests) | Klassifikationsverfahren |
| ROC (receiver operator characteristics) | Abbildung zur Darstellung von Klassifikationsergebnissen |
| Sensitivität | Anteil der korrekten Tests in der Gruppe mit vorgegebenener Erkrankung (infektiöse SIRS bzw. Sepsis) |
| Spezifizität | Anteil der korrekten Tests in der Gruppe ohne vorgegebenene Erkrankung (nicht-infektiöse SIRS) |
| SVM (support vector machines) | Klassifikationsverfahren |

Für eine schnelle Diagnosik hat sich in der Praxis herausgestellt, dass Echtzeit- oder Real-Time-Amplifikationsverfahren die bevorzugten Verfahren sind. Daher werden im Folgenden die Grundlagen, welche dem Fachmann wohlbekannt sind, kurz im Hinblick auf ihre Bedeutung für die vorliegende Erfindung zusammengefaßt.

Andere, dem Fachmann bekannten Verfahren, wie z.B. Sequenzierung, Mikroarray basierte Methoden, NASBA usw. sind ebenfalls möglich.

Mit Hilfe der Polymerase-Kettenreaktion (PCR) ist es möglich, spezifische Sequenzbereiche aus geringsten Ausgangsmengen von Nukleinsäuren in-vitro und zudem schnell zu amplifizieren, um sie so einer Analyse oder Weiterverarbeitung zugänglich zu machen. Ein doppelsträngiges DNA-Molekül wird durch Hitzeeinwirkung aufgeschmolzen (denaturiert). Die Einzelstränge dienen in der Folge als Matrize für die enzymatisch katalysierte Polymerisation von Desoxyribonukleotiden, wodurch wieder doppelsträngige DNA-Moleküle entstehen. Die als Primer bezeichneten Oligodesoxyribonukleotide definieren dabei den zu kopierenden Sequenzabschnitt, indem sie an Orten komplementärer Sequenz mit der Ziel-DNA hybridisieren und als Starter für die Polymerisation dienen. Der Prozess exponentieller Produktbildung wird von verschiedenen Faktoren begrenzt. Im Laufe der PCR geht die Netto-Produktbildung daher schließlich auf Null zurück und die Gesamtmenge an PCR-Produkt erreicht einen Plateauwert.

Geeignete PCR-Primer sind beispielsweise Primer mit den Sequenzen gemäß Tabelle 3. Es ist dem Fachmann jedoch bekannt, dass eine Vielzahl weiterer Primer zur Ausführung der vorliegenden Erfindung verwendet werden kann.

Seit ihrer Einführung in das molekularbiologische Methodenspektrum wurde eine nahezu unüberschaubare Vielzahl von technischen Varianten entwickelt. Heute ist die PCR eine der wichtigsten Methoden in der molekularen Biologie und der molekularen Medizin. Heute findet sie Verwendung in einem überaus breiten thematischen Spektrum, z. B. beim Nachweis von Viren oder Keimen, bei der Sequenzierung, dem Verwandtschaftsnachweis, der Erstellung von Transkriptionsprofilen und der Quantifizierung von Nukleinsäuren [Valasek und Repa, 2005; Klein, 2002]. Zudem lassen sich mit Hilfe der PCR in einfacher Weise beliebige Sequenzabschnitte des Nukleinsäurebestandes eines Organismus klonieren. Die Vielzahl entwickelter PCR-Varianten ermöglicht u. a. eine zielgerichtete oder zufällige Veränderung der DNA-Sequenz sowie sogar die Synthese größerer, in dieser Form zuvor nicht existenter Sequenzabfolgen.

Mit diesem klassischen Verfahren lassen sich hochsensitiv DNA und über die reverse Transkription (RT) auch RNA qualitativ nachweisen [Wong et al., 2005; Bustin 2002]. Eine Weiterentwicklung dieser Methode ist die Real-Time-PCR, die erstmals 1991 vorgestellt wurde und neben qualitativen Aussagen auch eine Quantifizierung ermöglicht.

Real-Time-PCR, auch quantitative PCR (qPCR) genannt, ist eine Methode zur Detektion und Quantifizierung von Nukleinsäuren in Echtzeit [Nolan et al., 2006]. In der Molekularbiologie gehört sie bereits seit einigen Jahren zu den etablierten Standardtechniken.

Im Gegensatz zur PCR findet hier die Detektion bereits während der Amplifikation statt. Basierend auf Fluoreszenz-markierten Sonden, den Fluorophoren, kann die Amplifikation in Echtzeit verfolgt werden. In jedem Reaktionszyklus nehmen die fluoreszierenden PCR-Produkte und damit die Intensität der lichtinduzierten Fluoreszenz-Emission zu. Da die Zunahme der Fluoreszenz und die Menge an neusynthetisierten PCR-Produkten über einen weiten Bereich proportional zueinander sind, kann aus den gewonnenen Daten die Ausgangsmenge des Templates bestimmt werden. Eine gelelektrophoretische Auftrennung der Amplifikate ist nicht mehr erforderlich. Die Ergebnisse sind direkt verfügbar, was eine deutliche Zeitersparnis mit sich bringt. Da die Reaktionen in geschlossenen Gefäßen ablaufen, und nach dem Start der PCR keine weiteren Pipettierschritte erforderlich sind, reduziert sich das Kontaminationsrisiko auf ein Minimum. Als Fluorophore werden entweder nukleinsäure-bindende Fluoreszenzfarbstoffe wie SYBRGreen oder sequenzspezifische Fluoreszenzsonden wie Taq-Man-Sonden, LightCycler-Sonden und Molecular Beacons eingesetzt [Kubista et al., 2006]. SYBRGreen ist ein Farbstoff, dessen Fluoreszenz stark zunimmt, sobald das Molekül an doppelsträngige DNA bindet. Diese kostengünstige Lösung ist besonders bei der parallelen Durchführung mehrerer Reaktionen mit unterschiedlichen Primerpaaren geeignet. Nachteile liegen in der geringen Spezifität, da SYBRGreen sequenzunspezifisch an jede doppelsträngige DNA bindet, sowie darin, dass keine Multiplex-Messungen durchgeführt werden können. Mit Hilfe einer Schmelzkurvenanalyse kann nach erfolgter PCR allerdings zwischen dem Zielprodukt und unspezifischer DNA differenziert werden: In Abhängigkeit der Nukleotidlänge und -zusammensetzung zerfällt jeder DNA-Doppelstrang bei einer für ihn charakteristischen Temperatur, der Schmelztemperatur, in seine zwei Einzelstränge. Da die doppelsträngige DNA von spezifischen PCR-Produkten einen höheren Schmelzpunkt hat als unspezifisch entstehende Primerdimere, ist eine Unterscheidung anhand der Fluoreszenzabnahme bei Zunahme der Temperatur möglich.

Im Gegensatz dazu ist der Nachweis mit fluoreszenzbasierten Sonden hochspezifisch, aber auch sehr kostenintensiv. Beim TaqMan-Prinzip enthält der PCR-Ansatz neben den PCR-Primern eine sequenzspezifische TaqMan-Hybridisierungssonde, die über einen Quencher und einen Reporterfarbstoff verfügt. Die Sonde ist komplementär zu einer Sequenz, die zwischen den Primern liegt. In freier Lösung wird die Fluoreszenz durch die räumliche Nähe des Quenchers unterdrückt. Nach dem FRET-(Fluoreszenz-Resonanz-Energie-Transfer)-Prinzip schluckt der Quencher die Fluoreszenzemission des angeregten Fluorophors. Hybridisiert diese Sonde jedoch mit der Zielsequenz, wird sie während der PCR von der Taq-Polymerase hydrolysiert, der Reporterfarbstoff wird räumlich vom Quencher entfernt und emittiert bei Anregung detektierbare Fluoreszenz. Beim LightCycler-Prinzip enthält der PCR-Ansatz neben den PCR-Primern zwei fluoreszenz-markierte Sonden (Donor- und Akzeptor- Fluoreszenzfarbstoff). Ein nach außen hin messbares Fluoreszenzsignal entsteht nur bei unmittelbar benachbarter Hybridisierung der beiden Sonden mit der spezifischen Zielsequenz. Im Rahmen einer anschließenden Schmelzkurvenanalyse können sogar das Vorliegen und die Art einzelner Punktmutationen innerhalb der Hybridisierungsbereiche der Sonden detektiert werden. Ein weiteres Beispiel sind die Molecular Beacons. Diese Oligonukleotide enthalten am 5'- und 3'-Ende zueinander komplementäre Sequenzen, die in ungebundenem Zustand hybridisieren und eine Haarnadelstruktur bilden. Reporterfluorophor und Quencher, lokalisiert an beiden Enden, sind so in direkter Nachbarschaft. Erst wenn die Sonde am Templat bindet, werden die beiden Farbstoffe räumlich getrennt, so dass nach Anregung wieder Fluoreszenz messbar ist. Scorpion- und Sunrise-Primer bilden zwei weitere Modifikationen für sequenzspezifische Sonden [Whitcombe et al,. 1999].

Die quantitative Bestimmung eines Templates kann mittels absoluter oder relativer Quantifizierung erfolgen. Bei der absoluten Quantifizierung findet die Messung anhand externer Standards, z.B. Plasmid-DNA in unterschiedlichen Verdünnungen, statt. Die relative Quantifizierung nutzt dagegen so genannte Housekeeping- oder Referenzgene als Referenz [Huggett et al., 2005]. Diese Referenzgene werden konstant exprimiert und bieten damit die Möglichkeit zur Normierung unterschiedlicher Expressionsanalysen. Die Auswahl der Housekeepinggene muss für jedes Experiment individuell erfolgen. Für die vorliegende Erfindung werden bevorzugt Housekeepinggene mit den Sequenzen gemäß Tabelle 2 verwendet.

Die generierten Experiment-Daten werden mit Hilfe der geräteeigenen Software ausgewertet. Für die grafische Darstellung wird die gemessene Fluoreszenzintensität gegen die Anzahl der Zyklen aufgetragen. Die resultierende Kurve unterteilt sich dabei in drei Bereiche. In der ersten Phase, also zu Beginn der Reaktion, überwiegt noch das Grundrauschen, ein Signal des PCR-Produktes ist noch nicht nachweisbar. Die zweite Phase entspricht der exponentiellen Wachstumsphase. In diesem Segment verdoppelt sich das DNA-Template annähernd in jedem Reaktionsschritt. Entscheidend für die Auswertung ist der Zyklus, bei dem detektierbare Fluoreszenz auftritt und die exponentielle Phase der Amplifikation beginnt. Dieser Threshold-Cycle(CT)-Wert oder auch Crossing Point liefert die Basis für die Berechnung der Ausgangsmenge an vorhandener Ziel-DNA. So ermittelt die Software bei einer absoluten Quantifizierung die Crossing Points der unterschiedlichen Referenz-Verdünnungen und quantifiziert anhand der errechneten Standardkurve die Template-Menge. In der letzten Phase erreicht die Reaktion schließlich ein Plateau.

Die quantitative PCR ist ein wichtiges Werkzeug für Genexpressionsstudien in der klinischen Forschung. Mit der Möglichkeit, mRNA exakt zu quantifizieren, lassen sich bei der Suche nach neuen Wirkstoffen die Auswirkungen bestimmter Faktoren auf Zellen analysieren, die Differenzierung von Vorläuferzellen in verschiedene Zelltypen beobachten oder die Genexpression in Wirtszellen als Antwort auf Infektionen nachverfolgen. Durch den Vergleich von Wildtyp- und Krebszellen auf RNA-Ebene können in der Zellkultur Gene identifiziert werden, die einen entscheidenden Einfluss auf Krebsentstehung haben. In der Routine-Labordiagnostik wird Real-Time-PCR vorrangig zum qualitativen und quantitativen Nachweis von Viren und Bakterien eingesetzt. In der klinischen Routine, insbesondere im Bereich der Intensivmedizin, braucht der Arzt einen schnellen und eindeutigen Befund. Auf Basis der Real-Time-PCR können Tests durchgeführt werden, die noch am gleichen Tag das Ergebnis liefern. Hiermit begründet sich ein enormer Fortschritt für die klinische Diagnostik der Sepsis.

Neben den hier beschriebenen technischen Varianten der PCR-Methode können auch sogenannte isothermale Amplifikationsverfahren wie beispielsweise NASBA oder SDA oder andere technische Varianten für die der Detektion vorausgehenden Vervielfältigung der Zielsequenz verwendet werden.

Ein bevorzugtes Verfahren zur Wahl der Multigenbiomarkersequenzen umfaßt die folgenden Schritte:

a. Patientenauswahl basiert auf dem extremen Gruppenverfahren;

b. Generierung von mindestens einem Multigenbiomarker;

c. Bestimmung finaler Multigenbiomarker.

Ein bevorzugtes Verfahren des dem *"in vitro* Diagnostic multivariate index assay" ähnlichen Tests umfaßt die folgenden Schritte:

a. Isolierung von Proben-Nukleinsäuren aus einer von einem Patienten stammenden Probe;

b. Erfassung von Genaktivitäten mittels Sequenzen von wenigstens einem Zustands- und/oder Untersuchungsfragespezifischen Multigenbiomarkers;

c. Erfassung von Genaktivitäten für wenigstens ein internes Referenzgen um die in b) erfassten Genaktivitäten zu normalisieren;

d. Verwendung einer Interpretationsfunktion für die in c) normalisierten Genaktivitäten um einen Zustands- und/oder Untersuchungsfrage-spezifischen Index abzuleiten.

Eine bevorzugte Ausführungsform der vorliegenden Erfindung liegt auch in einer Verwendung, bei welcher die Genaktivitäten mittels eines Hybridisierungsverfahrens, insbesondere auf wenigstens einem Mikroarray, bestimmt werden. Der Vorteil eines Mikroarrays liegt in der höheren Informationsdichte des Biochips im Vergleich zu den Amplifikationsverfahren. So ist es z.B. mühelos möglich, auf einem Mikroarray mehrere 100 Sonden bereitzustellen, um in einem einzigen Untersuchungsgang mehrere Fragestellungen gleichzeitig zu untersuchen.

Die mittels der Erfindung erhaltenen Genaktivitätsdaten können auch vorteilhaft für die elektronische Weiterverarbeitung, z. B. zur Aufzeichnung in der elektronischen Krankenakte verwendet werden.

Eine weitere Ausführungsform der Erfindung besteht in der Verwendung von rekombinant oder synthetisch hergestellten, spezifischen Nukleinsäuresequenzen, Partialsequenzen, einzeln oder in Teilmengen, als Multigenbiomarker in SepsisAssays und/oder zur Bewertung der Wirkung und Toxizität beim Wirkstoffscreening und/oder zur Herstellung von Therapeutika und von Stoffen und Stoffgemischen, die als Therapeutikum vorgesehen sind, zur Vorbeugung und Behandlung von SIRS und Sepsis.

Für die erfindungsgemäßen Verfahren (Arraytechnik und/oder Amplifikationsverfahren) wird die Probe ausgewählt aus: Gewebe, Körperflüssigkeiten, insbesondere Blut, Serum, Plasma, Urin, Speichel oder Zellen oder Zellkomponenten; oder eine Mischung davon.

Es ist bevorzugt, dass Proben, insbesondere Zellproben, einer lytischen Behandlung unterzogen werden, um deren Zellinhalte freizusetzen.

Es ist dem Fachmann klar, dass die in den Ansprüchen dargelegten einzelnen Merkmale der Erfindung ohne Einschränkung beliebig miteinander kombinierbar sind.

## Klassifikationsmethoden

Die Theorie des Lernens spielt eine Schlüsselrolle auf dem Gebiet der Statistik, Datenanalyse und künstlichen Intelligenz mit zahlreichen Anwendungen in Ingenieurwissenschaften. Klassifikationsverfahren werden hauptsächlich bei 2 unterschiedlichen Aufgaben verwendet, bei der Abgrenzung von vorher unbekannten Klassen (unüberwachtes Lernen, class discovery) und bei der Zuordnung von bestimmten Daten/Proben/Patienten zu einer bereit definierten Klasse (Klassenvorhersage, class prediction) [Golub et al., 1999].

In der Klassenvorhersage werden Daten/Proben/Patienten benutzt, die bereits existierenden bzw. definierten Klassen/Gruppen zugeordnet wurden (so genannter Trainingsdatensatz), um ein analytisches Verfahren (Klassifikationsalgorithmus) zu entwickeln, das die Unterschiede zwischen den Gruppen widerspiegelt. Unabhängige Proben (sogenannter Testdatensatz) werden verwendet, um die Trennungsgüte der Klassifikationsregel zu bewerten. Die Vorgehensweise kann in folgende Schritte eingeteilt werden:

1. Definiere einen idealen Daten/Proben/Patientensatz, um charakteristische Profile der zu detektierenden Gruppen zu bekommen.
2. Jede Gruppe wird dann geteilt, so dass 2 gleichwertige Teilmengen, ein Trainingsdatensatz und ein Testdatensatz, entstehen.
3. Profile für den Trainingsdatensatz enthalten idealerweise Daten, die eine maximale Differenz zwischen den Gruppen widerspiegeln.
4. Die Differenz zwischen den Gruppen wird mittels geeigneter Abstandsmaße quantifiziert und anhand eines Algorithmus bewertet. Dieser Algorithmus sollte zu einer Klassifikationsregel führen, die mit der höchsten Spezifität und Sensitivität die Daten in die richtige Klasse einordnet. Typische Vertreter solcher Algorithmen aus dem Bereich des überwachten Lernens sind die Diskriminanzanalyse (DA), Random Forests (RF), Generalized Partial Least Squares (GPLS), Support Vector Machines (SVM) oder k-Nearest Neighbors (kNN).
5. Abschließend wird die Güte der Klassifikationsregel am Testdatensatz geprüft.

## Definitionen:

**Diskriminanzanalyse (DA):** Bei der linearen Diskriminanzanalyse erhalten wir eine lineare, bei der quadratischen Diskriminanzanalyse (QDA) eine quadratische Diskriminanzfunktion. Die Diskriminanzfunktion bestimmt sich aus der Kovarianzmatrix und den Gruppenmittelwerten. Im Fall der quadratischen Diskriminanzanalyse wird zusätzlich angenommen, dass auch die Kovarianzmatrix zwischen den Gruppen variiert [Hastie et al.,2001].

**Random Forests (RF):** Die Klassifikation mittels Random Forests basiert auf der Kombination von Entscheidungsbäumen, [Breiman, 2001]. Der Ablauf des Algorithmus ist etwa folgendermaßen:

1. Wähle durch Ziehen mit Zurücklegen einen Trainingsdatensatz aus (Out-ofbag data).
2. An jedem Knoten des Entscheidungsbaums wähle zufällig Variablen aus. Berechne mit diesen Variablen die beste Aufteilung des Trainingsdatensatzes auf die Klassen.
3. Nachdem alle Entscheidungsbäume generiert wurden, fass die Klassenzuordnungen der einzelnen Entscheidungsbäume zu einer Klassenzuordnung zusammen.

**Generalized Partial Least Squares (GPLS):** Bei dem Generalized Partial Least Squares [Ding und Gentleman, 2004] Verfahren handelt es sich um eine sehr flexible Verallgemeinerung des multiplen Regressionsmodells. Aufgrund der großen Flexibilität kann diese Methode auch in vielen Situationen angewendet werden, in denen das klassische Modell versagt.

**Support Vector Machine (SVM):** Beim Support Vector Machine Klassifikator handelt es sich um einen verallgemeinerten linearen Klassifikator. Die Eingabedaten werden in einem höher dimensionalen Raum abgebildet und in diesem Raum wird eine optimale trennende (Hyper-)Ebene konstruiert. Diese im höherdimensionalen Raum linearen Schranken transformieren sich zu nichtlinearen Schranken im Raum der Eingabedaten, [Vapnik, 1999].

**k-nächsten Nachbarn (k-Nearest Neighbors, kNN):** Bei der Methode der k-nächsten Nachbarn, wird die Klassenzugehörigkeit einer Beobachtung (eines Patienten) anhand der sich in seiner Umgebung befindlichen k-nächsten Nachbarn entschieden. Die Nachbarschaft wird dabei in der Regel mit Hilfe des euklidischen Abstands bestimmt und die Klassenzugehörigkeit dann anhand eines Mehrheitsvotums entschieden [Hastie et al., 2001].

Im Folgenden ist ein generelles Konzept beschrieben, wie die erfindungsgemäßen Verfahren durchgeführt werden. Dabei ist dem Fachmann wohbekannt, dass geringfüge Anpassungen der statistischen Verfahren erforderlich sein können, wenn andere Patientenkollektive und/oder andere Fragestellungen untersucht werden sollen. Für die Generierung der Klasiifikationsregel werden verschiedene statistische Verfahren (Diskriminanzanalyse und/oder Random Forests u.a.) sowie Strategien verwendet (einfache und mehrfache Kreuzvalidierung, zufällige Bootstrapstichproben u.a.)

Basierend auf Genexpressionsdaten sollte ein Verfahren zur Bestimmung eines Multigenbiomarkers entwickelt werden, der eine infektiöse Komplikation wie beispielsweise Sepsis widerspiegelt. Der Biomarker und der zugehörige Index-Wert, auch "Score" genannt, bilden die Grundlage eines sogenannten "in vitro Diagnostic multivariate Index Assays" [IVDMIA, FDA-Guidelines, 2003] zur Verbesserung der Diagnose systemischer Infektionen. Die aus dem Verfahren resultierende Klassifikationsregel sollte insbesondere eine Differenzierung von SIRS-und Sepsis-Patienten mit - im Vergleich zum etablierten Biomarker Procalcitonin - verbesserter Sensitivität und Spezifität ermöglichen, ist jedoch nicht auf diese Fragestellung beschränkt.

Zur Entwicklung eines solchen Multigenbiomarkers sind die folgenden Schritte notwendig:

*1.Schritt:* Trainingsdatensatz. Um den Zusammenhang zwischen einer Genexpression bestimmter Gene und der untersuchten Erkrankung aufzudecken, werden Populationen (Kohorten) definiert, die ihr Vorhanden- bzw. Nichtvorhandensein am deutlichsten repräsentieren. Bei der Diagnose einer infektiösen Komplikation werden üblicherweise Sepsis-Patienten (infektiös) und Patienten mit einer sogenannten sterilen SIRS (nichtinfektiös) in die Studie aufgenommen. Entsprechend dieser Festlegung wird ein Plan zur Sammlung bzw. Auswahl der zugehörigen RNA-Proben festgelegt. Von den ausgewählten Proben werden die Genexpressionsprofile auf einer geeigneten Plattform gemessen, vorverarbeitet und einer Qualitätskontrolle unterzogen. Systematische Messfehler werden korrigiert und Ausreißer eliminiert.

*2. Schritt:* Genvorauswahl. Bei der Generierung eines formalen Klassifikators auf der Basis von Mikroarray-Daten ist die Genvorauswahl ein Schlüsselschritt, da nur ein geringer Anteil der gemessenen Gene einen Beitrag zur Gruppenunterscheidung leistet. Auch die meisten Klassifikationsverfahren setzen eine Genselektion voraus. Durch eine präzise Genauswahl kann das Klassifikationsverfahren so einfach wie möglich gestaltet und eine Überanpassung an die Trainingsdaten (Overfitting) vermieden werden. Zur Vorauswahl der Klassifikationsgene werden geeignete Filteroptionen wie die Schwelle der statistischen Inferenz, der minimale akzeptierte Abstand zwischen den Gruppen, die minimale Signalintensität u. a. festgelegt. Nur Gene, die solche Bedingungen erfüllen, werden für die Klassifikation betrachtet.

*3.Schritt:* Klassifikationsverfahren. Verschiedene Klassifikationsverfahren werden bzgl. ihrer Trennfähigkeit hinsichtlich der zu differenzierenden pathophysiologischen Zustände getestet. Dazu werden Methoden der Cross-Validierung verwendet. Ein Klassifikationsverfahren mit dem kleinsten Klassifikationsfehler wird ausgewählt, wobei die kleinste notwendige Anzahl von Genen gleich mitbestimmt wird. Als sinnvolle Regel hat sich herausgestellt, dass die Anzahl der Gene immer kleiner sein soll als die Anzahl der Proben im Trainingsdatensatz, um eine Überanpassung zu vermeiden. Abschließend wird die resultierende Klassifikationsregel definiert.

Patientenauswahl Die Patientenauswahl ist bei der Aufstellung des Trainingsdatensatzes bedeutsam. In einer Vorstudie im Rahmen der vorliegenden Erfindung wurde vorerst eine Sensitivität von ca. 75 % im Trainings-und ca. 65 % im Testdatensatz erreicht. Diese relativ geringe Klassifikationsgüte ließ sich aber nicht durch die schwache Optimierung des Klassifikators, sondern durch die nicht ausreichend präzise Auswahl von Sepsis-Patienten erklären. Dementsprechend wurden Sepsis-Patienten nach einer Peritonitis viel häufiger richtig klassifiziert als Sepsis-Patienten nach einer "VAP" (Ventilator-Associated Pneumonia). Tatsächlich liegt die infektiöse Komplikation nach einer Peritonitis an sich vor. Dagegen lässt sich bei VAP eine wirkliche Infektion von einer Kolonisation nur schwer unterscheiden [Mayhall, 2001].

Um die Güte der Patientenauswahl zu bewerten, kann das Prinzip der sogenannten Extremgruppen nützlich sein. Danach werden in einer Studie nur solche Patientengruppen berücksichtigt, die den untersuchten Effekt möglichst deutlich abbilden. Dabei repräsentieren die ausgewählten Stichproben einen idealisierten Fall, in dem viele in der Praxis auftretende Effekte (z.B. die Häufigkeit der Erkrankung) nicht berücksichtigt werden. Von Liu [Liu et al., 2005] wurde vorgeschlagen, für den Trainingsdatensatz eines auf Mikroarrays basierten Klassifikators Extremgruppen zu bilden. Am Beispiel der Überlebensanalyse von Krebspatienten wurde gezeigt, dass die Anwendung von extremen Gruppen (Patienten, die nach kurzer Zeit gestorben sind vs. Patienten, die lange überlebt haben) zu einer besseren Vorauswahl von Klassifikationsgenen und einer höheren Klassifikationgüte geführt hat, auch wenn der Trainingdatensatz aus weniger Profilen (Patienten) bestand, als im üblichen Fall, wenn alle Patienten (auch mit mittleren Überlebenszeiten) berücksicht wurden.

Im Folgenden wird ausgeführt, wie weit die Patientenauswahl die Generierung eines Multigenbiomarkers zur Diagnose der infektiösen Komplikation beeinflussen kann. In einer Studie der Anmelderin wurden Patienten, die nach einem schweren operativen Eingriff eine Sepsis entwickelt haben, untersucht. Es wurden Proben vom ersten Tag der Sepsis-Diagnose mit der Probe vom ersten post-operativen Tag verglichen. Die hier signifikant differentiell exprimierten Gene spiegeln aber einen Mischeffekt wider; die infektiöse Komplikation wird verdeckt durch Effekte wie Erholung nach dem operativen Stress oder die post-operative Behandlung. In der bereits erwähnten Pilotstudie wurden die Patienten mit einer klinischen (nicht immer mikrobiologisch gesicherten) Sepsisdiagnose in die Trainingspopulation eingeschlossen, was zu einer Durchmischung der beiden untersuchten Gruppen (Septiker und Kontrollen) führte und die Sensitivität verschlechterte. In dem Ausführungsbeispiel der US-Patentanmeldung Nr. 20060246495 wurde für die Auswahl der Sepsisgruppe ebenfalls die klinische Sepsisdiagnose verwendet. Darüber hinaus wurde die Schwere der Erkrankung zwischen der Gruppe der Sepsispatienten und der Kontrollgruppe der SIRS-Patienten nicht berücksichtigt. Dies kann der Grund der geringen Klassifikationsgüte und ihrer Abhängigkeit vom Klassifikationsalgorithmus sein. In die Studie von Johnson [Johnson et al., 2007] wurden Patienten nach einem Trauma in zwei Gruppen aufgeteilt, mit einer infektiösen Komplikation und ohne eine Infektion. Der Vorteil dieser Studie war, dass Patienten der beiden Gruppen sich in Komorbidität und Vorbehandlung wenig unterschieden. Die Vorauswahl ist aber nicht für alle Sepsispatienten repräsentativ und die Verallgemeinerung des hier aufgedeckten sepsisrelevanten Genexpressionsmusters auf Patienten mit anderem Hintergrund (auf andere Risikogruppen) ist nicht selbstverständlich. Im Allgemeinen muss davon ausgegangen werden, dass in Studien mit verschiedenen Risikogruppen auch verschiedene Klassifikatoren generiert werden müssen. In der Studie von Tang [Tang et al., 2007a] wurde das Prinzip der Extremgruppen indirekt angewandt, in dem nur Patienten mit einer mikrobiologisch gesicherten Sepsis-Diagnose im Trainingsdatensatz berücksichtigt wurden. Der Proben-Sammelplan führte aber zu einer kleinen Kontrollgruppe (ein Drittel der Proben: 14 aus 44). Dementsprechend wurde im Training eine Spezifität von 77% und in einem unabhängigen Testdatensatz (unter mehr realen Bedingungen) von lediglich 60% erreicht. Die Beschreibung der Patientengruppen in der SIRS-Lab Studie und der Studie von Tang [Tang et al., 2007a] lässt einen weiteren Einflussfaktor erkennen. Sie zeigt, dass die bzgl. des Infektionsfokus heterogenen Sepsisgruppen nicht ausbalanciert sind, sondern dass Gruppen mit verschiedenen Infektionsfoci unterschiedlich vertreten sind. Tatsächlich ist im Großteil der Fälle auf der Intensivstation (ITS) die Lunge (ca. 45-50 %) oder der Abdomen (ca. 25%) der Fokus der Infektion bei einer Sepsisdiagnose. Dementsprechend sind diese Patientengruppen in den Untersuchungen überrepräsentiert, viele andere Foci kommen dann nur vereinzelt vor. Ähnlich sind in den Kontrollgruppen insbesondere postoperative und Traumapatienten vertreten und andere Risikogruppen sind nur durch einzelne Patienten vertreten. Die dargestellte Analyse zeigt, dass in allen Studien die ausgewählten Patientengruppen die infektiöse Komplikation nicht eindeutig abbilden, wodurch die Klassifikationsschwächen erklärt werden können. Andererseits wird aus der Zusammenfassung deutlich, dass es bei der infektiösen Komplikation kaum möglich ist, alle Einflussfaktoren bei der Auswahl der Patientengruppen zu berücksichtigen. Aus diesem Grund wird der folgende Weg zur Patientenauswahl für den Trainingsdatensatz vorgeschlagen.

## Allgemeines zu Material und Methoden der vorliegenden Erfindung:

### Patientenauswahl

Die Auswahl der repräsentativen Stichproben stand im Mittelpunkt des beschriebenen Verfahrens. Es wurden in den Trainingsdatensatz Patienten mit einer mikrobiologischen gesicherten bzw. ausgeschlossenen Infektionsdiagnose aus jeweils zwei der am besten repräsentierten Sepsis- bzw. Kontrollsubgruppen eingeschlossen. Damit wird das Prinzip der Extremgruppen nicht nur für den Haupteffekt (infektiös vs. nicht infektiös) sondern auch für die Kontrolle der wichtigsten Einflussgrössen (Schichtung von Populationen) angewandt. Der Vorteil dieser Auswahl ist vorerst, dass man hier einen Klassifikator für die häufigsten Risiko bzw. Erkrankungsgruppen generiert. Darüber hinaus wird erwartet, dass sich ein Klassifikator, der die systemische Infektion für wenige aber sehr unterschiedliche Subgruppen widerspiegelt, sich auf weitere Patientengruppen anwenden lässt. Bei der Auswahl der Trainingsdaten wurde wie folgt vorgegangen. In die Patientendatenbank der Anmelderin wurden im Zeitrahmen von zweieinhalb Jahren 400 ITS-Patienten aufgenommen, bei denen ein Sepsis-Risiko vermutet wurde, und die zugehörigen klinischen Daten über den gesamten Aufenthalt detailliert dokumentiert. Die RNA-Proben wurden über ca. 7-14 Sepsis-relevante Tage gesammelt. In Annäherung an das PIRO-Konzept [Levy et al., 2003] wurden die Patienten retrospektiv nach folgenden Kriterien stratifiziert: (i) welche Indikation führte zur der Übernahme auf die Intensivstation (postoperative Komplikation, Trauma bzw. Polytrauma, akuter Sepsisverdacht), (ii) wurde eine infektiöse Komplikation diagnostiziert, was war der Infektionsfokus, (iii) wie war die Reaktion des Organismus (Anzahl der vorhanden SIRS-Kriterien , Schock-Behandlung, PCT-, CRP-Werte), (iv) wie schwer war die Erkrankung (SOFA, MODS-Score). Die Datenbankrecherche ergab, dass mit einer infektiösen Komplikation (Sepsis) insbesondere Patienten nach einer Pneumonie (40%) und nach einer Peritonitis (23%) in die Studie aufgenommen wurden. Weitere Fokuse kamen vereinzeln vor. Diese Daten entsprechen den epidemiologischen Studien der Deutschen Sepsisgesellschaft, womit die Sammlung als repräsentativ eingestuft wurde. Die Patientendaten dieser Gruppen wurden unabhängig von 2 Ärzten [nach ACCP/SCCM, 1992; Levy et al., 2003; Calandra und Cohen, 2005] geprüft und die finale Patientenauswahl festgelegt. Es wurden 29 Patienten mit einer mikrobiologisch gesicherten Diagnose ausgewählt und der erste septische Tag bestimmt. Die Zusammenfassung der Schwere-Kriterien ergab, dass bei den Patienten an diesem Tag eine schwere Sepsis bzw. ein septischer Schock diagnostiziert wurde. Sie erreichten einen durchschnittlichen SOFA- Wert von 10, die Summe akuter Organdysfunktionen betrug etwa 3. Als Kontrollgruppe wurden 29 Risiko-Patienten nach einer Bypass-Operation eingeschlossen. Es wurde der erste Tag mit einer ähnlichen Schwere wie bei den Sepsis-Gruppen bestimmt aber ohne Zeichen einer Infektion ausgewählt. Eine Aufstellung zu wichtigen klinischen und Labor-Parametern für die ausgewählten Patienten findet sich beispielhaft, jedoch ohne Einschränkung hierauf, in Tabelle 1.

Tabelle 1: Zusammenfassung der klinischen Parameter für Patienten des Trainingsdatensatzes. Die Werte entsprechen der Anzahl oder, mit Stern markiert, dem Median (Interquartilabstand) der Werte.

| | Sepsis | keine Sepsis |
|---|---|---|
| Patientenanzahl | 29 | 29 |
| Mortalität | 52% | 21% |
| Geschlecht (m/w) | 22/7 | 20/9 |
| Alter (y)* | 66 (13) | 68 (8) |
| SIRS-Kriterien* | 3 (0) | 3 (2) |
| SOFA-Score* | 10 (4) | 7 (4) |
| Anzahl der Organdysfunktionen* | 3 (2) | 2 (2) |
| PCT (ng/ml)* | 12 (24,32) | 1,82 (10,78) |
| CRP (mg/l)* | 194 (161) | 85,45 (88,675) |
| WBC (no/l)* | 12200 (11150) | 12800 (8700) |
| Apache II | 19 (6) | 13 (5) |
| Hypotensionsbehandlung | 90% | 48% |
| | *Sepsis-Fokus:* | *Indikation für ITS- Aufnahme:* |
| Peritonitis | 13 | Kardio-pulmonarer Bypass / ITS-Aufenthalt mehr als 3 Tage: 22 |
| Pneumonia | 8 | |
| Mediastinitis | 4 | |
| Myocarditis | 1 | Kardio-pulmonarer Bypass / ITS-Aufenthalt max. 3 Tage: 7 |
| Urosepsis | 1 | |
| Kneeempyem | 1 | |

Generierung des Klassifikators und Etablierung des SIQ-Scores

Auf dem Weg zur Klassifikatorentwicklung wurden folgende Schritte durchgeführt:

1. Schritt: Qualitätskontrolle: Aus der vom Expertenwissen bestätigten Vorauswahl aus einem Patientenkollektiv wurden die zugehörigen Genexpressionsdaten verschiedenen Ähnlichkeitsanalysen unterworfen, um untypische Hybridisierungsergebnisse auszuschließen [Buneß et al., 2005], wodurch die finale Trainingsdatenmatrix generiert wurde.

2. Schritt: Normalisierung bzw. Vorverarbeitung der Daten: Zum Normalisieren wurde für jede Probe der Mittelwert der 3 ausgewählten Housekeeper-Gene (R1, R2 und R3) berechnet. Von diesem Wert wurde der Ct-Wert jedes einzelnen Markers abgezogen. Jeder so gewonnene Delta Ct Wert spiegelt die relative Abundanz des Targettranskriptes bezogen auf den Kalibrator wider, wobei ein positiver Delta Ct Wert eine Abundanz höher als der Mittelwert der Referenzen und negativer Delta Ct Wert eine Abundanz kleiner als der Mittelwert der Referenzen bedeuten.

3. Schritt: Ranking: Um die Gen-Marker nach ihrer Trennungsgüte anzuordnen, wurde die lineare Diskriminanzanalyse (LDA) [Hastie et al., 2001] zusammen mit der Methode der vorwärts Selektion verwendet, wobei die Trennbarkeit mit dem F-Wert bewertet wurde [Hocking, R. R., 1976). Dieser Analyseschritt wurde für 1000 Bootstrap-Stichproben wiederholt. Die in jeder Wiederholung ermittelten Marker-Ranks wurden über die 1000 Läufe gemittelt und die Marker-Kandidaten wurden nach dem mittleren Rank aufsteigend angeordnet. Diese Anordnung bedeutet, dass der Marker mit dem kleinsten mittleren Rank der war, der am häufigsten den meisten Beitrag zur Trennungsgüte leistete und der Marker mit dem höchsten mittleren Rank für die Trennung in meisten Wiederholungen wenig beitrug.

4. Schritt: Klassifikation: Für die Marker, die in der Ranking-Analyse die besten Ergebnisse lieferten, wurde basierend auf der LDA eine Diskriminazfunktion bestimmt. Die zugehörigen Gewichte werden in der Tabelle 9 dargelegt.

5. Schritt: Interne Validierung: Um die Güte der Klassifikation für wachsende Anzahl von Markern zu beurteilen wurde die einfache Kreuzvalidierung verwendet.

6. Schritt: Etablierung des SIQ-Scores: Basierend auf der Diskriminanzfunktion wurde ein auf die Sepsis bezogener diagnostischer Parameter, ein sogenannter SIQ-Score (SIQ) wie folgt eingeführt. Für eine neue unabhängige Probe bekommt man i.a. als Klassifkationsergebnis einen dimensionsfreien Wert der Diskriminanzfunktion. Ein positiver Wert klassifiziert die Probe als infektiös und ein negativer Wert als nicht infektiös. Für typische Vertreter der jeweiligen Gruppe erhält man absolut höhere Werte, schwer klassifizierbare Proben erreichen Werte nahe Null. Der Streubereich der Diskriminanz-Werte entspricht i.a. der Variabilität der Datenmatrix. So erreichte man in der Klassifikation Diskriminanzwerte von ca. -5 bis 5. Um die Unterschiede deutlicher hervorzuheben, wurde der SIQ-Score (SIQ) als der 10-fache Wert der Diskriminanzfunktion mit den Gewichten aus der Tabelle 9 eingeführt. Dementsprechend variierten die SIQ-Werte der Testdaten von ca. -50 bis 50.

Die vorliegende Erfindung wird im Folgenden anhand von Beispielen und unter Bezug auf das Sequenzprotokoll, das ebenfalls ein Teil dieser Beschreibung ist, näher erläutert, ohne dass dies eine Einschränkung der Erfindung bedeutet.

## Ergebnisse

Im nächsten Schritt wurden die Genexpressionsdaten der Patientendatenbank der Anmelderin, die nicht im Trainingsdatensatz verwendet wurden, einer Klassifikation unterzogen. Dieser unabhängige Testdatensatz bestand aus 113 Proben von 65 Personen (vgl. Tabellen 4 und 5). Dabei wurden Proben von 38 Sepsis-Patienten untersucht, die ein breites Spektrum an klinischen Phänotypen mit dem Risiko eine generalisierte Infektion ausbilden, repräsentieren. Darüber hinaus wurden Proben über den SIRS-Verlauf von 22 postoperativen Patienten sowie 5 gesunden Probanden analysiert.

Für diesen unabhängigen Testdatensatz wurde die beste Klassifikationseffizienz von 81,4% mit 7 folgenden Markern erreicht: M6, M15, M9, M7, M2, M10, M4. Die ROC-Kurve zur Klassifikation von Testdaten wird in Fig. 1 dargestellt. Als Vergleich wird in Fig 4 die ROC-Kurve zur Klassifikation von Testdaten mittels PCT oder CRP dargestellt. Aus der Fig. 4 wird ersichtich, dass für beide Paramter die Fläche unter der Kurve, die die Güte der Klassifikation widerspiegelt, unter 70% liegt und damit diagnostisch wenig relevant ist.
In der Fig. 2 (Patient 8112) wird der Verlauf des SIQ-Scores für einen Patienten dargestellt, der postoperativ eine Sepsis entwickelt hat. Aus Fig. 2 wird ersichtlich, dass SIQ-Score die diagnostisch-relevante Schwelle bereits 2 Tage vor der klinischen Manifestation der Sepsis übersteigt. Der Verlauf weiterer Sepsis-relevanten klinischen Parametern (PCT, CRP, SOFA, Körpertemperatur, Schockbehandlung) wird als Vergleich mit dargestellt. In diesem Vergleich wird ersichtlich, dass SIQ-Score der einzige Parameter ist, der vorzeitig die infektiöse Komplikation widerspiegelt. Damit wird demonstriert, dass die beschriebene Erfindung für die frühe Erkennung von infektiösen Komplikationen, wie Sepsis und/oder generalisierter Infektion, angewendet werden kann.

In der Fig. 3 (Patient 7084) wird der Verlauf des SIQ-Scores für einen Patienten dargestellt, der postoperativ eine Sepsis entwickelt hat, in einen septischen Schock fiel, sich aber einer akuten Phase durch eine relevante Behandlung wieder erholt hat. Aus Fig. 3 wird ersichtlich, dass SIQ-Score ein Tag vor der klinischen Manifestation der Sepsis über den diagnostischen Schwellenwert ansteigt und in der akuten Phase über der Schwelle bleibt. Nach der akuten Phase fällt der SIQ-Score unter diese Schwelle. Damit wird demonstriert, dass die beschriebene Erfindung für die Verlaufskontrolle und/oder Therapiekontrolle von z.B. Antibiotika-Therapie und/oder adjunktive klinische Maßnahmen und/oder operative Sanierungsmaßnahmen angewendet werden kann.

Weitere Vorteile und Merkmale der vorliegenden Erfindung ergeben sich aufgrund der Beschreibung von Ausführungsbeipielen sowie anhand der Zeichnung.

Es zeigt:

Fig. 1 eine ROC-Kurve zur Klassifikation von Testdaten mittels SIQ-Scores;

Fig. 2 eine Darstellung eines beispielhaften Verlaufs eines erfindungsgemäßen Scores (SIQ-Score) und der sepsisrelevanten klinischen Parameter PCT und CRP (Fig. 2A) sowie SOFA-Score, Körpertemperatur und Katecholamindosierung (Fig. 2B) für einen ersten Patienten;

Fig. 3 eine Darstellung eines beispielhaften Verlaufs eines erfindungsgemäßen Scores (SIQ-Score) und der sepsisrelevanten klinischen Parameter PCT und CRP (Fig. 3A) sowie SOFA-Score, Körpertemperatur und Katecholamindosierung (Fig. 3B) für einen zweiten Patienten; und

Fig.4 eine ROC-Kurve zur Klasifikation von Testdaten mittels PCT oder CRP.

Die vorliegende Erfindung wird im Folgenden anhand von Beispielen und unter Bezug auf das Sequenzprotokoll, das ebenfalls ein Teil dieser Beschreibung ist, näher erläutert, ohne dass dies eine Einschränkung der Erfindung bedeutet.

Fig. 1 zeigt eine ROC-Kurve zur Klassifikation der Testdaten mittels des SIQ-Scores In Fig. 1 wird das Verhältnis zwischen richtig Positiven (Sensitivität) und falsch Positiven (1-Spezifität) markiert, grau gestrichelt für den Schwellenwert von Null und schwarz gestrichelt für die beste erreichte Klassifikation von 81,4%.

Fig. 2 zeigt einen Verlauf des SIQ-Scores eines beispielhaften Patienten und der Sepsis-relevanten klinischen Parameter PCT, CRP, SOFA, Körpertemperatur sowie die Dosierung von Katecholaminen (norepinephrine), die die Schock-Behandlung reflektiert. Im Teil A der Figur wurde die Skala jedes Parameters so angepasst, dass die schwarze horizontale Mittel-Linie den diagnostisch relevanten Schwellenwert markiert. Die Sepsis wurde am 6. Tag diagnostiziert, der SIQ-Score steigt bereits am 4. Tag über die Schwelle von -4.9.

Fig. 3 zeigt einen Verlauf des SIQ-Scores eines weiteren Patienten und der Sepsis-relevanten klinischen Parameter PCT, CRP, SOFA, Körpertemperatur sowie die Dosierung von Katecholaminen (norepinephrine), die die Schock-Behandlung reflektiert. Im Teil A der Figur wurde die Skala jedes Parameters so angepasst, dass die schwarze horizontale Mittel-Linie den diagnostisch relevanten Schwellenwert markiert. Die Sepsis wurde am 4. ITS-Tag diagnostiziert, der SIQ-Score steigt bereits am 3. Tag über die Schwelle von -4.9. Nach der akuten Phase, die mit dem Absetzen der Katecholamine (Schock-Behandlung) am 8 Tag beendet wird, fällt der SIQ-Score unter die Schwelle von -4.9.

Fig. 4 zeigt ROC-Kurven zur Klassifikation der Testdaten mittels der Paramter PCT oder CRP In der Fig. 4 wird in schwarz die ROC-Kurve zu PCT und in grau die ROC-Kurve zu CRPdargestellt, Die Fläche unter der Kurve, die die Güte der Klassifikation widerspiegelt, beträgt für PCT 56,8% und für CRP 66.9%.

Die nachfolgende Tabelle 2 gibt die eineindeutige Zuordnung der erfindungsgemäßen Marker-Polynucleotide zu ihren Transkriptvarianten/cis regulatorischen Sequenzen (Isoformen), der Gendatenbank-Zugriffsnummer und der SEQ ID des Sequenzprotokolls wieder.

**Tabelle 2**

| Marker und Referenzgene | **Transkriptvariante** | **Accession Number** | **Seq ID** |
|---|---|---|---|
| M2 | M2_1 | NM_001031700 | 4 |
| | M2_2 | NM_016613 | 25 |
| | M2_3 | NM_001128424 | 15 |

(fortgesetzt)

| Marker und Referenzgene | **Transkriptvariante** | **Accession Number** | **Seq ID** |
|---|---|---|---|
| M4 | M4_1 | NM_203330 | 35 |
| | M4_2 | NM_000611 | 3 |
| | M4_3 | NM_203329 | 34 |
| | M4_4 | NM_203331 | 36 |
| | M4_5 | NM_001127223 | 11 |
| | M4_6 | NM_001127225 | 12 |
| | M4_7 | NM_001127226 | 13 |
| | M4_8 | NM_001127227 | 14 |
| M6 | M6_1 | NM_001831 | 18 |
| | M6_2 | NM_203339 | 37 |
| M7 | M7_1 | NM_031311 | 28 |
| | M7_2 | NM_019049 | 26 |
| M9 | M9 | NM_006682 | 24 |
| M10 | M10 | NM_033554 | 32 |
| M15 | M15_1 | NM_003580 | 23 |
| | M15_2 | NM_001144772 | 16 |
| M3 | M3_A | NM_001123041 | 9 |
| | M3_B | NM_001123396 | 10 |
| M8 | M8 | NM_025209 | 27 |
| M12 | M12 | NM_002185 | 19 |
| M13 | M13 | NM_001080394 | 7 |
| M16 | M16 | NM_003268 | 22 |
| M17 | M17 | NM_182491 | 33 |

Tabelle 3 gibt für jeden der erfindungsgemäßen Marker-Polynucleotide die Primer (forward und reverse) für die quantitative PCR sowie das resultierende Amplikon und deren eineindeutige Zuordung zu der jeweiligen SEQ ID des Sequenzprotokolls wieder.

**Tabelle 3**

| Marker und Referenzgene | Primer für quantitative PCR/resultierende Amplikon | SEQ ID |
|---|---|---|
| M2 | M2-fw | 38 |
| | M2-rev | 39 |
| | M2-Amplikon | 40 |
| M4 | M4-fw | 41 |
| | M4-rev | 42 |
| | M4-Amplikon | 43 |

(fortgesetzt)

| Marker und Referenzgene | Primer für quantitative PCR/resultierende Amplikon | SEQ ID |
|---|---|---|
| M6 | M6-fw | 44 |
| | M6-rev | 45 |
| | M6-Amplikon | 46 |
| M7 | M7-fw | 47 |
| | M7-rev | 48 |
| | M7-Amplikon | 49 |
| M9 | M9-fw | 50 |
| | M9-rev | 51 |
| | M9-Amplikon | 52 |
| M10 | M10-fw | 53 |
| | M10-rev | 54 |
| | M10-Amplikon | 55 |
| M15 | M15-fw | 56 |
| | M15-rev | 57 |
| | M15-Amplikon | 58 |
| M3 | M3-fw | 59 |
| | M3-rev | 60 |
| | M3-Amplikon | 61 |
| M8 | M8-fw | 62 |
| | M8-rev | 63 |
| | M8-Amplikon | 64 |
| M12 | M12-fw | 65 |
| | M12-rev | 66 |
| | M12-Amplikon | 67 |
| M13 | M13-fw | 68 |
| | M13-rev | 69 |
| | M13-Amplikon | 70 |
| M16 | M16-fw | 71 |
| | M16-rev | 72 |
| | M16-Amplikon | 73 |
| M17 | M17-fw | 74 |
| | M17-rev | 75 |
| | M17-Amplikon | 76 |
| | | |

(fortgesetzt)

| Marker und Referenzgene | Primer für quantitative PCR/resultierende Amplikon | SEQ ID |
|---|---|---|
| R1 | R1-fw | 77 |
| | R1-rev | 78 |
| | R1-Amplikon | 79 |
| R2 | R2-fw | 80 |
| | R2-rev | 81 |
| | R2-Amplikon | 82 |
| R3 | R3-fw | 83 |
| | R3-rev | 84 |
| | R3-Amplikon | 85 |

**Biologische Plausibilität der identifizierten Biomarker**

Die beschriebenen Biomarker sind in funktioneller Hinsicht mit hoher Signifikanz immunologischen und inflammatorischen Signalwegen zuzuordnen. Eine wissensbasierte Analyse der Biomarkerpopulation wurde mit der Software Ingenuity Pathways Analysis (Ingenuity Systems, USA, www.ingenuity.com) durchgeführt, um den funktionellen Kontext der identifizierten Marker zu verdeutlichen. Basierend auf dem gesamten öffentlich verfügbaren Datenbankwissen werden die Marker in funktionelle Netzwerke und Kategorien eingeordnet. Die Hauptkategorien der vorliegenden Markerpopulation sind das Komplementsystem, Toll-like-Rezeptor-Signaltransduktion, Kommunikation zwischen Zellen der innaten und adaptiven Immunabwehr, TREM-1-Signaltransduktion, Ceramid-Signaltransduktion. Die Marker sind demnach mit hoher Signifikanz an immunologischen und entzündlichen Prozessen beteiligt, was die Relevanz für das Krankheitsbild der Sepsis untermauert. Damit konnte eine wichtige Grundvoraussetzung für Biomarker, das Vorhandensein biologischer Plausibilität, nachgewiesen werden.

Theragnostisches Potenzial der Biomarker am Beispiel der Koagulation:

Die Analyse der biologischen Plausibilität der Biomarker ergab für M6 und M9 eine funktionelle Rolle im Kontext der Koagulation und Fibrinolyse. Beide Prozesse gehören zu den am stärksten deregulierten physiologischen Funktionen in septischen Patienten. Eine Therapieoption von Patienten mit schwerer Sepsis und Organversagen stellt die Behandlung mit aktiviertem Protein C oder Thrombomodulin dar. M6 ist in septischen Patienten überexprimiert und gleichzeitig der negativen Transkriptionskontrolle durch aktiviertes Protein C unterworfen. M9 ist in septischen Patienten supprimiert und kann die zugeschriebene Aktivität der Prothrombin-Spaltung zur Bereitstellung von Thrombin möglicherweise nicht erfüllen. Thrombin wiederum ist nach Assoziation mit Thrombomodulin ein wichtiger Faktor für die Aktivierung von Protein C. Aufgrund dieser engen funktionellen Zusammenhänge erscheint es möglich, in entsprechenden klinischen Studien nach Mustern zu suchen, welche für den erfolgreichen Einsatz der o.g. therapeutischen Möglichkeiten charakteristisch sind. Dieser theragnostische Ansatz könnte es ermöglichen, die Responder zu identifizieren und Nonrespondern die u.U. gravierenden Nebenwirkungen zu ersparen. Die identifizierten Marker für die für eine solche Anwendung beinhalten somit auch das Potenzial für eine Entscheidungsfindung bezüglich spezieller Therapien des septischen Patienten.
Im Folgenden sind zunächst die klinisch relevanten Daten des untersuchten Patientenkollektives als Tabelle 4 gezeigt:

Tabelle 4

| Patient | Alter [Jahre ] | Geschlecht | Apache | postoperative Indikationen | nicht operative Indikationen | Aufhmediagnose | Überlebens -status (ITS) | Liegedauer [Tage] |
|---|---|---|---|---|---|---|---|---|
| 1013 | 59 | männlich | 22 | schwere Sepsis | | Sepsis, nicht näher bezeichnet | ja | 22 |
| 1015 | 71 | männlich | 29 | Eingriff Herzkranzgefäße, Thorax | | Instabile Angi pectoris | ja | 41 |
| 2042 | 81 | männlich | 15 | Eingriff Herzkranzgefäße | | Atherosklerotische Herzkrankheit: Ein-Gefäßerkrankung | ja | 7 |
| 5008 | 42 | männlich | 0 | | respiratorische Insuffiziens(Infektion), pankreatitis, akutes Organversagen (respiratorisch), akutes Organversagen (metabolisch), akutes Organversagen (rel) | Akute Pankreatitis | nein | 13 |
| 5009 | 57 | männlich | 21 | | schwere Sepsis, respiratorische Insuffiziens (Atemstillstand) , respiratorische Insuffiziens(Infektion), leberversagen, akutes Organversagen (respiratorisch), akutes Organversagen (metabolisch) | Sepsis, nicht näher bezeichnet | nein | 42 |
| 5010 | 67 | weiblich | 0 | schwere Sepsis, postoperativkardiovaskulaer, postoperativgastrointestil, postoperativ-metabolisch | | Akute Peritonitis | nein | 31 |

(fortgesetzt)

| Patient | Alter [Jahre ] | Geschlecht | Apache | postoperative Indikationen | nicht operative Indikationen | Aufhmediagnose | Überlebens -status (ITS) | Liegedauer [Tage] |
|---|---|---|---|---|---|---|---|---|
| 5018 | 71 | männlich | 28 | schwere Sepsis, Eingriff Herzkranzgefäße, postoperativ-kardiovaskulaer, postoperativrespiratorisch, postoperativ-rel | | Linksherzinsuffizienz | ja | 4 |
| 5019 | | weiblich | | neurochirurgisch | | Bandscheibenvorfall | ja | |
| 5020 | | männlich | | neurochirurgisch | | Bandscheibenvorfall | ja | |
| 5023 | | weiblich | | neurochirurgisch | | Bandscheibenvorfall | ja | |
| 6005 | 48 | weiblich | 17 | schwere Sepsis | | Akute Cholezystitis | nein | 28 |
| 6008 | 62 | weiblich | 14 | gastrointestil | | Peritonitis, nicht näher bezeichnet | ja | 13 |
| 6024 | 64 | männlich | 12 | | schwere Sepsis | Sepsis: Escherichia coli [E. coli] | ja | 6 |
| 6035 | 63 | männlich | 29 | schwere Sepsis, gastrointestil | | Perforation des Darmes (nichttraumatisch) | nein | 20 |
| 6036 | 33 | männlich | 9 | polytrauma | | Nicht näher bezeichnete multiple Verletzungen | ja | 20 |
| 6056 | 76 | weiblich | 23 | Eingriff Herzkranzgefäße, Eingriff Herzklappen | | Aortenklappenstenose | ja | 36 |
| 6061 | 68 | weiblich | 25 | Eingriff Herzkranzgefäße, Thorax | | Aortenklappenstenose | nein | 29 |
| 6063 | 59 | männlich | 17 | Wirbelsaeule | | Rückenmark-kompression, nicht näher bezeichnet | ja | 9 |
| 6064 | 78 | männlich | 16 | | DHI-Arrythmien | Sonstige näher bezeichnete Krankheiten des Pankreas | ja | 36 |
| 6070 | 66 | männlich | 14 | schwere Sepsis, Thorax | | Chronische Niereninsuffizienz, nicht näher bezeichnet | ja | 19 |

(fortgesetzt)

| Patient | Alter [Jahre ] | Geschlecht | Apache | postoperative Indikationen | nicht operative Indikationen | Aufhmediagnose | Überlebens -status (ITS) | Liegedauer [Tage] |
|---|---|---|---|---|---|---|---|---|
| 6075 | 73 | weiblich | 22 | gastrointestil | | Ileus, nicht näher bezeichnet | ja | 47 |
| 6104 | 40 | weiblich | 17 | | schwere Sepsis, respiratorische Insuffiziens (Asthma), respiratorische Insuffiziens (Aspiration), respiratorische Insuffiziens (Infektion) | Akute respiratorische Insuffizienz, anderenorts nicht klassifiziert | ja | 28 |
| 6120 | 54 | männlich | 18 | | schwere Sepsis | Perforation des Ösophagus | nein | 19 |
| 6124 | 69 | männlich | 15 | schwere Sepsis, Thorax | | Abnorme Befunde bei der bildgebenden Diagnostik der Lunge | ja | 13 |
| 6126 | 39 | männlich | 23 | schwere Sepsis, polytrauma | | Atemnotsyndrom des Erwachsenen [ARDS] | ja | 126 |
| 6141 | 70 | männlich | 21 | Thorax | | Emphysem, nicht näher bezeichnet | nein | 38 |
| 7023 | 75 | männlich | 21 | Eingriff Herzkranzgefäße | | | nein | 61 |
| 7040 | 70 | männlich | 21 | | | Sepsis, nicht näher bezeichnet | ja | 27 |
| 7052 | 67 | weiblich | 22 | | intrakranielle Blutung | Subarachnoidalblutung , von der A. communicans anterior ausgehend | ja | 33 |
| 7077 | 63 | männlich | 17 | | | Bösartige Neubildung: Mundboden, nicht näher bezeichnet | ja | 38 |
| 7079 | 77 | männlich | 26 | Eingriff Herzkranzgefäße | | | ja | 33 |

(fortgesetzt)

| Patient | Alter [Jahre ] | Geschlecht | Apache | postoperative Indikationen | nicht operative Indikationen | Aufhmediagnose | Überlebens -status (ITS) | Liegedauer [Tage] |
|---|---|---|---|---|---|---|---|---|
| 7084 | 69 | männlich | 17 | Eingriff Herzkranzgefäße | | Krankheiten der Mitral- und Trikuspidalklappe, kombiniert | ja | 24 |
| 7096 | 85 | männlich | 18 | | | Atherosklerose der Extremitäterterien: Becken-Bein-Typ, mit Gangrän | ja | 8 |
| 7105 | 75 | weiblich | 20 | schwere Sepsis | | Sepsis, nicht näher bezeichnet | ja | 10 |
| 7112 | 75 | weiblich | 27 | Eingriff Herzkranzgefäße | | Atherosklerotische Herzkrankheit: Drei-Gefäßerkrankung | nein | 67 |
| 7119 | 64 | weiblich | 14 | Eingriff Herzkranzgefäße | | Instabile Angi pectoris | ja | 6 |
| 7120 | 84 | weiblich | 21 | schwere Sepsis | | Bösartige Neubildung am Rektosigmoid, Übergang | nein | 13 |
| 714 | 79 | weiblich | 26 | gastrointestil | | Ulcus duodeni: Chronisch oder nicht näher bezeichnet, mit Perforation | nein | 7 |
| 749 | 75 | weiblich | 16 | Eingriff Herzklappen, Thorax | | Aortenklappenstenose | ja | 27 |
| 8009 | 60 | männlich | 9 | Eingriff Herzkranzgefäße | | Atherosklerotische Herzkrankheit: Ohne hämodymisch wirksame Stenosen | ja | 51 |
| 8011 | 64 | männlich | 4 | Eingriff Herzkranzgefäße | | Atherosklerotische Herzkrankheit: Ohne hämodymisch wirksame Stenosen | ja | 2 |
| 8026 | 68 | weiblich | 12 | Eingriff Herzkranzgefäße, Eingriff Herzklappen | | Atherosklerotische Herzkrankheit: Ein-Gefäßerkrankung | ja | 6 |

(fortgesetzt)

| Patient | Alter [Jahre ] | Geschlecht | Apache | postoperative Indikationen | nicht operative Indikationen | Aufhmediagnose | Überlebens -status (ITS) | Liegedauer [Tage] |
|---|---|---|---|---|---|---|---|---|
| 8034 | 77 | weiblich | 12 | Eingriff Herzkranzgefäße | | Atherosklerotische Herzkrankheit: Ohne hämodymisch wirksame Stenosen | ja | 2 |
| 8039 | 55 | weiblich | 16 | Eingriff Herzklappen | | Aortenklappenstenose | ja | 7 |
| 8044 | 70 | männlich | 9 | Eingriff Herzkranzgefäße | | Atherosklerotische Herzkrankheit: Ohne hämodymisch wirksame Stenosen | ja | 2 |
| 8052 | 71 | männlich | 11 | Eingriff Herzkranzgefäße | | Atherosklerotische Herzkrankheit: Ohne hämodymisch wirksame Stenosen | ja | 2 |
| 8056 | 70 | weiblich | 13 | Eingriff Herzklappen | | Mitralklappeninsuffizienz | ja | 5 |
| 8058 | 63 | weiblich | 21 | Eingriff Herzklappen | | Sonstige Aortenklappen-krankheiten | ja | 5 |
| 8073 | 82 | männlich | 15 | Eingriff Herzkranzgefäße | | Atherosklerotische Herzkrankheit: Ohne hämodymisch wirksame Stenosen | ja | 2 |
| 8086 | 78 | männlich | 13 | Eingriff Herzkranzgefäße | | Atherosklerotische Herzkrankheit: Ein-Gefäßerkrankung | ja | 6 |
| 8096 | 61 | männlich | 11 | Eingriff Herzkranzgefäße, Eingriff Herzklappen | | Mitralklappenstenose | nein | 12 |
| 8101 | 63 | männlich | 12 | Eingriff Herzkranzgefäße | | Atherosklerotische Herzkrankheit: Ohne hämodymisch wirksame Stenosen | nein | 8 |

(fortgesetzt)

| Patient | Alter [Jahre ] | Geschlecht | Apache | postoperative Indikationen | nicht operative Indikationen | Aufhmediagnose | Überlebens -status (ITS) | Liegedauer [Tage] |
|---|---|---|---|---|---|---|---|---|
| 8102 | 70 | männlich | 17 | Eingriff Herzkranzgefäße | | Atherosklerotische Herzkrankheit: Ein-Gefäßerkrankung | ja | 6 |
| 8103 | 54 | männlich | 6 | Eingriff Herzklappen | | Aortenklappenstenose | ja | 2 |
| 8108 | 66 | männlich | 11 | Eingriff Herzkranzgefäße | | Atherosklerotische Herzkrankheit: Ohne hämodymisch wirksame Stenosen | ja | 2 |
| 8111 | 65 | männlich | 16 | Eingriff Herzkranzgefäße | | Atherosklerotische Herzkrankheit: Ohne hämodymisch wirksame Stenosen | ja | 14 |
| 8112 | 76 | männlich | 13 | Eingriff Herzkranzgefäße | | Atherosklerotische Herzkrankheit: Ohne hämodymisch wirksame Stenosen | nein | 10 |
| 8116 | 80 | weiblich | 18 | Eingriff Herzkranzgefäße | | Atherosklerotische Herzkrankheit: Ein-Gefäßerkrankung | ja | 5 |
| 8122 | 67 | männlich | 17 | Eingriff Herzkranzgefäße | | Instabile Angi pectoris | ja | 5 |
| 920 | 74 | männlich | 23 | | schwere Sepsis | Sepsis, nicht näher bezeichnet | ja | 4 |

Tabelle 5: allgemeine Beschreibung der Patienten aus dem Testdatensatz, aufgezeichnet wurden allgemeinen klinischen Parametern, durch die die ITS-Behandlung begründet wird.

| Patient | IST-Tag | PCT [ng/ml] | CRP [mg/l] | SOFA-SCORE | Schwere der Erkrankung | Anz. ODF | Leukozyten-zahl | Gruppe | Anz. SIRS-Krit. | Noradrelin-dosis | CDCS | Wahrschein-lichkeit CDCS | Antibiotika |
|---|---|---|---|---|---|---|---|---|---|---|---|---|---|
| 1013 | 1 | 3.9 | | 8 | schwere Sepsis | 1 | 23800 | S | 3 | 0.04 | oberflaechliche chirurgische Wundinfektion, Spinalabszesse | definitiv, definitiv | Gentamicin, Flucloxacillin, Clindamycin, Ceftriaxon |
| 1015 | 10 | 5.12 | | 12 | sept. Schock | 2 | 11100 | S | 3 | 1.3 | Pneumonie | definitiv | Meropenem, Linezolid |
| 2042 | 2 | 10.8 | 97.6 | 9 | SIRS | 2 | 18600 | C | 3 | 0.26 | | | |
| 5008 | 6 | 10 | 200 | 11 | schwere Sepsis | 2 | 17500 | S | 2 | 0.2 | Pneumonie | wahrscheinlich | Oxacillin, Tiem, Klion, Diflucan |
| 5009 | 3 | 2 | 250 | 8 | schwere Sepsis | 1 | 7900 | S | 2 | 0.05 | Pneumonie, Gastroenteritis | wahrscheinlich, wahrscheinlich | Ampicillin, Ciprofloxacin, Unbekannt: fortum, Colimycin, unbekannt: V-fend, Herpesin |
| 5010 | 2 | 0 | 164 | 8 | sept. Schock | 3 | 11600 | S | 2 | 0.06 | Tracheobronchitis, Infektion des Gastrintestiltraktes, Intraabdominelle Infektion | unwahrscheinlich, definitiv, definitiv | Cefuroxim, Metronidazol, Tiem, Vancomycin, Sulperazon, Amikacin, Flucozol |
| 5018 | 2 | | 280 | | sept. Schock | 3 | 17000 | S | 2 | 0.3 | Endokarditis | wahrscheinlich | Amoxicillin, Gentamicin |
| 5019 | 1 | | | 0 | SIRS | | | C | | 0.3 | | | |
| 5020 | 1 | | | | SIRS | | | C | | 0.3 | | | |
| 5023 | 1 | | | | SIRS | | | C | | 0.3 | | | |

| | | | | | | | | | | | | | |
|---|---|---|---|---|---|---|---|---|---|---|---|---|---|
| 6005 | 2 | | 290.1 | 8 | schwere Sepsis | 2 | 6900 | S | 3 | 0.3 | Cholezystitis | definitiv | |
| 6008 | 2 | 6 | 111 | 7 | sept. Schock | 3 | 16800 | S | 3 | 0.56 | Intraabdominelle Infektion | definitiv | Cefuroxim, Metronidazol |
| 6024 | 2 | 2.46 | 49.8 | 10 | schwere Sepsis | 3 | 10400 | S | 2 | 0.03 | Meningitis / Ventrikulitis, Kathetersepsis | definitiv, wahrscheinlic h | Meropenem, Ceftriaxon |
| 6035 | 3 | 4.72 | 103 | 13 | sept. Schock | 3 | 16100 | S | 4 | 0.11 | Intraabdominelle Infektion | definitiv | Cefuroxim, Metronidazol |
| 6036 | 10 | 0.65 | | 8 | sept. Schock | 1 | 19200 | S | 2 | 0.16 | Pneumonie | wahrscheinlic h | Ciprofloxacin |
| 6056 | 14 | 2.32 | 94.2 | 10 | sept. Schock | 3 | 19300 | S | 4 | 0.31 | Pneumonie | wahrscheinlic h | Ciprofloxacin, Imipenem |
| 6061 | 10 | 0.52 | 78.8 | 8 | sept. Schock | 2 | 19900 | S | 4 | 0.21 | Pneumonie,Kathete r | wahrscheinlic h, wahrscheinlic h | Flucozol, Imipenem |
| 6063 | 2 | 4.07 | 236 | 13 | sept. Schock | 2 | 14200 | S | 4 | 0.17 | tiefe chirurgische Wundinfektion, Pneumonie, Tracheobronchitis | wahrscheinlic h, wahrscheinlic h, wahrscheinlic h | Ceftriaxon, Clindamycin |
| 6064 | 8 | 0.54 | 218 | 7 | sept. Schock | 3 | 17400 | S | 4 | 0.28 | tiefe chirurgische Wundinfektion, Intraabdominelle Infektion,Pankreatiti s | wahrscheinlic h, wahrscheinlic h, wahrscheinlic h | Levofloxacin |
| 6070 | 3 | 2.31 | 404 | 7 | schwere Sepsis | 2 | 10600 | S | 1 | 0.093 | Pneumonie, Tracheobronchitis | wahrscheinlic h, wahrscheinlic h | Piperacillin / Tazobactam, Vancomycin |
| 6075 | 3 | 37.6 | 269 | 9 | sept. Schock | 3 | 37900 | S | 3 | 0.43 | Intraabdominelle Infektion | wahrscheinlic h | Piperacillin / Tazobactam |
| 6104 | 7 | 32.9 | 325 | 6 | Sepsis | 1 | 11800 | S | 3 | | Pneumonie | wahrscheinlic h | Imipenem, Vancomycin |

| | | | | | | | | | | | | | |
|---|---|---|---|---|---|---|---|---|---|---|---|---|---|
| 6120 | 3 | 36.8 | 478 | 12 | sept. Schock | 3 | 11600 | S | 2 | 0.22 | Intraabdominelle Infektion | wahrscheinlich | Cefuroxim |
| 6124 | 5 | 0.3 | 159 | 9 | sept. Schock | 3 | 9100 | S | 2 | 0.22 | Pneumonie | definitiv | |
| 6126 | 10 | 86.2 | 80.4 | 10 | sept. Schock | 4 | 13700 | S | 4 | 0.22 | oberflaechliche chirurgische Wundinfektion | definitiv | |
| 6141 | 5 | 1.18 | 247 | 7 | sept. Schock | 3 | 13800 | S | 4 | 0.17 | Pneumonie | definitiv | Piperacillin / Tazobactam |
| 7023 | 9 | 0.3 | 124 | 10 | sept. Schock | 2 | 14200 | S | 4 | 0.13 | Pneumonie, Bakteriaemie | wahrscheinlich, unwahrscheinlich | Piperacillin / Tazobactam |
| 7040 | 2 | 13.5 | 304 | 11 | sept. Schock | 2 | 28400 | S | 3 | 0.36 | tiefe chirurgische Wundinfektion, Pneumonie, haematogen | definitiv, wahrscheinlich, wahrscheinlich | Meropenem, Vancomycin |
| 7052 | 12 | 0.45 | 229 | 9 | SIRS | 2 | 12400 | C | 2 | 0.082 | | | Levofloxacin |
| | 13 | 0.37 | 230 | 10 | SIRS | 2 | 14200 | C | 2 | 0.1 | | | Levofloxacin |
| | 14 | 0.47 | 234 | 8 | keine | 2 | 11500 | C | 1 | 0.082 | | | Levofloxacin |
| 7077 | 9 | 0.65 | 335 | 10 | SIRS | 2 | 13700 | C | 3 | 0.27 | | | Amoxicillin / Clavulansäure, Gentamicin |
| | 10 | 0.74 | 415 | 9 | sept. Schock | 2 | 16400 | S | 3 | 0.25 | Weichteilinfektion | definitiv | Amoxicillin / Clavulansäure, Gentamicin, Imipenem |
| | 11 | 0.66 | 378 | 10 | Sepsis | 2 | 123000 | S | 4 | 0.2 | Weichteilinfektion | definitiv | Gentamicin, Imipenem |

| | | | | | | | | | | | | | | |
|---|---|---|---|---|---|---|---|---|---|---|---|---|---|---|
| 7079 | 12 | 5.62 | 233 | 11 | sept. Schock | 3 | 37000 | S | 3 | 0.92 | Mediastinitis | definitiv | Levofloxacin, Vancomycin |
| 7084 | 2 | 6.11 | 135 | 7 | schwere SIRS | 1 | 13100 | C | 3 | 0.09 | | | Cefazolin |
| | 3 | 7.95 | 355 | 6 | SIRS | 1 | 14400 | C | 3 | 0.09 | | | Cefazolin |
| | 4 | 6.41 | 379 | 8 | sept. Schock | 1 | 10900 | S | 3 | 0.22 | Pneumonie | wahrscheinlich | Cefazolin, Piperacillin / Tazobactam |
| | 5 | 11.4 | 449 | 10 | sept. Schock | 2 | 10100 | S | 3 | 0.37 | Pneumonie | wahrscheinlich | Piperacillin / Tazobactam |
| 7096 | 2 | 1.24 | 134 | 7 | schwere SIRS | 2 | 12400 | C | 2 | 0.17 | | | |
| | 3 | 1.27 | 200 | 8 | schwere SIRS | 1 | 12700 | C | 3 | 0.062 | | | |
| | 4 | 0.62 | 164 | 6 | SIRS | 0 | 9600 | C | 2 | | | | |
| | 5 | 0.5 | 120 | 8 | schwere SIRS | 1 | 15700 | C | 3 | | | | |
| | 6 | 0.9 | 151 | 8 | schwere SIRS | 1 | 14800 | C | 3 | | | | Piperacillin / Tazobactam |
| | 7 | 0.96 | 177 | 7 | Sepsis | 0 | 15400 | S | 2 | | Pneumonie | wahrscheinlich | Piperacillin / Tazobactam |
| | 8 | 1.1 | 215 | 7 | Sepsis | 0 | 20800 | S | 2 | | Pneumonie | wahrscheinlich | Piperacillin / Tazobactam |

| | | | | | | | | | | | | | |
|---|---|---|---|---|---|---|---|---|---|---|---|---|---|
| 7105 | 1 | 3.29 | 311 | 12 | sept. Schock | 3 | 14700 | S | 3 | 0.25 | Myokarditis /Perikarditis | wahrscheinlich | Imipenem |
| 7112 | 23 | 0.9 | 56.7 | 9 | schwere Sepsis | 2 | 13200 | S | 4 | | Pneumonie | wahrscheinlich | Cefepim, Flucozol, Levofloxacin |
| 7119 | 3 | 1.31 | 288 | 7 | schwere SIRS | 2 | 19200 | C | 2 | 0.16 | | | Piperacillin / Tazobactam |
| | 4 | 0.61 | 295 | 5 | keine | 1 | 11900 | C | 1 | 0.01 | | | |
| | 5 | | 228 | 4 | SIRS | 0 | 9000 | C | 2 | | | | |
| 7120 | 2 | | 153 | 6 | sept. Schock | 2 | 13000 | S | 2 | 0.73 | Intraabdominelle Infektion | wahrscheinlich | Cefuroxim, Metronidazol |
| 714 | 1 | 0.89 | 111 | 9 | sept. Schock | 3 | 17000 | S | 4 | 0.87 | Intraabdominelle Infektion | definitiv | Metronidazol, Cefuroxim |
| 749 | 8 | 2.88 | 173 | 8 | Sepsis | 0 | 16000 | S | 3 | | Tracheobronchitis | wahrscheinlich | Piperacillin / Tazobactam |
| 8009 | 2 | 7.25 | 34.8 | 8 | SIRS | 2 | 10100 | C | 4 | 0.17 | | | |
| | 3 | 5.38 | 206 | 6 | SIRS | 1 | 5800 | C | 4 | 0.22 | | | Piperacillin / Tazobactam |
| | 4 | 4.4 | 256 | 8 | SIRS | 2 | 16600 | C | 4 | 0.23 | | | Piperacillin / Tazobactam |
| | 5 | 7.67 | 288 | 10 | SIRS | 4 | 18900 | C | 4 | 0.16 | | | Meropenem, Piperacillin / Tazobactam |

| | | | | | | | | | | | | | |
|---|---|---|---|---|---|---|---|---|---|---|---|---|---|
| | 6 | 6.56 | 136 | 10 | SIRS | 2 | 15800 | C | 4 | 0.2 | | | Meropenem |
| | 7 | 3.97 | 162 | 9 | SIRS | 4 | 23700 | C | 4 | 0.11 | | | Meropenem |
| | 8 | 2.47 | 207 | 11 | SIRS | 4 | 26200 | C | 4 | 0.39 | | | Meropenem |
| | 11 | 9.84 | 207 | 11 | sept. Schock | 4 | 28300 | S | 3 | 0.02 | Pneumonie, Intraabdominelle Infektion | definitiv, definitiv | Meropenem |
| 8011 | 2 | 0.3 | 82.9 | 5 | SIRS | 0 | 11400 | C | 2 | | | | |
| 8026 | 2 | 3.28 | 83.8 | 4 | SIRS | 2 | 8900 | C | 3 | | | | Cefazolin |
| | 3 | 3.01 | 205 | 7 | SIRS | 1 | 9200 | C | 3 | 0.052 | | | Cefazolin |
| | 4 | 1.55 | 70 | 5 | SIRS | 0 | 10800 | C | 3 | | | | Cefazolin |
| | 5 | 0.77 | 39.6 | 4 | SIRS | 2 | 6900 | C | 3 | | | | Cefazolin |
| | 6 | 0.35 | 23.6 | 3 | SIRS | 0 | 7200 | C | 2 | | | | Cefazolin |
| 8034 | 2 | | 42.5 | 1 | SIRS | 0 | 9000 | C | 2 | | | | |
| 8039 | 2 | 1.39 | 53.8 | 6 | SIRS | 3 | 22500 | C | 4 | 0.12 | | | Cefazolin |

| | | | | | | | | | | | | | |
|---|---|---|---|---|---|---|---|---|---|---|---|---|---|
| | 3 | 0.84 | 178 | 7 | SIRS | 2 | 19900 | C | 4 | | | | Cefazolin |
| | 4 | 0.86 | 197 | 8 | SIRS | 2 | 20800 | C | 4 | | | | Cefazolin, Piperacillin / Tazobactam |
| | 5 | 0.63 | 131 | 10 | SIRS | 2 | 17.4 | C | 3 | | | | Piperacillin / Tazobactam, Erythromycin |
| | 6 | 0.47 | 78.4 | 9 | SIRS | 2 | 14400 | C | 2 | | | | Piperacillin / Tazobactam |
| 8044 | 2 | 0.43 | 48.3 | 1 | SIRS | 0 | 10700 | C | 3 | | | | Cefazolin |
| 8052 | 2 | | 84.7 | 0 | SIRS | 0 | 8700 | C | 2 | | | | |
| 8056 | 3 | 0.82 | 128 | 5 | SIRS | 1 | 22000 | C | 2 | | | | Cefazolin |
| 8058 | 3 | 22.7 | 72.7 | 11 | SIRS | 5 | 6300 | C | 2 | | | | Cefazolin, Piperacillin / Tazobactam |
| 8073 | 2 | 0.5 | 67.5 | 4 | SIRS | 1 | 6800 | C | 2 | | | | |
| 8086 | 2 | 0.35 | 37.7 | 5 | SIRS | 3 | 12400 | C | 3 | 0.042 | | | |
| 8096 | 2 | 21.9 | 117 | 10 | SIRS | 3 | 15300 | C | 3 | 0.32 | | | Cefazolin |
| | 3 | 14.5 | 294 | 12 | SIRS | 5 | 13500 | C | 4 | 1.3 | | | Cefazolin |

| | | | | | | | | | | | | | |
|---|---|---|---|---|---|---|---|---|---|---|---|---|---|
| | 4 | 9.38 | 291 | 14 | SIRS | 5 | 13900 | C | 3 | 0.78 | | | Cefazolin |
| 8101 | 3 | 1.27 | 92.9 | 8 | SIRS | 3 | 17900 | C | 4 | 0.17 | | | Cefazolin, Piperacillin / Tazobactam |
| | 4 | 1.23 | 213 | 11 | SIRS | 5 | 15000 | C | 4 | 0.23 | | | Piperacillin / Tazobactam |
| | 5 | 1.42 | 195 | 11 | SIRS | 3 | 22600 | C | 4 | 0.46 | | | Piperacillin / Tazobactam |
| | 6 | 3.64 | 233 | 14 | sept. Schock | 5 | 39500 | S | 4 | 0.94 | | | Imipenem, Vancomycin, Piperacillin / Tazobactam |
| | 7 | 6.59 | 184 | 17 | sept. Schock | 5 | 33600 | S | 4 | 1 | | | Imipenem, Vancomycin |
| 8102 | 2 | 1.83 | 35.6 | 5 | SIRS | 3 | 13100 | C | 4 | 0.016 | | | |
| 8103 | 2 | 0.52 | 55.8 | 1 | SIRS | 0 | 6900 | C | 2 | | | | Cefazolin |
| 8108 | 2 | 0.3 | 73.7 | 3 | SIRS | 0 | 7300 | C | 2 | | | | |
| 8111 | 2 | 6.82 | 67 | 7 | SIRS | 3 | 19700 | C | 4 | | | | Cefazolin |
| | 4 | 3.82 | 182 | | SIRS | 3 | 13800 | C | 2 | 0.4 | Pneumonie | wahrscheinlich | Ciprofloxacin |
| | 5 | 2.24 | 133 | 8 | SIRS | 3 | 14300 | C | 4 | 0.12 | | | Ciprofloxacin |

| | | | | | | | | | | | | | |
|---|---|---|---|---|---|---|---|---|---|---|---|---|---|
| | 6 | 0.82 | 67 | 5 | SIRS | 2 | 8700 | C | 3 | | | | Ciprofloxacin |
| | 7 | 0.35 | 128 | 3 | schwere Sepsis | 1 | 10400 | S | 2 | | Tracheobronchitis | wahrscheinlich | Ciprofloxacin |
| | 8 | 0.3 | 98.2 | 5 | schwere Sepsis | 3 | 19800 | S | 4 | 0.089 | Tracheobronchitis | wahrscheinlich | Ciprofloxacin, Piperacillin / Tazobactam |
| 8112 | 2 | 2.55 | | | SIRS | 3 | 14100 | C | 4 | 0.066 | | | Cefazolin |
| | 3 | 1.49 | 168 | 9 | SIRS | 3 | 17400 | C | 4 | 0.11 | | | Cefazolin, Piperacillin / Tazobactam |
| | 4 | 1.06 | 175 | 10 | SIRS | 4 | 13000 | C | 4 | 0.2 | | | Piperacillin / Tazobactam |
| | 5 | 0.88 | 151 | 14 | SIRS | 2 | 12500 | C | 4 | 0.077 | | | Piperacillin / Tazobactam |
| | 6 | 0.64 | 128 | 12 | schwere Sepsis | 3 | 13300 | S | 4 | 0.09 | Pneumonie | wahrscheinlich | Piperacillin / Tazobactam |
| | 7 | 0.44 | 113 | 12 | sept. Schock | 3 | 9000 | S | 3 | 0.11 | Pneumonie | wahrscheinlich | |
| 8116 | 2 | | 81.1 | 10 | SIRS | 5 | 15100 | C | 3 | | | | |
| 8122 | 4 | 0.3 | 171 | 4 | SIRS | 3 | 11200 | C | 2 | | | | |
| 920 | 2 | 1.26 | 124 | 10 | schwere Sepsis | 5 | 10600 | S | 2 | 0.031 | tiefe chirurgische Wundinfektion | definitiv | Meropenem |

Tabelle 5 zeigt Sepsis-relevante klinische Parameter aus den Verlaufsbögen der Patienten aus dem Testdatensatz, aufgezeichnet wurden klinische Parameter, durch die der Krankheitsverlauf dokumentiert wird.

## Ausführungsbeispiele

### Beispiel 1: Aufstellung eines Klassifikators zur Identifizierung von SIRS- und Sepsis-Patienten mit hoher Sensitivität/Spezifität

Patienten-Gruppen

Im ersten Schritt der Analyse (Training) wurden Proben von Patienten einer IntensivStation (ITS) eingeschlossen. Für die Sepsis-Gruppe wurden Patienten mit einem mikrobiologisch bestätigten Infektionsfokus ausgesucht, wobei die Probe vom ersten Tag der Sepsis berücksichtigt wurde. In die Kontroll-Gruppe wurden Patienten nach einer schweren Herz-OP ausgewählt (Kardiopulmonaler Bypass, CPB), die postoperativ eine sterile SIRS entwickelt haben. Die Kontrollgruppe wurde an die Sepsis-Gruppe bzgl. Patientenzahl, Alter, Geschlechtsaufteilung und Erkrankungsschwere angepasst, so dass der wesentliche Unterschied zwischen den Gruppen im Vorhanden einer infektiösen Komplikation lag. Jeder Patient der Kontrollgruppe wurde mit je einer Probe vertreten. Der Trainingsdatensatz bestand aus 29 Sepsis- und 29 Kontrollfällen. Die wichtigsten klinischen Parameter wurden in der Tabelle 6 zusammengefasst.

Im zweiten Schritt (Validierung) wurde ein Testdatensatz untersucht, der aus 113 Proben von 65 Personen bestand (vgl. Tabellen 4 und 5). Dabei wurden Proben von weiteren Sepsis-Patienten untersucht, die ein breites Spektrum an klinischen Phänotypen mit dem Risiko eine generalisierte Infektion ausbilden, repräsentieren. Darüber hinaus wurden Proben über den Krankheitsverlauf von SIRS zu Sepsis ausgesucht. In die Analyse wurden höchstens Proben von den ersten 2 Tagen der Sepsis-Diagnose eingeschlossen.

Als Kontrolle dienten weitere Proben der SIRS-Patienten aus der Trainingsgruppe, technische Wiederholungen, Proben weiterer postoperativen Fälle und 5 gesunde Kontrollen. Auch mit dieser Auswahl der Kontrollen wird die Anwendbarkeit des Verfahrens in einem breit angelegten Phänotyp sichergestellt.

Messung der Genexpression

Aus dem Blut der Patienten wurde Total-RNA isoliert und in cDNA umgeschrieben. Diese wurde im Assay als Templat eingesetzt. Die Markerkandidaten zur Klassifizierung wurden in den Tabellen 2 und 3 zusammengefasst. Ans Ende der Tabelle wurden 3 sogenannte Referenzgene (auch Housekeepinggene) eingefügt (R1, R2 und R3). Sie ermöglichen eine relative Quantifizierung von Genexpression, die eine Aussage zur Abundanz des Targettranskriptes bezogen auf einen Kalibrator. Für jeden Organismus und jedes Gewebe sind solche Referenzgene spezifisch und müssen für die jeweilige Anwendung, hier die Unterscheidung einer infektiöser von einer nicht-infektiöser Ursache einer systemischen Entzündungreaktion anhand von humanen Vollblut-Proben, sorgfältig ausgewählt werden. Ausgehend von den Genexpressionsprofilen aus dem Vollblut der Sepsis- und Kontrollpatienten wurden die stabilsten Gene mit der geringsten Variabilität ausgewählt und in der quantitativen PCR zur Normalisierung eingesetzt.

Experimentelle Ausführung

Blutabnahme und RNA-Isolation:

Das Vollblut der Patienten wurde auf der Intensivstation von den Patienten in PAXgene tubes abgenommen (PreAnalytix, Hombrechtikon, CH) und nach den Herstellervorgaben bis zur Aufarbeitung gelagert. Mit PAXGene Blood-RNA-Kits wurde gemäß den Vorgaben des Herstellers (Qiagen, Hilden, BRD) die RNA isoliert und bis zur Analyse bei -80°C gelagert.

Reverse Transkription:

Von jeder Patienten-Probe wurden 0,5 μg der Total-RNA zu komplementärer DNA (cDNA) mit der reversen Transkriptase Superscript II (Invitrogen Deutschland, Karlsruhe, BRD) in einem 20 μl-Ansatz (enthält 1 μl 10 mM dNTP-Mix von Fermentas und 1 μl 0,5 μg/μl Oligo(dT)-Primer) umgeschrieben. Die RNA wurde anschließend durch alkalische Hydrolyse aus dem Ansatz entfernt. Die Reaktionsansätze wurden nicht aufgereinigt, sondern mit Wasser auf 50 μl aufgefüllt.

Real-Time-PCR

Verwendet wurde der Platinum SYBR Green qPCR SuperMix-UDG - Kit der Firma Invitrogen (Invitrogen Deutschland, Karlsruhe, BRD). Die Patienten-cDNA wurde 1:25 mit Wasser verdünnt, davon wurde jeweils 1 µl in die PCR eingesetzt. Die Proben wurden jeweils in 3 Replikaten pipettiert.

| | |
|---|---|
| PCR-Ansatz pro well (10 µl) | 2µl Templat-cDNA 1:100 |
| | 1 µl forward primer, 10 mM |
| | 1 µl reverse primer, 10 mM |
| | 1 µl Fluorescein Reference Dye |
| | 5 µl Platinum SYBR Green qPCR SuperMix-UDG |

Es wurde ein Mastermix ohne Templat hergestellt, dieser wurde in 9 pl-Aliquots in die PCR-Platte aliquotiert, dazu wurden jeweils die Patienten-cDNAs pipettiert.

Das sich daran anschließende PCR-Programm bestand aus den folgenden Schritten::

| | | |
|---|---|---|
| 95 °C | 2 min (Aktivierung der Polymerase) | |
| 95 °C | 10 sec (Denaturierung) | 40 x |
| 58 °C | 15 sec (Anlagerung) | |
| 72 °C | 20 sec (Extension) | |
| 55 °C- 95 °C | 10 sec (Erstellung der Schmelzkurve, Erhöhung der Anfangstemperatur nach jedem Schritt um 1 °C) | 41 x |

Verwendet wurde das iQ™5 Multicolor Real-Time-PCR Detection System der Firma BIORAD mit der dazugehörigen Auswertungssoftware. Die so genannten Ct-Werte (Anzahl der Zyklen) wurden als Messergebnis vom Programm automatisch im Bereich des linearen Anstiegs der Kurven berechnet. Die Messwerte wurden im String-Format abgespeichert.

<u>Datenanalyse:</u>

Die Datenanalyse wurde unter der freien Software R Project Version R 2.8.0 (R.app GUI 1.26 (5256), S.Urbanek & S.M.lacus, © R Foundation for Statistical Computing, 2008) durchgeführt, die unter www.r-project.org zur Verfügung steht.

<u>Daten-Vorverarbeitung:</u>

Die in die Analyse eingegangenen Datenmatrizen der gemessenen Ct-Werte wurden in den Tabellen 6 und 7 für jeweils den Training- und Testdatensatz dargelegt. Zum Normalisieren wurde für jede Probe der Mittelwert der 3 ausgewählten Housekeeper-Gene (R1, R2 und R3) berechnet. Von diesem Wert wurde der Ct-Wert jedes einzelnen Markers abgezogen. Jeder so gewonnene Delta Ct Wert spiegelt die relative Abundanz des Targettranskriptes bezogen auf den Kalibrator wider, wobei ein positiver Delta Ct Wert eine Abundanz höher als der Mittelwert der Referenzen und negativer Delta Ct Wert eine Abundanz kleiner als der Mittelwert der Referenzen bedeuten.

Tabelle 6: Ct-Werte des Trainingsdatensatzes pro Marker (Mittelwert der Dreifachbestimmung) und die Gruppenzugehörigkeit (letzte Spalte)

| | M2 | M3 | M4 | M5 | M6 | M8 | M9 | M10 | M12 | M13 | M15 | M16 | M17 | R1 | R2 | R3 | Gruppe |
|---|---|---|---|---|---|---|---|---|---|---|---|---|---|---|---|---|---|
| 2038_001 | 27.9 | 28.2 | 26.9 | 24.7 | 26.1 | 27.2 | 25.6 | 24.0 | 27.5 | 32.3 | 27.6 | 25.0 | 29.9 | 26.4 | 25.3 | 31.4 | keine Sepsis |
| 8001_001 | 26.1 | 28.2 | 25.9 | 22.9 | 24.8 | 26.7 | 24.0 | 24.1 | 25.2 | 31.5 | 25.6 | 24.1 | 27.2 | 25.2 | 23.1 | 30.0 | keine Sepsis |
| 8002_001 | 27.0 | 27.5 | 25.6 | 22.4 | 25.6 | 26.6 | 24.8 | 22.8 | 24.7 | 30.3 | 26.6 | 23.8 | 27.7 | 26.0 | 23.7 | 31.3 | keine Sepsis |
| 8009_001 | 28.3 | 28.4 | 28.1 | 26.3 | 27.9 | 27.4 | 25.7 | 25.1 | 25.9 | 32.2 | 28.2 | 27.3 | 29.4 | 27.6 | 27.2 | 32.5 | keine Sepsis |
| 8010_001 | 27.2 | 28.2 | 26.4 | 24.8 | 26.6 | 27.2 | 25.5 | 24.5 | 26.2 | 32.1 | 27.4 | 25.2 | 28.8 | 27.0 | 25.3 | 31.6 | keine Sepsis |
| 8011_001 | 25.5 | 26.8 | 26.1 | 23.8 | 25.3 | 27.3 | 24.4 | 24.4 | 25.4 | 31.1 | 27.0 | 25.2 | 28.1 | 26.0 | 25.0 | 30.1 | keine Sepsis |
| 8012_001 | 28.3 | 29.6 | 28.1 | 25.2 | 27.3 | 28.3 | 26.1 | 25.9 | 26.7 | 34.2 | 28.2 | 26.2 | 29.1 | 27.7 | 26.3 | 32.5 | keine Sepsis |
| 8025_002 | 26.8 | 28.8 | 25.9 | 24.8 | 25.4 | 26.7 | 25.8 | 24.6 | 24.9 | 32.4 | 27.1 | 24.9 | 29.0 | 26.3 | 25.3 | 29.9 | keine Sepsis |
| 8030_001 | 26.7 | 29.1 | 26.9 | 24.7 | 27.3 | 27.5 | 25.9 | 23.6 | 25.3 | 32.1 | 28.0 | 25.7 | 28.7 | 26.6 | 25.7 | 31.7 | keine Sepsis |
| 8032_003 | 26.9 | 27.9 | 28.3 | 26.7 | 26.6 | 27.4 | 26.2 | 24.4 | 25.9 | 32.5 | 28.7 | 25.6 | 28.4 | 26.3 | 26.3 | 32.6 | keine Sepsis |
| 8034_001 | 25.9 | 27.2 | 26.0 | 24.4 | 25.9 | 27.0 | 25.6 | 24.9 | 26.3 | 31.9 | 26.9 | 24.9 | 28.6 | 25.3 | 25.2 | 32.1 | keine Sepsis |
| 8044_001 | 26.3 | 27.2 | 25.4 | 24.4 | 25.2 | 26.3 | 24.5 | 24.7 | 25.8 | 30.7 | 25.8 | 24.7 | 26.8 | 25.7 | 25.2 | 30.5 | keine Sepsis |
| 8051_002 | 26.8 | 28.1 | 26.6 | 24.4 | 25.1 | 26.8 | 25.1 | 24.3 | 25.4 | 31.9 | 26.9 | 25.1 | 28.3 | 25.9 | 24.8 | 31.9 | keine Sepsis |
| 8052_001 | 27.4 | 28.7 | 27.8 | 24.8 | 25.9 | 26.6 | 25.2 | 24.7 | 26.4 | 31.7 | 27.7 | 25.9 | 28.9 | 26.5 | 25.9 | 33.2 | keine Sepsis |
| 8056_003 | 27.2 | 27.9 | 26.6 | 24.2 | 27.2 | 27.3 | 24.4 | 25.6 | 25.7 | 25.3 | 31.9 | 26.0 | 28.6 | 29.6 | 33.6 | 25.8 | keine Sepsis |
| 8058_001 | 27.5 | 29.1 | 27.5 | 26.0 | 27.3 | 28.8 | 26.3 | 24.9 | 27.0 | 32.2 | 27.7 | 25.2 | 29.5 | 27.2 | 26.2 | 32.2 | keine Sepsis |
| 8068_001 | 27.8 | 28.8 | 26.5 | 24.9 | 26.8 | 27.5 | 25.7 | 24.9 | 26.4 | 33.8 | 26.9 | 25.5 | 28.9 | 26.8 | 25.8 | 32.1 | keine Sepsis |
| 8073_001 | 26.8 | 27.9 | 27.3 | 24.8 | 26.0 | 27.4 | 25.0 | 23.3 | 25.6 | 33.2 | 26.9 | 25.2 | 29.1 | 26.3 | 24.9 | 31.5 | keine Sepsis |
| 8076_002 | 27.5 | 29.4 | 27.5 | 26.3 | 26.4 | 27.4 | 26.7 | 25.8 | 26.2 | 32.1 | 28.3 | 25.9 | 29.1 | 27.3 | 27.0 | 32.0 | keine Sepsis |
| 8084_003 | 28.7 | 29.4 | 27.1 | 25.9 | 26.3 | 27.4 | 25.6 | 24.2 | 25.7 | 33.5 | 28.1 | 26.7 | 28.4 | 26.3 | 26.1 | 32.4 | keine Sepsis |
| 8094_001 | 25.9 | 26.6 | 25.8 | 23.8 | 25.5 | 26.2 | 24.2 | 23.6 | 25.6 | 29.9 | 26.3 | 24.0 | 28.2 | 25.5 | 24.1 | 31.4 | keine Sepsis |
| 8096_001 | 27.5 | 28.5 | 25.7 | 24.1 | 26.8 | 26.9 | 25.8 | 24.3 | 25.8 | 33.1 | 26.7 | 25.5 | 28.2 | 26.4 | 25.4 | 31.5 | keine Sepsis |
| 8103_001 | 25.8 | 27.4 | 26.2 | 25.2 | 24.6 | 25.7 | 24.3 | 22.6 | 24.9 | 31.6 | 26.7 | 24.9 | 28.4 | 25.4 | 24.9 | 30.5 | keine Sepsis |
| 8108_001 | 26.1 | 27.6 | 26.0 | 25.0 | 25.9 | 27.0 | 24.9 | 24.3 | 26.2 | 32.3 | 27.2 | 24.9 | 29.0 | 26.3 | 25.4 | 32.3 | keine Sepsis |
| 8111_002 | 26.2 | 26.9 | 26.6 | 24.9 | 25.0 | 26.4 | 24.4 | 23.7 | 25.1 | 31.8 | 27.0 | 24.4 | 27.9 | 25.3 | 24.6 | 30.3 | keine Sepsis |
| 8112_002 | 27.4 | 28.3 | 25.6 | 24.3 | 25.8 | 25.7 | 25.3 | 24.7 | 25.0 | 32.2 | 26.3 | 24.0 | 28.9 | 26.0 | 24.5 | 31.4 | keine Sepsis |
| 8116_003 | 29.4 | 29.0 | 27.9 | 25.8 | 28.2 | 30.0 | 25.7 | 26.7 | 27.4 | 27.8 | 33.1 | 27.5 | 30.8 | 32.0 | 37.3 | 26.9 | keine Sepsis |
| 8122_001 | 28.1 | 29.4 | 27.1 | 24.0 | 26.5 | 27.1 | 24.9 | 24.9 | 26.6 | 33.4 | 27.2 | 25.7 | 28.2 | 26.4 | 25.3 | 32.0 | keine Sepsis |
| 814_001 | 26.5 | 27.3 | 26.0 | 25.8 | 25.9 | 24.5 | 24.4 | 23.4 | 23.1 | 31.0 | 25.8 | 25.7 | 27.5 | 25.2 | 24.5 | 30.0 | keine Sepsis |
| 1014_002 | 27.8 | 29.1 | 25.6 | 24.5 | 28.4 | 26.2 | 26.8 | 25.4 | 24.8 | 32.0 | 27.7 | 25.0 | 29.0 | 26.3 | 25.9 | 31.4 | Sepsis |
| 1020_001 | 29.2 | 28.6 | 23.9 | 22.8 | 28.7 | 28.2 | 26.0 | 26.3 | 27.6 | 31.7 | 28.0 | 23.8 | 28.5 | 26.2 | 23.7 | 30.1 | Sepsis |
| 1021_001 | 27.0 | 26.3 | 26.3 | 23.6 | 28.6 | 25.9 | 24.8 | 26.1 | 25.8 | 31.0 | 30.8 | 26.0 | 28.8 | 27.8 | 31.2 | 23.7 | Sepsis |
| 6008_001 | 27.7 | 28.6 | 25.3 | 23.0 | 26.5 | 27.3 | 25.3 | 23.6 | 25.1 | 32.0 | 27.2 | 24.6 | 29.2 | 26.2 | 24.5 | 30.9 | Sepsis |

(fortgesetzt)

| | M2 | M3 | M4 | M5 | M6 | M8 | M9 | M10 | M12 | M13 | M15 | M16 | M17 | R1 | R2 | R3 | Gruppe |
|---|---|---|---|---|---|---|---|---|---|---|---|---|---|---|---|---|---|
| 6009_001 | 28.9 | 29.8 | 25.0 | 23.6 | 28.6 | 27.3 | 26.6 | 27.3 | 25.2 | 31.9 | 27.4 | 24.1 | 28.9 | 25.5 | 24.6 | 30.7 | Sepsis |
| 6025_001 | 28.9 | 27.6 | 24.7 | 23.3 | 27.5 | 26.7 | 26.4 | 26.7 | 23.5 | 26.7 | 26.3 | 24.5 | 26.5 | 28.3 | 26.2 | 28.7 | Sepsis |
| 6032_001 | 28.5 | 30.4 | 27.0 | 23.7 | 27.9 | 29.0 | 26.6 | 25.2 | 25.3 | 34.6 | 28.0 | 25.3 | 29.0 | 27.4 | 25.2 | 32.1 | Sepsis |
| 6035_001 | 27.0 | 28.2 | 24.5 | 24.1 | 25.7 | 26.4 | 26.0 | 25.5 | 26.6 | 32.1 | 26.9 | 24.2 | 28.0 | 25.8 | 24.8 | 31.0 | Sepsis |
| 6040_001 | 27.4 | 28.1 | 23.6 | 21.7 | 28.4 | 26.1 | 27.1 | 23.8 | 24.4 | 29.6 | 26.0 | 25.3 | 26.5 | 25.5 | 25.3 | 30.1 | Sepsis |
| 6046_001 | 28.6 | 30.0 | 26.1 | 24.4 | 28.7 | 27.5 | 27.7 | 25.6 | 25.4 | 32.5 | 28.5 | 25.8 | 28.5 | 26.6 | 26.2 | 31.6 | Sepsis |
| 6048_001 | 29.6 | 30.7 | 27.0 | 26.7 | 29.7 | 29.4 | 28.2 | 26.9 | 26.9 | 34.5 | 28.9 | 26.3 | 29.8 | 26.8 | 27.4 | 32.7 | Sepsis |
| 6062_001 | 29.6 | 31.7 | 25.9 | 23.9 | 29.6 | 27.8 | 27.4 | 26.7 | 27.4 | 33.6 | 28.6 | 24.3 | 28.6 | 26.5 | 25.6 | 31.7 | Sepsis |
| 6065_001 | 28.1 | 28.6 | 26.2 | 24.4 | 28.9 | 30.0 | 26.5 | 26.2 | 26.5 | 34.8 | 27.7 | 24.9 | 29.4 | 26.4 | 25.7 | 32.5 | Sepsis |
| 6070_001 | 28.4 | 29.9 | 26.8 | 25.2 | 27.6 | 28.0 | 26.8 | 26.9 | 26.0 | 34.9 | 28.8 | 25.9 | 29.9 | 27.4 | 26.4 | 32.3 | Sepsis |
| 6073_001 | 29.7 | 31.6 | 26.3 | 24.9 | 29.7 | 29.3 | 28.9 | 26.9 | 25.7 | 33.7 | 29.9 | 25.9 | 29.9 | 27.4 | 27.6 | 32.4 | Sepsis |
| 6075_001 | 31.6 | 33.2 | 24.0 | 23.4 | 32.5 | 27.2 | 28.5 | 26.9 | 27.3 | 34.0 | 27.3 | 24.3 | 27.2 | 26.1 | 26.5 | 32.8 | Sepsis |
| 6078_001 | 27.3 | 30.0 | 24.7 | 24.6 | 28.0 | 27.8 | 26.4 | 25.2 | 26.4 | 31.6 | 28.0 | 24.8 | 28.0 | 26.7 | 26.2 | 32.6 | Sepsis |
| 6081_001 | 30.4 | 30.7 | 27.2 | 24.2 | 30.1 | 29.3 | 29.1 | 27.8 | 27.5 | 33.6 | 30.4 | 26.9 | 29.6 | 29.4 | 28.7 | 33.9 | Sepsis |
| 6082_001 | 30.5 | 32.5 | 27.8 | 26.1 | 29.6 | 30.4 | 28.0 | 28.0 | 28.5 | 33.0 | 29.8 | 27.8 | 30.6 | 28.1 | 26.4 | 31.5 | Sepsis |
| 6084_001 | 28.2 | 27.4 | 25.2 | 24.7 | 27.3 | 28.4 | 26.0 | 26.8 | 25.9 | 27.6 | 32.1 | 25.5 | 29.2 | 29.0 | 32.0 | 24.8 | Sepsis |
| 6085_001 | 29.1 | 30.0 | 27.2 | 24.4 | 28.1 | 28.5 | 27.1 | 26.0 | 26.0 | 33.5 | 29.1 | 25.2 | 29.3 | 27.5 | 26.1 | 32.0 | Sepsis |
| 6098_001 | 28.2 | 29.1 | 26.8 | 24.9 | 25.7 | 28.5 | 26.4 | 25.0 | 25.8 | 32.9 | 27.7 | 24.8 | 29.6 | 26.5 | 24.8 | 32.2 | Sepsis |
| 6104_001 | 28.4 | 27.7 | 26.7 | 23.9 | 26.8 | 27.9 | 26.3 | 24.1 | 25.8 | 25.2 | 31.3 | 25.7 | 28.4 | 29.8 | 32.4 | 25.0 | Sepsis |
| 6110_001 | 28.7 | 31.0 | 26.6 | 24.2 | 27.9 | 27.3 | 28.0 | 26.8 | 26.8 | 33.7 | 28.9 | 26.4 | 28.9 | 27.8 | 26.6 | 33.6 | Sepsis |
| 6115_001 | 30.1 | 31.2 | 28.8 | 24.9 | 27.9 | 29.3 | 26.7 | 26.1 | 27.5 | 34.1 | 29.2 | 27.3 | 31.1 | 28.4 | 26.6 | 32.4 | Sepsis |
| 6125_001 | 28.3 | 29.2 | 26.5 | 23.4 | 26.9 | 27.8 | 25.5 | 24.7 | 26.7 | 32.4 | 28.2 | 25.3 | 29.2 | 27.0 | 25.4 | 31.3 | Sepsis |
| 829_001 | 28.7 | 31.3 | 25.5 | 24.8 | 28.5 | 27.1 | 26.1 | 26.5 | 25.5 | 31.3 | 27.5 | 24.6 | 27.6 | 24.9 | 24.2 | 30.5 | Sepsis |
| 942_001 | 30.0 | 31.9 | 27.7 | 25.2 | 27.7 | 27.7 | 25.8 | 25.4 | 25.9 | 33.8 | 28.7 | 25.8 | 28.8 | 26.8 | 24.6 | 31.7 | Sepsis |
| 987_001 | 26.7 | 28.2 | 25.8 | 22.5 | 25.5 | 26.0 | 24.9 | 24.6 | 26.0 | 31.9 | 26.7 | 24.2 | 28.6 | 26.2 | 24.2 | 31.7 | Sepsis |

Tabelle 7: Ct-Werte des Testdatensatzes pro Marker (Mittelwert der Dreifachbestimmung, fehlende Werte wurden mit NA notiert und aus der Analyse ausgeschlossen) und die Gruppenzugehörigkeit (letzte Spalte). Die ersten 5 Proben gehören zu gesunden Probanden, die anderen zu den Patienten, die in der Tabelle 4 beschrieben wurden. Die zugehörige Experiment-ID setzt sich zusammen aus der Fallnummer und der Proben-ID (eingeführt mit 2 Nullen), eine zusätzliche 1 markiert eine Wiederholung

| Experiment ID | M2 | M3 | M4 | M5 | M6 | M8 | M9 | M10 | M12 | M13 | M15 | M16 | M17 | R1 | R2 | R3 | Gruppe |
|---|---|---|---|---|---|---|---|---|---|---|---|---|---|---|---|---|---|
| 12A | 27.84 | 28.1 | 27.5 | 26.6 | 26.2 | 24.2 | 24.7 | 22.7 | 23.9 | 31.4 | 27.3 | 27.8 | 28.8 | 25.7 | 24.2 | 30.2 | keine Sepsis |
| 2A | 26.72 | 28.2 | 27.3 | 27.7 | 26.7 | 24.5 | 24.4 | 22.5 | 23.6 | 32.6 | 26.9 | 27.9 | 29.4 | 26.5 | 24.9 | 31.1 | keine Sepsis |
| 2C | 28.18 | 28.4 | 27.8 | 26.0 | 27.0 | 26.2 | 24.3 | 25.1 | 24.9 | 31.1 | 27.2 | 28.2 | 29.4 | 26.3 | 24.8 | 31.6 | keine Sepsis |
| 7A | 27.31 | 27.6 | 27.4 | 26.1 | 25.3 | 24.9 | 24.0 | 22.9 | 22.8 | 30.2 | 26.4 | 27.5 | 28.7 | 25.2 | 24.1 | 30.8 | keine Sepsis |
| 7C | 27.94 | 27.6 | 27.3 | 25.4 | 25.4 | 25.5 | 24.0 | 23.0 | 23.9 | 30.8 | 26.1 | 27.2 | 28.7 | 25.0 | 23.8 | 30.2 | keine Sepsis |
| 8011_001_1 | 23.06 | 23.8 | 23.5 | 21.3 | 22.3 | 26.9 | 21.3 | 18.7 | 22.8 | 26.6 | 23.8 | 22.5 | 25.2 | 22.6 | 21.4 | 28.0 | keine Sepsis |
| 8034_001_1 | 25.82 | 26.6 | 25.5 | 23.8 | 25.7 | 26.7 | 24.3 | 24.7 | 25.7 | 31.5 | 25.7 | 24.4 | 28.4 | 25.2 | 24.6 | 30.8 | keine Sepsis |
| 8044_001_1 | 24.31 | 24.4 | 23.5 | 22.5 | 23.9 | 25.1 | 22.0 | 22.9 | 23.8 | 28.6 | 23.2 | 22.4 | 25.5 | 23.1 | 23.0 | 29.5 | keine Sepsis |
| 8052_001_1 | 26.64 | 27.2 | 27.1 | 23.6 | 25.3 | 26.7 | 24.3 | 24.0 | 24.9 | 32.2 | 26.3 | 25.2 | 28.0 | 26.0 | 25.0 | 31.6 | keine Sepsis |
| 8073_001_1 | 25.93 | 27.1 | 26.9 | 25.3 | 25.5 | 27.4 | 25.3 | 23.8 | 24.7 | 30.5 | 27.1 | 25.6 | 28.6 | 27.1 | 25.8 | 31.4 | keine Sepsis |
| 8103_001_1 | 23.68 | 23.9 | 22.7 | 21.4 | 21.7 | 23.2 | 22.1 | 19.9 | 21.7 | 27.9 | 21.3 | 21.6 | 26.0 | 23.3 | 18.9 | 27.3 | keine Sepsis |
| 8108_001_1 | 25.79 | 25.5 | 24.0 | 21.0 | 24.4 | 26.5 | 23.4 | 22.5 | 24.1 | 30.1 | 25.6 | 23.2 | 26.1 | 24.4 | 23.4 | 31.3 | keine Sepsis |
| 5019_001 | 27.41 | 28.0 | 27.2 | 25.5 | 26.3 | 25.5 | 24.6 | 24.4 | 24.5 | 32.4 | 27.2 | 26.0 | 28.8 | 26.5 | 25.5 | 32.0 | keine Sepsis |
| 5020_001 | 23.28 | 25.3 | 25.4 | 24.3 | 24.0 | 24.7 | 23.1 | 22.3 | 23.1 | 27.4 | 25.1 | 23.9 | 27.0 | 24.7 | 23.5 | 27.8 | keine Sepsis |
| 5023_001 | 25.14 | 25.5 | 25.4 | 25.1 | 24.6 | 25.0 | 22.9 | 22.8 | 23.3 | 31.6 | 26.0 | 23.3 | 27.5 | 24.5 | 24.0 | 30.2 | keine Sepsis |
| 8026_001 | 27.06 | 27.8 | 27.5 | 24.4 | 26.3 | 28.6 | 24.5 | 24.1 | 26.3 | 31.8 | 27.1 | 26.0 | 27.9 | 26.8 | 25.7 | 32.0 | keine Sepsis |
| 8056_002 | 26.30 | 28.4 | 25.9 | 25.2 | 26.3 | 27.7 | 25.5 | 23.9 | 26.1 | 31.4 | 27.3 | 24.5 | 27.7 | 25.6 | 26.0 | 31.8 | keine Sepsis |
| 8058_002 | 27.73 | 29.7 | 27.7 | 24.9 | 26.2 | 28.0 | 24.7 | 23.5 | 25.3 | 32.7 | 27.5 | 24.7 | 29.3 | 26.4 | 24.4 | 30.7 | keine Sepsis |
| 8086_001 | 25.46 | 25.9 | 25.5 | 24.1 | 24.4 | 26.2 | 23.1 | 22.9 | 23.7 | 30.7 | 25.1 | 24.1 | 27.6 | 24.9 | 24.0 | 30.2 | keine Sepsis |
| 8122_003 | 27.51 | 28.3 | 26.0 | 22.3 | 25.3 | 26.0 | 23.6 | 23.8 | 24.6 | 31.5 | 25.4 | 25.4 | 27.2 | 25.0 | 24.2 | 30.0 | keine Sepsis |
| 2042_001 | 28.20 | 31.6 | 28.5 | 24.3 | 28.3 | 30.3 | 26.8 | 25.4 | 27.8 | 34.1 | 29.1 | 25.8 | 29.9 | 29.1 | 26.4 | 33.4 | keine Sepsis |
| 8102_001 | 27.30 | 29.7 | 27.8 | 24.9 | 26.9 | 28.9 | 24.8 | 23.4 | 26.7 | 33.0 | 28.1 | 26.2 | 30.1 | 26.9 | 25.5 | 31.7 | keine Sepsis |
| 8111_001 | 25.79 | 27.0 | 26.7 | 23.7 | 25.0 | 27.0 | 23.9 | 24.8 | 26.0 | 32.0 | 26.5 | 24.5 | 29.1 | 26.0 | 24.4 | 31.4 | keine Sepsis |
| 8112_001 | 24.47 | 24.8 | 23.5 | 21.9 | 24.7 | 26.1 | 22.6 | 22.8 | 22.9 | 30.1 | 23.3 | 22.8 | 28.3 | 22.0 | 21.7 | 29.4 | keine Sepsis |
| 8116_001 | 26.60 | 30.1 | 27.8 | 24.6 | 26.7 | 28.7 | 25.8 | 24.5 | 26.6 | 32.8 | 28.1 | 25.9 | 29.8 | 27.0 | 25.9 | 32.1 | keine Sepsis |
| 8039_001 | 25.09 | 27.1 | 25.8 | 23.2 | 24.6 | 25.2 | 22.6 | 23.1 | 23.8 | 31.2 | 25.6 | 26.1 | 27.8 | 25.3 | 23.8 | 29.9 | keine Sepsis |
| 8039_002 | 25.19 | 26.7 | 24.9 | 23.1 | 24.1 | 28.7 | 22.9 | 26.3 | 22.9 | 30.1 | 25.1 | 25.6 | 27.8 | 24.7 | 23.5 | 30.3 | keine Sepsis |
| 8039_003 | 26.72 | 27.7 | 26.1 | 24.1 | 25.1 | 25.5 | 24.0 | 24.3 | 25.1 | 32.1 | 27.2 | 25.6 | 28.6 | 26.2 | 25.1 | 30.8 | keine Sepsis |
| 8039_004 | 27.61 | 30.2 | 26.3 | 24.3 | 26.5 | 30.6 | 24.4 | 27.6 | 25.4 | 33.2 | 27.3 | 26.5 | 28.7 | 26.3 | 25.2 | 31.3 | keine Sepsis |
| 8039_005 | 26.85 | 25.9 | 25.7 | 25.7 | 24.9 | 27.2 | 25.2 | 25.3 | 25.8 | 30.7 | 26.1 | 25.8 | 27.5 | 25.2 | 24.1 | 30.7 | keine Sepsis |
| 7052_001 | 28.40 | 29.9 | 26.8 | 24.2 | 27.7 | 26.6 | 25.7 | 24.3 | 25.0 | 34.3 | 28.0 | 26.5 | 28.6 | 26.6 | 25.8 | 31.2 | keine Sepsis |

(fortgesetzt)

| Experiment ID | M2 | M3 | M4 | M5 | M6 | M8 | M9 | M10 | M12 | M13 | M15 | M16 | M17 | R1 | R2 | R3 | Gruppe |
|---|---|---|---|---|---|---|---|---|---|---|---|---|---|---|---|---|---|
| 7052_002 | 29.62 | 31.3 | 27.1 | 24.0 | 29.0 | 27.2 | 26.4 | 25.3 | 26.2 | 33.2 | 29.0 | 26.2 | 29.8 | 26.9 | 25.9 | 31.7 | keine Sepsis |
| 7052_003 | 29.62 | 31.0 | 28.1 | 24.2 | 29.0 | 27.4 | 26.7 | 26.0 | 27.2 | 33.4 | 29.0 | 27.8 | 29.5 | 28.3 | 26.8 | 32.7 | keine Sepsis |
| 7119_001 | 26.68 | 27.4 | 26.5 | 24.2 | 25.2 | 26.1 | 24.5 | 23.3 | 24.4 | 31.2 | 26.2 | 24.5 | 28.4 | 25.9 | 23.8 | 31.5 | keine Sepsis |
| 7119_002 | 27.97 | 29.1 | 28.6 | 25.1 | 27.2 | 26.6 | 26.0 | 24.7 | 26.0 | 32.6 | 27.7 | 26.3 | 29.1 | 26.9 | 25.9 | 32.1 | keine Sepsis |
| 7119_003 | 28.56 | 29.1 | 28.3 | 24.8 | 26.8 | 26.7 | 25.6 | 24.7 | 25.3 | 31.8 | 27.6 | 27.1 | 29.4 | 26.6 | 25.7 | 30.7 | keine Sepsis |
| 8026_001_1 | 28.10 | 28.8 | 33.3 | 25.0 | 26.8 | 28.2 | 25.6 | 24.4 | 26.2 | 32.4 | 27.5 | 26.6 | 28.4 | 27.0 | 26.3 | 33.4 | keine Sepsis |
| 8026_002 | 29.11 | 29.6 | 32.7 | 25.1 | 27.5 | 28.6 | 26.0 | 25.2 | 26.1 | 32.9 | 27.8 | 26.1 | 29.0 | 27.1 | 25.7 | 32.5 | keine Sepsis |
| 8026_003 | 32.38 | 33.0 | 35.0 | 26.2 | 29.9 | 30.5 | 27.4 | 26.4 | 27.7 | 34.9 | 29.3 | 28.5 | 30.0 | 31.6 | 28.1 | 34.4 | keine Sepsis |
| 8026_004 | 29.79 | 30.5 | 32.2 | 25.4 | 28.7 | 28.9 | 27.7 | 26.8 | 27.0 | NA | 29.1 | 28.1 | 29.8 | 28.5 | 26.9 | 32.5 | keine Sepsis |
| 8026_005 | 28.06 | 28.6 | 32.0 | 24.5 | 26.1 | 27.0 | 25.3 | 24.0 | 24.9 | 32.5 | 27.4 | 26.7 | 24.9 | 25.7 | 24.8 | 31.5 | keine Sepsis |
| 7077_001 | 27.22 | 28.8 | 27.3 | 24.1 | 27.8 | 27.1 | 26.5 | 25.6 | 25.8 | 32.6 | 28.1 | 25.9 | 29.8 | 27.1 | 25.6 | 31.5 | keine Sepsis |
| 7077_002 | 25.50 | 27.6 | 25.8 | 23.0 | 26.2 | 25.5 | 25.3 | 24.5 | 25.3 | 31.5 | 27.4 | 25.2 | 28.4 | 25.1 | 24.8 | 30.4 | Sepsis |
| 7077_003 | 26.50 | 28.4 | 27.5 | 24.3 | 27.8 | 27.1 | 26.0 | 25.4 | 26.1 | 32.1 | 27.4 | 26.6 | 29.4 | 26.6 | 25.2 | 30.7 | Sepsis |
| 7084_001 | 26.16 | 28.2 | 27.0 | 23.6 | 26.1 | 27.4 | 25.3 | 24.2 | 27.0 | 33.3 | 26.8 | 24.7 | 29.9 | 26.0 | 25.5 | 32.0 | keine Sepsis |
| 7084_002 | 26.19 | 27.7 | 26.2 | 22.6 | 26.6 | 27.0 | 25.8 | 24.1 | 25.1 | 32.2 | 25.8 | 23.5 | 29.1 | 25.7 | 24.0 | 31.2 | keine Sepsis |
| 7084_003 | 27.42 | 30.0 | 28.2 | 23.9 | 28.6 | 28.5 | 27.0 | 25.6 | 26.2 | 32.9 | 26.9 | 24.6 | 30.3 | 26.5 | 25.1 | 31.5 | Sepsis |
| 7084_004 | 26.61 | 29.9 | 25.8 | 23.9 | 27.9 | 28.2 | 27.2 | 25.4 | 25.5 | 33.0 | 26.3 | 23.6 | 29.7 | 26.8 | 25.7 | 32.2 | Sepsis |
| 7096_001 | 25.59 | 27.0 | 26.9 | 23.2 | 25.3 | 26.0 | 23.7 | 23.3 | 24.6 | 31.4 | 26.7 | 25.4 | 28.7 | 25.8 | 23.8 | 29.9 | keine Sepsis |
| 7096_002 | 26.40 | 28.4 | 26.9 | 23.6 | 24.8 | 29.7 | 24.5 | 23.7 | 24.6 | 31.0 | 27.6 | 26.2 | 28.2 | 25.8 | 24.4 | 30.9 | keine Sepsis |
| 7096_003 | 27.50 | 29.5 | 27.6 | 23.8 | 26.0 | 26.4 | 24.6 | 23.9 | 25.1 | 32.5 | 27.8 | 26.7 | 28.9 | 26.5 | 25.0 | 31.2 | keine Sepsis |
| 7096_004 | 25.80 | 27.9 | 25.8 | 23.6 | 25.8 | 29.8 | 24.2 | 23.9 | 25.1 | 30.7 | 26.5 | 24.5 | 28.5 | 25.2 | 24.5 | 30.6 | keine Sepsis |
| 7096_005 | 26.01 | 27.7 | 26.3 | 23.2 | 25.2 | 26.1 | 24.1 | 23.8 | 24.0 | 31.7 | 26.3 | 24.5 | 29.1 | 25.6 | 23.6 | 30.3 | keine Sepsis |
| 7096_006 | 27.14 | 28.5 | 26.9 | 24.2 | 26.8 | 31.2 | 25.4 | 24.7 | 25.0 | 32.4 | 27.8 | 25.5 | 29.6 | 26.5 | 25.2 | 31.1 | Sepsis |
| 7096_007 | 25.97 | 27.5 | 24.9 | 22.7 | 25.3 | 25.6 | 24.3 | 23.9 | 24.6 | 31.3 | 26.5 | 23.8 | 28.4 | 25.0 | 23.8 | 30.2 | Sepsis |
| 8009_001_1 | 27.01 | 27.5 | 31.7 | 25.0 | 26.5 | 26.9 | 24.0 | 23.8 | 24.3 | 32.4 | 27.0 | 26.1 | 28.0 | 26.0 | 25.6 | 31.6 | keine Sepsis |
| 8009_002 | 25.06 | 26.4 | 24.9 | 22.9 | 24.7 | NA | 22.9 | 22.1 | 23.9 | 31.4 | 26.2 | 23.7 | 26.9 | 24.6 | 23.5 | 31.0 | keine Sepsis |
| 8009_003 | 24.75 | 24.6 | 21.8 | 20.7 | 24.6 | NA | 23.1 | 22.5 | 22.1 | 29.7 | 23.5 | 20.6 | 24.3 | 22.7 | 21.2 | 28.7 | keine Sepsis |
| 8009_004 | 27.57 | 28.9 | 29.6 | 23.6 | 26.8 | 27.2 | 25.7 | 23.8 | 23.7 | 33.1 | 26.8 | 23.4 | 27.8 | 26.0 | 24.4 | 31.8 | keine Sepsis |
| 8009_005 | 28.02 | 28.1 | 30.2 | 24.6 | 27.5 | 27.4 | 25.5 | 24.7 | 25.0 | 33.7 | 27.0 | 24.0 | 27.8 | 25.9 | 24.5 | 31.1 | keine Sepsis |
| 8009_006 | 27.17 | 28.1 | 28.9 | 24.6 | 26.5 | 26.3 | 25.6 | 24.0 | 24.9 | 32.2 | 26.5 | 23.3 | 27.5 | 25.3 | 23.9 | 30.7 | keine Sepsis |
| 8009_007 | 26.97 | 28.5 | 28.9 | 23.8 | 27.6 | 25.8 | 25.8 | 23.9 | 24.7 | 33.1 | 26.5 | 23.4 | 28.2 | 25.1 | 23.9 | 30.3 | keine Sepsis |
| 8009_010 | 29.35 | 29.7 | 30.3 | 25.8 | 28.6 | 28.1 | 26.6 | 25.7 | 26.8 | 34.4 | 27.8 | 24.9 | 29.1 | 26.6 | 25.4 | 32.8 | Sepsis |
| 8096_001_1 | 28.75 | 30.6 | 26.1 | 25.0 | 28.7 | 27.2 | 27.5 | 26.2 | 25.7 | 35.8 | 28.2 | 25.4 | 29.2 | 27.0 | 25.8 | 31.6 | keine Sepsis |

(fortgesetzt)

| Experiment ID | M2 | M3 | M4 | M5 | M6 | M8 | M9 | M10 | M12 | M13 | M15 | M16 | M17 | R1 | R2 | R3 | Gruppe |
|---|---|---|---|---|---|---|---|---|---|---|---|---|---|---|---|---|---|
| 8096_002 | 27.69 | 28.8 | 25.8 | 24.2 | 27.3 | 26.6 | 25.6 | 24.4 | 24.0 | 32.7 | 27.7 | 24.8 | 28.3 | 26.2 | 26.0 | 31.6 | keine Sepsis |
| 8096_003 | 28.48 | 31.4 | 27.0 | 23.1 | 28.0 | 26.1 | 26.5 | 26.2 | 24.5 | 33.2 | 28.8 | 26.6 | 28.8 | 26.4 | 25.6 | 31.2 | keine Sepsis |
| 8112_001_1 | 27.42 | 28.7 | 27.0 | 25.3 | 27.3 | 30.1 | 26.5 | 27.2 | 26.7 | 32.6 | 27.5 | 25.9 | 30.0 | 26.4 | 25.6 | 32.8 | keine Sepsis |
| 8112_002_1 | 27.36 | 28.9 | 26.8 | 25.4 | 26.6 | 31.7 | 26.3 | 26.7 | 26.5 | 32.7 | 27.4 | 25.2 | 29.4 | 26.5 | 25.2 | 32.3 | keine Sepsis |
| 8112_003 | 27.18 | 28.8 | 27.3 | 24.2 | 26.5 | 29.1 | 26.2 | 26.3 | 24.7 | 32.3 | 27.4 | 25.1 | 29.6 | 26.2 | 24.8 | 31.9 | keine Sepsis |
| 8112_004 | 27.83 | 29.8 | 27.7 | 25.9 | 26.9 | 32.0 | 27.0 | 26.7 | 25.5 | 33.1 | 28.0 | 25.9 | 29.0 | 26.6 | 24.9 | 31.4 | keine Sepsis |
| 8112_005 | 28.19 | 30.0 | 27.4 | 24.8 | 26.9 | 30.3 | 26.4 | 26.7 | 25.8 | 34.1 | 27.9 | 26.2 | 29.3 | 26.4 | 24.7 | 31.2 | Sepsis |
| 8112_006 | 28.64 | 30.2 | 27.7 | 26.0 | 27.6 | 32.2 | 26.9 | 27.1 | 26.4 | 34.2 | 28.0 | 26.9 | 30.3 | 27.1 | 25.8 | 32.7 | Sepsis |
| 8101_001 | 24.47 | 25.9 | 24.7 | 21.9 | 24.3 | 24.5 | 23.0 | 22.5 | 25.1 | 30.6 | 25.1 | 23.3 | 26.5 | 24.2 | 23.3 | 30.6 | keine Sepsis |
| 8101_002 | 24.87 | 27.0 | 25.3 | 22.8 | 25.2 | 24.8 | 23.9 | 23.2 | 24.0 | 30.8 | 25.6 | 23.7 | 26.7 | 24.5 | 23.5 | 17.6 | keine Sepsis |
| 8101_003 | 24.48 | 25.7 | 23.7 | 22.2 | 24.2 | 29.5 | 23.7 | 23.5 | 24.8 | 31.1 | 25.0 | 22.3 | 26.4 | 24.0 | 23.2 | 31.0 | keine Sepsis |
| 8101_004 | 24.88 | 26.9 | 24.1 | 22.3 | 24.6 | 24.9 | 24.2 | 23.7 | 23.6 | 30.2 | 24.9 | 22.2 | 25.7 | 24.1 | 22.6 | 29.2 | Sepsis |
| 8101_005 | 23.84 | 26.4 | 22.4 | 22.6 | 24.9 | 24.2 | 23.9 | 22.8 | 24.9 | 28.9 | 24.6 | 22.4 | 25.0 | 24.1 | 22.9 | 28.7 | Sepsis |
| 8111_003 | 25.77 | 26.7 | 24.8 | 23.1 | 24.9 | 24.9 | 24.2 | 24.0 | 26.1 | 31.0 | 25.3 | 23.3 | 27.3 | 24.4 | 23.1 | 30.1 | keine Sepsis |
| 8111_004 | 25.15 | 26.7 | 26.0 | 22.5 | 24.1 | 24.4 | 23.6 | 24.2 | 25.5 | 30.1 | 25.6 | 23.5 | 27.3 | 25.6 | 24.4 | 30.0 | keine Sepsis |
| 8111_005 | 25.32 | 26.6 | 26.8 | 22.7 | 24.7 | 25.1 | 23.6 | 23.2 | 24.2 | 30.4 | 25.8 | 24.4 | 27.5 | 25.1 | 24.3 | 31.4 | keine Sepsis |
| 8111_006 | 26.53 | 26.9 | 25.3 | 23.2 | 25.9 | 25.3 | 24.7 | 25.7 | 26.2 | 31.4 | 25.7 | 24.1 | 28.3 | 24.6 | 24.8 | 30.6 | Sepsis |
| 8111_007 | 25.88 | 26.8 | 26.5 | 23.6 | 25.8 | 26.5 | 25.6 | 26.0 | 27.1 | 29.4 | 26.4 | 23.6 | 28.4 | 26.0 | 24.1 | 28.5 | Sepsis |
| 6008_002 | 25.89 | 26.1 | 22.4 | 20.5 | 26.0 | 25.6 | 24.5 | 22.5 | 22.8 | 28.4 | 23.4 | 21.9 | 28.2 | 23.0 | 22.6 | 28.1 | Sepsis |
| 6063_001 | 26.45 | 26.8 | 25.0 | 23.0 | 25.6 | 25.7 | 24.3 | 23.3 | 25.0 | 30.9 | 25.9 | 23.5 | 28.3 | 25.2 | 23.6 | 30.0 | Sepsis |
| 6141_001 | 27.23 | 29.9 | 25.6 | 23.9 | 28.0 | 27.9 | 26.4 | 26.0 | 26.6 | 31.8 | 27.2 | 24.1 | 27.9 | 25.2 | 25.0 | 31.6 | Sepsis |
| 1013_001 | 28.86 | 30.9 | 26.9 | 23.6 | 28.4 | 27.1 | 26.0 | 25.6 | 26.3 | 33.3 | 28.1 | 25.0 | 25.5 | 26.6 | 24.6 | 31.6 | Sepsis |
| 6024_002 | 26.64 | 28.5 | 25.3 | 21.9 | 26.1 | 24.8 | 24.7 | 23.9 | 23.7 | 32.2 | 26.0 | 24.7 | 28.3 | 25.6 | 25.1 | 31.2 | Sepsis |
| 7040_001 | 28.55 | 31.1 | 26.1 | 23.7 | 28.4 | 26.3 | 26.8 | 25.7 | 26.1 | 33.9 | 27.6 | 24.7 | 28.5 | 26.1 | 25.0 | 31.5 | Sepsis |
| 7079_002 | 27.32 | 30.4 | 26.3 | 23.6 | 28.6 | 26.6 | 27.1 | 26.5 | 25.8 | 30.7 | 27.2 | 25.3 | 28.6 | 26.4 | 25.0 | 29.8 | Sepsis |
| 920_001 | 29.50 | 31.4 | 29.1 | 26.0 | 28.2 | 27.5 | 26.6 | 25.1 | 25.8 | 32.7 | 28.6 | 27.0 | 29.1 | 27.4 | 25.5 | 30.7 | Sepsis |
| 6036_001 | 28.82 | 30.0 | 26.8 | 24.6 | 28.7 | 26.5 | 27.1 | 25.3 | 24.0 | 34.7 | 27.7 | 26.7 | 29.5 | 26.8 | 25.9 | 31.3 | Sepsis |
| 6056_001 | 26.28 | 27.9 | 25.4 | 24.6 | 27.6 | 26.0 | 25.5 | 25.3 | 27.2 | 31.0 | 26.5 | 25.5 | 27.5 | 25.7 | 24.8 | 30.1 | Sepsis |
| 6061_001 | 28.63 | 29.0 | 27.0 | 25.2 | 28.2 | 26.5 | 26.3 | 25.6 | 26.2 | 32.6 | 26.7 | 25.5 | 30.0 | 26.4 | 25.0 | 30.8 | Sepsis |
| 7023_001 | 26.83 | 29.2 | 26.5 | 24.3 | 26.5 | 25.5 | 25.7 | 24.5 | 25.5 | 34.7 | 26.3 | 25.9 | 28.6 | 27.4 | 25.7 | 31.7 | Sepsis |
| 7112_001 | 28.60 | 29.5 | 31.3 | 25.9 | 27.9 | 27.9 | 25.4 | 26.3 | 25.3 | 33.8 | 27.0 | 24.7 | 30.1 | 26.1 | 24.9 | 31.6 | Sepsis |
| 6064_001 | 28.63 | 30.0 | 30.0 | 22.8 | 29.6 | 26.2 | 26.2 | 26.2 | 26.1 | 33.8 | 27.1 | 24.3 | 28.9 | 25.6 | 25.1 | 30.6 | Sepsis |
| 6120_001 | 27.58 | 30.7 | 25.7 | 23.4 | 29.0 | 27.8 | 27.2 | 27.3 | 27.2 | 33.7 | 28.1 | 24.6 | 28.6 | 26.4 | 25.3 | 31.4 | Sepsis |

(fortgesetzt)

| Experiment ID | M2 | M3 | M4 | M5 | M6 | M8 | M9 | M10 | M12 | M13 | M15 | M16 | M17 | R1 | R2 | R3 | Gruppe |
|---|---|---|---|---|---|---|---|---|---|---|---|---|---|---|---|---|---|
| 7105_001 | 27.19 | 28.6 | 32.3 | 22.7 | 26.6 | 26.4 | 25.1 | 25.1 | 24.6 | 31.7 | 27.7 | 24.4 | 29.1 | 26.0 | 25.2 | 30.8 | Sepsis |
| 7120_001 | 29.38 | 29.8 | 29.6 | 24.4 | 29.3 | 28.2 | 26.9 | 26.3 | 26.2 | 33.5 | 27.9 | 24.7 | 28.2 | 26.6 | 25.7 | 32.2 | Sepsis |
| 749_001 | 27.59 | 28.8 | 26.4 | 23.5 | 26.5 | 25.6 | 24.0 | 25.1 | 25.3 | 31.2 | 26.3 | 25.1 | 28.6 | 25.1 | 24.1 | 30.5 | Sepsis |
| 5008_001 | 26.87 | 27.9 | 31.2 | 22.0 | 26.5 | 26.1 | 24.5 | 24.0 | 25.5 | 32.3 | 27.2 | 24.8 | 28.3 | 25.6 | 24.3 | 30.8 | Sepsis |
| 5009_001 | 25.52 | 26.2 | 29.7 | 22.3 | 25.9 | 25.5 | 23.2 | 24.2 | 26.3 | 30.8 | 26.0 | 24.2 | 27.4 | 25.0 | 23.4 | 29.0 | Sepsis |
| 5010_001 | 27.96 | 29.1 | 29.5 | 22.8 | 28.8 | 27.4 | 28.5 | 26.3 | 27.0 | 32.6 | 27.9 | 25.6 | 27.8 | 26.1 | 26.7 | 31.9 | Sepsis |
| 5018_001 | 28.47 | 30.4 | 30.0 | 23.7 | 28.4 | 27.6 | 26.8 | 26.6 | 26.7 | 34.0 | 27.3 | 25.3 | 29.2 | 26.2 | 25.9 | 31.9 | Sepsis |
| 1015_001 | 29.77 | 30.5 | 26.7 | 24.8 | 28.8 | 28.3 | 26.7 | 26.1 | 26.8 | 34.5 | 28.3 | 25.0 | 29.3 | 26.9 | 25.5 | 30.5 | Sepsis |
| 6005_001 | 29.27 | 30.0 | 26.9 | 23.5 | 28.3 | 26.4 | 26.8 | 24.9 | 21.6 | 33.4 | 27.7 | 24.9 | 28.8 | 26.2 | 25.6 | 32.1 | Sepsis |
| 6035_002 | 28.80 | 28.0 | 26.3 | 25.0 | 28.0 | 27.4 | 26.9 | 26.2 | 25.7 | 32.8 | 27.1 | 25.6 | 29.1 | 26.6 | 25.3 | 31.6 | Sepsis |
| 6070_002 | 29.09 | 31.0 | 27.7 | 24.7 | 28.2 | 28.9 | 26.9 | 25.8 | 27.4 | 34.1 | 29.8 | 26.5 | 29.8 | 28.1 | 26.4 | 33.0 | Sepsis |
| 6075_002 | 29.35 | 30.6 | 25.6 | 25.8 | 28.9 | 28.7 | 26.8 | 26.4 | 27.2 | 32.6 | 28.0 | 24.6 | 28.5 | 26.3 | 25.4 | 31.6 | Sepsis |
| 6104_002 | 30.63 | 32.3 | 28.8 | 25.8 | 28.5 | 28.5 | 27.2 | 27.2 | 26.8 | 34.4 | 28.9 | 27.2 | 29.0 | 27.5 | 26.3 | 32.1 | Sepsis |
| 6124_001 | 30.50 | 31.3 | 26.7 | 24.8 | 29.1 | 26.7 | 28.1 | 25.0 | 26.1 | 32.7 | 28.3 | 26.0 | 28.5 | 27.0 | 26.4 | 31.9 | Sepsis |
| 6126_001 | 30.72 | 28.6 | 27.6 | 24.5 | 27.8 | 26.2 | 26.0 | 24.7 | 24.5 | 31.4 | 26.8 | 25.7 | 26.9 | 25.9 | 24.7 | 30.4 | Sepsis |
| 714_001 | 32.80 | 31.9 | 27.6 | 27.5 | 29.9 | 30.3 | 29.8 | 28.0 | 25.2 | NA | 29.5 | 26.8 | 31.6 | 28.9 | 28.0 | 33.5 | Sepsis |

Klassifikation:

Ziel der Klassifikation war die Bestimmung des Markersets, der die beste Trennung zwischen Proben von Patienten mit und ohne Sepsis ermöglicht.

Um die Gen-Marker nach ihrer Trennungsgüte anzuordnen, wurde die lineare Diskriminanzanalyse (LDA) [Hastie et al., 2001] zusammen mit der Methode der vorwärts Selektion verwendet, wobei die Trennbarkeit mit dem F-Wert bewertet wurde [Hocking, R. R., 1976].

Die Berechnung erfolgte unter der Verwendung der Funktion lda aus der R-Bibliothek MASS. Für p Marker wurden die Gewichte ($w_0,...,w_p$) der Diskriminanzfunktion $f_{LD}$, die durch die Formel

$$f_{LD}(x_1,\ldots,x_p)=\sum_{i=1}^{p}w_ix_i-w_0$$

definiert ist, aus den Trainingsdaten berechnet. Jede Trainings-Probe wurde nachhinein klassifiziert, wofür in der vorangegangenen Formel für $x_i$ die Delta Ct-Werte der Probe eingesetzt wurden. Die Gewichte der Diskriminanzfunktion wurden so berechnet, dass ein positiver Wert der Funktion die Zuordnung zur Gruppe mit einer infektiösen Komplikation und ein negativer Wert der Funktion die Zuordnung zur Gruppe ohne eine infektiöse Komplikation bedeuten.

Die Klassifikationsprozedur wurde für eine aufsteigende Anzahl von Markern wiederholt, wobei sukzessiv die Marker-Kandidaten in die Diskriminanzanalyse eingeschlossen wurden, die den höchsten Beitrag zur Trennungsgüte leisteten (vorwärts Selektion). Dieser Analyseschritt wurde für 1000 Bootstrap-Stichproben wiederholt, die durch zufälliges Ziehen mit Zurücklegen aus dem Trainingsdatensatzes gewonnen wurden. Die in jeder Wiederholung ermittelten Marker-Ranks wurden über die 1000 Läufe gemittelt. Die Marker-Kandidaten wurden nach dem mittleren Rank aufsteigend angeordnet. Diese Anordnung bedeutet, dass der Marker mit dem kleinsten mittleren Rank der war, der am häufigsten den meisten Beitrag zur Trennungsgüte leistete und der Marker mit dem höchsten mittleren Rank für die Trennung in meisten Wiederholungen wenig beitrug.

Die Güte des ermittelten Marker-Rankings wurde geprüft, in dem die Trennbarkeit der Trainingsgruppen für Markersets mit aufsteigender Markerzahl bewerten wurde. Dafür wurde die lineare Diskriminanzanalyse mit einer einfachen Kreuzvalidierung verwendet. Für $p$ Marker wurden die Gewichte ($w_0,...,w_p$) der Diskriminanzfunktion $f_{LD}$ aus dem reduzierten Trainingsdaten berechnet, in dem nacheinander eine Probe weggelassen wurde. Diese Probe wurde nachhinein klassifiziert, wofür in der vorangegangenen Formel für $x_i$ die Delta Ct-Werte der Probe eingesetzt wurden.

Um zu prüfen, dass die Trennungs-Güte nicht primär vom Klassifikationsverfahren sondern von der Marker-Auswahl abhängt, wurde abschließend die einfache Kreuzvalidierung auch für die quadratische Diskriminanzanalyse (QDA) [Hastie et al., 2001 in gleicher weise wiederholt. Die Berechnung erfolgte unter der Verwendung der Funktion qda aus der R-Bibliothek MASS.

Die Klassifikationsergebnisse des Trainingsdatensatzes wurden für die Matrix des Testdatensatzes validiert. Aus dem Trainingsdatensatz wurde für jedes Markersets mit aufsteigender Markerzahl die Diskriminanzfunktion bestimmt und zu Klassifikation der Test-Proben verwendet. Die Güte der Klassifikation wurde mittels der Receiver Operating Characteristic (ROC) - Kurve bewertet, in der die Rate der richtig Positiven (Sensitivität) gegen die Rate der falsch Positiven (1-Spezifität) für eine aufsteigenden Folge der Klassifikationsschwellen abgebildet wird [Fawcett T., 2006]. Für die Bewertung wurde aus jeder ROC-Kurve die Fläche unter dar Kurve (AUC) berechnet und die höchste erreichbare Klassifikationseffizienz (Anteil der korrekt klassifizierten Proben) bestimmt.

Ergebnisse

Die Ergebnisse der oben beschriebenen Klassifikationsanalyse wurden in der Tabelle 8 zusammengefasst. Die Ranking-Prozedur ergab die folgende Anordnung der Marker-Kandidaten: M6, M15, M9, M7, M2, M10, M4, M12, M17, M3, M8, M13, M16. Die Kreuzvalidierungsrate der LDA und der QDA stieg deutlich für die ersten 3 Marker auf 94.8%. Die beste Trennung der Trainingsgruppen mit 96,6 % wurde mit LDA für die ersten 6 Marker erreicht, bei der 56 aus 58 Proben richtig klassifiziert wurden (QDA liefert ein Maximum bei 3 Markern folgend mit 7 Markern). Für mehr als 7 Markern wurde keine Verbesserung der Klassifikation erreicht.

Für den unabhängigen Testdatensatz wurde die größte Fläche unter der ROC-Kurve von mehr als 85% für die ersten 6 und 7 Markern erreicht, die beste Klassifikation mit 81,4% lieferten die ersten 7 Markern.

Tabelle 8: Ergebnisse der Klassifikationsoptimierung. Fett markiert ist der maximale Wert der jeweiligen Spalte, der das beste Ergebnis bzgl. der Markerkombination widerspiegelt.

| | Trainingsdaten (n= 58) | | | Testdaten (n = 113) | |
|---|---|---|---|---|---|
| Markerranking | mittlerer Rank (1000 Bootstraps) | Kreuzvalidierungsrate (%) | | Fläche unter der ROC-Kurve, AUC (%) | Klassifikations-Effizienz (%) |
| | | LDA | QDA | | |
| M6 | 3.3 | 70.7 | 74.1 | 60.1 | 60.2 |
| M15 | 4.1 | 79.3 | 81.0 | 68.1 | 68.1 |
| M9 | 4.4 | 94.8 | 94.8 | 84.0 | 78.8 |
| M7 | 5.7 | 93.1 | 87.9 | 84.3 | 77.9 |
| M2 | 5.7 | 93.1 | 89.7 | 83.8 | 75.2 |
| M10 | 7.0 | **96.6** | 87.9 | 85.2 | 78.8 |
| M4 | 7.1 | 91.4 | 93.1 | **85.4** | **81.4** |
| M12 | 7.2 | 89.7 | 89.7 | 83.9 | 78.8 |
| M17 | 8.7 | 91.4 | 89.7 | 82.5 | 77.9 |
| M3 | 8.9 | 91.4 | 87.9 | 81.9 | 78.8 |
| M8 | 9.4 | 89.7 | 84.5 | 80.3 | 75.2 |
| M13 | 9.4 | 87.9 | 81.0 | 79.2 | 73.5 |
| M16 | 10.3 | 87.9 | 77.6 | 78.4 | 73.5 |

Da in der Klassifikationsanalyse die besten Ergebnisse überwiegend mit den ersten 7 Markern aus der obigen Tabelle erreicht wurden, wurde für weitere Klassifikationen die 7-Marker-LDA verwendet, deren Diskriminanzfunktion $f_{LD}$ aus dem Trainingsdatensatz bestimmt wurde. Die zugehörigen Gewichte ($w_0$,...,$w_7$) wurden in der Tabelle 9 dargelegt.

Basierend auf dieser Funktion wurde ein auf die Sepsis bezogener diagnostischer Parameter, ein sogenannter SIQ-Score (SIQ) wie folgt eingeführt. Für eine neue unabhängige Probe bekommt man als Klassifkationsergebnis einen dimensionsfreien Wert der Diskriminanzfunktion. Ein positiver Wert klassifiziert die Probe als infektiös und ein negativer Wert als nicht infektiös. Für typische Vertreter der jeweiligen Gruppe erhält man absolut höhere Werte, schwer klassifizierbare Proben erreichen Werte nahe Null. Der Streubereich der Diskriminanz-Werte entspricht i.a. der Variabilität der Delta Ct- Datenmatrix. So erreichte man in der Klassifikation Diskriminanzwerte von ca. -5 bis 5. Um die Unterschiede deutlicher hervorzuheben, wurde der SIQ-Score (SIQ) als der 10-fache Wert der Diskriminanzfunktion mit den Gewichten aus der Tabelle 9 eingeführt. Dementsprechend variierten die SIQ-Werte der Testdaten von ca. -50 bis 50.

Tabelle 9: Gewichte der Diskriminanzfunktion, die aus dem Trainingsdatensatz ermittelt wurden.

| w0 | w1 (M6) | w2 (M15) | w3 (M9) | w4 (M7) | w5 (M2) | w6 (M10) | w7 (M4) |
|---|---|---|---|---|---|---|---|
| 0.160 | 0.733 | -0.722 | -1.006 | 0.188 | -0.387 | -0.268 | 0.161 |

In der linearen Diskriminanzanalyse wird i.a. die Diskriminanzfunktion so bestimmt, dass die Trennungsschwelle zwischen den beiden Trainingsgruppen bei Null liegt. Mittels der ROC-Kurve kann bei einem unabhängigen Testdatensatz ermittelt werden, bei welchem Schwellen-Wert die beste Trennung der zugehörigen Test-Gruppen erreicht wird. In Fig. 1 wird die ROC-Kurve zur Klassifikation der Testdaten mittels des SIQ-Scores dargestellt und es wird das Verhältnis zwischen richtig Positiven (Sensitivität) und falsch Positiven (1-Spezifität) markiert, grau gestrichelt für den Schwellenwert von Null und schwarz gestrichelt für die beste erreichte Klassifikation von 81,4%. Dieses Klassifikationsergebnis wurde für den Schwellenwert von SIQ = - 4.9 erreicht. Aus Fig. 1 wurde ersichtlich, dass durch die Verschiebung der Schwelle von 0 auf -4.9 einen Sensitivität-Gewinn von ca. 63% auf über 80% zu Kosten der Spezifität von ca. 81% auf ca. 80% erreicht wird. Dieses Ergebnis spiegelt die Diskrepanz zwischen den vorausgewählten Patienten des Trainingsdatensatzes und dem heterogenen Kollektiv des Testdatensatzes wider. Die Klassifikationsgüte, die mit der aktualisierten Klassifikationsschwellen von SIQ = -4.9 erreicht wurde, wird in der Tabelle 10 dargestellt.

Damit wird demonstriert, dass die beschriebene Erfindung für die Unterscheidung zwischen infektiöser und nicht- infektiöser Genese bei systemischen Reaktionen des Organismus, wie z.B. SIRS, Sepsis, postoperative Komplikationen, chronischem und/oder akutem Organdysfunktion, Schock-Reaktion, Entzündungsreaktion und/oder Trauma, differentialdiagnostisch angewendet werden kann.

Tabelle 10: Güte der Klassifikation für den Testdatensatz mittels des SIQ-Scores bei einer Schwelle

| | | Goldstandard Sepsis (n) | Goldstandard keine Sepsis (n) | Summe (n) | Vorhersagewerte |
|---|---|---|---|---|---|
| SIQ-Score (Schwelle = -4.9) | positiv (n) | 32.7% (37) | 12.4% (14) | 45.1% (51) | 72.5% |
| | negativ (n) | 6.2% (7) | 48.7% (55) | 54.9% (62) | 88.7% |
| | Summe (n) | 38.9% (44) | 61.1% (69) | 100.0% (113) | |
| | | **Sensitivität 84.1%** | **Spezifität 79.7%** | **Effizienz 81.4%** | |

**Beispiel 2: Frühe Sepsis-Erkennung**

Im Testdatensatz des 1. Ausführungsbeispiels wurden für den Fall Nr. 8112 4 Proben vor und 2 Proben nach der Entwicklung der Sepsis untersucht (vgl. Tabellen 4 und 7). In der Klassifikationsanalyse wurde ein SIQ-Score über dem Wert von -4.9 bereits 2 Tage vor der klinischen Manifestation der Sepsis erreicht. Der Verlauf des im 1. Ausführungsbeispiel eingeführten SIQ-Scores sowie der Verlauf weiterer Sepsis-relevanten klinischen Parametern (PCT, CRP, SOFA, Körpertemperatur, Schockbehandlung) wird in Fig. 2 dargestellt. Aus Fig. 2 wird ersichtlich, dass SIQ-Score der einzige Parameter ist, der vorzeitig die infektiöse Komplikation widerspiegelt. Damit wird demonstriert, dass die beschriebene Erfindung für die frühe Erkennung von infektiösen Komplikationen, wie Sepsis und/oder generalisierter Infektion, angewendet werden kann.

**Beispiel 3: Beobachtungdes Therapieverlaufs**

Im Testdatensatz des 1. Ausführungsbeispiels wurden für den Fall Nr. 7084 2 Proben vor und 2 Proben nach der Entwicklung der Sepsis untersucht (vgl. Tabellen 4 und 7). Für das 3. Ausführungsbeispiel wurden 5 nachfolgenden Proben dieses Patienten in gleicher Weise, wie es im 1. Beispiel beschrieben wurde, vermessen und ausgewertet (vgl. Tabelle 11). In Fig. 3 wurden die ermittelten Werte des im 1. Ausführungsbeispiel eingeführten SIQ-Scores zusammen mit den Sepsis-relevanten klinischen Parameter PCT, CRP, SOFA, Körpertemperatur sowie der Dosierung von Katecholaminen (norepinephrine), die die Schock-Behandlung reflektiert, dargestellt. Aus Fig. 3 wird ersichtlich, dass SIQ-Score ein Tag vor der klinischen Manifestation der Sepsis über den Schwellenwert von - 4.9 ansteigt und in der akuten Phase über der Schwelle bleibt. Nach der klinischen Manifestation der Sepsis wurden eine entsprechende Antibiotika-Therapie und eine Schockbehandlung begonnen (vgl. Tabelle 4 und Fig. 3). Die akute Phase wurde mit dem Absetzen der Katecholamine (Schock-Behandlung) am 8. Tag beendet. Der verbesserte Zustand des Patienten spiegelt sich auch im Abfallen des SOFA-Scores ab dem 7. Tag wider. Nach der akuten Phase nimmt auch der SIQ-Score ab und fällt am 8. Tag unter die Schwelle von -4.9. Die Parameter PCT und CRP nehmen ebenfalls ab, bleiben aber über dem zugehörigen diagnostischen Entscheidungswert. Damit wird demonstriert, dass die beschriebene Erfindung für die Verlaufskontrolle und/oder Therapiekontrolle von z.B. Antibiotika-Therapie und/oder adjunktive klinische Maßnahmen und/oder operative Sanierungsmaßnahmen angewendet werden kann.

Tabelle 11: Ct-Werte des Trainingsdatensatzes pro Marker (Mittelwert der Dreifachbestimmung) und die Gruppenzugehörigkeit (letzte Spalte)

| Experiment ID | M2 | M3 | M4 | M5 | M6 | M8 | M9 | M10 | M12 | M13 | M15 | M16 | M17 | R1 | R2 | R3 | |
|---|---|---|---|---|---|---|---|---|---|---|---|---|---|---|---|---|---|
| 7084_001 | 26.2 | 28.2 | 27.0 | 23.6 | 26.1 | 27.4 | 25.3 | 24.2 | 27.0 | 33.3 | 26.8 | 24.7 | 29.9 | 26.0 | 25.5 | 32.0 | keine Sepsis |
| 7084_002 | 26.2 | 27.7 | 26.2 | 22.6 | 26.6 | 27.0 | 25.8 | 24.1 | 25.1 | 32.2 | 25.8 | 23.5 | 29.1 | 25.7 | 24.0 | 31.2 | keine Sepsis |
| 7084_003 | 27.4 | 30.0 | 28.2 | 23.9 | 28.6 | 28.5 | 27.0 | 25.6 | 26.2 | 32.9 | 26.9 | 24.6 | 30.3 | 26.5 | 25.1 | 31.5 | Sepsis |
| 7084_004 | 26.6 | 29.9 | 25.8 | 23.9 | 27.9 | 28.2 | 27.2 | 25.4 | 25.5 | 33.0 | 26.3 | 23.6 | 29.7 | 26.8 | 25.7 | 32.2 | Sepsis |
| 7084_005 | 24.8 | 28.3 | 25.1 | 22.8 | 27.2 | 27.6 | 26.4 | 24.6 | 24.1 | 29.5 | 26.1 | 22.4 | 28.4 | 25.7 | 24.1 | 28.7 | Sepsis |
| 7084_006 | 26.6 | 28.9 | 26.0 | 23.5 | 26.7 | 30.9 | 26.5 | 24.8 | 25.3 | 32.1 | 26.6 | 23.0 | 29.5 | 25.9 | 24.7 | 31.8 | Sepsis |
| 7084_007 | 25.4 | 27.5 | 25.2 | 23.7 | 26.7 | 28.0 | 25.0 | 23.8 | 22.1 | 31.5 | 25.7 | 21.9 | 28.8 | 25.0 | 23.3 | 31.7 | Sepsis |
| 7084_008 | 24.8 | 26.7 | 24.4 | 23.0 | 26.4 | 30.5 | 25.4 | 24.3 | 24.0 | 31.1 | 25.2 | 21.5 | 28.9 | 24.6 | 23.5 | 31.5 | Sepsis |
| 7084_009 | 25.9 | 28.0 | 25.8 | 23.7 | 27.4 | 28.3 | 25.4 | 24.3 | 25.9 | 32.3 | 26.8 | 23.6 | 29.3 | 26.3 | 24.6 | 31.7 | Sepsis |

## LITERATUR

ACCP/SCCM (1992), Crit. Care Med 20, 864-74

Alberti C, Brun-Buisson C, Goodman SV, Guidici D, Granton J, Moreno R, Smithies M, Thomas O, Artigas A, Le Gall JR; European Sepsis Group (2003) Influence of systemic inflammatory response syndrome and Sepsis on outcome of critically ill infected patients. Am J Respir Crit Care Med 168, 77-84.

Altschul et al.: J Mol Biol 215:403-410; 1990

Amin K, Kauffman CA (2003), Fever of unknown origin. Postgrad med114(3), 69-75

Baker SG, und Kramer B (2006), Identifying genes that contribute most to good classification in microarray. BMC Bioinformatics 7, 407

Benson D.A., Karsch-Mizrachi I., Lipman D.J., Ostell J., Sayers E.W. GenBank. Nucleic Acids Res. 2009 Jan;37(Database issue):D26-31.

Blake PG (2008) Complicated peritonitis - the biggest cause of technique failiure. Perit Dial Int. 28(4):327-328

Bone RC, Balk RA, Cerra FB, Dellinger EP, Fein AM, Knaus WA, Schein RM, Sibbald WJ, the ACCP/SCCM Consensus Conference Committee (1992) Definitions for Sepsis and organ failure and guidelines for the use of innovative therapies in Sepsis. Chest 101,1656-1662

Box GEP, Cox DR (1964) An analysis of transformations (with discussion). J Roy Stat Soc B 26, 211-252

Buneß A, Huber W, Steiner K, Sültmann H, Poustka A (2005) ArrayMagic: twocolour cDNA microarray quality control and preprocessing. Bioinformatics 21, 554 - 556.

Breiman L (2001) Random Forests. Machine Learning 45(1), 5-32

Brun-Buisson C, Doyon F, Carlet J, Dellamonica P, Gouin F, Lepoutre A, Mercier JC, Offenstadt G, Regnier B (1995) Incidence, risk factors, and outcome of severe Sepsis and septic shock in adults. A multicenter prospective study in intensive care units. French ICU Group for Severe Sepsis. JAMA 274, 968-974

Brun-Buisson C, Roudot-Thoraval F, Girou E, Grenier-Sennelier C, Durand-Zaleski I (2003) The costs of septic syndromes in the intensive care unit and influence of hospital-acquired Sepsis. Intensive Care Med 29, 1464-1471

Bustin SA (2002) Quantification of mRNA using real-time reverse transcription PCR (RT-PCR): trends and problems. J Mol Endicronol 29, 23-29

Calandra T, Cohen J. (2005) International Sepsis Forum Definition of Infection in the ICU Consensus Conference. Critical Care Med 33(7), 1538-48.

Carrigan SD Scott G Tabrizian M (2004) Toward resolving the challenges of Sepsis. Clin Chem 50(8), 1301-1314

DE 10 2007 036 678 Verwendung von Polynukleotiden zur Erfassung von Genaktivitäten für die Unterscheidung zwischen lokaler und systemischer Infektion

DE 102007036678.9 (nicht veröffentlicht)

Ding BY and Gentleman R (2004) Classification using generalized partial least squares. Bioconductor Project Working Papers. Paper 5. http://www.begress.com/bioconductor/paper5 9

Efron B (1979) Bootstrap Methods: Another Look at the Jackknife. The Annals of Statistics 7(1), 1-26

Fawcett, T. (2006). An introduction to ROC analysis. Pattern Recognition Letters, 27, 861-874.

FDA: In Vitro Diagnostic Multivariate Index Assays. Draft Guidance for Industry, Clinical Laboratories, and FDA Staff (2003) http://www.fda.gov/cdrh/oivd/guidance/1610.pdf

Feezor RJ, Baker HV, Xiao W et al. (2004) Genomic and Proteomic Determinants of Outcome in Patients Untergoing Thoracoabdominal Aortic Aneurysm Repair. J Immun 172, 7103-7109

Klein D (2002) Quantification using real-time PCR technology: applications and limitations. Trends Mol Med 8(6), 257-260

Koulaouzidis A, Bhat S, Saeed AA (2009) Spontaneous bacterial peritonitis. World J Gastroenterol. 15(9):1042-9.

Mayhall G (2001) Ventilator-Associated Pneumonia or Not? Contemporary Diagnosis. Emerging Infection Disease CDC 7(2)

Golub TR, Slonim DK, Tamayo P, et al. (1999) Molecular classification of cancer: class discovery and class prediction by gene expression monitoring. Science 286, 531-537

T. Hastie, R.Tibshirani, J. Friedman (2001) "The Elements of Statistical Learning", Springer series in statistics, ISBN 0-387-95284-5.

Hocking, R. R. (1976) "The Analysis and Selection of Variables in Linear Regression" Biometrics, 32.

Hollander M., Wolfe D., (1973), Nonparametric statistical inference. New York: John Wiley & Sons.

Huber W, von Heydebreck A, Sueltmann H, Poustka A, Vingron M (2003) Parameter estimation for the calibration and variance stabilization of microarray data. Stat Appl in Genetics and Mol Biology 2(1), Article 3

Huggett J, Dheda K, Bustin S et al. (2005) Real-time RT-PCr normalisation; strategies and considerations Genes Immun 6(4), 279-284

Increase in National Hospital Discharge Survey rates for septicemia--United States, 1979-1987. MMWR Morb Mortal Wkly Rep 1990 39, 31-34

Johnson SB, Lissauer M, Bochicchio GV, Moore R, Cross AS, Scalea TM. (2007) Gene Expression Profiles Differentiate Between Sterile SIRS and Early Sepsis Annals of Surgery 245(4), 611-621

Knaus WA , Draper EA, Wagner DP, Zimmermann JE (1985) Prognosis in acute organ-system failure. Ann Surg 202, 658-693

Kofoed K, Andersen O, Kronborg G, Tvede M, Petersen J, Eugen-Olsen J, Larsen K (2007), Use of plasma C-reactive protein, procalcitonin, neutrophils, macrophage migration inhibitory factor, soluble urokinase-type plasminogen activator receptor, and soluble triggering receptor expressed on myeloid cells-1 in combination to diagnose infections: a prospective study. Critical Care 11, R38

Kubista M, Andrade JM, Bengtsson M et al. (2006) The real-time polymerase chain reaction. Mol Aspects Med 27, 95-125

Kumar A, Roberts D, Wood KE et al. (2006) Duration of hypothension before initiation of effective antimicrobial therapy is the critical determinant of survival in human septic shock. Crit Care Med 34(6), 1589-1596

Le-Gall JR, Lemeshow S, Leleu G, Klar J, Huillard J, Rue M, Teres D, Artigas A (1995) Customized probability models for early severe Sepsis in adult intensive care patients. Intensive Care Unit Scoring Group. JAMA 273, 644-650

Levy MM, Fink MP, Marshall JC, Abraham E, Angus D, Cook D, Cohen J, Opal SM, Vincent JL, Ramsay G et al. (2003) 2001 SCCM/ESICM/ACCP/ATS/SIS International Sepsis Definitions Conference. Intensive Care Med 29, 530-538

Liu H, Li J, Wong L (2005) Use of extreme patient samples for outcome prediction from gene expresseion data. Bioinformatics 21(16), 3377-3384

MAQC Consortium. The MicroArray Quality Control (MAQC) project shows inter-and intraplatform reproducibility of gene expression measurements. Nat Biotechnol 2006, 24:, 1151-61

Mathiak G, Kabir K, Grass G, et al. (2003) Lipopolysaccharides from different bacterial sources elicit disparate cytokine responses in whole blood assays. Int J Mol Med 11(1), 41-44

Marshall JC, Vincent JL, Fink MP et al. Measures, markers, and mediators: toward a staging system for clinical sepsis. A report of the Fifth Toronto Sepsis Roundtable, Toronto, Ontario, Canada, October 25-26, 2000 und Crit Care Med. 2003, 31: 1560-1567

Mayhall CG (2001) Ventilator-Associated Pneumonia or Not? Contemporary Diagnosis. Emerg Infect Dis 7(2), 200-204

Nolan T, Hands RE, Bustin SA (2006) Quantification of mRNA using realt-time RT-PCR. Nat Protoc 1(3), 1559-1582

Opal SM, Lim Y-P, Siryaporn E, et al. (2005) Longitudinal studies of inter-alpha inhibitor proteins in severly septic patients : A potential clinical marker and mediator of severe sepsis. Clin Invest 35(2), 387-292

Pachot A, Lepape A, Vey S, et al. (2006) Systemic transcriptional analysis in survivor and non-survivor septic shock patients: a preliminary study. Immunol Lett 106(1), 63-71. Epub 2006 May 17

Pile JC (2006) Evaluating postoperative fever: a focused approach. Clev Clin J Med. 73 (supp.1) 62-66

Pruitt K.D., Tatusova T., Maglott D.R. NCBI reference sequences (RefSeq): a curated non-redundant sequence database of genomes, transcripts and proteins. Nucleic Acids Res. 2007 Jan;35(Database issue):D61-5.

Ramilo O, Allman W, Chung W, Mejias A, Ardura M, Glaser C, Wittkowski KM, Piqueras P, Bancherau J, Palucka K A, Chaussabel D, (2007) Gene expression patterns in blood leukocytes discriminate patients with acute infections. Blood 109, 2066-2077

R Development Core Team (2006) R: A language and environment for statistical computing. R Foundation for Statistical Computing, Vienna, Austria. ISBN 3-900051-07-0, URL http://www.R-project.org

Rocke DM, Durbin B. (2001) A model for measurement error for gene expression arrays. J Comput Biol 8, 557-569

Roth AR, Basello DO, (2003) Approach to the adult patient with fever of unknown origin. Am Fam Phys 68(11) 2223-2228

Ruokonen E et al. (1999) Procalcitonin concentrations in patients with neutropenic fever. Eur J Clin Microbiol Infect Dis 18(4), 283-5

Ruokonen E et al. (2002) Procalcitonin and neopterin as indicators of infection in critically ill patients. Acta Anaesthesiol Scand 46(4), 398-404

Simon L, Gauvin F, Amre DK, Saint-Lois P, Lacroix J, (2004) Serum procalcitonin and C-reactive protein levels as marker of bacterial infection: A systematic review and meta analysis. Clin Infec Dis 39, 206-217

Simon R. (2005) Roadmap for Developing and Validating Therapeutically Relevant Genomic Classifiers. J Clin Oncol 23, 7332-7341

Sponholz C, Sakr Y, Reinhart K, Brunkhorst F, (2006) Diagnostic value and prognostic implications of serum procalcitonin after cardiac surgery: a systematic review of the literature, Critical Care 10, R145

Suprin E et al. (2000) Procalcitonin: a valuable indicator of infection in a medical ICU Intensive Care Med 26(9), 1232-1238

Tang BMP, Eslick GD, Craig JC et al. (2007a) Accuracy of procalcitonin for sepsis diagnosis in critically ill patients : systematic review and meta-analysis. Lancet Infect Dis 7, 210-217

Tang BMP, McLean AS, Dawes IW, Huang SJ, Lin RCY (2007b) The Use of Gene-Expression Profiling to Identify Candidate Genes In Human Sepsis. American Journal of Respiratory and Critical Care Medicine 176(7), 676-684 The NCBI handbook [Internet]. Bethesda (MD): National Library of Medicine (US), National Center for Biotechnology Information; 2002 Oct.

US 20060246495 Diagnosis of sepsis

US 6960439 Identification, monitoring and treatment of disease and characterization of biological condition using gene expression profiles

Vandesompele J, Preter De K, Pattyn F, et al. (2002) Accurate normalisation of real-time quantitative PCR data by geometric averaging of multiple internal control genes. Genome Biology 3(7), research0034.1-0034.11

Valasek MA, Repa JJ (2005) The power of real-time PCR. Advan Physiol Educ 29, 151-159

Vapnik V., (1999). The Nature of Statistical Learning Theory. Springer, New York.

Whitcombe D, Theaker J, Guy SP et al. (1999) Detection of PCR products using selfprobing amplicons and fouoreschence. Nat Biotechnol 17, 904-907

WO 2006/100203 Verwendung von Geneaktivitäts-Klassifikatoren für die in vitro Klassifizierung von Genexpressionsprofilen von Patienten mit infektiösem/nichtinfektiösem

WO 2004/087949 Verfahren zur Erkennung akuter, generalisierter entzündlicher Zustände (SIRS), Sepsis, sepsisähnlichen Zuständen und systemischen Infektionen

WO 2005/083115 Verfahren zur Erkennung von Sepsis

WO 2005/106020 Verfahren zur Erstellung von Kriterien zur Vorhersage des Krankheitsverlaufs bei Sepsis

WO 2006/042581 Verfahren zur Unterscheidung zwischen nichtinfektiösen und infektiösen Ursachen eines Multiorganversagens

WO 2007/144105 Verfahren zur Feststellung der lokalen Entzündung eines Fiebers unklarer Genese

WO 2007/124820 Verfahren zur in vitro-überwachung postoperativer Veränderungen nach Lebertransplantation

WO 2003/084388 Early detection of sepsis

Wong ML, Medrano JF (2005) Real-time PCR for mRNA quantification. Biotechniques 39(1) 1-11

Zeni F, Freeman B, Natanson C (1997) Anti-inflammatory therapies to treat sepsis and septic shock: A reassessment. Crit Care Med 25(7), 1095-1100

## Patentansprüche

**1.** Verfahren zur in vitro Erfassung und/oder Früherkennung und/oder Unterscheidung und/oder Verlaufsbeobachtung und/oder Beurteilung von pathophysiologischen Zuständen, ausgewählt aus der Gruppe bestehend aus: SIRS, Sepsis und deren Schweregraden; septischem Schock, wobei das Verfahren folgende Schritte umfasst:

a) Isolierung von Probennukleinsäuren aus einer von einem Patienten stammenden Blutprobe;
b) Bestimmung von Genaktivitäten mittels

b.1 der drei Polynukleotide M6, M9 und M15;
b.2 der vier Polynukleotide M6, M9, M15 und M7;
b.3 der fünf Polynukleotide M6, M9, M15, M7 und M2;
b.4 der sechs Polynukleotide M6, M9, M15, M7, M2 und M10;
b.5 der sieben Polynukleotide M6, M9, M15, M7, M2, M10 und M4;
b.6 der acht Polynukleotide M6, M9, M15, M7, M2, M10, M4 und M12;
b.7 der neun Polynukleotide M6, M9, M15, M7, M2, M10, M4, M12 und M17;
b.8 der zehn Polynukleotide M6, M9, M15, M7, M2, M10, M4, M12, M17 und M3;
b.9 der elf Polynukleotide M6, M9, M15, M7, M2, M10, M4, M12, M17, M3 und M8;
b.10 der zwölf Polynukleotide M6, M9, M15, M7, M2, M10, M4, M12, M17, M3, M8 und M13; oder
b.11 der dreizehn Polynukleotide M6, M9, M15, M7, M2, M10, M4, M12, M17, M3, M8, M13 und M16;und/oder deren Transkripten, zur Bildung wenigstens eines für die Erfassung und/oder Unterscheidung und/oder den Verlauf von pathophysiologischen Zuständen eines Patienten charakteristischen Multigenbiomarkers; wobei die Polynukleotide gemäß folgenden Tabellen definiert sind:

| Polynukleotid | Transkriptvariante | Accession Number | Seq ID |
|---|---|---|---|
| M2 | M2_1 | NM_001031700 | 4 |
| | M2_2 | NM_016613 | 25 |
| | M2_3 | NM_001128424 | 15 |
| M4 | M4_1 | NM_203330 | 35 |
| | M4_2 | NM_000611 | 3 |
| | M4_3 | NM_203329 | 34 |
| | M4_4 | NM_203331 | 36 |
| | M4_5 | NM_001127223 | 11 |
| | M4_6 | NM_001127225 | 12 |
| | M4_7 | NM_001127226 | 13 |
| | M4_8 | NM_001127227 | 14 |
| M6 | M6_1 | NM_001831 | 18 |
| | M6_2 | NM_203339 | 37 |
| M7 | M7_1 | NM_031311 | 28 |
| | M7_2 | NM_019049 | 26 |
| M9 | M9 | NM_006682 | 24 |
| M10 | M10 | NM_033554 | 32 |
| M15 | M15_1 | NM_003580 | 23 |
| | M15_2 | NM_001144772 | 16 |
| M3 | M3_A | NM_001123041 | 9 |
| | M3_B | NM_001123396 | 10 |
| M8 | M8 | NM_025209 | 27 |
| M12 | M12 | NM_002185 | 19 |
| M13 | M13 | NM_001080394 | 7 |
| M16 | M16 | NM_003268 | 22 |

(fortgesetzt)

| Polynukleotid | Transkriptvariante | Accession Number | Seq ID |
|---|---|---|---|
| M17 | M17 | NM_182491 | 33 |

| Marker und Referenzgen | Primer für quantitative PCR/resultierende Amplikon | Seq ID |
|---|---|---|
| M2 | M2-fw | 38 |
| | M2-rev | 39 |
| | M2-Amplikon | 40 |
| M4 | M4-fw | 41 |
| | M4-rev | 42 |
| | M4-Amplikon | 43 |
| M6 | M6-fw | 44 |
| | M6-rev | 45 |
| | M6-Amplikon | 46 |
| M7 | M7-fw | 47 |
| | M7-rev | 48 |
| | M7-Amplikon | 49 |
| M9 | M9-fw | 50 |
| | M9-rev | 51 |
| | M9-Amplikon | 52 |
| M10 | M10-fw | 53 |
| | M10-rev | 54 |
| | M10-Amplikon | 55 |
| M15 | M15-fw | 56 |
| | M15-rev | 57 |
| | M15-Amplikon | 58 |
| M3 | M3-fw | 59 |
| | M3-rev | 60 |
| | M3-Amplikon | 61 |
| M8 | M8-fw | 62 |
| | M8-rev | 63 |
| | M8-Amplikon | 64 |
| M12 | M12-fw | 65 |
| | M12-rev | 66 |
| | M12-Amplikon | 67 |

(fortgesetzt)

| Marker und Referenzgen | Primer für quantitative PCR/resultierende Amplikon | Seq ID |
|---|---|---|
| M13 | M13-fw | 68 |
| | M13-rev | 69 |
| | M13-Amplikon | 70 |
| M16 | M16-fw | 71 |
| | M16-rev | 72 |
| | M16-Amplikon | 73 |
| M17 | M17-fw | 74 |
| | M17-rev | 75 |
| | M17-Amplikon | 76 |

c) Bestimmung von Genaktivitäten wenigstens eines internen Referenzgens, auf die die unter b) bestimmten Genaktivitäten bezogen, insbesondere normalisiert, werden;
d) Bilden eines Wertes aus den einzelnen bestimmten Genaktivitäten des Multigenbiomarkers, der den pathophysiologischen Zustand anzeigt.

**2.** Verfahren nach Anspruch 1, **dadurch gekennzeichnet, dass** das Referenzgen ausgewählt wird aus den Gruppen R1, R2 und R3 und/oder deren Transkripten, wobei die Referenzgene gemäß folgenden Tabellen definiert sind:

| Polynukleotid | Transkriptvariante | Accession Number | Seq ID |
|---|---|---|---|
| R1 | R1_A | NM_001228 | 17 |
| | R1_B | NM_033355 | 29 |
| | R1_C | NM_033356 | 30 |
| | R1_E | NM_033358 | 31 |
| | R1_F | NM_001080124 | 5 |
| | R1_G | NM_001080125 | 6 |
| R2 | R2_1 | NM_002209 | 20 |
| | R2_2 | NM_001114380 | 8 |
| R3 | R3 | NM_003082 | 21 |

| Marker und Referenzgen | Primer für quantitative PCR/resultierende Amplikon | Seq ID |
|---|---|---|
| R1 | R1-fw | 77 |
| | R1-rev | 78 |
| | R1-Amplikon | 79 |
| R2 | R2-fw | 80 |
| | R2-rev | 81 |
| | R2-Amplikon | 82 |

(fortgesetzt)

| Marker und Referenzgen | Primer für quantitative PCR/resultierende Amplikon | Seq ID |
|---|---|---|
| R3 | R3-fw | 83 |
| | R3-rev | 84 |
| | R3-Amplikon | 85 |

3. Verfahren nach Anspruch 1 oder 2, **dadurch gekennzeichnet, dass** die Probennukleinsäure RNA, insbesondere Gesamt-RNA oder mRNA, oder DNA, insbesondere cDNA, ist.

4. Verwendung von

b.1 der drei Polynukleotide M6, M9 und M15;
b.2 der vier Polynukleotide M6, M9, M15 und M7;
b.3 der fünf Polynukleotide M6, M9, M15, M7 und M2;
b.4 der sechs Polynukleotide M6, M9, M15, M7, M2 und M10;
b.5 der sieben Polynukleotide M6, M9, M15, M7, M2, M10 und M4;
b.6 der acht Polynukleotide M6, M9, M15, M7, M2, M10, M4 und M12;
b.7 der neun Polynukleotide M6, M9, M15, M7, M2, M10, M4, M12 und M17;
b.8 der zehn Polynukleotide M6, M9, M15, M7, M2, M10, M4, M12, M17 und M3;
b.9 der elf Polynukleotide M6, M9, M15, M7, M2, M10, M4, M12, M17, M3 und M8;
b.10 der zwölf Polynukleotide M6, M9, M15, M7, M2, M10, M4, M12, M17, M3, M8 und M13; oder
b.11 der dreizehn Polynukleotide M6, M9, M15, M7, M2, M10, M4, M12, M17, M3, M8, M13 und M16; und/oder deren Transkripten, wobei die Polynukleotide gemäß folgenden Tabellen definiert sind:

| Polynukleotid | Transkriptvariante | Accession Number | Seq ID |
|---|---|---|---|
| M2 | M2_1 | NM_001031700 | 4 |
| | M2_2 | NM_016613 | 25 |
| | M2_3 | NM_001128424 | 15 |
| M4 | M4_1 | NM_203330 | 35 |
| | M4_2 | NM_000611 | 3 |
| | M4_3 | NM_203329 | 34 |
| | M4_4 | NM_203331 | 36 |
| | M4_5 | NM_001127223 | 11 |
| | M4_6 | NM_001127225 | 12 |
| | M4_7 | NM 001127226 | 13 |
| | M4_8 | NM_001127227 | 14 |
| M6 | M6_1 | NM_001831 | 18 |
| | M6_2 | NM_203339 | 37 |
| M7 | M7_1 | NM_031311 | 28 |
| | M7_2 | NM_019049 | 26 |
| M9 | M9 | NM_006682 | 24 |
| M10 | M10 | NM_033554 | 32 |
| M15 | M15_1 | NM_003580 | 23 |
| | M15_2 | NM_001144772 | 16 |

(fortgesetzt)

| Polynukleotid | Transkriptvariante | Accession Number | Seq ID |
|---|---|---|---|
| M3 | M3_A | NM_001123041 | 9 |
| | M3_B | NM_001123396 | 10 |
| M8 | M8 | NM_025209 | 27 |
| M12 | M12 | NM_002185 | 19 |
| M13 | M13 | NM_001080394 | 7 |
| M16 | M16 | NM_003268 | 22 |
| M17 | M17 | NM_182491 | 33 |

| Marker und Referenzgen | Primer für quantitative PCR/resultierende Amplikon | Seq ID |
|---|---|---|
| M2 | M2-fw | 38 |
| | M2-rev | 39 |
| | M2-Amplikon | 40 |
| M4 | M4-fw | 41 |
| | M4-rev | 42 |
| | M4-Amplikon | 43 |
| M6 | M6-fw | 44 |
| | M6-rev | 45 |
| | M6-Amplikon | 46 |
| M7 | M7-fw | 47 |
| | M7-rev | 48 |
| | M7-Amplikon | 49 |
| M9 | M9-fw | 50 |
| | M9-rev | 51 |
| | M9-Amplikon | 52 |
| M10 | M10-fw | 53 |
| | M10-rev | 54 |
| | M10-Amplikon | 55 |
| M15 | M15-fw | 56 |
| | M15-rev | 57 |
| | M15-Amplikon | 58 |
| M3 | M3-fw | 59 |
| | M3-rev | 60 |
| | M3-Amplikon | 61 |
| M8 | M8-fw | 62 |
| | M8-rev | 63 |
| | M8-Amplikon | 64 |

(fortgesetzt)

| Marker und Referenzgen | Primer für quantitative PCR/resultierende Amplikon | Seq ID |
|---|---|---|
| M12 | M12-fw | 65 |
| | M12-rev | 66 |
| | M12-Amplikon | 67 |
| M13 | M13-fw | 68 |
| | M13-rev | 69 |
| | M13-Amplikon | 70 |
| M16 | M16-fw | 71 |
| | M16-rev | 72 |
| | M16-Amplikon | 73 |
| M17 | M17-fw | 74 |
| | M17-rev | 75 |
| | M17-Amplikon | 76 |

zur Bildung wenigstens eines Multigenbiomarkers zur Herstellung eines Multiplex-Assays als Hilfsmittel zur in vitro Erfassung und/oder Früherkennung und/oder Unterscheidung und/oder Verlaufsbeobachtung und/oder Beurteilung von pathophysiologischen Zuständen eines Patienten, wobei der pathophysiologische Zustand ausgewählt wird aus der Gruppe bestehend aus: SIRS, Sepsis und deren Schweregraden; sowie septischem Schock.

**5.** Verwendung nach Anspruch 4, **dadurch gekennzeichnet, dass** der Multigenbiomarker eine Kombination von mehreren Polynukleotid-, insbesondere Gensequenzen ist, anhand deren Genaktivitäten mittels einer Interpretationsfunktion eine Klassifikation durchgeführt und/oder ein Index gebildet wird.

**6.** Verwendung nach Anspruch 5, **dadurch gekennzeichnet, dass** die Genaktivitäten mittels enzymatischer Verfahren, insbesondere Amplifikationsverfahren, bevorzugt Polymereasekettenreaktion (PCR), vorzugsweise Real-Time-PCR, insbesondere sondenbasierte Verfahren wie Taq-Man, Scorpions, Molecular Beacons; und/oder mittels Hybridisierungsverfahren, insbesondere solchen auf Microarrays; und/oder direkter mRNA-Nachweis, insbesondere Sequenzierung oder Massenspektrometrie; und/oder isothermale Amplifikation, erfasst werden.

**7.** Verwendung nach Anspruch 5 oder 6, **dadurch gekennzeichnet, dass** aus den einzelnen bestimmten Genaktivitäten ein Index gebildet wird, der nach entsprechender Kalibrierung ein Maß für den Schweregrad und/oder den Verlauf des pathophysiologischen Zustands ist, wobei vorzugsweise der Index auf einer leicht interpretierbaren Skala angezeigt wird.

**8.** Verwendung nach einem der Ansprüche 5 bis 7, **dadurch gekennzeichnet, dass** man die erhaltenen Genaktivitätsdaten zur Herstellung von Software für die Beschreibung mindestens eines pathophysiologischen Zustands und/oder einer Untersuchungsfrage und/oder als Hilfsmittel für Diagnosezwecke und/oder für Patientendatenmangement-Systeme, insbesondere für die Verwendung zur Patientenstratifikation und als Einschlusskriterium für klinische Studien, einsetzt.

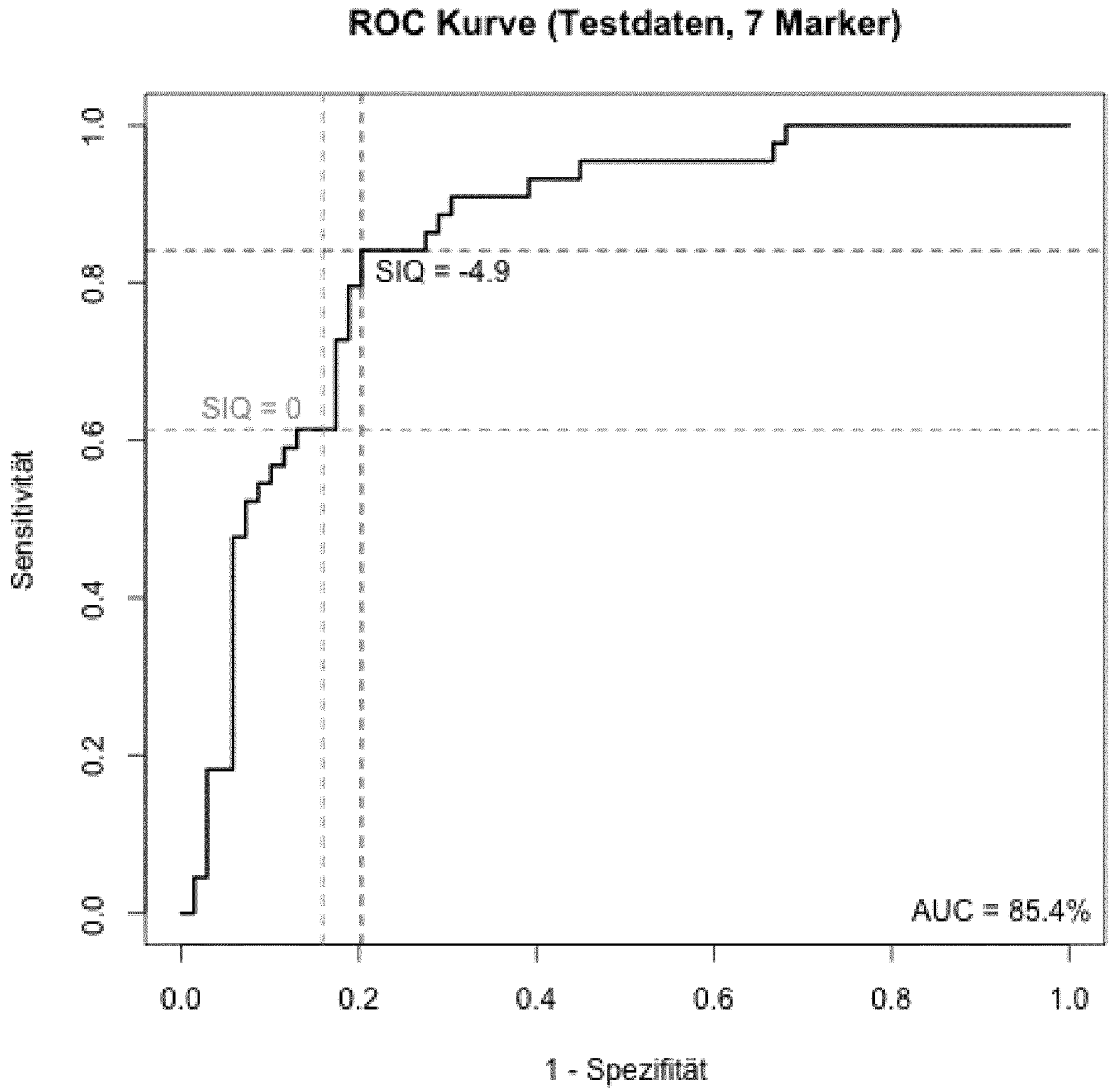
ROC Kurve (Testdaten, 7 Marker)
Sensitivität
1 - Spezifität
SIQ = -4.9
SIQ = 0
AUC = 85.4%
0.0
0.2
0.4
0.6
0.8
1.0

**Fig. 1**

(A)

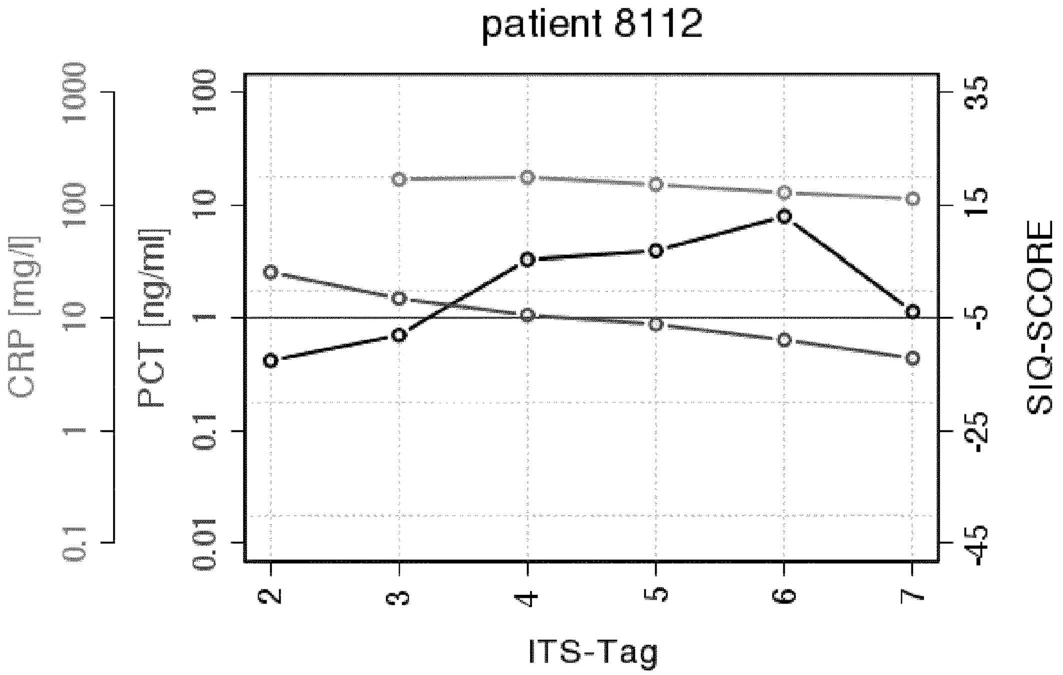
patient 8112
CRP [mg/l]
0.1
1
10
100
1000
PCT [ng/ml]
0.01
0.1
1
10
100
SIQ-SCORE
-45
-25
-5
15
35
2
3
4
5
6
7
ITS-Tag

(B)

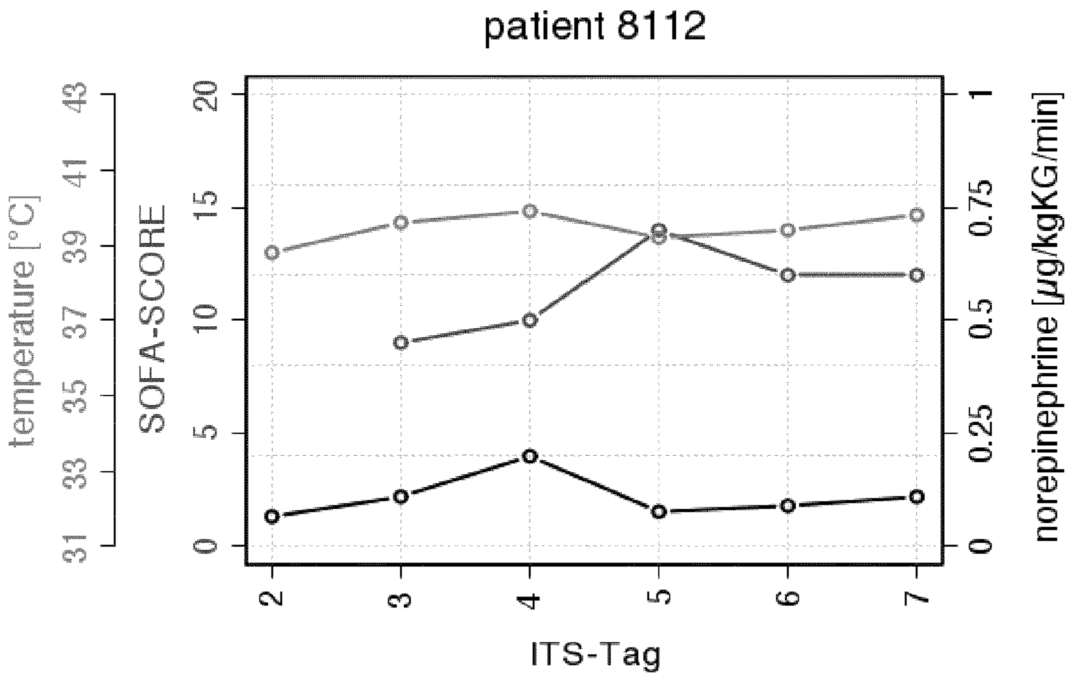
patient 8112
temperature [°C]
31
33
35
37
39
41
43
SOFA-SCORE
0
5
10
15
20
norepinephrine [μg/kgKG/min]
0
0.25
0.5
0.75
1
2
3
4
5
6
7
ITS-Tag

Fig. 2

(A)

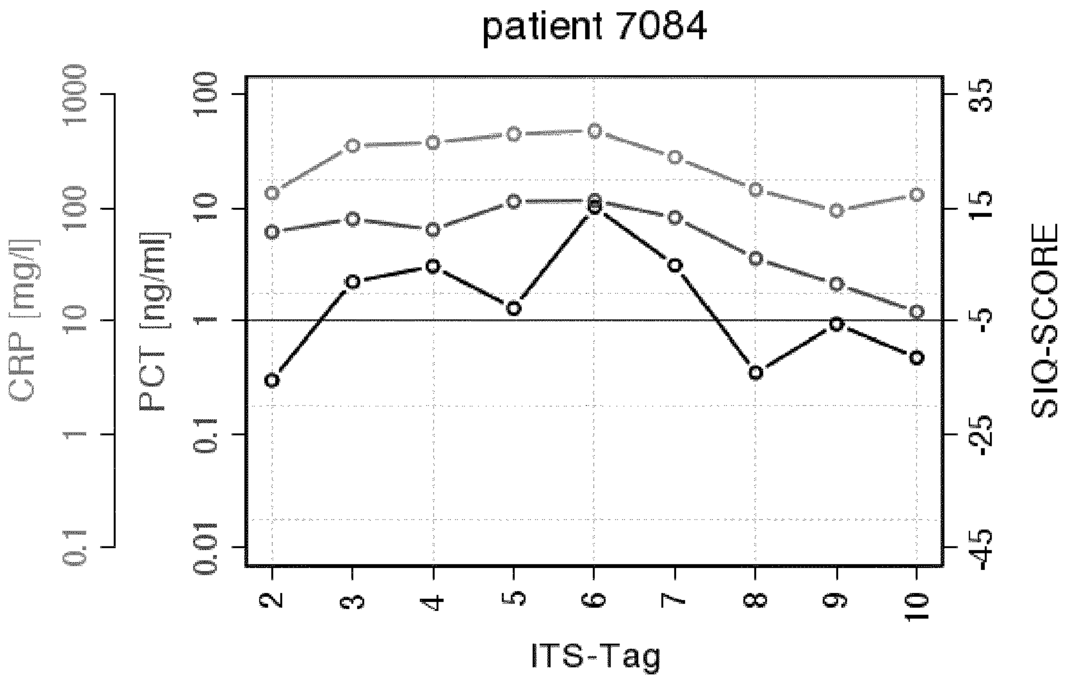
patient 7084
CRP [mg/l]
PCT [ng/ml]
SIQ-SCORE
ITS-Tag
0.1 1 10 100 1000
0.01 0.1 1 10 100
-45 -25 -5 15 35
2 3 4 5 6 7 8 9 10

(B)

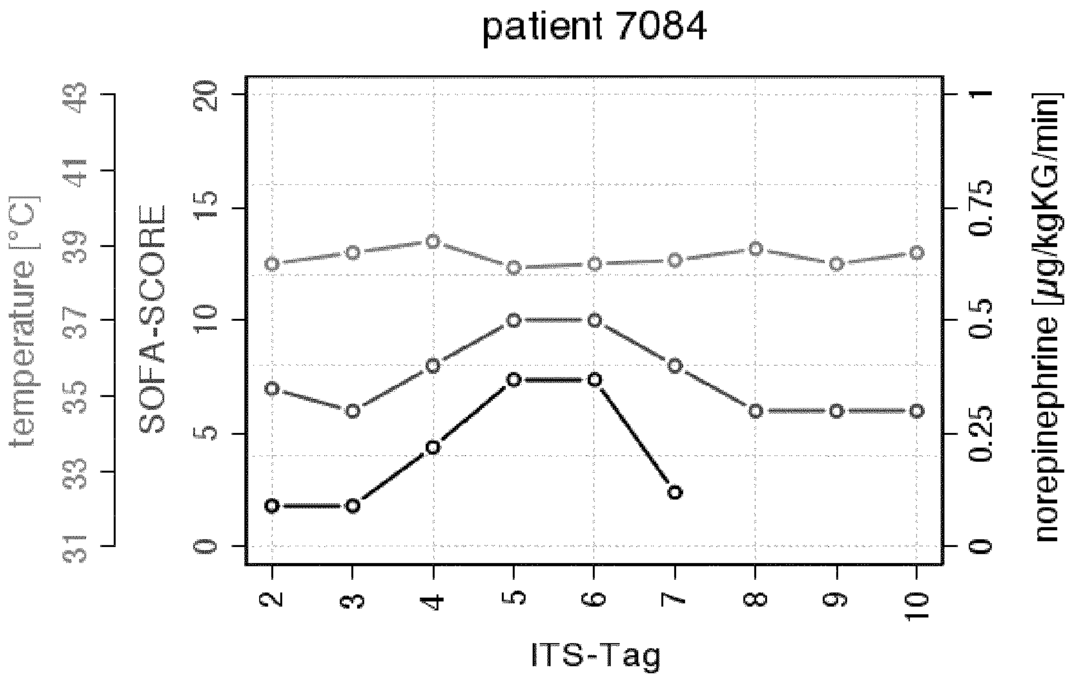
patient 7084
temperature [°C]
SOFA-SCORE
norepinephrine [μg/kgKG/min]
ITS-Tag
31 33 35 37 39 41 43
0 5 10 15 20
0 0.25 0.5 0.75 1
2 3 4 5 6 7 8 9 10

**Fig. 3**

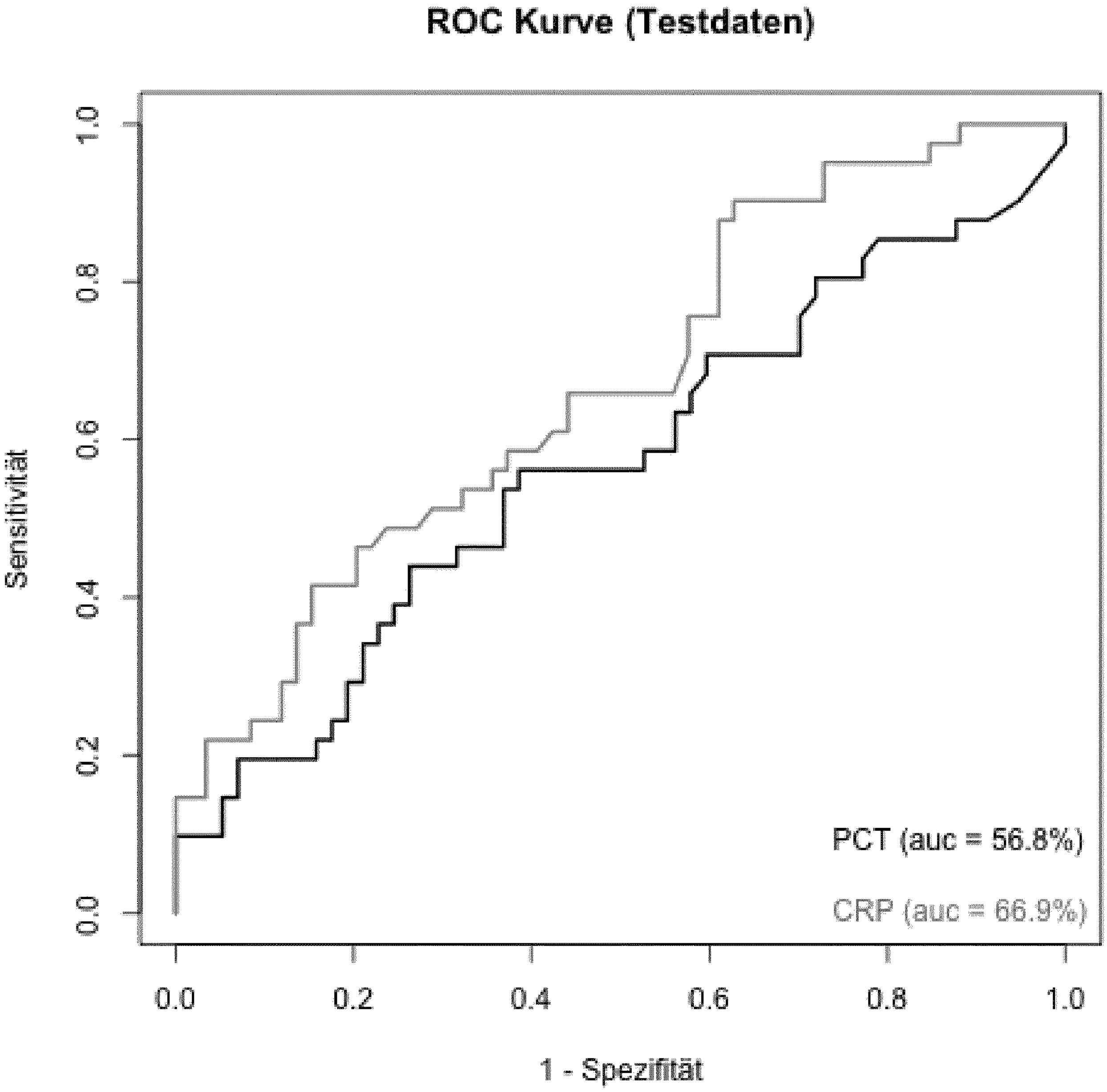
ROC Kurve (Testdaten)
Sensitivität
1.0
0.8
0.6
0.4
0.2
0.0
PCT (auc = 56.8%)
CRP (auc = 66.9%)
0.0
0.2
0.4
0.6
0.8
1.0
1 - Spezifität

Fig. 4

Europäisches Patentamt
European Patent Office
Office européen des brevets

# EUROPÄISCHER RECHERCHENBERICHT

Nummer der Anmeldung
EP 15 18 4023

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y<br>A | WO 2009/018962 A1 (SIRS LAB GMBH [DE]; RUSSWUM STEFAN [DE]) 12. Februar 2009 (2009-02-12)<br>* Seite 28 - Seite 33; Ansprüche 1-26; Tabelle 5; Sequenzen 45, 46, 62 *<br>* das ganze Dokument *<br>----- | 4-8<br>1-3 | INV. C12Q1/68 |
| Y | WO 2004/087949 A2 (SIRS LAB GMBH [DE]; RUSSWURM STEFAN [DE]; REINHART KONRAD [DE]; SALUZ) 14. Oktober 2004 (2004-10-14)<br>* Ansprüche 1-29; Sequenzen 2492,6421 *<br>* das ganze Dokument *<br>----- | 4-8 | |
| Y | WO 2005/106020 A1 (SIRS LAB GMBH [DE]; RUSSWURM STEFAN [DE]; DEIGNER HANS-PETER [DE]) 10. November 2005 (2005-11-10)<br>* Ansprüche 1--24; Sequenz 108 *<br>* das ganze Dokument *<br>----- | 4-8 | |
| A | WO 2008/107114 A2 (SIRS LAB GMBH [DE]; RUSSWURM STEFAN [DE]; SALUZ HANS PETER [DE]; DEIGN) 12. September 2008 (2008-09-12)<br>* das ganze Dokument *<br>----- | 2 | RECHERCHIERTE SACHGEBIETE (IPC)<br>C12Q |
| A | WO 2004/103294 A2 (TANOX INC [US]; FUNG SEK CHUNG [US]; MOLLNES TOM EIRIK [NO]) 2. Dezember 2004 (2004-12-02)<br>* Anspruch 2 *<br>-----<br>-/-- | 1-8 | |

Der vorliegende Recherchenbericht wurde für alle Patentansprüche erstellt

1

| Recherchenort | Abschlußdatum der Recherche | Prüfer |
|---|---|---|
| München | 7. Januar 2016 | Schmitt, Anja |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.....
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 15 18 4023

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | S. SHRIVASTAVA ET AL: "The Interface Between Coagulation and Immunity", AMERICAN JOURNAL OF TRANSPLANTATION, Bd. 7, Nr. 3, 1. März 2007 (2007-03-01), Seiten 499-506, XP055236693, DK ISSN: 1600-6135, DOI: 10.1111/j.1600-6143.2006.01653.x * das ganze Dokument * ----- | 1-8 | |
| A | F.P.H.TH.M A A.N R C.E R.J ROMIJN STURK BOING NIEUWLAND HACK BERCKMANS ET AL: "Cell-derived microparticles circulate in healthy humans and support low grade thrombin generation", THROMBOSIS AND HAEMOSTASIS, Bd. 85, Nr. 4, 1. Januar 2001 (2001-01-01) , Seiten 639-646, XP055236696, DE ISSN: 0340-6245 * das ganze Dokument * ----- | 1-8 | RECHERCHIERTE SACHGEBIETE (IPC) |
| A | ANAND GHANEKAR ET AL: "Endothelial induction of fgl2 contributes to thrombosis during acute vascular xenograft rejection", THE JOURNAL OF IMMUNOLOGY, THE AMERICAN ASSOCIATION OF IMMUNOLOGISTS, US, Bd. 172, Nr. 9, 1. Mai 2004 (2004-05-01), Seiten 5693-5701, XP002659723, ISSN: 0022-1767 * das ganze Dokument * ----- | 1-8 | |
| E | WO 2009/115478 A2 (SIRS LAB GMBH [DE]; RUSSWURM STEFAN [DE]) 24. September 2009 (2009-09-24) * Ansprüche 1-15; Tabelle 32 * ----- | 1-8 | |

Der vorliegende Recherchenbericht wurde für alle Patentansprüche erstellt

1

| Recherchenort | Abschlußdatum der Recherche | Prüfer |
|---|---|---|
| München | 7. Januar 2016 | Schmitt, Anja |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 15 18 4023

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-01-2016

| Im Recherchenbericht angeführtes Patentdokument | Datum der Veröffentlichung | Mitglied(er) der Patentfamilie | Datum der Veröffentlichung |
|---|---|---|---|
| WO 2009018962 A1 | 12-02-2009 | CA 2699433 A1 | 12-02-2009 |
| | | DE 102007036678 A1 | 05-02-2009 |
| | | EP 2179054 A1 | 28-04-2010 |
| | | JP 2010535017 A | 18-11-2010 |
| | | US 2010203534 A1 | 12-08-2010 |
| | | WO 2009018962 A1 | 12-02-2009 |
| WO 2004087949 A2 | 14-10-2004 | EP 1611255 A2 | 04-01-2006 |
| | | EP 2366799 A2 | 21-09-2011 |
| | | US 2008070235 A1 | 20-03-2008 |
| | | WO 2004087949 A2 | 14-10-2004 |
| WO 2005106020 A1 | 10-11-2005 | CA 2561817 A1 | 10-11-2005 |
| | | DE 102004015605 A1 | 20-10-2005 |
| | | US 2010086909 A1 | 08-04-2010 |
| | | WO 2005106020 A1 | 10-11-2005 |
| WO 2008107114 A2 | 12-09-2008 | AT 493517 T | 15-01-2011 |
| | | CA 2679017 A1 | 12-09-2008 |
| | | DE 102007010252 A1 | 04-09-2008 |
| | | EP 2118315 A2 | 18-11-2009 |
| | | EP 2392668 A2 | 07-12-2011 |
| | | ES 2358182 T3 | 06-05-2011 |
| | | ES 2524643 T3 | 10-12-2014 |
| | | JP 5430407 B2 | 26-02-2014 |
| | | JP 2010519893 A | 10-06-2010 |
| | | US 2010184608 A1 | 22-07-2010 |
| | | WO 2008107114 A2 | 12-09-2008 |
| WO 2004103294 A2 | 02-12-2004 | AU 2004241069 A1 | 02-12-2004 |
| | | AU 2010249190 A1 | 06-01-2011 |
| | | CA 2524534 A1 | 02-12-2004 |
| | | CN 1787741 A | 14-06-2006 |
| | | CN 102258784 A | 30-11-2011 |
| | | DK 1628530 T3 | 23-07-2012 |
| | | EP 1628530 A2 | 01-03-2006 |
| | | EP 2266606 A1 | 29-12-2010 |
| | | ES 2387275 T3 | 19-09-2012 |
| | | ES 2522525 T3 | 14-11-2014 |
| | | JP 4768620 B2 | 07-09-2011 |
| | | JP 2006528981 A | 28-12-2006 |
| | | JP 2011105747 A | 02-06-2011 |
| | | MX PA05011886 A | 17-02-2006 |
| | | US 2007274989 A1 | 29-11-2007 |
| | | WO 2004103294 A2 | 02-12-2004 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR. EP 15 18 4023

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-01-2016

| Im Recherchenbericht angeführtes Patentdokument | Datum der Veröffentlichung | Mitglied(er) der Patentfamilie | Datum der Veröffentlichung |
|---|---|---|---|
| WO 2009115478 A2 | 24-09-2009 | CA 2718741 A1 | 24-09-2009 |
| | | DE 102008000715 A1 | 24-09-2009 |
| | | GB 2470707 A | 01-12-2010 |
| | | JP 2011517401 A | 09-06-2011 |
| | | US 2011098195 A1 | 28-04-2011 |
| | | WO 2009115478 A2 | 24-09-2009 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2004087949 A
- DE 102007036678
- WO 2007124820 A
- WO 03084388 A
- US 6960439 B
- WO 2005083115 A
- WO 05106020 A
- WO 2006042581 A
- WO 2006100203 A
- WO 2007144105 A
- US 20060246495 A
- WO 2005106020 A
- WO 2003084388 A


### In der Beschreibung aufgeführte Nicht-Patentliteratur


- **ALTSCHUL et al.** *J Mol Biol,* 1990, vol. 215, 403-410
- *Crit. Care Med,* 1992, vol. 20, 864-74
- **ALBERTI C ; BRUN-BUISSON C ; GOODMAN SV ; GUIDICI D ; GRANTON J ; MORENO R ; SMITHIES M ; THOMAS O ; ARTIGAS A ; LE GALL JR.** European Sepsis Group (2003) Influence of systemic inflammatory response syndrome and Sepsis on outcome of critically ill infected patients. *Am J Respir Crit Care Med,* 2003, vol. 168, 77-84
- **AMIN K ; KAUFFMAN CA.** Fever of unknown origin. *Postgrad med,* 2003, vol. 114 (3), 69-75
- **BAKER SG ; KRAMER B.** Identifying genes that contribute most to good classification in microarray. *BMC Bioinformatics,* 2006, vol. 7, 407
- **BENSON D.A. ; KARSCH-MIZRACHI I. ; LIPMAN D.J. ; OSTELL J. ; SAYERS E.W.** *Nucleic Acids Res.,* Januar 2009, vol. 37, D26-31
- **BLAKE PG.** Complicated peritonitis - the biggest cause of technique failiure. *Perit Dial Int.,* 2008, vol. 28 (4), 327-328
- **BONE RC ; BALK RA ; CERRA FB ; DELLINGER EP ; FEIN AM ; KNAUS WA ; SCHEIN RM ; SIBBALD WJ.** the ACCP/SCCM Consensus Conference Committee (1992) Definitions for Sepsis and organ failure and guidelines for the use of innovative therapies in Sepsis. *Chest,* 1992, vol. 101, 1656-1662
- **BOX GEP ; COX DR.** An analysis of transformations (with discussion). *J Roy Stat Soc B,* 1964, vol. 26, 211-252
- **BUNEß A ; HUBER W ; STEINER K ; SÜLTMANN H ; POUSTKA A.** ArrayMagic: twocolour cDNA microarray quality control and preprocessing. *Bioinformatics,* 2005, vol. 21, 554-556
- **BREIMAN L.** Random Forests. *Machine Learning,* 2001, vol. 45 (1), 5-32
- **BRUN-BUISSON C ; DOYON F ; CARLET J ; DELLAMONICA P ; GOUIN F ; LEPOUTRE A ; MERCIER JC ; OFFENSTADT G ; REGNIER B.** Incidence, risk factors, and outcome of severe Sepsis and septic shock in adults. A multicenter prospective study in intensive care units. French ICU Group for Severe Sepsis. *JAMA,* 1995, vol. 274, 968-974
- **BRUN-BUISSON C ; ROUDOT-THORAVAL F ; GIROU E ; GRENIER-SENNELIER C ; DURAND-ZALESKI I.** The costs of septic syndromes in the intensive care unit and influence of hospital-acquired Sepsis. *Intensive Care Med,* 2003, vol. 29, 1464-1471
- **BUSTIN SA.** Quantification of mRNA using real-time reverse transcription PCR (RT-PCR): trends and problems. *J Mol Endicronol,* 2002, vol. 29, 23-29
- **CALANDRA T ; COHEN J.** International Sepsis Forum Definition of Infection in the ICU Consensus Conference. *Critical Care Med,* 2005, vol. 33 (7), 1538-48
- **CARRIGAN SD ; SCOTT G ; TABRIZIAN M.** Toward resolving the challenges of Sepsis. *Clin Chem,* 2004, vol. 50 (8), 1301-1314
- **DING BY ; GENTLEMAN R.** Classification using generalized partial least squares. *Bioconductor Project Working Papers,* 2004, http://www.begress.com/bioconductor/paper5 9
- Bootstrap Methods: Another Look at the Jackknife. **EFRON B.** The Annals of Statistics. 1979, vol. 7, 1-26
- **FAWCETT, T.** An introduction to ROC analysis. *Pattern Recognition Letters,* 2006, vol. 27, 861-874
- In Vitro Diagnostic Multivariate Index Assays. *Draft Guidance for Industry,* 2003, http://www.fda.gov/cdrh/oivd/guidance/1610.pdf

- **FEEZOR RJ ; BAKER HV ; XIAO W et al.** Genomic and Proteomic Determinants of Outcome in Patients Untergoing Thoracoabdominal Aortic Aneurysm Repair. *J Immun,* 2004, vol. 172, 7103-7109
- **KLEIN D.** Quantification using real-time PCR technology: applications and limitations. *Trends Mol Med,* 2002, vol. 8 (6), 257-260
- **KOULAOUZIDIS A ; BHAT S ; SAEED AA.** Spontaneous bacterial peritonitis. *World J Gastroenterol.,* 2009, vol. 15 (9), 1042-9
- **MAYHALL G.** Ventilator-Associated Pneumonia or Not? Contemporary Diagnosis. *Emerging Infection Disease CDC,* 2001, vol. 7 (2
- **GOLUB TR ; SLONIM DK ; TAMAYO P et al.** Molecular classification of cancer: class discovery and class prediction by gene expression monitoring. *Science,* 1999, vol. 286, 531-537
- **T. HASTIE ; R.TIBSHIRANI ; J. FRIEDMAN.** The Elements of Statistical Learning. *Springer series in statistics,* 2001, ISBN 0-387-95284-5
- **HOCKING, R. R.** The Analysis and Selection of Variables in Linear Regression. *Biometrics,* 1976, 32
- **HOLLANDER M. ; WOLFE D.** Nonparametric statistical inference. John Wiley & Sons, 1973
- **HUBER W ; VON HEYDEBRECK A ; SUELTMANN H ; POUSTKA A ; VINGRON M.** Parameter estimation for the calibration and variance stabilization of microarray data. *Stat Appl in Genetics and Mol Biology,* 2003, vol. 2 (1
- **HUGGETT J ; DHEDA K ; BUSTIN S et al.** Real-time RT-PCr normalisation; strategies and considerations. *Genes Immun,* 2005, vol. 6 (4), 279-284
- Increase in National Hospital Discharge Survey rates for septicemia--United States. *1979-1987. MMWR Morb Mortal Wkly Rep,* 1990, vol. 39, 31-34
- Gene Expression Profiles Differentiate Between Sterile SIRS and Early Sepsis. **JOHNSON SB ; LISSAUER M ; BOCHICCHIO GV ; MOORE R ; CROSS AS ; SCALEA TM.** Annals of Surgery. 2007, vol. 245, 611-621
- **KNAUS WA ; DRAPER EA ; WAGNER DP ; ZIMMERMANN JE.** Prognosis in acute organ-system failure. *Ann Surg,* 1985, vol. 202, 658-693
- **KOFOED K ; ANDERSEN O ; KRONBORG G ; TVEDE M ; PETERSEN J ; EUGEN-OLSEN J ; LARSEN K.** Use of plasma C-reactive protein, procalcitonin, neutrophils, macrophage migration inhibitory factor, soluble urokinase-type plasminogen activator receptor, and soluble triggering receptor expressed on myeloid cells-1 in combination to diagnose infections: a prospective study. *Critical Care,* 2007, vol. 11, R38
- **KUBISTA M ; ANDRADE JM ; BENGTSSON M et al.** The real-time polymerase chain reaction. *Mol Aspects Med,* 2006, vol. 27, 95-125
- **KUMAR A ; ROBERTS D ; WOOD KE et al.** Duration of hypothension before initiation of effective antimicrobial therapy is the critical determinant of survival in human septic shock. *Crit Care Med,* 2006, vol. 34 (6), 1589-1596
- **LE-GALL JR ; LEMESHOW S ; LELEU G ; KLAR J ; HUILLARD J ; RUE M ; TERES D ; ARTIGAS A.** Customized probability models for early severe Sepsis in adult intensive care patients. Intensive Care Unit Scoring Group. *JAMA,* 1995, vol. 273, 644-650
- **LEVY MM ; FINK MP ; MARSHALL JC ; ABRAHAM E ; ANGUS D ; COOK D ; COHEN J ; OPAL SM ; VINCENT JL ; RAMSAY G et al.** 2001 SCCM/ESICM/ACCP/ATS/SIS International Sepsis Definitions Conference. *Intensive Care Med,* 2003, vol. 29, 530-538
- **LIU H ; LI J ; WONG L.** Use of extreme patient samples for outcome prediction from gene expresseion data. *Bioinformatics,* 2005, vol. 21 (16), 3377-3384
- The MicroArray Quality Control (MAQC) project shows inter-and intraplatform reproducibility of gene expression measurements. *Nat Biotechnol,* 2006, vol. 24, 1151-61
- **MATHIAK G ; KABIR K ; GRASS G et al.** Lipopolysaccharides from different bacterial sources elicit disparate cytokine responses in whole blood assays. *Int J Mol Med,* 2003, vol. 11 (1), 41-44
- **MARSHALL JC ; VINCENT JL ; FINK MP et al.** *Measures, markers, and mediators: toward a staging system for clinical sepsis. A report of the Fifth Toronto Sepsis Roundtable,* 25. Oktober 2000
- *Crit Care Med.,* 2003, vol. 31, 1560-1567
- **MAYHALL CG.** Ventilator-Associated Pneumonia or Not? Contemporary Diagnosis. *Emerg Infect Dis,* 2001, vol. 7 (2), 200-204
- **NOLAN T ; HANDS RE ; BUSTIN SA.** Quantification of mRNA using realt-time RT-PCR. *Nat Protoc,* 2006, vol. 1 (3), 1559-1582
- **OPAL SM ; LIM Y-P ; SIRYAPORN E et al.** Longitudinal studies of inter-alpha inhibitor proteins in severly septic patients : A potential clinical marker and mediator of severe sepsis. *Clin Invest,* 2005, vol. 35 (2), 387-292
- **PACHOT A ; LEPAPE A ; VEY S et al.** Systemic transcriptional analysis in survivor and non-survivor septic shock patients: a preliminary study. *Immunol Lett,* 17. Mai 2006, vol. 106 (1), 63-71
- **PILE JC.** Evaluating postoperative fever: a focused approach. *Clev Clin J Med.,* 2006, vol. 73 (1), 62-66
- **PRUITT K.D. ; TATUSOVA T. ; MAGLOTT D.R.** NCBI reference sequences (RefSeq): a curated non-redundant sequence database of genomes, transcripts and proteins. *Nucleic Acids Res.,* Januar 2007, vol. 35, D61-5

- **RAMILO O ; ALLMAN W ; CHUNG W ; MEJIAS A ; ARDURA M ; GLASER C ; WITTKOWSKI KM ; PIQUERAS P ; BANCHERAU J ; PALUCKA K A.** Gene expression patterns in blood leukocytes discriminate patients with acute infections. *Blood,* 2007, vol. 109, 2066-2077
- R: A language and environment for statistical computing. *R Foundation for Statistical Computing, Vienna, Austria,* 2006, ISBN 3-900051-07-0, http://www.R-project.org
- **ROCKE DM ; DURBIN B.** A model for measurement error for gene expression arrays. *J Comput Biol,* 2001, vol. 8, 557-569
- **ROTH AR ; BASELLO DO.** Approach to the adult patient with fever of unknown origin. *Am Fam Phys,* 2003, vol. 68 (11), 2223-2228
- **RUOKONEN E et al.** Procalcitonin concentrations in patients with neutropenic fever. *Eur J Clin Microbiol Infect Dis,* 1999, vol. 18 (4), 283-5
- **RUOKONEN E et al.** Procalcitonin and neopterin as indicators of infection in critically ill patients. *Acta Anaesthesiol Scand,* 2002, vol. 46 (4), 398-404
- **SIMON L ; GAUVIN F ; AMRE DK ; SAINT-LOIS P ; LACROIX J.** Serum procalcitonin and C-reactive protein levels as marker of bacterial infection: A systematic review and meta analysis. *Clin Infec Dis,* 2004, vol. 39, 206-217
- **SIMON R.** Roadmap for Developing and Validating Therapeutically Relevant Genomic Classifiers. *J Clin Oncol,* 2005, vol. 23, 7332-7341
- **SPONHOLZ C ; SAKR Y ; REINHART K ; BRUNKHORST F.** Diagnostic value and prognostic implications of serum procalcitonin after cardiac surgery: a systematic review of the literature. *Critical Care,* 2006, vol. 10, R145
- **SUPRIN E et al.** Procalcitonin: a valuable indicator of infection in a medical ICU. *Intensive Care Med,* 2000, vol. 26 (9), 1232-1238
- **TANG BMP ; ESLICK GD ; CRAIG JC et al.** Accuracy of procalcitonin for sepsis diagnosis in critically ill patients : systematic review and meta-analysis. *Lancet Infect Dis,* 2007, vol. 7, 210-217
- **TANG BMP ; MCLEAN AS ; DAWES IW ; HUANG SJ ; LIN RCY.** The Use of Gene-Expression Profiling to Identify Candidate Genes In Human Sepsis. *American Journal of Respiratory and Critical Care Medicine,* 2007, vol. 176 (7), 676-684
- The NCBI handbook. National Center for Biotechnology Information, Oktober 2002
- **VANDESOMPELE J ; PRETER DE K ; PATTYN F et al.** Accurate normalisation of real-time quantitative PCR data by geometric averaging of multiple internal control genes. *Genome Biology,* 2002, vol. 3 (7
- **VALASEK MA ; REPA JJ.** The power of real-time PCR. *Advan Physiol Educ,* 2005, vol. 29, 151-159
- **VAPNIK V.** The Nature of Statistical Learning Theory. Springer, 1999
- **WHITCOMBE D ; THEAKER J ; GUY SP et al.** Detection of PCR products using selfprobing amplicons and fouoreschence. *Nat Biotechnol,* 1999, vol. 17, 904-907
- **WONG ML ; MEDRANO JF.** Real-time PCR for mRNA quantification. *Biotechniques,* 2005, vol. 39 (1), 1-11
- **ZENI F ; FREEMAN B ; NATANSON C.** Anti-inflammatory therapies to treat sepsis and septic shock: A reassessment. *Crit Care Med,* 1997, vol. 25 (7), 1095-1100

SEQUENCE LISTING

<110>  SIRS-Lab GmbH

<120>  Verfahren zur in vitro Erfassung und Untersuchung von pathophysiologischen Zuständen infektiöser und nicht-infektiöser Genese

<130>

<140>  SL0636
<141>

<160>  85

<170>  Patent Prepare  0.6.0

<210>  1
<211>  267
<212>  DNA
<213>  Homo sapiens

<400>  1
tttgagaatt gaaactttga tttattggct aggggagaga gatgaaaaga ggatcactct     60

ggtagcagga gttttgggca tgtccctctg agatccattt gatgaaaaca ttttttaaaa    120

catggtaggg cctcattttg cacattttct tcacggtgtc atcaaataaa catcatggta    180

aatataaact tgttcgcccg tcagaaagcg gcgccgggag ggagcgagcg tgagaaagtg    240

agcgtgtgcg ctgccggaga gcacgac                                        267

<210>  2
<211>  581
<212>  DNA
<213>  Homo sapiens

<400>  2
atatatccag tgctttgcaa gtgctctgcg caccttgtct cactccatcc tgacaataat     60

cctgggaggt gtgtgcaatt actacgacta ctctcttttt tatagatcat taaattcaga    120

actaaggagt taagtaactt gtccaagttg ttcacacagt gaagggaggg gccaagatat    180

gatggctggg agtctaattg cagttccctg agccatgtgc ctttctcttc actgaggact    240

gccccattct tgagtgccaa acgtcactag taacagggtg tgcctagata atttatgatc    300

caaactgagt cagtttggaa agtgaaaggg aaacttacat ataatccctc cgggacaatg    360

agcaaaaact aggactgtcc ccagacaaat gtgaacatac atatcatcac ttaaattaaa    420

atggctatga gaaagaaaga gggggagaaa cagtcttgcg ggtgtgaagt cccatgacca    480

gccatgtcaa aagaaggtaa agaagtcaag aaaaagccat gaagcccatt tgatttcatt    540

tttctgaaaa taggctcaag agggaataaa ttagaaactc a                        581

<210>  3
<211>  7635
<212>  mRNA

<213>  Homo sapiens

<400>  3

```
gggggccgggg ggcggagcct tgcgggctgg agcgaaagaa tgcgggggct gagcgcagaa      60
gcggctcgag gctggaagag gatcttgggc gccgccaggt tctgtggaca atcacaatgg     120
gaatccaagg agggtctgtc ctgttcgggc tgctgctcgt cctggctgtc ttctgccatt     180
caggtcatag cctgcagtgc tacaactgtc ctaacccaac tgctgactgc aaaacagccg     240
tcaattgttc atctgatttt gatgcgtgtc tcattaccaa agctgggtta caagtgtata     300
acaagtgttg gaagtttgag cattgcaatt tcaacgacgt cacaacccgc ttgagggaaa     360
atgagctaac gtactactgc tgcaagaagg acctgtgtaa ctttaacgaa cagcttgaaa     420
atggtgggac atccttatca gagaaaacag ttcttctgct ggtgactcca tttctggcag     480
cagcctggag ccttcatccc taagtcaaca ccaggagagc ttctcccaaa ctccccgttc     540
ctgcgtagtc cgctttctct tgctgccaca ttctaaaggc ttgatatttt ccaaatggat     600
cctgttggga aagaataaaa ttagcttgag caacctggct aagatagagg ggctctggga     660
gactttgaag accagtcctg tttgcaggga agccccactt gaaggaagaa gtctaagagt     720
gaagtaggtg tgacttgaac tagattgcat gcttcctcct ttgctcttgg gaagaccagc     780
tttgcagtga cagcttgagt gggttctctg cagccctcag attatttttc ctctggctcc     840
ttggatgtag tcagttagca tcattagtac atctttggag ggtggggcag gagtatatga     900
gcatcctctc tcacatggaa cgctttcata aacttcaggg atcccgtgtt gccatggagg     960
catgccaaat gttccatatg tgggtgtcag tcagggacaa caagatcctt aatgcagagc    1020
tagaggactt ctggcaggga agtggggaag tgttccagat agcagggcat gaaaacttag    1080
agaggtacaa gtggctgaaa atcgagtttt tcctctgtct ttaaatttta tatgggcttt    1140
gttatcttcc actggaaaag tgtaatagca tacatcaatg gtgtgttaaa gctatttcct    1200
tgcctttttt ttattggaat ggtaggatat cttggctttg ccacacacag ttacagagtg    1260
aacactctac tacatgtgac tggcagtatt aagtgtgctt attttaaatg ttactggtag    1320
aaaggcagtt caggtatgtg tgtatatagt atgaatgcag tggggacacc ctttgtggtt    1380
acagtttgag acttccaaag gtcatcctta ataacaacag atctgcaggg gtatgtttta    1440
ccatctgcat ccagcctcct gctaactcct agctgactca gcatagattg tataaaatac    1500
ctttgtaacg gctcttagca cactcacaga tgtttgaggc tttcagaagc tcttctaaaa    1560
aatgatacac acctttcaca agggcaaact tttccttttt ccctgtgtat tctagtgaat    1620
gaatctcaag attcagtaga cctaatgaca tttgtatttt atgatcttgg ctgtatttaa    1680
tggcataggc tgacttttgc agatggagga atttcttgat taatgttgaa aaaaaaccct    1740
tgattatact ctgttggaca aaccgagtgc aatgaatgat gcttttctga aaatgaaata    1800
```

taacaagtgg gtgaatgtgg ttatggccga aaaggatatg cagtatgctt aatggtagca 1860
actgaaagaa gacatcctga gcagtgccag ctttcttctg ttgatgccgt tccctgaaca 1920
taggaaaata gaaacttgct tatcaaaact tagcattacc ttggtgctct gtgttctctg 1980
ttagctcagt gtctttcctt acatcaatag gttttttttt ttttttttgg cctgaggaag 2040
tactgaccat gcccacagcc accggctgag caaagaagct catttcatgt gagttctaag 2100
gaatgagaaa caattttgat gaatttaagc agaaaatgaa tttctgggaa ctttttttggg 2160
ggcggggggg tggggaattc agccacactc cagaaagcca ggagtcgaca gttttggaag 2220
cctctctcag gattgagatt ctaggatgag attggcttac tgctatcttg tgtcatgtac 2280
ccactttttg gccagactac actgggaaga aggtagtcct ctaaagcaaa atctgagtgc 2340
cactaaatgg ggagatgggg ctgttaagct gtccaaatca acaagggtca tataaatggc 2400
cttaaacttt ggggttgctt tctgcaaaaa gttgctgtga ctcatgccat agacaaggtt 2460
gagtgcctgg acccaaaggc aatactgtaa tgtaaagaca tttatagtac taggcaaaca 2520
gcaccccagg tactccaggc cctcctggct ggagagggct gtggcaatag aaaattagtg 2580
ccaactgcag tgagtcagcc taggttaaat agagagtgta agagtgctgg acaggaacct 2640
ccaccctcat gtcacatttc ttcaatgtga cccttctggc ccctctcctc ctgacagcgg 2700
aacaatgact gccccgatag gtgaggctgg aggaagaatc agtcctgtcc ttggcaagct 2760
cttcactatg acagtaaagg ctctctgcct gctgccaagg cctgtgactt tctaacctgg 2820
cctcacgctg ggtaagctta aggtagaggt gcaggattag caagcccacc tggctaccag 2880
gccgacagct acatcctcca actgaccctg atcaacgaag agggattcat gtgtctgtct 2940
cagttggttc caaatgaaac cagggagcag gggagttagg aatcgaacac cagtcatgcc 3000
tactggctct ctgctcgaga gccaataccc tgtgccctcc actcatctgg atttacagga 3060
actgtcatag tgttcagtat tgggtggtga taagcccatt ggattgtccc cttgggggga 3120
tgagctaggg gtgcaaggaa cacctgatga gtagataagt ggagctcatg gtatttcctg 3180
aaagatgcta atctatttgc caaacttggt cttgaatgta ctggggcttt caaggtatgg 3240
gtatattttt cttgtgtcct tgcagttagc cccatgtct tatgtgtgtc ctgaaaaaat 3300
aagagcctgc ccaagacttt gggcctcttg acagaattaa ccacttttat acatctgagt 3360
tctcttggta agttctttag cagtgttcaa agtctactag ctcgcattag tttctgttgc 3420
tgccaacaga tctgaactaa tgctaacaga tccccctgag ggattcttga tgggctgagc 3480
agctggctgg agctagtact gactgacatt cattgtgatg agggcagctt tctggtacag 3540
gattctaagc tctatgtttt atatacattt tcatctgtac ttgcacctca ctttacacaa 3600
gaggaaacta tgcaaagtta gctggatcgc tcaaggtcac ttaggtaagt tggcaagtcc 3660
atgcttccca ctcagctcct caggtcagca agtctacttc tctgcctatt ttgtatactc 3720

tctttaatat gtgcctagct ttggaaagtc tagaatgggt ccctggtgcc tttttacttt 3780
gaagaaatca gtttctgcct ctttttggaa aagaaaacaa agtgcaattg ttttttactg 3840
gaaagttacc caatagcatg aggtgaacag gacgtagtta ggccttcctg taaacagaaa 3900
atcatatcaa aacactatct tcccatctgt ttctcaatgc ctgctacttc ttgtagatat 3960
ttcatttcag gagagcagca gttaaacccg tggattttgt agttaggaac ctgggttcaa 4020
accctcttcc actaattggc tatgtctctg gacaagtttt tttttttttt tttttttaaa 4080
ccctttctga actttcactt tctatgtcta cctcaaagaa ttgttgtgag gcttgagata 4140
atgcatttgt aaagggtctg ccagatagga agatgctagt tatggattta caaggttgtt 4200
aaggctgtaa gagtctaaaa cctacagtga atcacaatgc atttaccccc actgacttgg 4260
acataagtga aaactagcca gaagtctctt tttcaaatta cttacaggtt attcaatata 4320
aaatttttgt aatggataat cttatttatc taaactaaag cttcctgttt atacacactc 4380
ctgttattct gggataagat aaatgaccac agtaccttaa tttctaggtg ggtgcctgtg 4440
atggttcatt gtaggtaagg acattttctc tttttcagca gctgtgtagg tccagagcct 4500
ctgggagagg agggggtag catgcaccca gcaggggact gaactgggaa actcaaggtt 4560
ctttttactg tggggtagtg agctgccttt ctgtgatcgg tttccctagg gatgttgctg 4620
ttcccctcct tgctattcgc agctacatac aacgtggcca accccagtag gctgatccta 4680
tatatgatca gtgctggtgc tgactctcaa tagccccacc caagctggct ataggtttac 4740
agatacatta attaggcaac ctaaaatatt gatgctggtg ttggtgtgac ataatgctat 4800
ggccagaact gaaacttaga gttataattc atgtattagg gttctccaga gggacagaat 4860
tagtaggata tatgtatata tgaaagggag gttattaggg agaactggct cccacagtta 4920
gaaggcgaag tcgcacaata ggccgtctgc aagctgggtt agagagaagc cagtagtggc 4980
tcagcctgag ttcaaaaacc tcaaaactgg ggaagctgac agtgcagcca gccttcagtc 5040
tgtggccaaa ggcccaagag cccctggcaa ccaacccact ggtgcaagtc ctagattcca 5100
aaggctgaag aacctggagt ctgatgtcca agagcaggaa gagtggaaga aagccagaag 5160
actcagcaaa caaggtagac agtgtctacc accatagtgg ccataccaaa gaggctaccg 5220
attccttcct gctacctgga tccctgaagt tgccctggtc tctgcacctt ctaaacctag 5280
ttcttaagag ctttccatta catgagctgt ctcaaagccc tccaataaat tctcagtgta 5340
agcttctgtt gcttgtggac agaaaattct gacagaccta ccctataagt gttactgtca 5400
ggataacatg agaacgcaca acagtaagtg gtcactaagt gttagctacg gttattttgc 5460
ccaaggtagc atggctagtt gatgccggtt gatggggctt aaacccagct ccctcatctt 5520
ccaggcctct gtactcccta ttccactaaa ctacctctca ggtttatttt tttaaattct 5580

tactctgcaa gtacatagga ccacatttac ctgggaaaac aagaataaag gctgctctgc 5640
atttttttaga aactttttttg aaagggagat gggaatgcct gcacccccaa gtccagacca 5700
acacaatggt taattgagat gaataataaa ggaaagactg ttctgggctt cccagaatag 5760
cttggtcctt aaattgtggc acaaacaacc tcctgtcaga gccagcctcc tgccaggaag 5820
aggggtagga gactagaggc cgtgtgtgca gccttgccct gaaggctagg gtgacaattt 5880
ggaggctgtc caaacaccct ggcctctaga gctggcctgt ctatttgaaa tgccggctct 5940
gatgctaatc ggcgaccctc aggcaagtta cttaacctta catgcctcag ttttctcatc 6000
tggaaaatga gaaccctagg tttagggttg ttagaaaagt taaatgagtt aagacaagtg 6060
cctgggacac agtagcctct tgtgtgtgtt tatcattatg tcctcagcag gtcgtagaag 6120
cagcttctca ggtgtgaggc tggcgcgatt atctggagtg ggttgggttt tctaggatgg 6180
accccctgct gcattttcct cattcatcca ccagggctta atggggaatc aaggaatcca 6240
tgtgtaactg tataataact gtagccacac tccaatgacc acctactagt tgtccctggc 6300
actgcttata catatgtcca tcaaatcaat cctatgaagt agatactgtc ttcattttat 6360
agatcagaga caattggggt tcagagagct gatgtgattt tcccagggtc acagagagtc 6420
ccagattcag gcacaactct tgtattccaa gacacaacca ctacatgtcc aaaggctgcc 6480
cagagccacc gggcacggca aattgtgaca tatccctaaa gaggctgagc acctggtcag 6540
gatctgatgg ctgacagtgt gtccagatgc agagctggag tgggggaggg gaagggggc 6600
tccttgggac agagaaggct ttctgtgctt tctctgaagg gagcagtctg aggaccaagg 6660
gaacccggca aacagcacct caggtactcc aggccctcct ggctggagag ggctgtggca 6720
atggaaaatt agtgccaact gcaatgagtc agcctcggtt aaatagagag tgaagaatgc 6780
tggacaggaa cctccaccct catgtcacat ttcttcagtg tgacccttct ggcccctctc 6840
ctcctgacag cggaacaatg actgccccga taggtgaggc tggaggaaga atcagtcctg 6900
tccttggcaa gctcttcact atgacagtaa aggctctctg cctgctgcca aggcctgtga 6960
ctttctaacc tggcctcacg ctgggtaagc ttaaggtaga ggtgcaggat tagcaagccc 7020
acctggctac caggccgaca gctacatctt tcaactgacc ctgatcaacg aagagggact 7080
tgtgtctctc agttggttcc aaatgaaacc agggagcagg ggcgttagga agctccaaca 7140
ggatggtact taatggggca tttgagtgga gaggtaggtg acatagtgct ttggagccca 7200
gggagggaaa ggttctgctg aagttgaatt caagactgtt ctttcatcac aaacttgagt 7260
ttcctggaca tttgtttgca gaaacaaccg tagggttttg ccttaacctc gtgggtttat 7320
tattacctca tagggacttt gcctcctgac agcagtttat gggtgttcat tgtggcactt 7380
gagttttctt gcatacttgt tagagaaacc aagtttgtca tcaacttctt atttaacccc 7440
ctggctataa cttcatggat tatgttataa ttaagccatc cagagtaaaa tctgtttaga 7500

ttatcttgga gtaaggggga aaaaatctgt aatttttttct cctcaactag atatatacat 7560

aaaaaatgat tgtattgctt catttaaaaa atataacgca aaatctcttt tccttctaaa 7620

aaaaaaaaaa aaaaa 7635

<210> 4
<211> 4833
<212> mRNA
<213> Homo sapiens

<400> 4
gtgaactgtt gcaccgtgca attgcacact ataaatgtct ttccttatct gtgtgtactc 60

ttatctcact gttctatttt ttctcctcat ttatattaac tctttcttac cttttttttct 120

gaacttctag gccttctctt tccagaactg gtggaagaca aatgaaacgg ccaagatggt 180

aagaaacaag ccgcatttct ccttggggag actgataatt taaaaggttt gttgtgtcag 240

aaacattccc agcttcatca ccaacccttt ccttccacct ctgcccactg gagaccactt 300

atatcccgaa gcggacgcgg cagctgaagt caggaaacca tgcatcacat tagcaggagc 360

caactgcaga ctttaaactc cgttcaacat gtggatgcgg cagagaaatg acctgtccag 420

acaagccggg gcagctcata aactggttca tctgctccct gtgcgtcccg cgggtgcgta 480

agctctggag cagccggcgt ccaaggaccc ggagaaacct tctgctgggc actgcgtgtg 540

ccatctactt gggcttcctg gtgagccagg tggggagggc ctctctccag catggacagg 600

cggctgagaa ggggccacat cgcagccgcg acaccgccga gccatccttc cctgagatac 660

ccctggatgg taccctggcc cctccagagt cccagggcaa tgggtccact ctgcagccca 720

atgtggtgta cattacccta cgctccaagc gcagcaagcc ggccaatatc cgtggcaccg 780

tgaagcccaa gcgcaggaaa aagcatgcag tggcatcggc tgccccaggg caggaggctt 840

tggtcggacc atcccttcag ccgcaggaag cggcaaggga agctgatgct gtagcacctg 900

ggtacgctca gggagcaaac ctggttaaga ttggagagcg accctggagg ttggtgcggg 960

gtccgggagt gcgagccggg ggcccagact tcctgcagcc cagctccagg gagagcaaca 1020

ttaggatcta cagcgagagc gccccctcct ggctgagcaa agatgacatc cgaagaatgc 1080

gactcttggc ggacagcgca gtggcagggc tccggcctgt gtcctctagg agcggagccc 1140

gtttgctggt gctggagggg ggcgcacctg gcgctgtgct ccgctgtggc cctagcccct 1200

gtgggcttct caagcagccc ttggacatga gtgaggtgtt tgccttccac ctagacagga 1260

tcctggggct caacaggacc ctgccgtctg tgagcaggaa agcagagttc atccaagcag 1320

cagcagcagc gtgtctttcc atgcgcttgg cattctttat tttcccagcc tgggaggata 1380

tgagagttcc agggaaatgc tgtattggac atgcaagact cacctgggga acttatcagc 1440

agttgctgaa acagaaatgc tggcagaatg gccgagtacc caagcctgaa tcgggttgta 1500

ctgaaataca tcatcatgag tggtccaaga tggcactctt tgattttttg ttacagattt 1560

ataatcgctt agatacaaat tgctgtggat tcagacctcg caaggaagat gcctgtgtac 1620

agaatggatt gaggccaaaa tgtgatgacc aaggttctgc ggctctagca cacattatcc 1680

agcgaaagca tgacccaagg catttggttt ttatagacaa caagggtttc tttgacagga 1740

gtgaagataa cttaaacttc aaattgttag aaggcatcaa agagtttcca gcttctgcag 1800

tttctgtttt gaagagccag cacttacggc agaaacttct tcagtctctg tttcttgata 1860

aagtgtattg ggaaagtcaa ggaggtagac aaggaattga aaagcttatc gatgtaatag 1920

aacacagagc caaaattctt atcacctata tcaatgcaca cggggtcaaa gtattaccta 1980

tgaatgaatg acaaaagaat cttctggcta gggtgttaga tatatttatg catttttggt 2040

tttgttttta aatcaagcac atcaacctca agcccgttta gcaatgaggc agtgtagatg 2100

aatacgtaaa ataaatgact ttaaccaagt agctataatg ggacttagca ctgtatgcat 2160

acttaaaaag gttttgaaaa acaaactact tgagaaatat ttgtttatat ttttctctaa 2220

catcatgcta tgtgtcagtc tgaacatctg acaacagaaa tttcagttat tattctagct 2280

aagttttgaa aacatttgtc atgctgttta atagaaaact gcaaaccaga gacactgact 2340

ccattaataa accatatttt gtgccgtttt gactgttctg accaaatact aatgggaaca 2400

attcttgacg tttttctgtt gctgattgtt aacatagagc agtctctaca ctaccctgag 2460

gcaactctac attggaacac tgaggcttac agcctgcaag agcatcagag ctgaccatac 2520

atttaaacag aaatgctggt ttatttgcaa aatcaccagt atattttcta ttgtgtctat 2580

aaaaaatcag tcatttaagt acaagaatca tattttccat tcctttttag aaatttattt 2640

tgttgtccct atggaaatca ttcacatctg acaatttata tgttaaagag ttttactctc 2700

tctattttgg tccaatttgt atctagtggc tgagaaatta aataattcta aagtatgaag 2760

ttacctatct gaaaatgtac ttacagagta tcattttaaa atggatgtct ctttaaaaat 2820

tttgttactt ttaccaacaa tgtaatataa tttatgtata ttttattaat aatagtgaat 2880

tccttaaaat ttgttctatg tacttatatt taatttgatt taatggttac tgcccagata 2940

ttgagaattg gttcaaatat tgagtgtgtt tcaatatatt atctggctta tttcaacatg 3000

agtaatatga gcaaaataag ttaaaacctg cgtctgatca attttcctca tgactagaac 3060

taaaacagta aatttggaca atattaagcc tcaaataatc atctccaaac tccttctaac 3120

actttttaaa tcagattgga agacatggac aaatcaggtt catgtgttgc atctttatgt 3180

cctttgccaa tatccaagat catcacatat ggtagatatt cacatggagt ttcaaattca 3240

gaatagatta ccattacctt cctgccctta cacatcctac tccttattta aaagttctat 3300

ttgtgacttt tcatttcctg aaagtttaaa aatacaattt gagaatgttt ataatacatt 3360

ctctcctgtc ttttcacggt tacgtctgtt attgctgaaa tacaccacat tttctttgtt 3420

ctggtcaagg ttaactcaat atctgtgtga aagagaacta ctaacaacgt tacaatagag 3480

gctagatttg aaaaaaaaaa tctatagatc taattgatac aattgtagaa caaaatgtca 3540

aaataatgtt ttaagtataa gagaagatgg accaaggaga gagagatcat ttgaaaatct 3600

aattgtagct tttctaggct cacattcatg tactactttt agcaccctta tgggctgtgc 3660

tcgccccctg gacagttgag ctttggatta tcttcctctt caattttccc tctattgacc 3720

cgagtgtctc cctctgcttc tacagattta tagtactcct tggctctttt gagtctccac 3780

ttttactcac tgtctctggg atttttaaga tccttttctt ctcttataaa tcatcctctt 3840

aatgaaaatt agcctaacaa aagtttggag actggaatcc tactttgagc cactgacttg 3900

aaataactct tttggcaagt tgcctgacat cctgtcttac caaggtggca tatttgcatt 3960

tttactgctt aaaacatttt ttttttttta ccatctttat ccaaatttat catattgatg 4020

gtaggactaa caggcttttt agaagctggc tttaactttg agtctcaagc tacaatgctg 4080

ttgggcagcc tggtcttccc acgtgagggt ttaactttgt ttatttgcct ccagttattc 4140

caaaatgctt attaaatgaa agtcccagga acatgtttat tttagtcacc tttgcttttt 4200

aacaattttg ttttgtaatc aatgagtaat tcatgatgaa ttatttttga ctaatggata 4260

gccgaaggcc aggcttttaa ttctaatagg taatgttctt cttttgtctt attgaaacaa 4320

tgagaatact ctgtgcattt caaatgcact ccgattatgc tgtggtttta ttcacataag 4380

cacaatatgt gttttattta taacttcata acaaacttat aatataataa tttaccttag 4440

cagacatgca aaagcttatt cttgtgtgac ttactttctt taagctaata atataaaaat 4500

aaatatgtat cttaaaaatc tataataaaa cattagaaat taaagatatg tgctttttat 4560

tttgcagatg agttcatttg cttttgtaga tgtgttttca gagctaggta cagaggaatg 4620

tttgctacct ttagcggtga aaaaagaaag agagtcaaga attttgttgg attgtgtttg 4680

tgtgtgcata tatttgatat catcattata tttgtaatct ttggacttgt aatcatagcc 4740

tgtttattct actgtgccat taaatatact ttaccttata cataacgaat aaaataccta 4800

gaagtagatt tatttacaaa aaaaaaaaaa aaa 4833

<210> 5
<211> 2750
<212> mRNA
<213> Homo sapiens

<400> 5
gtgctctgag tttttggttt ctgtttcacc ttgtgtctga gctggtctga aggctggttg 60

ttcagactga gcttcctgcc tgcctgtacc ccgccaacag cttcagaaga aggagcagcc 120

cctgggtgcg tccactttct gggcacgtga ggttgggcct tggccgcctg agcccttgag 180

ttggtcactt gaaccttggg aatattgaga ttatattctc ctgcctttta aaaagatgga 240

cttcagcaga aatctttatg atattgggga acaactggac agtgaagatc tggcctccct 300

caagttcctg agcctggact acattccgca aaggaagcaa gaacccatca aggatgcctt 360

gatgttattc cagagactcc aggaaaagag aatgttggag gaaagcaatc tgtccttcct 420

gaaggagctg ctcttccgaa ttaatagact ggatttgctg attacctacc taaacactag 480

aaaggaggag atggaaaggg aacttcagac accaggcagg gctcaaattt ctgcctacag 540

ggtcatgctc tatcagattt cagaagaagt gagcagatca gaattgaggt cttttaagtt 600

tcttttgcaa gaggaaatct ccaaatgcaa actggatgat gacatgaacc tgctggatat 660

tttcatagag atggagaaga gggtcatcct gggagaagga aagttggaca tcctgaaaag 720

agtctgtgcc caaatcaaca agagcctgct gaagataatc aacgactatg aagaattcag 780

caaaggggag gagttgtgtg gggtaatgac aatctcggac tctccaagag aacaggatag 840

tgaatcacag actttggaca aagtttacca aatgaaaagc aaacctcggg gatactgtct 900

gatcatcaac aatcacaatt ttgcaaaagc acgggagaaa gtgcccaaac ttcacagcat 960

tagggacagg aatggaacac acttggatgc aggggctttg accacgacct ttgaagagct 1020

tcattttgag atcaagcccc acgatgactg cacagtagag caaatctatg agattttgaa 1080

aatctaccaa ctcatggacc acagtaacat ggactgcttc atctgctgta tcctctccca 1140

tggagacaag ggcatcatct atggcactga tggacaggag gcccccatct atgagctgac 1200

atctcagttc actggtttga agtgcccttc ccttgctgga aaacccaaag tgtttttat 1260

tcaggcttgt caggggggata actaccagaa aggtatacct gttgagactg attcagagga 1320

gcaaccctat ttagaaatgg atttatcatc acctcaaacg agatatatcc cggatgaggc 1380

tgactttctg ctggggatgg ccactgtgaa taactgtgtt tcctaccgaa accctgcaga 1440

gggaacctgg tacatccagt cactttgcca gagcctgaga gagcgatgtc ctcgaggcga 1500

tgatattctc accatcctga ctgaagtgaa ctatgaagta agcaacaagg atgacaagaa 1560

aaacatgggg aaacagatgc ctcagcctac tttcacacta agaaaaaaac ttgtcttccc 1620

ttctgattga tggtgctatt ttgtttgttt tgttttgttt tgtttttttg agacagaatc 1680

tcgctctgtc gcccaggctg gagtgcagtg gcgtgatctc ggctcaccgc aagctccgcc 1740

tcccgggttc aggccattct cctgcctcag cctcccgagt agctgggact acaggggccc 1800

gccaccacac ctggctaatt ttttaaaaat atttttagta gagacagggt ttcactgtgt 1860

tagccagggt ggtcttgatc tcctgacctc gtgatccacc cacctcggcc tcccaaagtg 1920

ctgggattac aggcgtgagc caccgcgcct ggccgatggt actatttaga tataacacta 1980

tgtttattta ctaattttct agattttcta ctttattaat tgttttgcac ttttttataa 2040

gagctaaagt taaataggat attaacaaca ataacactgt ctcctttctc ttatgcttaa 2100

ggctttggga atgtttttag ctggtggcaa taaataccag acacgtacaa aatccagcta 2160

tgaatataga gggcttatga ttcagattgt tatctatcaa ctataagccc actgttaata 2220

ttctattaac tttaattctc tttcaaagct aaattccaca ctaccacatt aaaaaaatta 2280

gaaagtagcc acgtatggtg gctcatgtct ataatcccag cactttggga ggttgaggtg 2340

ggaggattgc ttgaacccaa gaggtcaagg ctgcagtgag ccatgttcac accgctgcac 2400

tcaagcttgg gtgacagaac aagaccccgt ctcaaaaaaa attttttttt taataaaaca 2460

aaatttgttt gaaatctttt aaaaattcaa atgattttta caagttttaa ataagctctc 2520

cccaaacttg ctttatgcct tcttattgct tttatgatat atatatgctt ggctaactat 2580

atttgctttt tgctaacaat gctctggggt ctttttatgc atttgcattt gctctttcat 2640

ctctgcttgg attattttaa atcattagga attaagttat ctttaaaatt taagtatctt 2700

ttttcaaaaa catttttttaa tagaataaaa tataatttga tcttattaaa 2750

<210> 6
<211> 2938
<212> mRNA
<213> Homo sapiens

<400> 6
gagtgagtca tctctgttct gctttaggag taaagtttac cctgcagttc cttctgtggt 60

gaagttttct ctttctctcg gagaccagat tctgcctttc tgctggaggg aagtgttttc 120

acaggttctc ctccttttat cttttgtgtt ttttttcaag ccctgctgaa tttgctagtc 180

aactcaacag gaagtgaggc catggaggga ggcagaagag ccagggtggt tattgaaagt 240

aaaagaaact tcttcctggg agcctttccc accccttcc ctgctgagca cgtggagtta 300

ggcaggttag gggactcgga gactgcgatg gtgccaggaa agggtggagc ggattatatt 360

ctcctgcctt ttaaaaagat ggacttcagc agaaatcttt atgatattgg ggaacaactg 420

gacagtgaag atctggcctc cctcaagttc ctgagcctgg actacattcc gcaaaggaag 480

caagaaccca tcaaggatgc cttgatgtta ttccagagac tccaggaaaa gagaatgttg 540

gaggaaagca atctgtcctt cctgaaggag ctgctcttcc gaattaatag actggatttg 600

ctgattacct acctaaacac tagaaaggag gagatggaaa gggaacttca gacaccaggc 660

agggctcaaa tttctgccta cagggtcatg ctctatcaga tttcagaaga agtgagcaga 720

tcagaattga ggtcttttaa gtttcttttg caagaggaaa tctccaaatg caaactggat 780

gatgacatga acctgctgga tattttcata gagatggaga agagggtcat cctgggagaa 840

ggaaagttgg acatcctgaa aagagtctgt gcccaaatca acaagagcct gctgaagata 900

atcaacgact atgaagaatt cagcaaagag agaagcagca gccttgaagg aagtcctgat 960

gaattttcaa atggggagga gttgtgtggg gtaatgacaa tctcggactc tccaagagaa 1020

caggatagtg aatcacagac tttggacaaa gtttaccaaa tgaaaagcaa acctcgggga 1080
tactgtctga tcatcaacaa tcacaatttt gcaaaagcac gggagaaagt gcccaaactt 1140
cacagcatta gggacaggaa tggaacacac ttggatgcag gggctttgac cacgaccttt 1200
gaagagcttc attttgagat caagccccac gatgactgca cagtagagca aatctatgag 1260
attttgaaaa tctaccaact catggaccac agtaacatgg actgcttcat ctgctgtatc 1320
ctctcccatg gagacaaggg catcatctat ggcactgatg gacaggaggc ccccatctat 1380
gagctgacat ctcagttcac tggtttgaag tgcccttccc ttgctggaaa acccaaagtg 1440
ttttttattc aggcttgtca gggggataac taccagaaag gtatacctgt tgagactgat 1500
tcagaggagc aaccctattt agaaatggat ttatcatcac ctcaaacgag atatatcccg 1560
gatgaggctg actttctgct ggggatggcc actgtgaata actgtgtttc ctaccgaaac 1620
cctgcagagg gaacctggta catccagtca ctttgccaga gcctgagaga gcgatgtcct 1680
cgaggcgatg atattctcac catcctgact gaagtgaact atgaagtaag caacaaggat 1740
gacaagaaaa acatggggaa acagatgcct cagcctactt tcacactaag aaaaaaactt 1800
gtcttccctt ctgattgatg gtgctatttt gtttgttttg ttttgttttg tttttttgag 1860
acagaatctc gctctgtcgc ccaggctgga gtgcagtggc gtgatctcgg ctcaccgcaa 1920
gctccgcctc ccgggttcag gccattctcc tgcctcagcc tcccgagtag ctgggactac 1980
aggggcccgc caccacacct ggctaatttt ttaaaaatat ttttagtaga gacagggttt 2040
cactgtgtta gccagggtgg tcttgatctc ctgacctcgt gatccaccca cctcggcctc 2100
ccaaagtgct gggattacag gcgtgagcca ccgcgcctgg ccgatggtac tatttagata 2160
taacactatg tttatttact aattttctag attttctact ttattaattg ttttgcactt 2220
ttttataaga gctaaagtta aataggatat taacaacaat aacactgtct cctttctctt 2280
atgcttaagg ctttgggaat gtttttagct ggtggcaata aataccagac acgtacaaaa 2340
tccagctatg aatatagagg gcttatgatt cagattgtta tctatcaact ataagcccac 2400
tgttaatatt ctattaactt taattctctt tcaaagctaa attccacact accacattaa 2460
aaaaattaga aagtagccac gtatggtggc tcatgtctat aatcccagca ctttgggagg 2520
ttgaggtggg aggattgctt gaacccaaga ggtcaaggct gcagtgagcc atgttcacac 2580
cgctgcactc aagcttgggt gacagaacaa gaccccgtct caaaaaaaat ttttttttta 2640
ataaaacaaa atttgtttga aatcttttaa aaattcaaat gatttttaca agttttaaat 2700
aagctctccc caaacttgct ttatgccttc ttattgcttt tatgatatat atatgcttgg 2760
ctaactatat ttgctttttg ctaacaatgc tctggggtct ttttatgcat ttgcatttgc 2820
tctttcatct ctgcttggat tattttaaat cattaggaat taagttatct ttaaaattta 2880

agtatctttt ttcaaaaaca ttttttaata gaataaaata taatttgatc ttattaaa 2938

<210> 7
<211> 3220
<212> mRNA
<213> Homo sapiens

<400> 7
ctcccggaga tgccccgcgg cagccgcgct cggggctcta agagaaaaag gagttggaat 60
acagaatgcc catcctttcc aggagaaaga ccactgcagg tcagaagagc aggtctcagg 120
acagcagggg cagctgcctc tctctctgaa gcatggctca ggtgtggaga agggtttcag 180
aacacttctg ggaatccgtc attaacagct gaagagaaga cgattacaga aaagcacctt 240
gaattatgcc ctagacccaa gcaagaaacc accacatcta aaagcaccag tgggcttaca 300
gacataacat ggagctccag tggaagtgat ttgtcggatg aagataagac actttctcag 360
ttacagagag atgaattaca gtttatcgac tgggagattg acagtgacag ggcagaggct 420
agtgactgtg atgaatttga agatgacgag ggtgctgtgg aaatctcaga ctgtgcttct 480
tgtgcaagta atcagtcttt gacaagtgat gagaagctgt cggagcttcc caagccaagt 540
tctatagaaa ttttagagta ttcatcagat agtgaaaaag aagatgattt ggaaaatgtc 600
ctactcattg attcagaatc ccctcacaaa taccacgtgc agtttgcatc ggatgcaaga 660
cagattatgg agagactgat agatccaagg acaaaatcaa cagagaccat tttgcataca 720
cctcagaaac ccacagctaa gtttcccagg actccagaaa attcagcaaa gaagaagctt 780
ttaagaggtg gactagcaga aagactaaat ggactgcaga atcgagagag atctgctatt 840
tctttgtgga gacatcaatg tatttcttac caaaagacac tttcaggtag aaaatctggt 900
gtattaactg tgaaaatttt agagctgcat gaggaatgtg ccatgcaagt tgccatgtgt 960
gagcagttat tggggtcacc agccaccagc tcctcccaaa gtgtggctcc caggcctgga 1020
gctggcctga aagttctctt caccaaggag actgcaggct acctcagggg ccgtccccag 1080
gacactgtcc ggatcttccc tccctggcaa aaactgatta ttccaagtgg aagttgccct 1140
gttattctga atacttactt ttgtgagaaa gttgttgcca aagaagattc agaaaaaact 1200
tgtgaagtgt actgtccgga catacccctt ccaagaagaa gcatctcttt ggcccagatg 1260
tttgtaatta agggtctaac aaataattca cctgaaatcc aggttgtgtg tagtggtgta 1320
gccactacag ggacagcctg gacccatggg cacaaagaag caaaacagcg catcccaacc 1380
agcactcccc tgagggattc tctcctggat gtggtggaaa gccagggagc tgcctcgtgg 1440
ccaggagctg gagtccgagt ggtggtgcaa agagtgtatt ctcttcccag cagagacagc 1500
accaggggtc agcagggggc cagctcagga cacacagacc cagctggaac tcgagcctgc 1560
cttctggtac aagatgcctg tggaatgttc ggtgaagtgc acttggagtt caccatgtcg 1620

aaggcaagac agttggaagg gaagtcttgc agcctggtgg gaatgaaggt tctacagaaa 1680

gtcaccagag gaaggacagc ggggattttc agtttgattg acaccctgtg gcccccagcg 1740

atacctctga aaacacctgg ccgcgaccag ccctgtgaag agataaaaac tcatctgcct 1800

cctccagcct tgtgttacat cctcacagct catccaaatc tgggacaaat tgatataatt 1860

gacgaagacc ccatttataa gctttaccag cctccagtta cccgctgctt aagagacatt 1920

ctccagatga atgatcttgg tacccgttgc agtttctatg ccacggtgat ttaccaaaaa 1980

ccacagctga agagtctgct gcttctggag caaagggaga tctggctgct agtgaccgat 2040

gtcactctgc aaacgaagga ggagagagac cccaggctcc ccaaaaccct gctggtctat 2100

gtggccccct tgtgtgtgct gggctctgaa gtcctggagg cactcgctgg ggctgcccct 2160

cacagcctct tcttcaagga cgctctccgt gaccagggtc ggattgtttg tgctgaacga 2220

actgtcctct tgcttcagaa gccccttttg agtgtggtct ctggtgcaag ttcctgtgag 2280

ctgcctggcc cggtgatgct cgacagcctg gactctgcaa cacctgtcaa ctccatctgc 2340

agtgttcaag gcactgtggt tggcgtggac gagagcactg ctttctcatg gcctgtgtgt 2400

gacatgtgtg gcaacgggag attggaacag aggccggaag acagaggcgc cttttcctgt 2460

ggggactgct cccgggtggt cacatctcct gttctcaaga ggcacctgca ggtcttcctg 2520

gactgccgct caagaccgca gtgcagagtg aaggtcaagc tgttgcagcg cagcatttcc 2580

tccctgctga ggtttgccgc cggtgaagat gggagctacg aagtgaagag tgtcctcgga 2640

aaggaagtgg ggttgttaaa ttgttttgtc cagtccgtaa ccgcccaccc gaccagctgc 2700

attggattgg aggaaatcga gcttctgagt gcaggagggg cctctgcaga acactagcgg 2760

ttgccgcagg atctgtgaac tttgcaatgt ggctgcaagg gtggtggtgg tggtggtgat 2820

ttggggtagt tatttgttaa ctatggacac agtgaacgta gtttacgatc ttgaaatgaa 2880

acttagattt ttctggggaa atgttcagat acagttttgt gaactgtaaa tcaaaatacc 2940

tttttctaca gtttatcttt tattttctgc aaatttagga acatatttac tcgttttcac 3000

attgaatctt aagtttaagc tcttcatttg gtatttaggc aatatatgag aaaaaaattt 3060

tttttgttca tttgtaattt taacaagttg aacattttac catgattgaa catgttttta 3120

ttacagtatt taacattccc ccaaagaata ccctgcaaag tgtaaacctt tgtcccatac 3180

tgtgatatta ctgttctgct acaataaatg tcaaacctaa 3220

<210> 8
<211> 4974
<212> mRNA
<213> Homo sapiens

<400> 8
acacctccct ccccgcctgc cagtgtcacc agcctgttgc ctctgtgaga aagtaccact 60

```
gtaagaggcc aaagggcatg atcattttcc tctttcaccc tgtctaggtt gccagcaaat     120
cccacgggcc tcctgacgct gcccctgggg ccacaggtcc ctcgagtgct ggaaggatga     180
aggattcctg catcactgtg atggccatgg cgctgctgtc tgggttcttt ttcttcgcgc     240
cggcctcgag ctacaacctg gacgtgcggg gcgcgcggag cttctcccca ccgcgcgccg     300
ggaggcactt tggataccgc gtcctgcagg tcggaaacgg ggtcatcgtg ggagctccag     360
gggaggggaa cagcacagga agcctctatc agtgccagtc gggcacagga cactgcctgc     420
cagtcaccct gagaggttcc aactatacct ccaagtactt gggaatgacc ttggcaacag     480
accccacaga tggaagcatt ttgtttgctg ctgttcagtt ttccacaagc tacaaaacag     540
aatttgattt ctcagattat gttaaacgga aggaccctga tgctctgctg aagcatgtaa     600
agcacatgtt gctgttgacc aatacctttg gtgccatcaa ttatgtcgcg acagaggtgt     660
tccgggagga gctgggggcc cggccagatg ccaccaaagt gcttatcatc atcacggatg     720
gggaggccac tgacagtggc aacatcgatg cggccaaaga catcatccgc tacatcatcg     780
ggattggaaa gcattttcag accaaggaga gtcaggagac cctccacaaa tttgcatcaa     840
aacccgcgag cgagtttgtg aaaattctgg acacatttga gaagctgaaa gatctattca     900
ctgagctgca gaagaagatc tatgtcattg agggcacaag caaacaggac ctgacttcct     960
tcaacatgga gctgtcctcc agcggcatca gtgctgacct cagcaggggc catgcagtcg    1020
tgggggcagt aggagccaag gactgggctg gggctttcct tgacctgaag gcagacctgc    1080
aggatgacac atttattggg aatgaaccat tgacaccaga agtgagagca ggctatttgg    1140
gttacaccgt gacctggctg ccctcccggc aaaagacttc gttgctggcc tcgggagccc    1200
ctcgatacca gcacatgggc cgagtgctgc tgttccaaga gccacagggc ggaggacact    1260
ggagccaggt ccagacaatc catgggaccc agattggctc ttatttcggt ggggagctgt    1320
gtggcgtcga cgtggaccaa gatggggaga cagagctgct gctgattggt gccccactgt    1380
tctatgggga gcagagagga ggccgggtgt ttatctacca gagaagacag ttggggtttg    1440
aagaagtctc agagctgcag ggggaccccg gctacccact cgggcggttt ggagaagcca    1500
tcactgctct gacagacatc aacggcgatg ggctggtaga cgtggctgtg gggggcccctc   1560
tggaggagca gggggctgtg tacatcttca atgggaggca cgggggggctt agtccccagc   1620
caagtcagcg gatagaaggg acccaagtgc tctcaggaat tcagtggttt ggacgctcca    1680
tccatggggt gaaggacctt gaaggggatg gcttggcaga tgtggctgtg gggggctgaga   1740
gccagatgat cgtgctgagc tcccggcccg tggtggatat ggtcaccctg atgtccttct    1800
ctccagctga gatcccagtg catgaagtgg agtgctccta ttcaaccagt aacaagatga    1860
aagaaggagt taatatcaca atctgtttcc agatcaagtc tctcatcccc cagttccaag    1920
gccgcctggt tgccaatctc acttacactc tgcagctgga tggccaccgg accagaagac    1980
```

gggggttgtt cccaggaggg agacatgaac tcagaaggaa tatagctgtc accaccagca 2040

tgtcatgcac tgacttctca tttcatttcc cggtatgtgt tcaagacctc atctccccca 2100

tcaatgtttc cctgaatttc tctctttggg aggaggaagg gacaccgagg gaccaaaggg 2160

cgggcaagga cataccgccc atcctgagac cctccctgca ctcggaaacc tgggagatcc 2220

cttttgagaa gaactgtggg gaggacaaga agtgtgaggc aaacttgaga gtgtccttct 2280

ctcctgcaag atccagagcc ctgcgtctaa ctgcttttgc cagcctctct gtggagctga 2340

gcctgagtaa cttggaagaa gatgcttact gggtccagct ggacctgcac ttcccccggg 2400

gactctcctt ccgcaaggtg gagatgctga agccccatag ccagatacct gtgagctgcg 2460

aggagcttcc tgaagagtcc aggcttctgt ccagggcatt atcttgcaat gtgagctctc 2520

ccatcttcaa agcaggccac tcggttgctc tgcagatgat gtttaataca ctggtaaaca 2580

gctcctgggg ggactcggtt gaattgcacg ccaatgtgac ctgtaacaat gaggactcag 2640

acctcctgga ggacaactca gccactacca tcatccccat cctgtacccc atcaacatcc 2700

tcatccagga ccaagaagac tccacactct atgtcagttt caccccccaaa ggccccaaga 2760

tccaccaagt caagcacatg taccaggtga ggatccagcc ttccatccac gaccacaaca 2820

tacccaccct ggaggctgtg gttggggtgc cacagcctcc cagcgagggg cccatcacac 2880

accagtggag cgtgcagatg gagcctcccg tgccctgcca ctatgaggat ctggagaggc 2940

tcccggatgc agctgagcct tgtctccccg gagccctgtt ccgctgccct gttgtcttca 3000

ggcaggagat cctcgtccaa gtgatcggga ctctggagct ggtgggagag atcgaggcct 3060

cttccatgtt cagcctctgc agctccctct ccatctcctt caacagcagc aagcatttcc 3120

acctctatgg cagcaacgcc tccctggccc aggttgtcat gaaggttgac gtggtgtatg 3180

agaagcagat gctctacctc tacgtgctga gcggcatcgg ggggctgctg ctgctgctgc 3240

tcattttcat agtgctgtac aaggttggtt tcttcaaacg gaacctgaag gagaagatgg 3300

aggctggcag aggtgtcccg aatggaatcc ctgcagaaga ctctgagcag ctggcatctg 3360

ggcaagaggc tggggatccc ggctgcctga agcccctcca tgagaaggac tctgagagtg 3420

gtggtggcaa ggactgagtc caggcctgtg aggtgcagag tgcccagaac tggactcagg 3480

atgcccaggg ccactctgcc tctgcctgca ttctgccgtg tgccctcggg cgagtcactg 3540

cctctccctg gccctcagtt tccctatctc gaacatggaa ctcattcctg cctgtctcct 3600

ttgcaggctc atagggaaga cctgctgagg gaccagccaa gagggctgca aaagtgaggg 3660

cttgtcatta ccagacggtt caccagcctc tcttggtttc cttccttgga agagaatgtc 3720

tgatctaaat gtggagaaac tgtagtctca ggacctaggg atgttctggc cctcacccct 3780

gccctgggat gtccacagat gcctccaccc cccagaacct gtccttgcac actcccctgc 3840

actggagtcc agtctcttct gctggcagaa agcaaatgtg acctgtgtca ctacgtgact 3900

gtggcacacg ccttgttctt ggccaaagac caaattcctt ggcatgcctt ccagcaccct 3960

gcaaaatgag accctcgtgg ccttccccag cctcttctag agccgtgatg cctccctgtt 4020

gaagctctgg tgacaccagc ctttctccca ggccaggctc cttcctgtct tcctgcattc 4080

acccagacag ctccctctgc ctgaaccttc catctcgcca cccctccttc cttgaccagc 4140

agatcccagc tcacgtcaca cttggttggg tcctcacatc tttcacactt ccaccagcct 4200

gcactactcc ctcaaagcac acgtcatgtt tcttcatccg gcagcctgga tgtttttttcc 4260

ctgtttaatg attgacgtac ttagcagcta tctctcagtg aactgtgagg gtaaaggcta 4320

tacttgtctt gttcaccttg ggatgatgcc tcatgatatg tcagggcgtg ggacatctag 4380

taggtgcttg acataatttc actgaattaa tgacagagcc agtgggaaga tacagaaaaa 4440

gaggggctgg gctgggcgcg gtggttcacg cctgtaatcc cagcactttg ggaggccaag 4500

gagggtggat cacctgaggt caggagttag aggccagcct ggcgaaaccc catctctact 4560

aaaaatacaa aatccaggcg tggtggcaca cacctgtagt cccagctact caggaggttg 4620

aggtaggaga attgcttgaa cctgggaggt ggaggttgca gtgagccaag attgcgccat 4680

tgcactccag cctgggcaac acagcgagac tccgtctcaa ggaaaaaata aaaataaaaa 4740

gcgggcacgg gcccgtgaca tccccaccct tggaggctgt cttctcaggc tctgccctgc 4800

cctagctcca caccctctcc caggacccat cacgcctgtg cagtggcccc cacagaaaga 4860

ctgagctcaa ggtgggaacc acgtctgcta acttggagcc ccagtgccaa gcacagtgcc 4920

tgcatgtatt tatccaataa atgtgaaatt ctgtccaaaa aaaaaaaaaa aaaa 4974

<210> 9
<211> 2689
<212> mRNA
<213> Homo sapiens

<400> 9
tttattctct ggaacatgaa acattctgtt gtgctcatat catgcaaatt atcactagta 60

ggagagcaga gagtggaaat gttccaggta taaagaccca caagataaag aagctcagag 120

tcgttagaaa caggagcaga tgtacagggt ttgcctgact cacactcaag gttgcataag 180

caagatttca aaattaatcc tattctggag acctcaaccc aatgtacaat gttcctgact 240

ggaaaagaag aactatattt ttctgatttt ttttttcaaa tctttaccat tagttgccct 300

gtatctccgc cttcactttc tgcaggaaac tttatttcct acttctgcat gccaagtttc 360

tacctctaga tctgtttggt tcagttgctg agaagcctga cataccagga ctgcctgaga 420

caagccacaa gctgaacaga gaaagtggat tgaacaagga cgcatttccc cagtacatcc 480

acaacatgct gtccacatct cgttctcggt ttatcagaaa taccaacgag agcggtgaag 540

aagtcaccac ctttttttgat tatgattacg gtgctccctg tcataaattt gacgtgaagc 600
aaattggggc ccaactcctg cctccgctct actcgctggt gttcatcttt ggttttgtgg 660
gcaacatgct ggtcgtcctc atcttaataa actgcaaaaa gctgaagtgc ttgactgaca 720
tttacctgct caacctggcc atctctgatc tgctttttct tattactctc ccattgtggg 780
ctcactctgc tgcaaatgag tgggtctttg ggaatgcaat gtgcaaatta ttcacagggc 840
tgtatcacat cggttatttt ggcggaatct tcttcatcat cctcctgaca atcgatagat 900
acctggctat tgtccatgct gtgtttgctt taaaagccag gacggtcacc tttggggtgg 960
tgacaagtgt gatcacctgg ttggtggctg tgtttgcttc tgtcccagga atcatcttta 1020
ctaaatgcca gaaagaagat tctgtttatg tctgtggccc ttattttcca cgaggatgga 1080
ataatttcca cacaataatg aggaacattt tggggctggt cctgccgctg ctcatcatgg 1140
tcatctgcta ctcgggaatc ctgaaaaccc tgcttcggtg tcgaaacgag aagaagaggc 1200
atagggcagt gagagtcatc ttcaccatca tgattgttta ctttctcttc tggactccct 1260
ataatattgt cattctcctg aacaccttcc aggaattctt cggcctgagt aactgtgaaa 1320
gcaccagtca actggaccaa gccacgcagg tgacagagac tcttgggatg actcactgct 1380
gcatcaatcc catcatctat gccttcgttg gggagaagtt cagaagcctt tttcacatag 1440
ctcttggctg taggattgcc ccactccaaa aaccagtgtg tggaggtcca ggagtgagac 1500
caggaaagaa tgtgaaagtg actacacaag gactcctcga tggtcgtgga aaaggaaagt 1560
caattggcag agcccctgaa gccagtcttc aggacaaaga aggagcctag agacagaaat 1620
gacagatctc tgctttggaa atcacacgtc tggcttcaca gatgtgtgat tcacagtgtg 1680
aatcttggtg tctacgttac caggcaggaa ggctgagagg agagagactc cagctgggtt 1740
ggaaaacagt attttccaaa ctaccttcca gttcctcatt tttgaataca ggcatagagt 1800
tcagactttt tttaaatagt aaaaataaaa ttaaagctga aaactgcaac ttgtaaatgt 1860
ggtaaagagt tagtttgagt tactatcatg tcaaacgtga aaatgctgta ttagtcacag 1920
agataattct agctttgagc ttaagaattt tgagcaggtg gtatgtttgg gagactgctg 1980
agtcaaccca atagttgttg attggcagga gttggaagtg tgtgatctgt gggcacatta 2040
gcctatgtgc atgcagcatc taagtaatga tgtcgtttga atcacagtat acgctccatc 2100
gctgtcatct cagctggatc tccattctct caggcttgct gccaaaagcc ttttgtgttt 2160
tgttttgtat cattatgaag tcatgcgttt aatcacattc gagtgtttca gtgcttcgca 2220
gatgtccttg atgctcatat tgttccctat tttgccagtg ggaactccta aatcaagttg 2280
gcttctaatc aaagctttta aaccctattg gtaaagaatg gaaggtggag aagctccctg 2340
aagtaagcaa agactttcct cttagtcgag ccaagttaag aatgttctta tgttgcccag 2400
tgtgtttctg atctgatgca agcaagaaac actgggcttc tagaaccagg caacttggga 2460

actagactcc caagctggac tatggctcta ctttcaggcc acatggctaa agaaggtttc 2520

agaaagaagt ggggacagag cagaactttc accttcatat atttgtatga tcctaatgaa 2580

tgcataaaat gttaagttga tggtgatgaa atgtaaatac tgtttttaac aactatgatt 2640

tggaaaataa atcaatgcta taactatgtt gaaaaaaaa aaaaaaaaa 2689

<210> 10
<211> 2335
<212> mRNA
<213> Homo sapiens

<400> 10
tttattctct ggaacatgaa acattctgtt gtgctcatat catgcaaatt atcactagta 60

ggagagcaga gagtggaaat gttccaggta taaagaccca caagataaag aagctcagag 120

tcgttagaaa caggagcaga tgtacagggt ttgcctgact cacactcaag gttgcataag 180

caagatttca aaattaatcc tattctggag acctcaaccc aatgtacaat gttcctgact 240

ggaaaagaag aactatattt ttctgatttt tttttcaaa tctttaccat tagttgccct 300

gtatctccgc cttcactttc tgcaggaaac tttatttcct acttctgcat gccaagtttc 360

tacctctaga tctgtttggt tcagttgctg agaagcctga cataccagga ctgcctgaga 420

caagccacaa gctgaacaga gaaagtggat tgaacaagga cgcatttccc cagtacatcc 480

acaacatgct gtccacatct cgttctcggt ttatcagaaa taccaacgag agcggtgaag 540

aagtcaccac ctttttgat tatgattacg gtgctccctg tcataaattt gacgtgaagc 600

aaattggggc ccaactcctg cctccgctct actcgctggt gttcatcttt ggttttgtgg 660

gcaacatgct ggtcgtcctc atcttaataa actgcaaaaa gctgaagtgc ttgactgaca 720

tttacctgct caacctggcc atctctgatc tgctttttct tattactctc ccattgtggg 780

ctcactctgc tgcaaatgag tgggtctttg ggaatgcaat gtgcaaatta ttcacagggc 840

tgtatcacat cggttatttt ggcggaatct tcttcatcat cctcctgaca atcgatagat 900

acctggctat tgtccatgct gtgtttgctt taaaagccag gacggtcacc tttggggtgg 960

tgacaagtgt gatcacctgg ttggtggctg tgtttgcttc tgtcccagga atcatcttta 1020

ctaaatgcca gaaagaagat tctgtttatg tctgtggccc ttattttcca cgaggatgga 1080

ataatttcca cacaataatg aggaacattt tggggctggt cctgccgctg ctcatcatgg 1140

tcatctgcta ctcgggaatc ctgaaaaccc tgcttcggtg tcgaaacgag aagaagaggc 1200

atagggcagt gagagtcatc ttcaccatca tgattgttta ctttctcttc tggactccct 1260

ataatattgt cattctcctg aacaccttcc aggaattctt cggcctgagt aactgtgaaa 1320

gcaccagtca actggaccaa gccacgcagg tgacagagac tcttgggatg actcactgct 1380

gcatcaatcc catcatctat gccttcgttg ggagaagtt cagaaggtat ctctcggtgt 1440

tcttccgaaa gcacatcacc aagcgcttct gcaaacaatg tccagttttc tacagggaga 1500

cagtggatgg agtgacttca acaaacacgc cttccactgg ggagcaggaa gtctcggctg 1560

gtttataaaa cgaggagcag tttgattgtt gtttataaag ggagataaca atctgtatat 1620

aacaacaaac ttcaagggtt tgttgaacaa tagaaacctg taaagcaggt gcccaggaac 1680

ctcagggctg tgtgtactaa tacagactat gtcacccaat gcatatccaa catgtgctca 1740

gggaataatc cagaaaaact gtgggtagag actttgactc tccagaaagc tcatctcagc 1800

tcctgaaaaa tgcctcatta ccttgtgcta atcctctttt tctagtcttc ataatttctt 1860

cactcaatct ctgattctgt caatgtcttg aaatcaaggg ccagctggag gtgaagaaga 1920

gaatgtgaca ggcacagatg aatgggagtg agggatagtg gggtcagggc tgagaggaga 1980

aggagggaga catgagcatg gctgagcctg gacaaagaca aaggtgagca aagggctcac 2040

gcattcagcc aggagatgat actggtcctt agccccatct gccacgtgta tttaaccttg 2100

aagggttcac caggtcaggg agagtttggg aactgcaata acctgggagt tttggtggag 2160

tccgatgatt ctcttttgca taagtgcatg acatattttt gctttattac agtttatcta 2220

tggcacccat gcaccttaca tttgaaatct atgaaatatc atgctccatt gttcagatgc 2280

ttcttaggcc acatccccct gtctaaaaat tcagaaaatt tttgtttata aaaga 2335

<210> 11
<211> 7801
<212> mRNA
<213> Homo sapiens

<400> 11
atttgactca ctgaccctga tggggaagat agtgttattc ccacagtgtg gtagtacaca 60

ctggggctta tagggactga gcctactcaa gggtatatgg tgctgtgggt cagagctggg 120

gcatggccca gggattcagt gtgccttgac tccccctgta aatgttcctc tcagaagcct 180

tcttggcctt ccagcccttg gtttttgaga caaccagcag tcatttgttc gttcctgaca 240

ttccttcctg tcccttcctt ccaggttctg tggacaatca caatgggaat ccaaggaggg 300

tctgtcctgt tcgggctgct gctcgtcctg gctgtcttct gccattcagg tcatagcctg 360

cagtgctaca actgtcctaa cccaactgct gactgcaaaa cagccgtcaa ttgttcatct 420

gattttgatg cgtgtctcat taccaaagct gggttacaag tgtataacaa gtgttggaag 480

tttgagcatt gcaatttcaa cgacgtcaca acccgcttga gggaaaatga gctaacgtac 540

tactgctgca agaaggacct gtgtaacttt aacgaacagc ttgaaaatgg tgggacatcc 600

ttatcagaga aaacagttct tctgctggtg actccatttc tggcagcagc ctggagcctt 660

catccctaag tcaacaccag gagagcttct cccaaactcc ccgttcctgc gtagtccgct 720

ttctcttgct gccacattct aaaggcttga tattttccaa atggatcctg ttgggaaaga 780

ataaaattag cttgagcaac ctggctaaga tagaggggct ctgggagact ttgaagacca 840
gtcctgtttg cagggaagcc ccacttgaag gaagaagtct aagagtgaag taggtgtgac 900
ttgaactaga ttgcatgctt cctcctttgc tcttgggaag accagctttg cagtgacagc 960
ttgagtgggt tctctgcagc cctcagatta tttttcctct ggctccttgg atgtagtcag 1020
ttagcatcat tagtacatct ttggagggtg gggcaggagt atatgagcat cctctctcac 1080
atggaacgct ttcataaact tcagggatcc cgtgttgcca tggaggcatg ccaaatgttc 1140
catatgtggg tgtcagtcag ggacaacaag atccttaatg cagagctaga ggacttctgg 1200
cagggaagtg ggaagtgttt ccagatagca gggcatgaaa acttagagag gtacaagtgg 1260
ctgaaaatcg agtttttcct ctgtctttaa attttatatg ggctttgtta tcttccactg 1320
gaaaagtgta atagcataca tcaatggtgt gttaaagcta tttccttgcc tttttttat 1380
tggaatggta ggatatcttg gctttgccac acacagttac agagtgaaca ctctactaca 1440
tgtgactggc agtattaagt gtgcttattt taaatgttac tggtagaaag gcagttcagg 1500
tatgtgtgta tatagtatga atgcagtggg gacacccttt gtggttacag tttgagactt 1560
ccaaaggtca tccttaataa caacagatct gcaggggtat gttttaccat ctgcatccag 1620
cctcctgcta actcctagct gactcagcat agattgtata aaatacettt gtaacggctc 1680
ttagcacact cacagatgtt tgaggctttc agaagctctt ctaaaaaatg atacacacct 1740
ttcacaaggg caaacttttt ccttttccct gtgtattcta gtgaatgaat ctcaagattc 1800
agtagaccta atgacatttg tattttatga tcttggctgt atttaatggc ataggctgac 1860
ttttgcagat ggaggaattt cttgattaat gttgaaaaaa aacccttgat tatactctgt 1920
tggacaaacc gagtgcaatg aatgatgctt ttctgaaaat gaaatataac aagtgggtga 1980
atgtggttat ggccgaaaag gatatgcagt atgcttaatg gtagcaactg aaagaagaca 2040
tcctgagcag tgccagcttt cttctgttga tgccgttccc tgaacatagg aaaatagaaa 2100
cttgcttatc aaaacttagc attaccttgg tgctctgtgt tctctgttag ctcagtgtct 2160
ttccttacat caataggttt tttttttttt ttttggcctg aggaagtact gaccatgccc 2220
acagccaccg gctgagcaaa gaagctcatt tcatgtgagt tctaaggaat gagaaacaat 2280
tttgatgaat ttaagcagaa aatgaatttc tgggaacttt tttgggggcg ggggggtggg 2340
gaattcagcc acactccaga aagccaggag tcgacagttt tggaagcctc tctcaggatt 2400
gagattctag gatgagattg gcttactgct atcttgtgtc atgtacccac tttttggcca 2460
gactacactg ggaagaaggt agtcctctaa agcaaaatct gagtgccact aaatggggag 2520
atggggctgt taagctgtcc aaatcaacaa gggtcatata aatggcctta aactttgggg 2580
ttgctttctg caaaaagttg ctgtgactca tgccatagac aaggttgagt gcctggaccc 2640

aaaggcaata ctgtaatgta aagacattta tagtactagg caaacagcac cccaggtact 2700
ccaggccctc ctggctggag agggctgtgg caatagaaaa ttagtgccaa ctgcagtgag 2760
tcagcctagg ttaaatagag agtgtaagag tgctggacag gaacctccac cctcatgtca 2820
catttcttca atgtgaccct tctggcccct ctcctcctga cagcggaaca atgactgccc 2880
cgataggtga ggctggagga agaatcagtc ctgtccttgg caagctcttc actatgacag 2940
taaaggctct ctgcctgctg ccaaggcctg tgactttcta acctggcctc acgctgggta 3000
agcttaaggt agaggtgcag gattagcaag cccacctggc taccaggccg acagctacat 3060
cctccaactg accctgatca acgaagaggg attcatgtgt ctgtctcagt tggttccaaa 3120
tgaaaccagg gagcagggga gttaggaatc gaacaccagt catgcctact ggctctctgc 3180
tcgagagcca ataccctgtg ccctccactc atctggattt acaggaactg tcatagtgtt 3240
cagtattggg tggtgataag cccattggat tgtccccttg gggggatgag ctaggggtgc 3300
aaggaacacc tgatgagtag ataagtggag ctcatggtat ttcctgaaag atgctaatct 3360
atttgccaaa cttggtcttg aatgtactgg gggcttcaag gtatgggtat atttttcttg 3420
tgtccttgca gttagccccc atgtcttatg tgtgtcctga aaaaataaga gcctgcccaa 3480
gactttgggc ctcttgacag aattaaccac ttttatacat ctgagttctc ttggtaagtt 3540
ctttagcagt gttcaaagtc tactagctcg cattagtttc tgttgctgcc aacagatctg 3600
aactaatgct aacagatccc cctgagggat tcttgatggg ctgagcagct ggctggagct 3660
agtactgact gacattcatt gtgatgaggg cagctttctg gtacaggatt ctaagctcta 3720
tgttttatat acattttcat ctgtacttgc acctcacttt acacaagagg aaactatgca 3780
aagttagctg gatcgctcaa ggtcacttag gtaagttggc aagtccatgc ttcccactca 3840
gctcctcagg tcagcaagtc tacttctctg cctattttgt atactctctt taatatgtgc 3900
ctagctttgg aaagtctaga atgggtccct ggtgcctttt tactttgaag aaatcagttt 3960
ctgcctcttt ttggaaaaga aaacaaagtg caattgtttt ttactggaaa gttacccaat 4020
agcatgaggt gaacaggacg tagttaggcc ttcctgtaaa cagaaaatca tatcaaaaca 4080
ctatcttccc atctgtttct caatgcctgc tacttcttgt agatatttca tttcaggaga 4140
gcagcagtta aacccgtgga ttttgtagtt aggaacctgg gttcaaaccc tcttccacta 4200
attggctatg tctctggaca agtttttttt tttttttttt tttaaaccct ttctgaactt 4260
tcactttcta tgtctacctc aaagaattgt tgtgaggctt gagataatgc atttgtaaag 4320
ggtctgccag ataggaagat gctagttatg gatttacaag gttgttaagg ctgtaagagt 4380
ctaaaaccta cagtgaatca caatgcattt acccccactg acttggacat aagtgaaaac 4440
tagccagaag tctctttttc aaattactta caggttattc aatataaaat ttttgtaatg 4500
gataatctta tttatctaaa ctaaagcttc ctgtttatac acactcctgt tattctggga 4560

taagataaat gaccacagta ccttaatttc taggtgggtg cctgtgatgg ttcattgtag 4620

gtaaggacat tttctctttt tcagcagctg tgtaggtcca gagcctctgg gagaggaggg 4680

gggtagcatg cacccagcag gggactgaac tgggaaactc aaggttcttt ttactgtggg 4740

gtagtgagct gcctttctgt gatcggtttc cctagggatg ttgctgttcc cctccttgct 4800

attcgcagct acatacaacg tggccaaccc cagtaggctg atcctatata tgatcagtgc 4860

tggtgctgac tctcaatagc cccacccaag ctggctatag gtttacagat acattaatta 4920

ggcaacctaa aatattgatg ctggtgttgg tgtgacataa tgctatggcc agaactgaaa 4980

cttagagtta taattcatgt attagggttc tccagaggga cagaattagt aggatatatg 5040

tatatatgaa agggaggtta ttagggagaa ctggctccca cagttagaag gcgaagtcgc 5100

acaataggcc gtctgcaagc tgggttagag agaagccagt agtggctcag cctgagttca 5160

aaaacctcaa aactggggaa gctgacagtg cagccagcct tcagtctgtg gccaaaggcc 5220

caagagcccc tggcaaccaa cccactggtg caagtcctag attccaaagg ctgaagaacc 5280

tggagtctga tgtccaagag caggaagagt ggaagaaagc cagaagactc agcaaacaag 5340

gtagacagtg tctaccacca tagtggccat accaaagagg ctaccgattc cttcctgcta 5400

cctggatccc tgaagttgcc ctggtctctg caccttctaa acctagttct taagagcttt 5460

ccattacatg agctgtctca aagccctcca ataaattctc agtgtaagct tctgttgctt 5520

gtggacagaa aattctgaca gacctaccct ataagtgtta ctgtcaggat aacatgagaa 5580

cgcacaacag taagtggtca ctaagtgtta gctacggtta ttttgcccaa ggtagcatgg 5640

ctagttgatg ccggttgatg gggcttaaac ccagctccct catcttccag gcctctgtac 5700

tccctattcc actaaactac ctctcaggtt tattttttta aattcttact ctgcaagtac 5760

ataggaccac atttacctgg gaaaacaaga ataaaggctg ctctgcattt tttagaaact 5820

tttttgaaag ggagatggga atgcctgcac ccccaagtcc agaccaacac aatggttaat 5880

tgagatgaat aataaaggaa agactgttct gggcttccca gaatagcttg gtccttaaat 5940

tgtggcacaa acaacctcct gtcagagcca gcctcctgcc aggaagaggg gtaggagact 6000

agaggccgtg tgtgcagcct tgccctgaag gctagggtga caatttggag gctgtccaaa 6060

caccctggcc tctagagctg gcctgtctat ttgaaatgcc ggctctgatg ctaatcggcg 6120

accctcaggc aagttactta accttacatg cctcagtttt ctcatctgga aaatgagaac 6180

cctaggttta gggttgttag aaaagttaaa tgagttaaga caagtgcctg ggacacagta 6240

gcctcttgtg tgtgtttatc attatgtcct cagcaggtcg tagaagcagc ttctcaggtg 6300

tgaggctggc gcgattatct ggagtgggtt gggttttcta ggatggaccc cctgctgcat 6360

tttcctcatt catccaccag ggcttaatgg ggaatcaagg aatccatgtg taactgtata 6420

ataactgtag ccacactcca atgaccacct actagttgtc cctggcactg cttatacata 6480

tgtccatcaa atcaatccta tgaagtagat actgtcttca ttttatagat cagagacaat 6540

tggggttcag agagctgatg tgattttccc agggtcacag agagtcccag attcaggcac 6600

aactcttgta ttccaagaca caaccactac atgtccaaag gctgcccaga gccaccgggc 6660

acggcaaatt gtgacatatc cctaaagagg ctgagcacct ggtcaggatc tgatggctga 6720

cagtgtgtcc agatgcagag ctggagtggg ggaggggaag gggggctcct tgggacagag 6780

aaggctttct gtgctttctc tgaagggagc agtctgagga ccaagggaac ccggcaaaca 6840

gcacctcagg tactccaggc cctcctggct ggagagggct gtggcaatgg aaaattagtg 6900

ccaactgcaa tgagtcagcc tcggttaaat agagagtgaa gaatgctgga caggaacctc 6960

caccctcatg tcacatttct tcagtgtgac ccttctggcc cctctcctcc tgacagcgga 7020

acaatgactg ccccgatagg tgaggctgga ggaagaatca gtcctgtcct tggcaagctc 7080

ttcactatga cagtaaaggc tctctgcctg ctgccaaggc ctgtgacttt ctaacctggc 7140

ctcacgctgg gtaagcttaa ggtagaggtg caggattagc aagcccacct ggctaccagg 7200

ccgacagcta catctttcaa ctgaccctga tcaacgaaga gggacttgtg tctctcagtt 7260

ggttccaaat gaaaccaggg agcaggggcg ttaggaagct ccaacaggat ggtacttaat 7320

ggggcatttg agtggagagg taggtgacat agtgctttgg agcccaggga gggaaaggtt 7380

ctgctgaagt tgaattcaag actgttcttt catcacaaac ttgagtttcc tggacatttg 7440

tttgcagaaa caaccgtagg gttttgcctt aacctcgtgg gtttattatt acctcatagg 7500

gactttgcct cctgacagca gtttatgggt gttcattgtg gcacttgagt tttcttgcat 7560

acttgttaga gaaaccaagt ttgtcatcaa cttcttattt aacccccctgg ctataacttc 7620

atggattatg ttataattaa gccatccaga gtaaaatctg tttagattat cttggagtaa 7680

gggggaaaaa atctgtaatt ttttctcctc aactagatat atacataaaa aatgattgta 7740

ttgcttcatt taaaaaatat aacgcaaaat ctcttttcct tctaaaaaaa aaaaaaaaaa 7800

a 7801

<210> 12
<211> 7694
<212> mRNA
<213> Homo sapiens

<400> 12
atttgactca ctgaccctga tggggaagat agtgttattc ccacagtgtg gtagtacaca 60

ctggggctta tagggactga gcctactcaa gggtatatgg tgctgtgggt cagagctggg 120

gcatggccca gggattcagt gtgccttgac tccccctgtt ctgtggacaa tcacaatggg 180

aatccaagga gggtctgtcc tgttcgggct gctgctcgtc ctggctgtct tctgccattc 240

```
aggtcatagc ctgcagtgct acaactgtcc taacccaact gctgactgca aaacagccgt    300
caattgttca tctgattttg atgcgtgtct cattaccaaa gctgggttac aagtgtataa    360
caagtgttgg aagtttgagc attgcaattt caacgacgtc acaacccgct tgagggaaaa    420
tgagctaacg tactactgct gcaagaagga cctgtgtaac tttaacgaac agcttgaaaa    480
tggtgggaca tccttatcag agaaaacagt tcttctgctg gtgactccat ttctggcagc    540
agcctggagc cttcatccct aagtcaacac caggagagct tctcccaaac tccccgttcc    600
tgcgtagtcc gctttctctt gctgccacat tctaaaggct tgatattttc caaatggatc    660
ctgttgggaa agaataaaat tagcttgagc aacctggcta agatagaggg gctctgggag    720
actttgaaga ccagtcctgt ttgcagggaa gccccacttg aaggaagaag tctaagagtg    780
aagtaggtgt gacttgaact agattgcatg cttcctcctt tgctcttggg aagaccagct    840
ttgcagtgac agcttgagtg ggttctctgc agccctcaga ttatttttcc tctggctcct    900
tggatgtagt cagttagcat cattagtaca tctttggagg gtggggcagg agtatatgag    960
catcctctct cacatggaac gctttcataa acttcaggga tcccgtgttg ccatggaggc   1020
atgccaaatg ttccatatgt gggtgtcagt cagggacaac aagatcctta atgcagagct   1080
agaggacttc tggcagggaa gtggggaagt gttccagata gcagggcatg aaaacttaga   1140
gaggtacaag tggctgaaaa tcgagttttt cctctgtctt taaattttat atgggctttg   1200
ttatcttcca ctggaaaagt gtaatagcat acatcaatgg tgtgttaaag ctatttcctt   1260
gcctttttttt tattggaatg gtaggatatc ttggctttgc cacacacagt tacagagtga   1320
acactctact acatgtgact ggcagtatta agtgtgctta ttttaaatgt tactggtaga   1380
aaggcagttc aggtatgtgt gtatatagta tgaatgcagt ggggacaccc tttgtggtta   1440
cagtttgaga cttccaaagg tcatccttaa taacaacaga tctgcagggg tatgttttac   1500
catctgcatc cagcctcctg ctaactccta gctgactcag catagattgt ataaaatacc   1560
tttgtaacgg ctcttagcac actcacagat gtttgaggct ttcagaagct cttctaaaaa   1620
atgatacaca cctttcacaa gggcaaactt tttccttttc cctgtgtatt ctagtgaatg   1680
aatctcaaga ttcagtagac ctaatgacat ttgtatttta tgatcttggc tgtatttaat   1740
ggcataggct gacttttgca gatggaggaa tttcttgatt aatgttgaaa aaaaaccctt   1800
gattatactc tgttggacaa accgagtgca atgaatgatg cttttctgaa aatgaaatat   1860
aacaagtggg tgaatgtggt tatggccgaa aaggatatgc agtatgctta atggtagcaa   1920
ctgaaagaag acatcctgag cagtgccagc tttcttctgt tgatgccgtt ccctgaacat   1980
aggaaaatag aaacttgctt atcaaaactt agcattacct tggtgctctg tgttctctgt   2040
tagctcagtg tctttcctta catcaatagg tttttttttt tttttttggc ctgaggaagt   2100
actgaccatg cccacagcca ccggctgagc aaagaagctc atttcatgtg agttctaagg   2160
```

aatgagaaac aattttgatg aatttaagca gaaaatgaat ttctgggaac ttttttgggg 2220
gcggggggt ggggaattca gccacactcc agaaagccag gagtcgacag ttttggaagc 2280
ctctctcagg attgagattc taggatgaga ttggcttact gctatcttgt gtcatgtacc 2340
cactttttgg ccagactaca ctgggaagaa ggtagtcctc taaagcaaaa tctgagtgcc 2400
actaaatggg gagatggggc tgttaagctg tccaaatcaa caagggtcat ataaatggcc 2460
ttaaactttg gggttgcttt ctgcaaaaag ttgctgtgac tcatgccata gacaaggttg 2520
agtgcctgga cccaaaggca atactgtaat gtaaagacat ttatagtact aggcaaacag 2580
caccccaggt actccaggcc ctcctggctg gagagggctg tggcaataga aaattagtgc 2640
caactgcagt gagtcagcct aggttaaata gagagtgtaa gagtgctgga caggaacctc 2700
caccctcatg tcacatttct tcaatgtgac ccttctggcc cctctcctcc tgacagcgga 2760
acaatgactg ccccgatagg tgaggctgga ggaagaatca gtcctgtcct tggcaagctc 2820
ttcactatga cagtaaaggc tctctgcctg ctgccaaggc ctgtgacttt ctaacctggc 2880
ctcacgctgg gtaagcttaa ggtagaggtg caggattagc aagcccacct ggctaccagg 2940
ccgacagcta catcctccaa ctgaccctga tcaacgaaga gggattcatg tgtctgtctc 3000
agttggttcc aaatgaaacc agggagcagg ggagttagga atcgaacacc agtcatgcct 3060
actggctctc tgctcgagag ccaataccct gtgccctcca ctcatctgga tttacaggaa 3120
ctgtcatagt gttcagtatt gggtggtgat aagcccattg gattgtcccc ttggggggat 3180
gagctagggg tgcaaggaac acctgatgag tagataagtg gagctcatgg tatttcctga 3240
aagatgctaa tctatttgcc aaacttggtc ttgaatgtac tgggggcttc aaggtatggg 3300
tatatttttc ttgtgtcctt gcagttagcc cccatgtctt atgtgtgtcc tgaaaaaata 3360
agagcctgcc caagactttg ggcctcttga cagaattaac cacttttata catctgagtt 3420
ctcttggtaa gttctttagc agtgttcaaa gtctactagc tcgcattagt ttctgttgct 3480
gccaacagat ctgaactaat gctaacagat ccccctgagg gattcttgat gggctgagca 3540
gctggctgga gctagtactg actgacattc attgtgatga gggcagcttt ctggtacagg 3600
attctaagct ctatgtttta tatacatttt catctgtact tgcacctcac tttacacaag 3660
aggaaactat gcaaagttag ctggatcgct caaggtcact taggtaagtt ggcaagtcca 3720
tgcttcccac tcagctcctc aggtcagcaa gtctacttct ctgcctattt tgtatactct 3780
ctttaatatg tgcctagctt tggaaagtct agaatgggtc cctggtgcct ttttactttg 3840
aagaaatcag tttctgcctc tttttggaaa agaaaacaaa gtgcaattgt tttttactgg 3900
aaagttaccc aatagcatga ggtgaacagg acgtagttag gccttcctgt aaacagaaaa 3960
tcatatcaaa acactatctt cccatctgtt tctcaatgcc tgctacttct tgtagatatt 4020

tcatttcagg agagcagcag ttaaacccgt ggattttgta gttaggaacc tgggttcaaa 4080
ccctcttcca ctaattggct atgtctctgg acaagttttt tttttttttt ttttttaaac 4140
cctttctgaa ctttcacttt ctatgtctac ctcaaagaat tgttgtgagg cttgagataa 4200
tgcatttgta aagggtctgc cagataggaa gatgctagtt atggatttac aaggttgtta 4260
aggctgtaag agtctaaaac ctacagtgaa tcacaatgca tttacccccca ctgacttgga 4320
cataagtgaa aactagccag aagtctcttt ttcaaattac ttacaggtta ttcaatataa 4380
aatttttgta atggataatc ttatttatct aaactaaagc ttcctgttta tacacactcc 4440
tgttattctg ggataagata aatgaccaca gtaccttaat ttctaggtgg gtgcctgtga 4500
tggttcattg taggtaagga cattttctct ttttcagcag ctgtgtaggt ccagagcctc 4560
tgggagagga gggggggtagc atgcacccag caggggactg aactgggaaa ctcaaggttc 4620
tttttactgt ggggtagtga gctgcctttc tgtgatcggt ttccctaggg atgttgctgt 4680
tcccctcctt gctattcgca gctacataca acgtggccaa ccccagtagg ctgatcctat 4740
atatgatcag tgctggtgct gactctcaat agccccaccc aagctggcta taggtttaca 4800
gatacattaa ttaggcaacc taaaatattg atgctggtgt tggtgtgaca taatgctatg 4860
gccagaactg aaacttagag ttataattca tgtattaggg ttctccagag ggacagaatt 4920
agtaggatat atgtatatat gaaagggagg ttattaggga gaactggctc ccacagttag 4980
aaggcgaagt cgcacaatag gccgtctgca agctgggtta gagagaagcc agtagtggct 5040
cagcctgagt tcaaaaacct caaaactggg gaagctgaca gtgcagccag ccttcagtct 5100
gtggccaaag gcccaagagc ccctggcaac caacccactg gtgcaagtcc tagattccaa 5160
aggctgaaga acctggagtc tgatgtccaa gagcaggaag agtggaagaa agccagaaga 5220
ctcagcaaac aaggtagaca gtgtctacca ccatagtggc cataccaaag aggctaccga 5280
ttccttcctg ctacctggat ccctgaagtt gccctggtct ctgcaccttc taaacctagt 5340
tcttaagagc tttccattac atgagctgtc tcaaagccct ccaataaatt ctcagtgtaa 5400
gcttctgttg cttgtggaca gaaaattctg acagacctac cctataagtg ttactgtcag 5460
gataacatga gaacgcacaa cagtaagtgg tcactaagtg ttagctacgg ttattttgcc 5520
caaggtagca tggctagttg atgccggttg atggggctta aacccagctc cctcatcttc 5580
caggcctctg tactccctat tccactaaac tacctctcag gtttattttt ttaaattctt 5640
actctgcaag tacataggac cacatttacc tgggaaaaca agaataaagg ctgctctgca 5700
ttttttagaa actttttttga aagggagatg ggaatgcctg cacccccaag tccagaccaa 5760
cacaatggtt aattgagatg aataataaag gaaagactgt tctgggcttc ccagaatagc 5820
ttggtcctta aattgtggca caaacaacct cctgtcagag ccagcctcct gccaggaaga 5880
ggggtaggag actagaggcc gtgtgtgcag ccttgccctg aaggctaggg tgacaatttg 5940

gaggctgtcc aaacaccctg gcctctagag ctggcctgtc tatttgaaat gccggctctg 6000
atgctaatcg gcgaccctca ggcaagttac ttaaccttac atgcctcagt tttctcatct 6060
ggaaaatgag aaccctaggt ttagggttgt tagaaaagtt aaatgagtta agacaagtgc 6120
ctgggacaca gtagcctctt gtgtgtgttt atcattatgt cctcagcagg tcgtagaagc 6180
agcttctcag gtgtgaggct ggcgcgatta tctggagtgg gttgggtttt ctaggatgga 6240
ccccctgctg cattttcctc attcatccac cagggcttaa tggggaatca aggaatccat 6300
gtgtaactgt ataataactg tagccacact ccaatgacca cctactagtt gtccctggca 6360
ctgcttatac atatgtccat caaatcaatc ctatgaagta gatactgtct tcattttata 6420
gatcagagac aattggggtt cagagagctg atgtgatttt cccagggtca cagagagtcc 6480
cagattcagg cacaactctt gtattccaag acacaaccac tacatgtcca aaggctgccc 6540
agagccaccg ggcacggcaa attgtgacat atccctaaag aggctgagca cctggtcagg 6600
atctgatggc tgacagtgtg tccagatgca gagctggagt gggggagggg aagggggcct 6660
ccttgggaca gagaaggctt tctgtgcttt ctctgaaggg agcagtctga ggaccaaggg 6720
aacccggcaa acagcacctc aggtactcca ggccctcctg gctggagagg gctgtggcaa 6780
tggaaaatta gtgccaactg caatgagtca gcctcggtta aatagagagt gaagaatgct 6840
ggacaggaac ctccaccctc atgtcacatt tcttcagtgt gacccttctg gcccctctcc 6900
tcctgacagc ggaacaatga ctgccccgat aggtgaggct ggaggaagaa tcagtcctgt 6960
ccttggcaag ctcttcacta tgacagtaaa ggctctctgc ctgctgccaa ggcctgtgac 7020
tttctaacct ggcctcacgc tgggtaagct taaggtagag gtgcaggatt agcaagccca 7080
cctggctacc aggccgacag ctacatcttt caactgaccc tgatcaacga agagggactt 7140
gtgtctctca gttggttcca aatgaaacca gggagcaggg gcgttaggaa gctccaacag 7200
gatggtactt aatggggcat ttgagtggag aggtaggtga catagtgctt tggagcccag 7260
ggagggaaag gttctgctga agttgaattc aagactgttc tttcatcaca aacttgagtt 7320
tcctggacat ttgtttgcag aaacaaccgt agggttttgc cttaacctcg tgggtttatt 7380
attacctcat agggactttg cctcctgaca gcagtttatg ggtgttcatt gtggcacttg 7440
agttttcttg catacttgtt agagaaacca agtttgtcat caacttctta tttaaccccc 7500
tggctataac ttcatggatt atgttataat taagccatcc agagtaaaat ctgtttagat 7560
tatcttggag taagggggaa aaaatctgta attttttctc ctcaactaga tatatacata 7620
aaaaatgatt gtattgcttc atttaaaaaa tataacgcaa aatctctttt ccttctaaaa 7680
aaaaaaaaaa aaaa 7694

<210> 13

<211> 7629
<212> mRNA
<213> Homo sapiens

<400> 13
atttgactca ctgaccctga tggggaagat agtgttattc ccacagtgtg gtagtacaca 60

ctggggctta tagggactga gcctactcaa gggttctgtg gacaatcaca atgggaatcc 120

aaggagggtc tgtcctgttc gggctgctgc tcgtcctggc tgtcttctgc cattcaggtc 180

atagcctgca gtgctacaac tgtcctaacc caactgctga ctgcaaaaca gccgtcaatt 240

gttcatctga ttttgatgcg tgtctcatta ccaaagctgg gttacaagtg tataacaagt 300

gttggaagtt tgagcattgc aatttcaacg acgtcacaac ccgcttgagg gaaaatgagc 360

taacgtacta ctgctgcaag aaggacctgt gtaactttaa cgaacagctt gaaaatggtg 420

ggacatcctt atcagagaaa acagttcttc tgctggtgac tccatttctg gcagcagcct 480

ggagccttca tccctaagtc aacaccagga gagcttctcc caaactcccc gttcctgcgt 540

agtccgcttt ctcttgctgc cacattctaa aggcttgata ttttccaaat ggatcctgtt 600

gggaaagaat aaaattagct tgagcaacct ggctaagata gaggggctct gggagacttt 660

gaagaccagt cctgtttgca gggaagcccc acttgaagga agaagtctaa gagtgaagta 720

ggtgtgactt gaactagatt gcatgcttcc tcctttgctc ttgggaagac cagctttgca 780

gtgacagctt gagtgggttc tctgcagccc tcagattatt tttcctctgg ctccttggat 840

gtagtcagtt agcatcatta gtacatcttt ggagggtggg gcaggagtat atgagcatcc 900

tctctcacat ggaacgcttt cataaacttc agggatcccg tgttgccatg gaggcatgcc 960

aaatgttcca tatgtgggtg tcagtcaggg acaacaagat ccttaatgca gagctagagg 1020

acttctggca gggaagtggg gaagtgttcc agatagcagg gcatgaaaac ttagagaggt 1080

acaagtggct gaaaatcgag tttttcctct gtctttaaat tttatatggg ctttgttatc 1140

ttccactgga aaagtgtaat agcatacatc aatggtgtgt taaagctatt tccttgcctt 1200

ttttttattg gaatggtagg atatcttggc tttgccacac acagttacag agtgaacact 1260

ctactacatg tgactggcag tattaagtgt gcttatttta aatgttactg gtagaaaggc 1320

agttcaggta tgtgtgtata tagtatgaat gcagtgggga caccctttgt ggttacagtt 1380

tgagacttcc aaaggtcatc cttaataaca acagatctgc aggggtatgt tttaccatct 1440

gcatccagcc tcctgctaac tcctagctga ctcagcatag attgtataaa ataccttttgt 1500

aacggctctt agcacactca cagatgtttg aggctttcag aagctcttct aaaaaatgat 1560

acacaccttt cacaagggca aactttttcc ttttccctgt gtattctagt gaatgaatct 1620

caagattcag tagacctaat gacatttgta ttttatgatc ttggctgtat ttaatggcat 1680

aggctgactt ttgcagatgg aggaatttct tgattaatgt tgaaaaaaaa cccttgatta 1740

tactctgttg gacaaaccga gtgcaatgaa tgatgctttt ctgaaaatga aatataacaa 1800
gtgggtgaat gtggttatgg ccgaaaagga tatgcagtat gcttaatggt agcaactgaa 1860
agaagacatc ctgagcagtg ccagctttct tctgttgatg ccgttccctg aacataggaa 1920
aatagaaact tgcttatcaa aacttagcat taccttggtg ctctgtgttc tctgttagct 1980
cagtgtcttt ccttacatca ataggttttt tttttttttt ttggcctgag gaagtactga 2040
ccatgcccac agccaccggc tgagcaaaga agctcatttc atgtgagttc taaggaatga 2100
gaaacaattt tgatgaattt aagcagaaaa tgaatttctg ggaacttttt tgggggcggg 2160
ggggtgggga attcagccac actccagaaa gccaggagtc gacagttttg gaagcctctc 2220
tcaggattga gattctagga tgagattggc ttactgctat cttgtgtcat gtacccactt 2280
tttggccaga ctacactggg aagaaggtag tcctctaaag caaaatctga gtgccactaa 2340
atggggagat ggggctgtta agctgtccaa atcaacaagg gtcatataaa tggccttaaa 2400
ctttggggtt gctttctgca aaaagttgct gtgactcatg ccatagacaa ggttgagtgc 2460
ctggacccaa aggcaatact gtaatgtaaa gacatttata gtactaggca aacagcaccc 2520
caggtactcc aggccctcct ggctggagag ggctgtggca atagaaaatt agtgccaact 2580
gcagtgagtc agcctaggtt aaatagagag tgtaagagtg ctggacagga acctccaccc 2640
tcatgtcaca tttcttcaat gtgacccttc tggcccctct cctcctgaca gcggaacaat 2700
gactgccccg ataggtgagg ctggaggaag aatcagtcct gtccttggca agctcttcac 2760
tatgacagta aaggctctct gcctgctgcc aaggcctgtg actttctaac ctggcctcac 2820
gctgggtaag cttaaggtag aggtgcagga ttagcaagcc cacctggcta ccaggccgac 2880
agctacatcc tccaactgac cctgatcaac gaagagggat tcatgtgtct gtctcagttg 2940
gttccaaatg aaaccaggga gcaggggagt taggaatcga acaccagtca tgcctactgg 3000
ctctctgctc gagagccaat accctgtgcc ctccactcat ctggatttac aggaactgtc 3060
atagtgttca gtattgggtg gtgataagcc cattggattg tccccttggg ggatgagct 3120
aggggtgcaa ggaacacctg atgagtagat aagtggagct catggtattt cctgaaagat 3180
gctaatctat ttgccaaact tggtcttgaa tgtactgggg gcttcaaggt atgggtatat 3240
ttttcttgtg tccttgcagt tagcccccat gtcttatgtg tgtcctgaaa aaataagagc 3300
ctgcccaaga ctttgggcct cttgacagaa ttaaccactt ttatacatct gagttctctt 3360
ggtaagttct ttagcagtgt tcaaagtcta ctagctcgca ttagtttctg ttgctgccaa 3420
cagatctgaa ctaatgctaa cagatccccc tgagggattc ttgatgggct gagcagctgg 3480
ctggagctag tactgactga cattcattgt gatgagggca gctttctggt acaggattct 3540
aagctctatg ttttatatac attttcatct gtacttgcac ctcactttac acaagaggaa 3600
actatgcaaa gttagctgga tcgctcaagg tcacttaggt aagttggcaa gtccatgctt 3660

cccactcagc tcctcaggtc agcaagtcta cttctctgcc tattttgtat actctcttta 3720

atatgtgcct agctttggaa agtctagaat gggtccctgg tgccttttta ctttgaagaa 3780

atcagtttct gcctcttttt ggaaaagaaa acaaagtgca attgtttttt actggaaagt 3840

tacccaatag catgaggtga acaggacgta gttaggcctt cctgtaaaca gaaaatcata 3900

tcaaaacact atcttcccat ctgtttctca atgcctgcta cttcttgtag atatttcatt 3960

tcaggagagc agcagttaaa cccgtggatt ttgtagttag gaacctgggt tcaaaccctc 4020

ttccactaat tggctatgtc tctggacaag tttttttttt tttttttttt taaacccttt 4080

ctgaactttc actttctatg tctacctcaa agaattgttg tgaggcttga gataatgcat 4140

ttgtaaaggg tctgccagat aggaagatgc tagttatgga tttacaaggt tgttaaggct 4200

gtaagagtct aaaacctaca gtgaatcaca atgcatttac ccccactgac ttggacataa 4260

gtgaaaacta gccagaagtc tctttttcaa attacttaca ggttattcaa tataaaattt 4320

ttgtaatgga taatcttatt tatctaaact aaagcttcct gtttatacac actcctgtta 4380

ttctgggata agataaatga ccacagtacc ttaatttcta ggtgggtgcc tgtgatggtt 4440

cattgtaggt aaggacattt tctctttttc agcagctgtg taggtccaga gcctctggga 4500

gaggaggggg gtagcatgca cccagcaggg gactgaactg ggaaactcaa ggttcttttt 4560

actgtggggt agtgagctgc ctttctgtga tcggtttccc tagggatgtt gctgttcccc 4620

tccttgctat tcgcagctac atacaacgtg gccaacccca gtaggctgat cctatatatg 4680

atcagtgctg gtgctgactc tcaatagccc cacccaagct ggctataggt ttacagatac 4740

attaattagg caacctaaaa tattgatgct ggtgttggtg tgacataatg ctatggccag 4800

aactgaaact tagagttata attcatgtat tagggttctc cagagggaca gaattagtag 4860

gatatatgta tatatgaaag ggaggttatt agggagaact ggctcccaca gttagaaggc 4920

gaagtcgcac aataggccgt ctgcaagctg ggttagagag aagccagtag tggctcagcc 4980

tgagttcaaa aacctcaaaa ctggggaagc tgacagtgca gccagccttc agtctgtggc 5040

caaaggccca agagcccctg gcaaccaacc cactggtgca agtcctagat tccaaaggct 5100

gaagaacctg gagtctgatg tccaagagca ggaagagtgg aagaaagcca gaagactcag 5160

caaacaaggt agacagtgtc taccaccata gtggccatac caaagaggct accgattcct 5220

tcctgctacc tggatccctg aagttgccct ggtctctgca ccttctaaac ctagttctta 5280

agagctttcc attacatgag ctgtctcaaa gccctccaat aaattctcag tgtaagcttc 5340

tgttgcttgt ggacagaaaa ttctgacaga cctaccctat aagtgttact gtcaggataa 5400

catgagaacg cacaacagta agtggtcact aagtgttagc tacggttatt ttgcccaagg 5460

tagcatggct agttgatgcc ggttgatggg gcttaaaccc agctccctca tcttccaggc 5520

ctctgtactc cctattccac taaactacct ctcaggttta ttttttttaaa ttcttactct 5580

gcaagtacat aggaccacat ttacctggga aaacaagaat aaaggctgct ctgcattttt 5640

tagaaacttt tttgaaaggg agatgggaat gcctgcaccc ccaagtccag accaacacaa 5700

tggttaattg agatgaataa taaaggaaag actgttctgg gcttcccaga atagcttggt 5760

ccttaaattg tggcacaaac aacctcctgt cagagccagc ctcctgccag gaagaggggt 5820

aggagactag aggccgtgtg tgcagccttg ccctgaaggc tagggtgaca atttggaggc 5880

tgtccaaaca ccctggcctc tagagctggc ctgtctattt gaaatgccgg ctctgatgct 5940

aatcggcgac cctcaggcaa gttacttaac cttacatgcc tcagttttct catctggaaa 6000

atgagaaccc taggtttagg gttgttagaa aagttaaatg agttaagaca agtgcctggg 6060

acacagtagc ctcttgtgtg tgtttatcat tatgtcctca gcaggtcgta gaagcagctt 6120

ctcaggtgtg aggctggcgc gattatctgg agtgggttgg gttttctagg atggaccccc 6180

tgctgcattt tcctcattca tccaccaggg cttaatgggg aatcaaggaa tccatgtgta 6240

actgtataat aactgtagcc acactccaat gaccacctac tagttgtccc tggcactgct 6300

tatacatatg tccatcaaat caatcctatg aagtagatac tgtcttcatt ttatagatca 6360

gagacaattg gggttcagag agctgatgtg attttcccag ggtcacagag agtcccagat 6420

tcaggcacaa ctcttgtatt ccaagacaca accactacat gtccaaaggc tgcccagagc 6480

caccgggcac ggcaaattgt gacatatccc taaagaggct gagcacctgg tcaggatctg 6540

atggctgaca gtgtgtccag atgcagagct ggagtggggg aggggaaggg gggctccttg 6600

ggacagagaa ggctttctgt gctttctctg aagggagcag tctgaggacc aagggaaccc 6660

ggcaaacagc acctcaggta ctccaggccc tcctggctgg agagggctgt ggcaatggaa 6720

aattagtgcc aactgcaatg agtcagcctc ggttaaatag agagtgaaga atgctggaca 6780

ggaacctcca ccctcatgtc acatttcttc agtgtgaccc ttctggcccc tctcctcctg 6840

acagcggaac aatgactgcc ccgataggtg aggctggagg aagaatcagt cctgtccttg 6900

gcaagctctt cactatgaca gtaaaggctc tctgcctgct gccaaggcct gtgactttct 6960

aacctggcct cacgctgggt aagcttaagg tagaggtgca ggattagcaa gcccacctgg 7020

ctaccaggcc gacagctaca tctttcaact gaccctgatc aacgaagagg gacttgtgtc 7080

tctcagttgg ttccaaatga aaccagggag caggggcgtt aggaagctcc aacaggatgg 7140

tacttaatgg ggcatttgag tggagaggta ggtgacatag tgctttggag cccagggagg 7200

gaaaggttct gctgaagttg aattcaagac tgttctttca tcacaaactt gagtttcctg 7260

gacatttgtt tgcagaaaca accgtagggt tttgccttaa cctcgtgggt ttattattac 7320

ctcataggga ctttgcctcc tgacagcagt ttatgggtgt tcattgtggc acttgagttt 7380

tcttgcatac ttgttagaga aaccaagttt gtcatcaact tcttatttaa ccccctggct 7440

ataacttcat ggattatgtt ataattaagc catccagagt aaaatctgtt tagattatct 7500

tggagtaagg gggaaaaaat ctgtaatttt ttctcctcaa ctagatatat acataaaaaa 7560

tgattgtatt gcttcattta aaaaatataa cgcaaaatct cttttccttc taaaaaaaaa 7620

aaaaaaaaa 7629

<210> 14
<211> 7573
<212> mRNA
<213> Homo sapiens

<400> 14
attatacttc ttacagtgag gagccagagc ttccaggttc tgtggacaat cacaatggga 60

atccaaggag ggtctgtcct gttcgggctg ctgctcgtcc tggctgtctt ctgccattca 120

ggtcatagcc tgcagtgcta caactgtcct aacccaactg ctgactgcaa aacagccgtc 180

aattgttcat ctgattttga tgcgtgtctc attaccaaag ctgggttaca agtgtataac 240

aagtgttgga agtttgagca ttgcaatttc aacgacgtca caacccgctt gagggaaaat 300

gagctaacgt actactgctg caagaaggac ctgtgtaact ttaacgaaca gcttgaaaat 360

ggtgggacat ccttatcaga gaaaacagtt cttctgctgg tgactccatt tctggcagca 420

gcctggagcc ttcatcccta agtcaacacc aggagagctt ctcccaaact ccccgttcct 480

gcgtagtccg ctttctcttg ctgccacatt ctaaaggctt gatattttcc aaatggatcc 540

tgttgggaaa gaataaaatt agcttgagca acctggctaa gatagagggg ctctgggaga 600

ctttgaagac cagtcctgtt tgcagggaag ccccacttga aggaagaagt ctaagagtga 660

agtaggtgtg acttgaacta gattgcatgc ttcctccttt gctcttggga agaccagctt 720

tgcagtgaca gcttgagtgg gttctctgca gccctcagat tatttttcct ctggctcctt 780

ggatgtagtc agttagcatc attagtacat ctttggaggg tggggcagga gtatatgagc 840

atcctctctc acatggaacg ctttcataaa cttcagggat cccgtgttgc catggaggca 900

tgccaaatgt tccatatgtg ggtgtcagtc agggacaaca agatccttaa tgcagagcta 960

gaggacttct ggcagggaag tggggaagtg ttccagatag cagggcatga aaacttagag 1020

aggtacaagt ggctgaaaat cgagtttttc ctctgtcttt aaattttata tgggctttgt 1080

tatcttccac tggaaaagtg taatagcata catcaatggt gtgttaaagc tatttccttg 1140

cctttttttt attggaatgg taggatatct tggctttgcc acacacagtt acagagtgaa 1200

cactctacta catgtgactg gcagtattaa gtgtgcttat tttaaatgtt actggtagaa 1260

aggcagttca ggtatgtgtg tatatagtat gaatgcagtg ggacaccct ttgtggttac 1320

agtttgagac ttccaaaggt catccttaat aacaacagat ctgcaggggt atgttttacc 1380

atctgcatcc agcctcctgc taactcctag ctgactcagc atagattgta taaaatacct 1440

ttgtaacggc tcttagcaca ctcacagatg tttgaggctt tcagaagctc ttctaaaaaa 1500
tgatacacac ctttcacaag ggcaaacttt ttccttttcc ctgtgtattc tagtgaatga 1560
atctcaagat tcagtagacc taatgacatt tgtattttat gatcttggct gtatttaatg 1620
gcataggctg acttttgcag atggaggaat ttcttgatta atgttgaaaa aaaacccttg 1680
attatactct gttggacaaa ccgagtgcaa tgaatgatgc ttttctgaaa atgaaatata 1740
acaagtgggt gaatgtggtt atggccgaaa aggatatgca gtatgcttaa tggtagcaac 1800
tgaaagaaga catcctgagc agtgccagct ttcttctgtt gatgccgttc cctgaacata 1860
ggaaaataga aacttgctta tcaaaactta gcattacctt ggtgctctgt gttctctgtt 1920
agctcagtgt ctttccttac atcaataggt tttttttttt tttttggcc tgaggaagta 1980
ctgaccatgc ccacagccac cggctgagca aagaagctca tttcatgtga gttctaagga 2040
atgagaaaca attttgatga atttaagcag aaaatgaatt tctgggaact tttttggggg 2100
cgggggggtg gggaattcag ccacactcca gaaagccagg agtcgacagt tttggaagcc 2160
tctctcagga ttgagattct aggatgagat tggcttactg ctatcttgtg tcatgtaccc 2220
actttttggc cagactacac tgggaagaag gtagtcctct aaagcaaaat ctgagtgcca 2280
ctaaatgggg agatggggct gttaagctgt ccaaatcaac aagggtcata taaatggcct 2340
taaactttgg ggttgctttc tgcaaaaagt tgctgtgact catgccatag acaaggttga 2400
gtgcctggac ccaaaggcaa tactgtaatg taaagacatt tatagtacta ggcaaacagc 2460
accccaggta ctccaggccc tcctggctgg agagggctgt ggcaatagaa aattagtgcc 2520
aactgcagtg agtcagccta ggttaaatag agagtgtaag agtgctggac aggaacctcc 2580
accctcatgt cacatttctt caatgtgacc cttctggccc ctctcctcct gacagcggaa 2640
caatgactgc cccgataggt gaggctggag gaagaatcag tcctgtcctt ggcaagctct 2700
tcactatgac agtaaaggct ctctgcctgc tgccaaggcc tgtgactttc taacctggcc 2760
tcacgctggg taagcttaag gtagaggtgc aggattagca agcccacctg gctaccaggc 2820
cgacagctac atcctccaac tgaccctgat caacgaagag ggattcatgt gtctgtctca 2880
gttggttcca aatgaaacca gggagcaggg gagttaggaa tcgaacacca gtcatgccta 2940
ctggctctct gctcgagagc caataccctg tgccctccac tcatctggat ttacaggaac 3000
tgtcatagtg ttcagtattg ggtggtgata agcccattgg attgtcccct tgggggggatg 3060
agctaggggt gcaaggaaca cctgatgagt agataagtgg agctcatggt atttcctgaa 3120
agatgctaat ctatttgcca aacttggtct tgaatgtact gggggcttca aggtatgggt 3180
atatttttct tgtgtccttg cagttagccc ccatgtctta tgtgtgtcct gaaaaaataa 3240
gagcctgccc aagactttgg gcctcttgac agaattaacc acttttatac atctgagttc 3300

tcttggtaag ttctttagca gtgttcaaag tctactagct cgcattagtt tctgttgctg 3360
ccaacagatc tgaactaatg ctaacagatc cccctgaggg attcttgatg ggctgagcag 3420
ctggctggag ctagtactga ctgacattca ttgtgatgag ggcagctttc tggtacagga 3480
ttctaagctc tatgttttat atacattttc atctgtactt gcacctcact ttacacaaga 3540
ggaaactatg caaagttagc tggatcgctc aaggtcactt aggtaagttg gcaagtccat 3600
gcttcccact cagctcctca ggtcagcaag tctacttctc tgcctatttt gtatactctc 3660
tttaatatgt gcctagcttt ggaaagtcta gaatgggtcc ctggtgcctt tttactttga 3720
agaaatcagt ttctgcctct ttttggaaaa gaaaacaaag tgcaattgtt ttttactgga 3780
aagttaccca atagcatgag gtgaacagga cgtagttagg ccttcctgta aacagaaaat 3840
catatcaaaa cactatcttc ccatctgttt ctcaatgcct gctacttctt gtagatattt 3900
catttcagga gagcagcagt taaacccgtg gattttgtag ttaggaacct gggttcaaac 3960
cctcttccac taattggcta tgtctctgga caagtttttt tttttttttt tttttaaacc 4020
ctttctgaac tttcactttc tatgtctacc tcaaagaatt gttgtgaggc ttgagataat 4080
gcatttgtaa agggtctgcc agataggaag atgctagtta tggatttaca aggttgttaa 4140
ggctgtaaga gtctaaaacc tacagtgaat cacaatgcat ttaccccccac tgacttggac 4200
ataagtgaaa actagccaga agtctctttt tcaaattact tacaggttat tcaatataaa 4260
atttttgtaa tggataatct tatttatcta aactaaagct tcctgtttat acacactcct 4320
gttattctgg gataagataa atgaccacag taccttaatt tctaggtggg tgcctgtgat 4380
ggttcattgt aggtaaggac attttctctt tttcagcagc tgtgtaggtc cagagcctct 4440
gggagaggag gggggtagca tgcacccagc aggggactga actgggaaac tcaaggttct 4500
ttttactgtg ggtagtgag ctgcctttct gtgatcggtt tccctaggga tgttgctgtt 4560
cccctccttg ctattcgcag ctacatacaa cgtggccaac cccagtaggc tgatcctata 4620
tatgatcagt gctggtgctg actctcaata gccccaccca agctggctat aggtttacag 4680
atacattaat taggcaacct aaaatattga tgctggtgtt ggtgtgacat aatgctatgg 4740
ccagaactga aacttagagt tataattcat gtattagggt tctccagagg gacagaatta 4800
gtaggatata tgtatatatg aaagggaggt tattagggag aactggctcc cacagttaga 4860
aggcgaagtc gcacaatagg ccgtctgcaa gctgggttag agagaagcca gtagtggctc 4920
agcctgagtt caaaaacctc aaaactgggg aagctgacag tgcagccagc cttcagtctg 4980
tggccaaagg cccaagagcc cctggcaacc aacccactgg tgcaagtcct agattccaaa 5040
ggctgaagaa cctggagtct gatgtccaag agcaggaaga gtggaagaaa gccagaagac 5100
tcagcaaaca aggtagacag tgtctaccac catagtggcc ataccaaaga ggctaccgat 5160
tccttcctgc tacctggatc cctgaagttg ccctggtctc tgcaccttct aaacctagtt 5220

cttaagagct ttccattaca tgagctgtct caaagccctc caataaattc tcagtgtaag 5280
cttctgttgc ttgtggacag aaaattctga cagacctacc ctataagtgt tactgtcagg 5340
ataacatgag aacgcacaac agtaagtggt cactaagtgt tagctacggt tattttgccc 5400
aaggtagcat ggctagttga tgccggttga tggggcttaa acccagctcc ctcatcttcc 5460
aggcctctgt actccctatt ccactaaact acctctcagg tttatttttt taaattctta 5520
ctctgcaagt acataggacc acatttacct gggaaaacaa gaataaaggc tgctctgcat 5580
tttttagaaa ctttttttgaa agggagatgg gaatgcctgc accccccaagt ccagaccaac 5640
acaatggtta attgagatga ataataaagg aaagactgtt ctgggcttcc cagaatagct 5700
tggtccttaa attgtggcac aaacaacctc ctgtcagagc cagcctcctg ccaggaagag 5760
gggtaggaga ctagaggccg tgtgtgcagc cttgccctga aggctagggt gacaatttgg 5820
aggctgtcca aacaccctgg cctctagagc tggcctgtct atttgaaatg ccggctctga 5880
tgctaatcgg cgaccctcag gcaagttact taaccttaca tgcctcagtt ttctcatctg 5940
gaaaatgaga accctaggtt tagggttgtt agaaaagtta aatgagttaa gacaagtgcc 6000
tgggacacag tagcctcttg tgtgtgttta tcattatgtc ctcagcaggt cgtagaagca 6060
gcttctcagg tgtgaggctg gcgcgattat ctggagtggg ttgggttttc taggatggac 6120
cccctgctgc attttcctca ttcatccacc agggcttaat ggggaatcaa ggaatccatg 6180
tgtaactgta taataactgt agccacactc caatgaccac ctactagttg tccctggcac 6240
tgcttataca tatgtccatc aaatcaatcc tatgaagtag atactgtctt cattttatag 6300
atcagagaca attggggttc agagagctga tgtgattttc ccagggtcac agagagtccc 6360
agattcaggc acaactcttg tattccaaga cacaaccact acatgtccaa aggctgccca 6420
gagccaccgg gcacggcaaa ttgtgacata tccctaaaga ggctgagcac ctggtcagga 6480
tctgatggct gacagtgtgt ccagatgcag agctggagtg gggagggga aggggggctc 6540
cttgggacag agaaggcttt ctgtgctttc tctgaaggga gcagtctgag gaccaaggga 6600
acccggcaaa cagcacctca ggtactccag gccctcctgg ctggagaggg ctgtggcaat 6660
ggaaaattag tgccaactgc aatgagtcag cctcggttaa atagagagtg aagaatgctg 6720
gacaggaacc tccaccctca tgtcacattt cttcagtgtg acccttctgg cccctctcct 6780
cctgacagcg gaacaatgac tgccccgata ggtgaggctg gaggaagaat cagtcctgtc 6840
cttggcaagc tcttcactat gacagtaaag gctctctgcc tgctgccaag gcctgtgact 6900
ttctaacctg gcctcacgct gggtaagctt aaggtagagg tgcaggatta gcaagcccac 6960
ctggctacca ggccgacagc tacatctttc aactgaccct gatcaacgaa gagggacttg 7020
tgtctctcag ttggttccaa atgaaaccag ggagcagggg cgttaggaag ctccaacagg 7080

atggtactta atggggcatt tgagtggaga ggtaggtgac atagtgcttt ggagcccagg 7140

gagggaaagg ttctgctgaa gttgaattca agactgttct ttcatcacaa acttgagttt 7200

cctggacatt tgtttgcaga aacaaccgta gggttttgcc ttaacctcgt gggtttatta 7260

ttacctcata gggactttgc ctcctgacag cagtttatgg gtgttcattg tggcacttga 7320

gttttcttgc atacttgtta gagaaaccaa gtttgtcatc aacttcttat ttaacccccct 7380

ggctataact tcatggatta tgttataatt aagccatcca gagtaaaatc tgtttagatt 7440

atcttggagt aagggggaaa aaatctgtaa ttttttctcc tcaactagat atatacataa 7500

aaaatgattg tattgcttca tttaaaaaat ataacgcaaa atctcttttc cttctaaaaa 7560

aaaaaaaaaa aaa 7573

<210> 15
<211> 4809
<212> mRNA
<213> Homo sapiens

<400> 15
gtgaactgtt gcaccgtgca attgcacact ataaatgtct ttccttatct gtgtgtactc 60

ttatctcact gttctatttt ttctcctcat ttatattaac tctttcttac cttttttttct 120

gaacttctag gccttctctt tccagaactg gtggaagaca aatgaaacgg ccaagatggt 180

aagaaacaag ccgcatttct ccttggggag actgataatt taaaaggttt gttgtgtcag 240

aaacattccc agcttcatca ccaacccttt ccttccacct ctgcccactg gagaccactt 300

atatcccgaa gcggacgcgg cagctgaagt caggaaacca tgcatcacat tagcaggagc 360

caactgcaga ctttaaactc cgttcaacat gtggatgcgg cagagaaatg acctgtccag 420

acaagccggg gcagctcata aactggttca tctgctccct gtgcgtcccg cgggtgcgta 480

agctctggag cagccggcgt ccaaggaccc ggagaaacct tctgctgggc actgcgtgtg 540

ccatctactt gggcttcctg gtgagccagg tggggagggc ctctctccag catggacagg 600

cggctgagaa ggggccacat cgcagccgcg acaccgccga gccatccttc cctgagatac 660

ccctggatgg taccctggcc cctccagagt cccagggcaa tgggtccact ctgcagccca 720

atgtggtgta cattacccta cgctccaagc gcagcaagcc ggccaatatc cgtggcaccg 780

tgaagcccaa gcgcaggaaa aagcatgcag tggcatcggc tgccccaggg caggaggctt 840

tggtcggacc atcccttcag ccgcaggaag cggcaaggga agctgatgct gtagcacctg 900

ggtacgctca gggagcaaac ctggttaaga ttggagagcg accctggagg ttggtgcggg 960

gtccgggagt gcgagccggg ggcccagact tcctgcagcc cagctccagg gagagcaaca 1020

ttaggatcta cagcgagagc gccccctcct ggctgagcaa agatgacatc cgaagaatgc 1080

gactcttggc ggacagcgca gtggcagggc tccggcctgt gtcctctagg agcggagccc 1140

gtttgctggt gctggagggg ggcgcacctg gcgctgtgct ccgctgtggc cctagcccct 1200

gtgggcttct caagcagccc ttggacatga gtgaggtgtt tgccttccac ctagacagga 1260

tcctggggct caacaggacc ctgccgtctg tgagcaggaa agcagagttc atccaagatg 1320

gccgcccatg ccccatcatt ctttgggatg catctttatc ttcagcaagt aatgacaccc 1380

attcttctgt taagctcacc tggggaactt atcagcagtt gctgaaacag aaatgctggc 1440

agaatggccg agtacccaag cctgaatcgg gttgtactga aatacatcat catgagtggt 1500

ccaagatggc actctttgat tttttgttac agatttataa tcgcttagat acaaattgct 1560

gtggattcag acctcgcaag gaagatgcct gtgtacagaa tggattgagg ccaaaatgtg 1620

atgaccaagg ttctgcggct ctagcacaca ttatccagcg aaagcatgac ccaaggcatt 1680

tggtttttat agacaacaag ggtttctttg acaggagtga agataactta aacttcaaat 1740

tgttagaagg catcaaagag tttccagctt ctgcagtttc tgttttgaag agccagcact 1800

tacggcagaa acttcttcag tctctgtttc ttgataaagt gtattgggaa agtcaaggag 1860

gtagacaagg aattgaaaag cttatcgatg taatagaaca cagagccaaa attcttatca 1920

cctatatcaa tgcacacggg gtcaaagtat tacctatgaa tgaatgacaa aagaatcttc 1980

tggctagggt gttagatata tttatgcatt tttggttttg tttttaaatc aagcacatca 2040

acctcaagcc cgtttagcaa tgaggcagtg tagatgaata cgtaaaataa atgactttaa 2100

ccaagtagct ataatgggac ttagcactgt atgcatactt aaaaaggttt tgaaaaacaa 2160

actacttgag aaatatttgt ttatattttt ctctaacatc atgctatgtg tcagtctgaa 2220

catctgacaa cagaaatttc agttattatt ctagctaagt tttgaaaaca tttgtcatgc 2280

tgtttaatag aaaactgcaa accagagaca ctgactccat taataaacca tattttgtgc 2340

cgttttgact gttctgacca aatactaatg ggaacaattc ttgacgtttt tctgttgctg 2400

attgttaaca tagagcagtc tctacactac cctgaggcaa ctctacattg gaacactgag 2460

gcttacagcc tgcaagagca tcagagctga ccatacattt aaacagaaat gctggtttat 2520

ttgcaaaatc accagtatat tttctattgt gtctataaaa aatcagtcat ttaagtacaa 2580

gaatcatatt ttccattcct ttttagaaat ttattttgtt gtccctatgg aaatcattca 2640

catctgacaa tttatatgtt aaagagtttt actctctcta ttttggtcca atttgtatct 2700

agtggctgag aaattaaata attctaaagt atgaagttac ctatctgaaa atgtacttac 2760

agagtatcat tttaaaatgg atgtctcttt aaaaattttg ttacttttac caacaatgta 2820

atataattta tgtatatttt attaataata gtgaattcct taaaatttgt tctatgtact 2880

tatatttaat ttgatttaat ggttactgcc cagatattga gaattggttc aaatattgag 2940

tgtgtttcaa tatattatct ggcttatttc aacatgagta atatgagcaa aataagttaa 3000

aacctgcgtc tgatcaattt tcctcatgac tagaactaaa acagtaaatt tggacaatat 3060

taagcctcaa ataatcatct ccaaactcct tctaacactt tttaaatcag attggaagac 3120
atggacaaat caggttcatg tgttgcatct ttatgtcctt tgccaatatc caagatcatc 3180
acatatggta gatattcaca tggagtttca aattcagaat agattaccat taccttcctg 3240
cccttacaca tcctactcct tatttaaaag ttctatttgt gacttttcat ttcctgaaag 3300
tttaaaaata caatttgaga atgtttataa tacattctct cctgtctttt cacggttacg 3360
tctgttattg ctgaaataca ccacattttc tttgttctgg tcaaggttaa ctcaatatct 3420
gtgtgaaaga gaactactaa caacgttaca atagaggcta gatttgaaaa aaaaaatcta 3480
tagatctaat tgatacaatt gtagaacaaa atgtcaaaat aatgttttaa gtataagaga 3540
agatggacca aggagagaga gatcatttga aaatctaatt gtagcttttc taggctcaca 3600
ttcatgtact acttttagca cccttatggg ctgtgctcgc cccctggaca gttgagcttt 3660
ggattatctt cctcttcaat tttccctcta ttgacccgag tgtctccctc tgcttctaca 3720
gatttatagt actccttggc tcttttgagt ctccactttt actcactgtc tctgggattt 3780
ttaagatcct tttcttctct tataaatcat cctcttaatg aaaattagcc taacaaaagt 3840
ttggagactg gaatcctact ttgagccact gacttgaaat aactcttttg gcaagttgcc 3900
tgacatcctg tcttaccaag gtggcatatt tgcattttta ctgcttaaaa catttttttt 3960
tttttaccat ctttatccaa atttatcata ttgatggtag gactaacagg ctttttagaa 4020
gctggcttta actttgagtc tcaagctaca atgctgttgg gcagcctggt cttcccacgt 4080
gagggtttaa ctttgtttat ttgcctccag ttattccaaa atgcttatta aatgaaagtc 4140
ccaggaacat gtttatttta gtcacctttg ctttttaaca attttgtttt gtaatcaatg 4200
agtaattcat gatgaattat ttttgactaa tggatagccg aaggccaggc ttttaattct 4260
aataggtaat gttcttcttt tgtcttattg aaacaatgag aatactctgt gcatttcaaa 4320
tgcactccga ttatgctgtg gttttattca cataagcaca atatgtgttt tatttataac 4380
ttcataacaa acttataata taataattta ccttagcaga catgcaaaag cttattcttg 4440
tgtgacttac tttctttaag ctaataatat aaaaataaat atgtatctta aaaatctata 4500
ataaaacatt agaaattaaa gatatgtgct ttttattttg cagatgagtt catttgcttt 4560
tgtagatgtg ttttcagagc taggtacaga ggaatgtttg ctacctttag cggtgaaaaa 4620
agaaagagag tcaagaattt tgttggattg tgtttgtgtg tgcatatatt tgatatcatc 4680
attatatttg taatctttgg acttgtaatc atagcctgtt tattctactg tgccattaaa 4740
tatactttac cttatacata acgaataaaa tacctagaag tagatttatt tacaaaaaaa 4800
aaaaaaaaa 4809

<210> 16

<211> 3522
<212> mRNA
<213> Homo sapiens

<400> 16
aggtcggggt gagaggccgg gggcctggtc tgggccggag cctgccgggg acaggaactg 60

gatgacgggc caggccagat tccaccgcgg agccgaggca tgccgagcta tccggcagtg 120

caggtcccgg cggccgcggg cagctcaccc ggaaggccgc gcggggtccg ggcgtggatc 180

ccggcacgcg agcccgggcg ttcgcagggg aggattttcc ttgctgctgc ttaacttgga 240

ggagtactac tttgaacagc atagagccaa tcacattttg cacaagggca gtcaccatga 300

aaggaaaatc agaggctcct taaaaatatg ttcaaaatcg gtgatttttg aaccagattc 360

aatatcccag cccatcatca agattccttt gagagactgt ataaaaatag gaaagcatgg 420

agaaaatgga gccaatagac acttcacaaa ggcaaaatct ggggggtattt cactcatttt 480

cagtcaggta tatttcatta aagaacataa tgttgttgca ccatataaaa tagaaagggg 540

caaaatggaa tatgtttttg aattggatgt tcccgggaaa gtggaagatg ttgtggagac 600

gttgcttcag cttcacagag catcctgcct tgacaaattg ggtgaccaaa ccgccatgat 660

aacagctatt ttgcagtctc gtttagctag aacatcattt gacaaaaaca ggttccaaaa 720

catttctgaa aagctgcaca tggaatgcaa agcagaaatg gtgacgcctc tggtgactaa 780

tcctggacac gtgtgcatca cggacacaaa cctgtatttt cagcccctca acggctaccc 840

gaaacctgtg gtccagataa cactccaaga tgtccgccgc atctacaaaa ggaggcacgg 900

cctcatgcct ctgggcttgg aagtattttg cacagaagat gatctgtgtt ccgacatcta 960

cctaaagttc tatgaacctc aagatagaga tgatctctat ttttacattg ccacatacct 1020

agagcaccat gtggcggagc acactgctga gagctacatg ctgcagtggc agcgtggaca 1080

cctttccaac tatcagtacc tccttcacct caacaacctg gccgaccgca gctgcaacga 1140

cctctcccag taccctgtgt ttccatggat aatacatgat tattccagct cagaactaga 1200

tttgtcaaat ccaggaacct tccgggatct cagtaagcca gtaggggccc taaataagga 1260

acggctggag agactactga cacgctacca ggaaatgcct gaaccaaagt tcatgtatgg 1320

gagtcactac tcttccccgg gttatgtact tttttatctt gttaggattg caccagagta 1380

tatgctgtgc ctgcagaatg gaagatttga taatgcagat agaatgttca acagtattgc 1440

agaaacttgg aaaaactgtc tggatggtgc aacggatttt aaagagttaa ttccagaatt 1500

ctatggtgat gatgtgagct ttctagtcaa tagcctgaag ttggatttgg gaaagagaca 1560

aggaggacag atggttgacg acgtggagct tccccccttgg gcttccagtc ccgaggactt 1620

tctccagaag agcaaagatg cattggaaag caattatgtg tctgaacacc ttcacgagtg 1680

gattgatcta atatttggct acaaacaaaa agggagtgat gcagttgggg cccataatgt 1740

```
atttcatccc ctgacctatg aaggaggtgt agacttgaac agcatccagg atcctgatga   1800
gaaggtagcc atgcttacgc aaatcttgga atttgggcag acaccaaaac aactatttgt   1860
gacaccacat cctcgaagga tcaccccaaa gtttaaaagt ttgtcccaga cctccagtta   1920
taatgcttct atggcagatt ccccaggtga agagtctttt gaagacctga ccgaagaaag   1980
caaaacactg gcctggaata acatcaccaa actgcagtta cacgagcact ataaaatcca   2040
caaagaagca gttactggaa tcacggtctc tcgcaatgga tcttcagtat tcacaacatc   2100
ccaagattcc accttgaaga tgttttctaa agaatcaaaa atgctacaaa gaagtatatc   2160
attttcaaat atggctttat cgtcttgttt acttttacca ggagatgcca ctgtcataac   2220
ttcttcatgg gataataatg tctattttta ttccatagca tttggaagac gccaggacac   2280
gttaatggga catgatgatg ctgttagtaa gatctgttgg catgacaaca ggctatattc   2340
tgcatcgtgg gactctacag tgaaggtgtg gtctggtgtt cctgcagaga tgccaggcac   2400
caaaagacac cactttgact tgctggccga gctggaacat gatgtcagtg tagatacaat   2460
cagtttaaat gctgcaagca cactgttagt ttccggcacc aaagaaggca cagtgaatat   2520
ttgggacctc acaacggcca ccttaatgca ccagattcca tgccattcag ggattgtatg   2580
tgacactgct tttagcccag atagtcgcca tgtcctcagc acaggaacag atggctgtct   2640
taatgtcatt gatgtgcaga caggaatgct catctcctcc atgacatcag atgagcccca   2700
gaggtgcttt gtctgggatg gaaattccgt tttatctggc agtcagtctg gtgaactgct   2760
cgtttgggac ctccttggag caaaaatcag tgagagaata cagggccaca caggtgctgt   2820
gacatgtata tggatgaatg aacagtgtag cagtatcatc acaggagggg aagacagaca   2880
aattatattc tggaaattgc agtattaagt gccttttcct ctcctgaata ttaaattgaa   2940
ctctatttaa tgcattttta aaccaaactt ttaaacggac tggtgaatgt gcaatgttag   3000
taattagaag ttttaccaca tggaaaattt gtggttttaa actttctaaa tcatggtgac   3060
ttcattgaaa gccattagtt gctattctct tagggcagat aaaatgcggc tgtgttagga   3120
aaaacatgtt acactgtaag gcagatgatc gtccccgtat gatgattgtc agaagacagg   3180
actaagtagc agagaatagc taagagataa attgggctgg ggaaacttgt cagaaagcac   3240
tgaacaatta agaaattttc caagaaaatg tgcagtattc tctgctactt ctgaatctgt   3300
tttgtcttcc taatctatca caattgccac ccatcgggtt ttgggtgtgt gttttcatag   3360
cgtggttact ttctataatg ctgtacccag attctaagaa cctggagaag gattagcagt   3420
tcttagtaag tttactgtgt ataggaacgg tttgtatttc attacagcta ttcatctttt   3480
ctacattaaa aatatttttc tctaaagaaa aaaaaaaaaa aa                      3522
```

<210> 17
<211> 2914

<212> mRNA
<213> Homo sapiens

<400> 17

```
gtgctctgag tttttggttt ctgtttcacc ttgtgtctga gctggtctga aggctggttg      60
ttcagactga gcttcctgcc tgcctgtacc ccgccaacag cttcagaaga aggtgactgg     120
tggctgcctg aggaatacca gtgggcaaga gaattagcat ttctggagca tctgctgtct     180
gagcagcccc tgggtgcgtc cactttctgg gcacgtgagg ttgggccttg gccgcctgag     240
cccttgagtt ggtcacttga accttgggaa tattgagatt atattctcct gccttttaaa     300
aagatggact tcagcagaaa tctttatgat attggggaac aactggacag tgaagatctg     360
gcctccctca agttcctgag cctggactac attccgcaaa ggaagcaaga acccatcaag     420
gatgccttga tgttattcca gagactccag gaaaagagaa tgttggagga aagcaatctg     480
tccttcctga aggagctgct cttccgaatt aatagactgg atttgctgat tacctaccta     540
aacactagaa aggaggagat ggaaagggaa cttcagacac caggcagggc tcaaatttct     600
gcctacaggt tccacttctg ccgcatgagc tgggctgaag caaacagcca gtgccagaca     660
cagtctgtac ctttctggcg gagggtcgat catctattaa taagggtcat gctctatcag     720
atttcagaag aagtgagcag atcagaattg aggtctttta agtttctttt gcaagaggaa     780
atctccaaat gcaaactgga tgatgacatg aacctgctgg atattttcat agagatggag     840
aagagggtca tcctgggaga aggaaagttg gacatcctga aaagagtctg tgcccaaatc     900
aacaagagcc tgctgaagat aatcaacgac tatgaagaat tcagcaaagg ggaggagttg     960
tgtggggtaa tgacaatctc ggactctcca agagaacagg atagtgaatc acagactttg    1020
gacaaagttt accaaatgaa aagcaaacct cggggatact gtctgatcat caacaatcac    1080
aattttgcaa aagcacggga gaaagtgccc aaacttcaca gcattaggga caggaatgga    1140
acacacttgg atgcaggggc tttgaccacg acctttgaag agcttcattt tgagatcaag    1200
ccccacgatg actgcacagt agagcaaatc tatgagattt tgaaaatcta ccaactcatg    1260
gaccacagta acatggactg cttcatctgc tgtatcctct cccatggaga caagggcatc    1320
atctatggca ctgatggaca ggaggccccc atctatgagc tgacatctca gttcactggt    1380
ttgaagtgcc cttcccttgc tggaaaaccc aaagtgtttt ttattcaggc ttgtcagggg    1440
gataactacc agaaaggtat acctgttgag actgattcag aggagcaacc ctatttagaa    1500
atggatttat catcacctca aacgagatat atcccggatg aggctgactt tctgctgggg    1560
atggccactg tgaataactg tgtttcctac cgaaaccctg cagagggaac ctggtacatc    1620
cagtcacttt gccagagcct gagagagcga tgtcctcgag gcgatgatat tctcaccatc    1680
ctgactgaag tgaactatga agtaagcaac aaggatgaca agaaaaacat ggggaaacag    1740
atgcctcagc ctactttcac actaagaaaa aaacttgtct tcccttctga ttgatggtgc    1800
```

tattttgttt gttttgtttt gttttgtttt tttgagacag aatctcgctc tgtcgcccag 1860

gctggagtgc agtggcgtga tctcggctca ccgcaagctc cgcctcccgg gttcaggcca 1920

ttctcctgcc tcagcctccc gagtagctgg gactacaggg gcccgccacc acacctggct 1980

aattttttaa aaatattttt agtagagaca gggtttcact gtgttagcca gggtggtctt 2040

gatctcctga cctcgtgatc cacccacctc ggcctcccaa agtgctggga ttacaggcgt 2100

gagccaccgc gcctggccga tggtactatt tagatataac actatgttta tttactaatt 2160

ttctagattt tctactttat taattgtttt gcactttttt ataagagcta aagttaaata 2220

ggatattaac aacaataaca ctgtctcctt tctcttatgc ttaaggcttt gggaatgttt 2280

ttagctggtg gcaataaata ccagacacgt acaaaatcca gctatgaata tagagggctt 2340

atgattcaga ttgttatcta tcaactataa gcccactgtt aatattctat taactttaat 2400

tctctttcaa agctaaattc cacactacca cattaaaaaa attagaaagt agccacgtat 2460

ggtggctcat gtctataatc ccagcacttt gggaggttga ggtgggagga ttgcttgaac 2520

ccaagaggtc aaggctgcag tgagccatgt tcacaccgct gcactcaagc ttgggtgaca 2580

gaacaagacc ccgtctcaaa aaaaattttt tttttaataa aacaaaattt gtttgaaatc 2640

ttttaaaaat tcaaatgatt tttacaagtt ttaaataagc tctccccaaa cttgctttat 2700

gccttcttat tgcttttatg atatatatat gcttggctaa ctatatttgc tttttgctaa 2760

caatgctctg ggtctttttt atgcatttgc atttgctctt tcatctctgc ttggattatt 2820

ttaaatcatt aggaattaag ttatctttaa aatttaagta tctttttttca aaaacatttt 2880

ttaatagaat aaaatataat ttgatcttat taaa 2914

<210> 18
<211> 2859
<212> mRNA
<213> Homo sapiens

<400> 18
ctttccgcgg cattctttgg gcgtgagtca tgcaggtttg cagccagccc caaagggggt 60

gtgtgcgcga gcagagcgct ataaatacgg cgcctcccag tgcccacaac gcggcgtcgc 120

caggaggagc gcgcgggcac agggtgccgc tgaccgaggc gtgcaaagac tccagaattg 180

gaggcatgat gaagactctg ctgctgtttg tggggctgct gctgacctgg gagagtgggc 240

aggtcctggg ggaccagacg gtctcagaca atgagctcca ggaaatgtcc aatcagggaa 300

gtaagtacgt caataaggaa attcaaaatg ctgtcaacgg ggtgaaacag ataaagactc 360

tcatagaaaa aacaaacgaa gagcgcaaga cactgctcag caacctagaa gaagccaaga 420

agaagaaaga ggatgcccta aatgagacca gggaatcaga gacaaagctg aaggagctcc 480

caggagtgtg caatgagacc atgatggccc tctgggaaga gtgtaagccc tgcctgaaac 540

agacctgcat gaagttctac gcacgcgtct gcagaagtgg ctcaggcctg gttggccgcc 600
agcttgagga gttcctgaac cagagctcgc ccttctactt ctggatgaat ggtgaccgca 660
tcgactccct gctggagaac gaccggcagc agacgcacat gctggatgtc atgcaggacc 720
acttcagccg cgcgtccagc atcatagacg agctcttcca ggacaggttc ttcacccggg 780
agccccagga tacctaccac tacctgccct tcagcctgcc ccaccggagg cctcacttct 840
tctttcccaa gtcccgcatc gtccgcagct tgatgccctt ctctccgtac gagcccctga 900
acttccacgc catgttccag cccttccttg agatgataca cgaggctcag caggccatgg 960
acatccactt ccatagcccg gccttccagc acccgccaac agaattcata cgagaaggcg 1020
acgatgaccg gactgtgtgc cgggagatcc gccacaactc cacgggctgc ctgcggatga 1080
aggaccagtg tgacaagtgc cgggagatct tgtctgtgga ctgttccacc aacaacccct 1140
cccaggctaa gctgcggcgg gagctcgacg aatccctcca ggtcgctgag aggttgacca 1200
ggaaatacaa cgagctgcta aagtcctacc agtggaagat gctcaacacc tcctccttgc 1260
tggagcagct gaacgagcag tttaactggg tgtcccggct ggcaaacctc acgcaaggcg 1320
aagaccagta ctatctgcgg gtcaccacgg tggcttccca cacttctgac tcggacgttc 1380
cttccggtgt cactgaggtg gtcgtgaagc tctttgactc tgatcccatc actgtgacgg 1440
tccctgtaga agtctccagg aagaacccta aatttatgga gaccgtggcg gagaaagcgc 1500
tgcaggaata ccgcaaaaag caccgggagg agtgagatgt ggatgttgct tttgcaccta 1560
cgggggcatc tgagtccagc tccccccaag atgagctgca gccccccaga gagagctctg 1620
cacgtcacca agtaaccagg ccccagcctc caggccccca actccgccca gcctctcccc 1680
gctctggatc ctgcactcta acactcgact ctgctgctca tgggaagaac agaattgctc 1740
ctgcatgcaa ctaattcaat aaaactgtct tgtgagctga tcgcttggag ggtcctcttt 1800
ttatgttgag ttgctgcttc ccggcatgcc ttcattttgc tatgggggc aggcaggggg 1860
gatggaaaat aagtagaaac aaaaaagcag tggctaagat ggtataggga ctgtcatacc 1920
agtgaagaat aaaagggtga agaataaaag ggatatgatg acaaggttga tccacttcaa 1980
gaattgcttg ctttcaggaa gagagatgtg tttcaacaag ccaactaaaa tatattgctg 2040
caaatggaag cttttctgtt ctattataaa actgtcgatg tattctgacc aaggtgcgac 2100
aatctcctaa aggaatacac tgaaagttaa ggagaagaat cagtaagtgt aaggtgtact 2160
tggtattata atgcataatt gatgttttcg ttatgaaaac atttggtgcc cagaagtcca 2220
aattatcagt tttatttgta agagctattg cttttgcagc ggttttattt gtaaaagctg 2280
ttgatttcga gttgtaagag ctcagcatcc caggggcatc ttcttgactg tggcatttcc 2340
tgtccaccgc cggtttatat gatcttcata cctttccctg gaccacaggc gtttctcggc 2400

ttttagtctg aaccatagct gggctgcagt accctacgct gccagcaggt ggccatgact 2460

acccgtggta ccaatctcag tcttaaagct caggcttttc gttcattaac attctctgat 2520

agaattctgg tcatcagatg tactgcaatg gaacaaaact catctggctg catcccaggt 2580

gtgtagcaaa gtccacatgt aaatttatag cttagaatat tcttaagtca ctgtcccttg 2640

tctctctttg aagttataaa caacaaactt aaagcttagc ttatgtccaa ggtaagtatt 2700

ttagcatggc tgtcaaggaa attcagagta aagtcagtgt gattcactta atgatataca 2760

ttaattagaa ttatggggtc agaggtattt gcttaagtga tcataattgt aaagtatatg 2820

tcacattgtc acattaatgt caaaaaaaaa aaaaaaaaa 2859

<210> 19
<211> 1809
<212> mRNA
<213> Homo sapiens

<400> 19
gtcttcctcc ctccctccct tcctcttact ctcattcatt tcatacacac tggctcacac 60

atctactctc tctctctatc tctctcagaa tgacaattct aggtacaact tttggcatgg 120

ttttttcttt acttcaagtc gtttctggag aaagtggcta tgctcaaaat ggagacttgg 180

aagatgcaga actggatgac tactcattct catgctatag ccagttggaa gtgaatggat 240

cgcagcactc actgacctgt gcttttgagg acccagatgt caacatcacc aatctggaat 300

ttgaaatatg tggggccctc gtggaggtaa agtgcctgaa tttcaggaaa ctacaagaga 360

tatatttcat cgagacaaag aaattcttac tgattggaaa gagcaatata tgtgtgaagg 420

ttggagaaaa gagtctaacc tgcaaaaaaa tagacctaac cactatagtt aaacctgagg 480

ctccttttga cctgagtgtc gtctatcggg aaggagccaa tgactttgtg gtgacattta 540

atacatcaca cttgcaaaag aagtatgtaa aagttttaat gcacgatgta gcttaccgcc 600

aggaaaagga tgaaaacaaa tggacgcatg tgaatttatc cagcacaaag ctgacactcc 660

tgcagagaaa gctccaaccg gcagcaatgt atgagattaa agttcgatcc atccctgatc 720

actattttaa aggcttctgg agtgaatgga gtccaagtta ttacttcaga actccagaga 780

tcaataatag ctcaggggag atggatccta tcttactaac catcagcatt ttgagttttt 840

tctctgtcgc tctgttggtc atcttggcct gtgtgttatg gaaaaaaagg attaagccta 900

tcgtatggcc cagtctcccc gatcataaga agactctgga acatctttgt aagaaaccaa 960

gaaaaaattt aaatgtgagt ttcaatcctg aaagtttcct ggactgccag attcataggg 1020

tggatgacat tcaagctaga gatgaagtgg aaggttttct gcaagatacg tttcctcagc 1080

aactagaaga atctgagaag cagaggcttg gaggggatgt gcagagcccc aactgcccat 1140

ctgaggatgt agtcatcact ccagaaagct ttggaagaga ttcatccctc acatgcctgg 1200

```
ctgggaatgt cagtgcatgt gacgccccta ttctctcctc ttccaggtcc ctagactgca   1260

gggagagtgg caagaatggg cctcatgtgt accaggacct cctgcttagc cttgggacta   1320

caaacagcac gctgccccct ccattttctc tccaatctgg aatcctgaca ttgaacccag   1380

ttgctcaggg tcagcccatt cttacttccc tgggatcaaa tcaagaagaa gcatatgtca   1440

ccatgtccag cttctaccaa aaccagtgaa gtgtaagaaa cccagactga acttaccgtg   1500

agcgacaaag atgatttaaa agggaagtct agagttccta gtctccctca cagcacagag   1560

aagacaaaat tagcaaaacc ccactacaca gtctgcaaga ttctgaaaca ttgctttgac   1620

cactcttcct gagttcagtg gcactcaaca tgagtcaaga gcatcctgct tctaccatgt   1680

ggatttggtc acaaggttta aggtgaccca atgattcagc tatttaaaaa aaaaagagga   1740

aagaatgaaa gagtaaagga aatgattgag gagtgaggaa ggcaggaaga gagcatgaga   1800

ggaaaaaaa                                                           1809


<210>  20
<211>  5226
<212>  mRNA
<213>  Homo sapiens

<400>  20
acacctccct ccccgcctgc cagtgtcacc agcctgttgc ctctgtgaga aagtaccact     60

gtaagaggcc aaagggcatg atcattttcc tctttcaccc tgtctaggtt gccagcaaat    120

cccacgggcc tcctgacgct gcccctgggg ccacaggtcc ctcgagtgct ggaaggatga    180

aggattcctg catcactgtg atggccatgg cgctgctgtc tgggttcttt ttcttcgcgc    240

cggcctcgag ctacaacctg gacgtgcggg gcgcgcggag cttctcccca ccgcgcgccg    300

ggaggcactt tggataccgc gtcctgcagg tcggaaacgg ggtcatcgtg ggagctccag    360

gggaggggaa cagcacagga agcctctatc agtgccagtc gggcacagga cactgcctgc    420

cagtcaccct gagaggttcc aactatacct ccaagtactt gggaatgacc ttggcaacag    480

accccacaga tggaagcatt ttggcctgtg accctgggct gtctcgaacg tgtgaccaga    540

acacctatct gagtggcctg tgttacctct tccgccagaa tctgcagggt cccatgctgc    600

aggggcgccc tggttttcag gaatgtatca agggcaacgt agacctggta tttctgtttg    660

atggttcgat gagcttgcag ccagatgaat ttcagaaaat tctggacttc atgaaggatg    720

tgatgaagaa actcagcaac acttcgtacc agtttgctgc tgttcagttt tccacaagct    780

acaaaacaga atttgatttc tcagattatg ttaaacggaa ggaccctgat gctctgctga    840

agcatgtaaa gcacatgttg ctgttgacca ataccttgg tgccatcaat tatgtcgcga     900

cagaggtgtt ccgggaggag ctgggggccc ggccagatgc caccaaagtg cttatcatca    960

tcacggatgg ggaggccact gacagtggca acatcgatgc ggccaaagac atcatccgct   1020
```

acatcatcgg gattggaaag cattttcaga ccaaggagag tcaggagacc ctccacaaat 1080

ttgcatcaaa acccgcgagc gagtttgtga aaattctgga cacatttgag aagctgaaag 1140

atctattcac tgagctgcag aagaagatct atgtcattga gggcacaagc aaacaggacc 1200

tgacttcctt caacatggag ctgtcctcca gcggcatcag tgctgacctc agcaggggcc 1260

atgcagtcgt gggggcagta ggagccaagg actgggctgg gggctttctt gacctgaagg 1320

cagacctgca ggatgacaca tttattggga atgaaccatt gacaccagaa gtgagagcag 1380

gctatttggg ttacaccgtg acctggctgc cctcccggca aaagacttcg ttgctggcct 1440

cgggagcccc tcgataccag cacatgggcc gagtgctgct gttccaagag ccacagggcg 1500

gaggacactg gagccaggtc cagacaatcc atgggaccca gattggctct tatttcggtg 1560

gggagctgtg tggcgtcgac gtggaccaag atggggagac agagctgctg ctgattggtg 1620

ccccactgtt ctatggggag cagagaggag gccgggtgtt tatctaccag agaagacagt 1680

tggggtttga agaagtctca gagctgcagg gggaccccgg ctacccactc gggcggtttg 1740

gagaagccat cactgctctg acagacatca acggcgatgg gctggtagac gtggctgtgg 1800

gggcccctct ggaggagcag gggggctgtgt acatcttcaa tgggaggcac gggggggctta 1860

gtccccagcc aagtcagcgg atagaaggga cccaagtgct ctcaggaatt cagtggtttg 1920

gacgctccat ccatggggtg aaggaccttg aaggggatgg cttggcagat gtggctgtgg 1980

gggctgagag ccagatgatc gtgctgagct cccggcccgt ggtggatatg gtcaccctga 2040

tgtccttctc tccagctgag atcccagtgc atgaagtgga gtgctcctat tcaaccagta 2100

acaagatgaa agaaggagtt aatatcacaa tctgtttcca gatcaagtct ctcatccccc 2160

agttccaagg ccgcctggtt gccaatctca cttacactct gcagctggat ggccaccgga 2220

ccagaagacg gggggttgttc ccaggaggga gacatgaact cagaaggaat atagctgtca 2280

ccaccagcat gtcatgcact gacttctcat ttcatttccc ggtatgtgtt caagacctca 2340

tctcccccat caatgtttcc ctgaatttct ctctttggga ggaggaaggg acaccgaggg 2400

accaaagggc gcagggcaag gacataccgc ccatcctgag accctccctg cactcggaaa 2460

cctgggagat ccccttttgag aagaactgtg gggaggacaa gaagtgtgag gcaaacttga 2520

gagtgtcctt ctctcctgca agatccagag ccctgcgtct aactgctttt gccagcctct 2580

ctgtggagct gagcctgagt aacttggaag aagatgctta ctgggtccag ctggacctgc 2640

acttcccccc gggactctcc ttccgcaagg tggagatgct gaagccccat agccagatac 2700

ctgtgagctg cgaggagctt cctgaagagt ccaggcttct gtccagggca ttatcttgca 2760

atgtgagctc tcccatcttc aaagcaggcc actcggttgc tctgcagatg atgtttaata 2820

cactggtaaa cagctcctgg ggggactcgg ttgaattgca cgccaatgtg acctgtaaca 2880

atgaggactc agacctcctg gaggacaact cagccactac catcatcccc atcctgtacc 2940

ccatcaacat cctcatccag gaccaagaag actccacact ctatgtcagt ttcaccccca 3000
aaggccccaa gatccaccaa gtcaagcaca tgtaccaggt gaggatccag ccttccatcc 3060
acgaccacaa catacccacc ctggaggctg tggttggggt gccacagcct cccagcgagg 3120
ggcccatcac acaccagtgg agcgtgcaga tggagcctcc cgtgccctgc cactatgagg 3180
atctggagag gctcccggat gcagctgagc cttgtctccc cggagccctg ttccgctgcc 3240
ctgttgtctt caggcaggag atcctcgtcc aagtgatcgg gactctggag ctggtgggag 3300
agatcgaggc ctcttccatg ttcagcctct gcagctccct ctccatctcc ttcaacagca 3360
gcaagcattt ccacctctat ggcagcaacg cctccctggc ccaggttgtc atgaaggttg 3420
acgtggtgta tgagaagcag atgctctacc tctacgtgct gagcggcatc gggggggctgc 3480
tgctgctgct gctcattttc atagtgctgt acaaggttgg tttcttcaaa cggaacctga 3540
aggagaagat ggaggctggc agaggtgtcc cgaatggaat ccctgcagaa gactctgagc 3600
agctggcatc tgggcaagag gctggggatc ccggctgcct gaagcccctc catgagaagg 3660
actctgagag tggtggtggc aaggactgag tccaggcctg tgaggtgcag agtgcccaga 3720
actggactca ggatgcccag ggccactctg cctctgcctg cattctgccg tgtgccctcg 3780
ggcgagtcac tgcctctccc tggccctcag tttccctatc tcgaacatgg aactcattcc 3840
tgcctgtctc ctttgcaggc tcatagggaa gacctgctga gggaccagcc aagagggctg 3900
caaaagtgag ggcttgtcat taccagacgg ttcaccagcc tctcttggtt tccttccttg 3960
gaagagaatg tctgatctaa atgtggagaa actgtagtct caggacctag ggatgttctg 4020
gccctcaccc ctgccctggg atgtccacag atgcctccac cccccagaac ctgtccttgc 4080
acactcccct gcactggagt ccagtctctt ctgctggcag aaagcaaatg tgacctgtgt 4140
cactacgtga ctgtggcaca cgccttgttc ttggccaaag accaaattcc ttggcatgcc 4200
ttccagcacc ctgcaaaatg agaccctcgt ggccttcccc agcctcttct agagccgtga 4260
tgcctccctg ttgaagctct ggtgacacca gcctttctcc caggccaggc tccttcctgt 4320
cttcctgcat tcacccagac agctccctct gcctgaacct tccatctcgc cacccctcct 4380
tccttgacca gcagatccca gctcacgtca cacttggttg ggtcctcaca tctttcacac 4440
ttccaccagc ctgcactact ccctcaaagc acacgtcatg tttcttcatc cggcagcctg 4500
gatgtttttt ccctgtttaa tgattgacgt acttagcagc tatctctcag tgaactgtga 4560
gggtaaaggc tatacttgtc ttgttcacct tgggatgatg cctcatgata tgtcagggcg 4620
tgggacatct agtaggtgct tgacataatt tcactgaatt aatgacagag ccagtgggaa 4680
gatacagaaa aagaggggct gggctgggcg cggtggttca cgcctgtaat cccagcactt 4740
tgggaggcca aggagggtgg atcacctgag gtcaggagtt agaggccagc ctggcgaaac 4800

cccatctcta ctaaaaatac aaaatccagg cgtggtggca cacacctgta gtcccagcta 4860

ctcaggaggt tgaggtagga gaattgcttg aacctgggag gtggaggttg cagtgagcca 4920

agattgcgcc attgcactcc agcctgggca acacagcgag actccgtctc aaggaaaaaa 4980

taaaaataaa aagcgggcac gggcccgtga catccccacc cttggaggct gtcttctcag 5040

gctctgccct gccctagctc cacaccctct cccaggaccc atcacgcctg tgcagtggcc 5100

cccacagaaa gactgagctc aaggtgggaa ccacgtctgc taacttggag ccccagtgcc 5160

aagcacagtg cctgcatgta tttatccaat aaatgtgaaa ttctgtccaa aaaaaaaaaa 5220

aaaaaa 5226

<210> 21
<211> 2684
<212> mRNA
<213> Homo sapiens

<400> 21
acgccgtagg gggcgggccg ttcgggcttg gtttcctggg cgaccaccgc tggctagtcc 60

gttagaggcg tgcgggcttc ggaggcgtgc gggcttcggg tgccatgggg actcctcccg 120

gcctgcagac cgactgcgag gcgctgctca gccgcttcca ggagacggac agtgtacgct 180

tcgaggactt cacggagctc tggagaaaca tgaagttcgg gactatcttc tgtggcagaa 240

tgagaaattt agaaaagaac atgtttacaa aagaagcttt agctttggct tggcgatatt 300

ttttacctcc atacaccttc cagatcagag ttggtgcttt gtatctgcta tatggattat 360

ataataccca actgtgtcaa ccaaaacaaa agatcagagt tgccctgaag gattgggatg 420

aagttttaaa atttcagcaa gatttagtaa atgcacagca ttttgatgca gcttatattt 480

ttaggaagct acgactagac agagcatttc actttacagc aatgcccaaa ttgctgtcat 540

ataggatgaa gaaaaaaatt caccgagctg aagttacaga agaatttaag gacccaagtg 600

atcgtgtgat gaaacttatc acttctgatg tattagagga aatgctgaat gttcatgatc 660

attatcagaa catgaaacat gtaatttcag ttgataagtc caagccagat aaagccctca 720

gcttgataaa ggatgatttt tttgacaata ttaagaacat agttttggag catcagcagt 780

ggcacaaaga cagaaagaat ccatccttaa agtcaaaaac taatgatgga gaagaaaaaa 840

tggaaggaaa ttcacaagaa acggagagat gtgaaagggc agaatcatta gcgaaaataa 900

aatcaaaggc cttttcagtt gtcatacagg catccaaatc aagaaggcat cgtcaagtca 960

aactcgactc ttctgactct gattctgcat ctggtcaagg gcaagtcaaa gcaactagga 1020

aaaaagagaa gaaagaaaga ttgaaaccag caggaaggaa gatgtctctc agaaacaaag 1080

gcaatgtgca gaatatacac aaggaagata aacctttaag tctgagtatg cctgtaatta 1140

cagaagaaga agagaatgaa agtttgagtg gaacagagtt cactgcatcc aagaagagga 1200

gaaaacactg aacaaagagc ctggtgtagt ttttaatttt gagttttctg acagaagaaa 1260

agattgatat tttgtgtatt gaacaggaag actgccagta ttaaaaaaat ccttctggga 1320

atctgtaggt tatttcttgg aaattgcaat acgtagttct agaataaaag tacaaaaaat 1380

tagaataaga attctttaac attttcttta atgatttgca taaatggaga taaaacttgt 1440

atttagtatg taatagaaaa aattctgtta ttcgcagatt gttactattt cctataaggt 1500

tttgtgatac tatactgtcc taatacagtc tggtaatact attctatttt atttaaaata 1560

tttttattg aaatattaat gtttattaca tgcaaataac tattttgtat ctacagtcgg 1620

ataatggatt ttttattttg tatatttatt ctattttgta tattgttaag tgcaataaag 1680

tttttgcctt gctttatttt ttaatacata aaacttacat tctcataacg tgattgataa 1740

cttaggaagt tcacaatgta ttttctactt ctgcaattaa atattcttta gtgcttgttt 1800

attattacta aatactaatt aagtactaac aagtacttaa atactaatgt attaagtatt 1860

taagtacttt ctaataaaat ctttaacaat aataatgtaa atttcagaat gtgtctctgg 1920

tacagaatag ttgatattaa cagaaaaaaa aaaatctgta gcttcatgaa tatgccactc 1980

tgttaatttc ttgttccaga cattttaata gagattgctt gagccatgtt gtttgaattg 2040

ctgccaatag cagaccatat ccctatcatg ttgttggctc aactgttttt tttttttccc 2100

taatagagat ggagtatcgc tatgttgctc aggctggtct tgaactcctg ggctcaagct 2160

atcctcctgc ctcagcctcc caaagtactg ggattatagg tgtgagctac tgtacccagc 2220

cttaacctgt ttcacagttg attatacttc atgctgtttt ccagcatggt attattaagg 2280

gatttaaagt ttgggttgca tgcctgtaat cccagcattt tgggaggccg aggtgggcgg 2340

atcacgaggt caggagatcg agaccatcgt gactaacaca gtgaaacccc gtctctaata 2400

aaaatacgaa aaattagcca ggcgtggtgg cgggcgcctg taatcccagc tactcgggaa 2460

gctgaggcag gagaatggtg tgaacccagt gagccgagat cgtgccactg cactccagcc 2520

tgggcaacag agtgagactt cgtctcaaaa aaaaaaaaaa gtttgggttg aagatcaaat 2580

tcgtgatatc tctatatcta atctttaaaa atcagaatgc taatgctgac gcaaataaaa 2640

ttttcattta ttagcaaaaa aaaaaaaaaa aaaaaaaaaa aaaa 2684

<210> 22
<211> 3431
<212> mRNA
<213> Homo sapiens

<400> 22
ggttttcagg agcccgagcg agggcgccgc ttttgcgtcc gggaggagcc aaccgtggcg 60

caggcggcgc ggggaggcgt cccagagtct cactctgccg cccaggctgg actgcagtga 120

cacaatctcg gctgactgca accactgcct ccagggttca agcgattctc ttgcctcagc 180

```
ctcccaagta gctgggatta cagattgatg ttcatgttcc tgacactact acaagattca     240
tactcctgat gctactgaca acgtggcttc tccacagtca ccaaaccagg gatgctatac     300
tggacttccc tactctcatc tgctccagcc ccctgacctt atagttgccc agctttcctg     360
gcaattgact ttgcccatca atacacagga tttagcatcc agggaagatg tcggagcctc     420
agatgttaat tttctaattg agaatgttgg cgctgtccga acctggagac aggaaaacaa     480
aaagtccttt ctcctgattc accaaaaaat aaaatactga ctaccatcac tgtgatgaga     540
ttcctatagt ctcaggaact gaagtcttta aacaaccagg gaccctctgc ccctagaata     600
agaacatact agaagtccct tctgctagga caacgaggat catgggagac cacctggacc     660
ttctcctagg agtggtgctc atggccggtc ctgtgtttgg aattccttcc tgctcctttg     720
atggccgaat agccttttat cgtttctgca acctcaccca ggtcccccag gtcctcaaca     780
ccactgagag gctcctgctg agcttcaact atatcaggac agtcactgct tcatccttcc     840
cctttctgga acagctgcag ctgctggagc tcgggagcca gtatacccc ttgactattg      900
acaaggaggc cttcagaaac ctgcccaacc ttagaatctt ggacctggga agtagtaaga     960
tatacttctt gcatccagat gcttttcagg gactgttcca tctgtttgaa cttagactgt    1020
atttctgtgg tctctctgat gctgtattga aagatggtta tttcagaaat ttaaaggctt    1080
taactcgctt ggatctatcc aaaaatcaga ttcgtagcct ttaccttcat ccttcatttg    1140
ggaagttgaa ttccttaaag tccatagatt tttcctccaa ccaaatattc cttgtatgtg    1200
aacatgagct cgagccccta caagggaaaa cgctctcctt ttttagcctc gcagctaata    1260
gcttgtatag cagagtctca gtggactggg gaaaatgtat gaacccattc agaaacatgg    1320
tgctggagat actagatgtt tctggaaatg gctggacagt ggacatcaca ggaaacttta    1380
gcaatgccat cagcaaaagc caggccttct ctttgattct tgcccaccac atcatgggtg    1440
ccgggtttgg cttccataac atcaaagatc ctgaccagaa cacatttgct ggcctggcca    1500
gaagttcagt gagacacctg gatctttcac atgggtttgt cttctccctg aactcacgag    1560
tctttgagac actcaaggat ttgaaggttc tgaaccttgc ctacaacaag ataaataaga    1620
ttgcagatga agcattttac ggacttgaca acctccaagt tctcaatttg tcatataacc    1680
ttctgggggga actttacagt tcgaatttct atggactacc taaggtagcc tacattgatt   1740
tgcaaaagaa tcacattgca ataattcaag accaaacatt caaattcctg gaaaaattac    1800
agaccttgga tctccgagac aatgctctta caaccattca ttttattcca agcatacccg    1860
atatcttctt gagtggcaat aaactagtga ctttgccaaa gatcaacctt acagcgaacc    1920
tcatccactt atcagaaaac aggctagaaa atctagatat tctctacttt ctcctacggg    1980
tacctcatct ccagattctc attttaaatc aaaatcgctt ctcctcctgt agtggagatc    2040
aaaccccttc agagaatccc agcttagaac agcttttcct tggagaaaat atgttgcaac    2100
```

ttgcctggga aactgagctc tgttgggatg tttttgaggg actttctcat cttcaagttc 2160

tgtatttgaa tcataactat cttaattccc ttccaccagg agtatttagc catctgactg 2220

cattaagggg actaagcctc aactccaaca ggctgacagt tctttctcac aatgatttac 2280

ctgctaattt agagatcctg gacatatcca ggaaccagct cctagctcct aatcctgatg 2340

tatttgtatc acttagtgtc ttggatataa ctcataacaa gttcatttgt gaatgtgaac 2400

ttagcacttt tatcaattgg cttaatcaca ccaatgtcac tatagctggg cctcctgcag 2460

acatatattg tgtgtaccct gactcgttct ctggggtttc cctcttctct ctttccacgg 2520

aaggttgtga tgaagaggaa gtcttaaagt ccctaaagtt ctcccttttc attgtatgca 2580

ctgtcactct gactctgttc ctcatgacca tcctcacagt cacaaagttc cggggcttct 2640

gttttatctg ttataagaca gcccagagac tggtgttcaa ggaccatccc cagggcacag 2700

aacctgatat gtacaaatat gatgcctatt tgtgcttcag cagcaaagac ttcacatggg 2760

tgcagaatgc tttgctcaaa cacctggaca ctcaatacag tgaccaaaac agattcaacc 2820

tgtgctttga agaaagagac tttgtcccag gagaaaaccg cattgccaat atccaggatg 2880

ccatctggaa cagtagaaag atcgtttgtc ttgtgagcag acacttcctt agagatggct 2940

ggtgccttga agccttcagt tatgcccagg gcaggtgctt atctgacctt aacagtgctc 3000

tcatcatggt ggtggttggg tccttgtccc agtaccagtt gatgaaacat caatccatca 3060

gaggctttgt acagaaacag cagtatttga ggtggcctga ggatctccag gatgttggct 3120

ggtttcttca taaactctct caacagatac taaagaaaga aaaagaaaag aagaaagaca 3180

ataacattcc gttgcaaact gtagcaacca tctcctaatc aaaggagcaa tttccaactt 3240

atctcaagcc acaaataact cttcactttg tatttgcacc aagttatcat tttggggtcc 3300

tctctggagg tttttttttt ctttttgcta ctatgaaaac aacataaatc tctcaatttt 3360

cgtatcaaca ccatgttctg tctcactaac ctccaaatgg aaaataatag atctagaaaa 3420

ttgcaactgc c 3431

<210> 23
<211> 3582
<212> mRNA
<213> Homo sapiens

<400> 23
gccggagtcc ccacgcgagg atgctgcggt gagcgcggcc gggacgccgc gtcgggctct 60

cggccgccca gcggcgccgc aggggaagcc aggccggcag ccgcgcgctc cccagccggc 120

caccaatccc gccctccccg gctcctccgg acctcctcgg caggcggcgg cggggcctgc 180

ctgtgcgctc tgcgcccgcc cgcgcctacc ctccatggcg tttatccgga agaagcagca 240

ggagcagcag ctgcagctct actccaagga gagattttcc ttgctgctgc ttaacttgga 300

ggagtactac tttgaacagc atagagccaa tcacattttg cacaagggca gtcaccatga 360
aaggaaaatc agaggctcct taaaaatatg ttcaaaatcg gtgatttttg aaccagattc 420
aatatcccag cccatcatca agattccttt gagagactgt ataaaaatag gaaagcatgg 480
agaaaatgga gccaatagac acttcacaaa ggcaaaatct gggggtattt cactcatttt 540
cagtcaggta tatttcatta aagaacataa tgttgttgca ccatataaaa tagaaagggg 600
caaaatggaa tatgtttttg aattggatgt tcccgggaaa gtggaagatg ttgtggagac 660
gttgcttcag cttcacagag catcctgcct tgacaaattg ggtgaccaaa ccgccatgat 720
aacagctatt ttgcagtctc gtttagctag aacatcattt gacaaaaaca ggttccaaaa 780
catttctgaa aagctgcaca tggaatgcaa agcagaaatg gtgacgcctc tggtgactaa 840
tcctggacac gtgtgcatca cggacacaaa cctgtatttt cagcccctca acggctaccc 900
gaaacctgtg gtccagataa cactccaaga tgtccgccgc atctacaaaa ggaggcacgg 960
cctcatgcct ctgggcttgg aagtattttg cacagaagat gatctgtgtt ccgacatcta 1020
cctaaagttc tatgaacctc aagatagaga tgatctctat ttttacattg ccacatacct 1080
agagcaccat gtggcggagc acactgctga gagctacatg ctgcagtggc agcgtggaca 1140
cctttccaac tatcagtacc tccttcacct caacaacctg gccgaccgca gctgcaacga 1200
cctctcccag taccctgtgt ttccatggat aatacatgat tattccagct cagaactaga 1260
tttgtcaaat ccaggaacct tccgggatct cagtaagcca gtaggggccc taaataagga 1320
acggctggag agactactga cacgctacca ggaaatgcct gaaccaaagt tcatgtatgg 1380
gagtcactac tcttccccgg gttatgtact tttttatctt gttaggattg caccagagta 1440
tatgctgtgc ctgcagaatg gaagatttga taatgcagat agaatgttca acagtattgc 1500
agaaacttgg aaaaactgtc tggatggtgc aacggatttt aaagagttaa ttccagaatt 1560
ctatggtgat gatgtgagct ttctagtcaa tagcctgaag ttggatttgg gaaagagaca 1620
aggaggacag atggttgacg acgtggagct tccccccttgg gcttccagtc ccgaggactt 1680
tctccagaag agcaaagatg cattggaaag caattatgtg tctgaacacc ttcacgagtg 1740
gattgatcta atatttggct acaaacaaaa agggagtgat gcagttgggg cccataatgt 1800
atttcatccc ctgacctatg aaggaggtgt agacttgaac agcatccagg atcctgatga 1860
gaaggtagcc atgcttacgc aaatcttgga atttgggcag acaccaaaac aactatttgt 1920
gacaccacat cctcgaagga tcaccccaaa gtttaaaagt ttgtcccaga cctccagtta 1980
taatgcttct atggcagatt ccccaggtga agagtctttt gaagacctga ccgaagaaag 2040
caaaacactg gcctggaata acatcaccaa actgcagtta cacgagcact ataaaatcca 2100
caaagaagca gttactggaa tcacggtctc tcgcaatgga tcttcagtat tcacaacatc 2160

ccaagattcc accttgaaga tgttttctaa agaatcaaaa atgctacaaa gaagtatatc 2220

attttcaaat atggctttat cgtcttgttt acttttacca ggagatgcca ctgtcataac 2280

ttcttcatgg gataataatg tctattttta ttccatagca tttggaagac gccaggacac 2340

gttaatggga catgatgatg ctgttagtaa gatctgttgg catgacaaca ggctatattc 2400

tgcatcgtgg gactctacag tgaaggtgtg gtctggtgtt cctgcagaga tgccaggcac 2460

caaaagacac cactttgact tgctggccga gctggaacat gatgtcagtg tagatacaat 2520

cagtttaaat gctgcaagca cactgttagt ttccggcacc aaagaaggca cagtgaatat 2580

ttgggacctc acaacggcca ccttaatgca ccagattcca tgccattcag ggattgtatg 2640

tgacactgct tttagcccag atagtcgcca tgtcctcagc acaggaacag atggctgtct 2700

taatgtcatt gatgtgcaga caggaatgct catctcctcc atgacatcag atgagcccca 2760

gaggtgcttt gtctgggatg gaaattccgt tttatctggc agtcagtctg gtgaactgct 2820

cgtttgggac ctccttggag caaaaatcag tgagagaata cagggccaca caggtgctgt 2880

gacatgtata tggatgaatg aacagtgtag cagtatcatc acaggagggg aagacagaca 2940

aattatattc tggaaattgc agtattaagt gccttttcct ctcctgaata ttaaattgaa 3000

ctctatttaa tgcattttta aaccaaactt ttaaacggac tggtgaatgt gcaatgttag 3060

taattagaag ttttaccaca tggaaaattt gtggttttaa actttctaaa tcatggtgac 3120

ttcattgaaa gccattagtt gctattctct tagggcagat aaaatgcggc tgtgttagga 3180

aaaacatgtt acactgtaag gcagatgatc gtccccgtat gatgattgtc agaagacagg 3240

actaagtagc agagaatagc taagagataa attgggctgg ggaaacttgt cagaaagcac 3300

tgaacaatta agaaattttc caagaaaatg tgcagtattc tctgctactt ctgaatctgt 3360

tttgtcttcc taatctatca caattgccac ccatcgggtt ttgggtgtgt gttttcatag 3420

cgtggttact ttctataatg ctgtacccag attctaagaa cctggagaag gattagcagt 3480

tcttagtaag tttactgtgt ataggaacgg tttgtatttc attacagcta ttcatctttt 3540

ctacattaaa aatatttttc tctaaagaaa aaaaaaaaaa aa 3582

<210> 24
<211> 4268
<212> mRNA
<213> Homo sapiens

<400> 24
gccctgcgca ctccctgctg gggtgagcag cactgtaaag atgaagctgg ctaactggta 60

ctggctgagc tcagctgttc ttgccactta cggtttttttg gttgtggcaa acaatgaaac 120

agaggaaatt aaagatgaaa gagcaaagga tgtctgccca gtgagactag aaagcagagg 180

gaaatgcgaa gaggcagggg agtgccccta ccaggtaagc ctgcccccct tgactattca 240

gctcccgaag caattcagca ggatcgagga ggtgttcaaa gaagtccaaa acctcaagga 300
aatcgtaaat agtctaaaga aatcttgcca agactgcaag ctgcaggctg atgacaacgg 360
agacccaggc agaaacggac tgttgttacc cagtacagga gccccgggag aggttggtga 420
taacagagtt agagaattag agagtgaggt taacaagctg tcctctgagc taaagaatgc 480
caaagaggag atcaatgtac ttcatggtcg cctggagaag ctgaatcttg taaatatgaa 540
caacatagaa aattatgttg acagcaaagt ggcaaatcta acatttgttg tcaatagttt 600
ggatggcaaa tgttcaaagt gtcccagcca agaacaaata cagtcacgtc cagttcaaca 660
tctaatatat aaagattgct ctgactacta cgcaataggc aaaagaagca gtgagaccta 720
cagagttaca cctgatccca aaaatagtag ctttgaagtt tactgtgaca tggagaccat 780
gggggggaggc tggacagtgc tgcaggcacg tctcgatggg agcaccaact tcaccagaac 840
atggcaagac tacaaagcag gctttggaaa cctcagaagg gaattttggc tggggaacga 900
taaaattcat cttctgacca agagtaagga aatgattctg agaatagatc ttgaagactt 960
taatggtgtc gaactatatg ccttgtatga tcagttttat gtggctaatg agtttctcaa 1020
atatcgttta cacgttggta actataatgg cacagctgga gatgcattac gtttcaacaa 1080
acattacaac cacgatctga agtttttcac cactccagat aaagacaatg atcgatatcc 1140
ttctgggaac tgtgggctgt actacagttc aggctggtgg tttgatgcat gtctttctgc 1200
aaacttaaat ggcaaatatt atcaccaaaa atacagaggt gtccgtaatg ggattttctg 1260
gggtacctgg cctggtgtaa gtgaggcaca ccctggtggc tacaagtcct ccttcaaaga 1320
ggctaagatg atgatcagac ccaagcactt taagccataa atcactctgt tcattcctcc 1380
aggtattcgt tatctaatag ggcaattaat tccttcagca ctttagaata tgccttgttt 1440
catatttttc atagctaaaa aatgatgtct gacggctagg ttcttatgct acacagcatt 1500
tgaaataaag ctgaaaaaca atgcatttta aaggagtcct ttgttgttat gctgttatcc 1560
aatgaacact tgcaagcaat tagcaatatt gagaattata cattagattt acaattcttt 1620
taatttctat tgaaactttt tctattgctt gtattacttg ctgtatttaa aaaataattg 1680
ttggctgggt gtggtagctc acgcctgtaa tcccagcact ttggaatgtc aaggcaggca 1740
gatcacttga ggtcaggagt ttgagaccag cctggccaaa catgtgaaac gctgtctcta 1800
ttaaaaatac aaaaattagc cgggcatggt ggtacatgcc tgtaatccta gctacttggg 1860
aggctgaggc aggagaatcg cttgaacctg agaggaagag gttgcagtga gccaagactg 1920
agccactgca ctccagcatg ggtgacagag aaaactctgt ctcaaacaaa aaaataataa 1980
aatttattca gtaggctgga ttctacacaa agtaatctgt atttgggcca tgatttaagc 2040
acatctgaag gtatatcact cttttcaggc tataattatt tgggtaatct tcattctgag 2100
acaaacttaa tctatatcat ttactttgca acagaacaac cctacagcat tttggttccc 2160

agactaaggg aactaatatc tatataatta aacttgttca tttatcattc atgaaatata 2220
aaatacttgt catttaaacc gtttaaaaat gtggtagcat aatgtcaccc caaaaagcat 2280
tcagaaagca atgtaactgt gaagaccagg gtttaaaggt aattcattta tagtttataa 2340
ctccttagat gtttgatgtt gaaaactgct ttaacatgaa aattatcttc ctctgctctg 2400
tgtgaacaat agcttttaat ttaagattgc tcactactgt actagactac tggtaggttt 2460
ttttgggggg ggtgggtagg gatatgtggg taatgaagca tttacttaca ggctatcata 2520
ctctgaggcc aattttatct ccaaagcaat aatatcatta agtgattcac ttcatagaag 2580
gctaagtttc tctaggacag atagaaaaca tgaattttga aatatataga acagtagtta 2640
aaatactata tatttcaacc ctggctggta gattgcttat tttactatca gaaactaaaa 2700
gatagatttt tacccaaaca gaagtatctg taatttttat aattcatcaa ttctggaatg 2760
ctatatataa tatttaaaag actttttaaa tgtgtttaat ttcatcatcg taaaaaggga 2820
tcatctcaga gagaacagca gtattctgcg tatttttaaa aatgctctag agtaacattt 2880
gaagtaattc actgtagtgt atgccagtcc tagaaataat ttttttaatt tctggtgtct 2940
gtttctaata cactaaccaa gttttcaaaa tatatttaca aagatgcatc tttacccatt 3000
attttaaaat gattaaggag gatagttgct tcaggtaaca agctaatttt tcaaatatta 3060
ggcccttaca gaactattta gtcaaaaagt aagatattcc tttaaaatat ataacccaaa 3120
gctttcagtt aaacatgata tatcacaaat actattaaaa tgttaaagag aaatgcaaat 3180
agcattaaat gatgaccaaa atgtaaaata ttgtagattt caaaagctgt gtctctatta 3240
ggtgggatac caaatgtaaa tgatgtaact gacgttgttt tttacttttt actttttaaa 3300
aaagactaaa aacgttttga tattatacaa tgtatttgtt tcagataagg tcattgtcat 3360
ttagtatata taattaatat atgtacaagt ttaagtaaat tcctgtgagt aaaaatggac 3420
ttatcacaaa acatagttct aaagaaaggt atatgctcat atacacggtg tccattaatt 3480
taatgggaac taggtataac ttcaggagaa tttggcaaat aattcattaa tccatgtaaa 3540
tattcaaaag cttgttctat ccacattatt tcaagggatc actttatttt tcattatact 3600
ttcacagcac ttttctagta aattctgtaa cacagaaatt ccattttgga atcatttcat 3660
gttaccaata attctagact tttataacat ttaacatgtt gatggaaata gattacatct 3720
gctagaacct tttgccttaa ctattcacca atatatgcta atattcataa atatggattg 3780
actgtttaca aacattagaa tcttgtcttg gttccatttt gatggctaat atttgttatc 3840
ttaattaaga ctatttctga ggtcatgatt acttgaaaat attgactaaa actgggtcct 3900
tagaaattcc aggtggagct gatttaccta tgactgaggg gaaaaaaaaa tcaaatttta 3960
ctgataatag taatgctcca aatgaattaa tgacacatct gttcaataaa taaagagctt 4020

aaatatacaa aacataagaa atctgggcaa caaaacttgt ggtctttact tttgaatagc 4080

tacccaagaa aaggttttaa aggtaaaagt tatgagtaat gtcatcacaa taagctcttg 4140

tttaaaattc ttttctttta tgtataatta ggtttatgtt tcatgtcttt ttaaaacctt 4200

ataaaagatt taattatcac atctattctt caatgtggaa atattaaata ttgttggttg 4260

taaaataa 4268

<210> 25
<211> 4854
<212> mRNA
<213> Homo sapiens

<400> 25
gtttttaaaa gctttgtatc tcttaaaacc atgcagcagt cagtttccaa gttttgcttt 60

gcaatcagta gttttcaagg gagcttttaa agctgaactg aaatgtttga aatgtggaac 120

actcttgacc atgaaatatg ttctacttac atgcctcagc ctttaaaagt tctttgcatt 180

agagtcaagg attacattct tcctggagcc aagcatgggg ccagctgtaa acaagccgca 240

tttctccttg gggagactga taatttaaaa ggtttgttgt gtcagaaaca ttcccagctt 300

catcaccaac cctttccttc cacctctgcc cactggagac cacttatatc ccgaagcgga 360

cgcggcagct gaagtcagga aaccatgcat cacattagca ggagccaact gcagacttta 420

aactccgttc aacatgtgga tgcggcagag aaatgacctg tccagacaag ccggggcagc 480

tcataaactg gttcatctgc tccctgtgcg tcccgcgggt gcgtaagctc tggagcagcc 540

ggcgtccaag gacccggaga aaccttctgc tgggcactgc gtgtgccatc tacttgggct 600

tcctggtgag ccaggtgggg agggcctctc tccagcatgg acaggcggct gagaaggggc 660

cacatcgcag ccgcgacacc gccgagccat ccttccctga gatacccctg gatggtaccc 720

tggcccctcc agagtcccag ggcaatgggt ccactctgca gcccaatgtg gtgtacatta 780

ccctacgctc caagcgcagc aagccggcca atatccgtgg caccgtgaag cccaagcgca 840

ggaaaaagca tgcagtggca tcggctgccc cagggcagga ggctttggtc ggaccatccc 900

ttcagccgca ggaagcggca agggaagctg atgctgtagc acctgggtac gctcagggag 960

caaacctggt taagattgga gagcgaccct ggaggttggt gcggggtccg ggagtgcgag 1020

ccgggggccc agacttcctg cagcccagct ccagggagag caacattagg atctacagcg 1080

agagcgcccc ctcctggctg agcaaagatg acatccgaag aatgcgactc ttggcggaca 1140

gcgcagtggc agggctccgg cctgtgtcct ctaggagcgg agcccgtttg ctggtgctgg 1200

aggggggcgc acctggcgct gtgctccgct gtggccctag cccctgtggg cttctcaagc 1260

agcccttgga catgagtgag gtgtttgcct tccacctaga caggatcctg gggctcaaca 1320

ggaccctgcc gtctgtgagc aggaaagcag agttcatcca agatggccgc ccatgcccca 1380

tcattctttg ggatgcatct ttatcttcag caagtaatga cacccattct tctgttaagc 1440
tcacctgggg aacttatcag cagttgctga aacagaaatg ctggcagaat ggccgagtac 1500
ccaagcctga atcgggttgt actgaaatac atcatcatga gtggtccaag atggcactct 1560
ttgatttttt gttacagatt tataatcgct tagatacaaa ttgctgtgga ttcagacctc 1620
gcaaggaaga tgcctgtgta cagaatggat tgaggccaaa atgtgatgac caaggttctg 1680
cggctctagc acacattatc cagcgaaagc atgacccaag gcatttggtt tttatagaca 1740
acaagggttt ctttgacagg agtgaagata acttaaactt caaattgtta gaaggcatca 1800
aagagtttcc agcttctgca gtttctgttt tgaagagcca gcacttacgg cagaaacttc 1860
ttcagtctct gtttcttgat aaagtgtatt gggaaagtca aggaggtaga caaggaattg 1920
aaaagcttat cgatgtaata gaacacagag ccaaaattct tatcacctat atcaatgcac 1980
acggggtcaa agtattacct atgaatgaat gacaaaagaa tcttctggct agggtgttag 2040
atatatttat gcatttttgg ttttgttttt aaatcaagca catcaacctc aagcccgttt 2100
agcaatgagg cagtgtagat gaatacgtaa aataaatgac tttaaccaag tagctataat 2160
gggacttagc actgtatgca tacttaaaaa ggttttgaaa aacaaactac ttgagaaata 2220
tttgtttata tttttctcta acatcatgct atgtgtcagt ctgaacatct gacaacagaa 2280
atttcagtta ttattctagc taagttttga aaacatttgt catgctgttt aatagaaaac 2340
tgcaaaccag agacactgac tccattaata aaccatattt tgtgccgttt tgactgttct 2400
gaccaaatac taatgggaac aattcttgac gtttttctgt tgctgattgt taacatagag 2460
cagtctctac actaccctga ggcaactcta cattggaaca ctgaggctta cagcctgcaa 2520
gagcatcaga gctgaccata catttaaaca gaaatgctgg tttatttgca aaatcaccag 2580
tatattttct attgtgtcta taaaaaatca gtcatttaag tacaagaatc atattttcca 2640
ttccttttta gaaatttatt ttgttgtccc tatggaaatc attcacatct gacaatttat 2700
atgttaaaga gttttactct ctctattttg gtccaatttg tatctagtgg ctgagaaatt 2760
aaataattct aaagtatgaa gttacctatc tgaaaatgta cttacagagt atcattttaa 2820
aatggatgtc tctttaaaaa ttttgttact tttaccaaca atgtaatata atttatgtat 2880
attttattaa taatagtgaa ttccttaaaa tttgttctat gtacttatat ttaatttgat 2940
ttaatggtta ctgcccagat attgagaatt ggttcaaata ttgagtgtgt ttcaatatat 3000
tatctggctt atttcaacat gagtaatatg agcaaaataa gttaaaacct gcgtctgatc 3060
aattttcctc atgactagaa ctaaaacagt aaatttggac aatattaagc ctcaaataat 3120
catctccaaa ctccttctaa cactttttaa atcagattgg aagacatgga caaatcaggt 3180
tcatgtgttg catctttatg tcctttgcca atatccaaga tcatcacata tggtagatat 3240
tcacatggag tttcaaattc agaatagatt accattacct tcctgccctt acacatccta 3300

ctccttattt aaaagttcta tttgtgactt ttcatttcct gaaagtttaa aaatacaatt 3360

tgagaatgtt tataatacat tctctcctgt cttttcacgg ttacgtctgt tattgctgaa 3420

atacaccaca ttttctttgt tctggtcaag gttaactcaa tatctgtgtg aaagagaact 3480

actaacaacg ttacaataga ggctagattt gaaaaaaaaa atctatagat ctaattgata 3540

caattgtaga acaaaatgtc aaaataatgt tttaagtata agagaagatg gaccaaggag 3600

agagagatca tttgaaaatc taattgtagc ttttctaggc tcacattcat gtactacttt 3660

tagcaccctt atgggctgtg ctcgccccct ggacagttga gctttggatt atcttcctct 3720

tcaattttcc ctctattgac ccgagtgtct ccctctgctt ctacagattt atagtactcc 3780

ttggctcttt tgagtctcca cttttactca ctgtctctgg gatttttaag atccttttct 3840

tctcttataa atcatcctct taatgaaaat tagcctaaca aaagtttgga gactggaatc 3900

ctactttgag ccactgactt gaaataactc ttttggcaag ttgcctgaca tcctgtctta 3960

ccaaggtggc atatttgcat ttttactgct taaaacattt tttttttttt accatcttta 4020

tccaaattta tcatattgat ggtaggacta acaggctttt tagaagctgg ctttaacttt 4080

gagtctcaag ctacaatgct gttgggcagc ctggtcttcc cacgtgaggg tttaactttg 4140

tttatttgcc tccagttatt ccaaaatgct tattaaatga aagtcccagg aacatgttta 4200

ttttagtcac ctttgctttt taacaatttt gttttgtaat caatgagtaa ttcatgatga 4260

attatttttg actaatggat agccgaaggc caggctttta attctaatag gtaatgttct 4320

tcttttgtct tattgaaaca atgagaatac tctgtgcatt tcaaatgcac tccgattatg 4380

ctgtggtttt attcacataa gcacaatatg tgttttattt ataacttcat aacaaactta 4440

taatataata atttacctta gcagacatgc aaaagcttat tcttgtgtga cttactttct 4500

ttaagctaat aatataaaaa taaatatgta tcttaaaaat ctataataaa acattagaaa 4560

ttaaagatat gtgcttttta ttttgcagat gagttcattt gcttttgtag atgtgttttc 4620

agagctaggt acagaggaat gtttgctacc tttagcggtg aaaaaagaaa gagagtcaag 4680

aattttgttg gattgtgttt gtgtgtgcat atatttgata tcatcattat atttgtaatc 4740

tttggacttg taatcatagc ctgtttattc tactgtgcca ttaaatatac tttaccttat 4800

acataacgaa taaaatacct agaagtagat ttatttacaa aaaaaaaaaa aaaa 4854

<210> 26
<211> 1779
<212> mRNA
<213> Homo sapiens

<400> 26
gtgactgggt ggggctgcct cacttctgcc tgatttggga agcgctgcaa ggacaaccgg 60

ctggggtcct tgcgcgccgc ggctcaggga ggagcaccga ctgcgccgcg taagtgccgc 120

ctgccctgcg tgggtcgtgc cagctcagcg ggacaggtcc tcgcctcggt ccctcggact 180

tagggagcgc ggggcagacc ctgagagatg gttggtgcca tgtggaaggt gattgtttcg 240

ctggtcctgt tgatgcctgg cccctgtgat gggctgtttc gctccctata cagaagtgtt 300

tccatgccac ctaagggaga ctcaggacag ccattatttc tcacccctta cattgaagct 360

gggaagatcc aaaaaggaag agaattgagt ttggtcggcc ctttcccagg actgaacatg 420

aagagttatg ccggcttcct caccgtgaat aagacttaca acagcaacct cttcttctgg 480

ttcttcccag ctcagataca gccagaagat gccccagtag ttctctggct acagggtggg 540

ccgggaggtt catccatgtt tggactcttt gtggaacatg ggccttatgt tgtcacaagt 600

aacatgacct tgcgtgacag agacttcccc tggaccacaa cgctctccat gctttacatt 660

gacaatccag tgggcacagg cttcagtttt actgatgata cccacggata tgcagtcaat 720

gaggacgatg tagcacggga tttatacagt gcactaattc agtttttcca gatatttcct 780

gaatataaaa ataatgactt ttatgtcact ggggagtctt atgcagggaa atatgtgcca 840

gccattgcac acctcatcca ttccctcaac cctgtgagag aggtgaagat caacctgaac 900

ggaattgcta ttggagatgg atattctgat cccgaatcaa ttataggggg ctatgcagaa 960

ttcctgtacc aaattggctt gttggatgag aagcaaaaaa agtacttcca gaagcagtgc 1020

catgaatgca tagaacacat caggaagcag aactggtttg aggcctttga aatactggat 1080

aaactactag atggcgactt aacaagtgat ccttcttact tccagaatgt tacaggatgt 1140

agtaattact ataacttttt gcggtgcacg gaacctgagg atcagcttta ctatgtgaaa 1200

tttttgtcac tcccagaggt gagacaagcc atccacgtgg ggaatcagac ttttaatgat 1260

ggaactatag ttgaaaagta cttgcgagaa gatacagtac agtcagttaa gccatggtta 1320

actgaaatca tgaataatta taaggttctg atctacaatg gccaactgga catcatcgtg 1380

gcagctgccc tgacagagcg ctccttgatg ggcatggact ggaaaggatc ccaggaatac 1440

aagaaggcag aaaaaaaagt ttggaagatc tttaaatctg acagtgaagt ggctggttac 1500

atccggcaag cgggtgactt ccatcaggta attattcgag gtggaggaca tattttaccc 1560

tatgaccagc ctctgagagc ttttgacatg attaatcgat tcatttatgg aaaaggatgg 1620

gatccttatg ttggataaac taccttccca aaagagaaca tcagaggttt tcattgctga 1680

aaagaaaatc gtaaaaacag aaaatgtcat aggaataaaa aaattatctt ttcatatctg 1740

caagattttt ttcatcaata aaaattatcc ttgaaacaa 1779

<210> 27
<211> 2913
<212> mRNA
<213> Homo sapiens

<400> 27

```
gaatggctgg ggttggctga agagcgcaca gtggagtttt aatgtccgcc atgttggcca     60
tggcgtattg aagagagcga gcgagagagg agcggagcgg cacagcctcc caccctcccg    120
gctggtgtta gtgcccggac ggcgggctct gcgctccgcc cctcaagtcc ccggcagcgg    180
ttggcgagtg gggccgaac ccccggttct ccatgatccc gctggccggg gccgtttccc    240
cagagcggag aggtatctgc tgcgcctgag atgagtaaac tgtcgtttcg ggcgcgggcg    300
ctagacgcct cgaagccgct gccggttttc cgctgtgagg atctgcccga cctgcacgaa    360
tacgcctcga taaacagggc cgtgccgcag atgcccaccg gaatggagaa ggaagaggag    420
tcggaacatc atcttcagcg ggctatttca gcacagcagg tgtatggcga gaagagggat    480
aatatggtta taccggtccc agaggcagaa agtaatattg cttactatga gtctatatat    540
cctggggaat ttaagatgcc aaagcagctc attcacatac agccttttag tttggatgct    600
gaacagcctg attatgattt ggattctgaa gatgaagtat ttgtgaataa actgaaaaag    660
aaaatggaca tctgcccatt gcaatttgag gagatgattg accgcctaga aaaaggcagt    720
ggtcagcagc cagtcagtct gcaggaagcc aaactactgc taaaagaaga tgatgaacta    780
attagagaag tttatgaata ttggattaaa aagagaaaaa actgtcgagg gccatctctt    840
attccatcag taaaacaaga gaagcgagat ggttccagca caaatgatcc ttatgtggct    900
tttagaaggc gtactgaaaa aatgcagact cgaaaaaatc gcaaaaatga tgaagcctct    960
tacgaaaaaa tgcttaagct gcgacgagat ctaagtcgag ctgttactat tctagagatg   1020
ataaaaagaa gagaaaaaag taaaagagag ctattgcact taacactgga aattatggaa   1080
aagaggtata atttgggcga ctacaatgga gagatcatgt ctgaggttat ggcacagaga   1140
cagccaatga aacctactta tgccatcccc atcatcccta ttactaatag cagtcaattt   1200
aaacaccagg aagcaatgga tgtgaaggag ttcaaagtta ataagcaaga taaagccgat   1260
cttatccgac cgaaacggaa atatgaaaag aagcccaaag tcttaccatc gtctgccgct   1320
gctactcccc aacagacgag tcctgctgca ctgccagtct tcaatgctaa agatctgaat   1380
cagtatgact ttcccagctc agacgaagaa cctctctccc aggttttgtc tggctcttcg   1440
gaagctgagg aagacaatga tcctgatggt ccttttgctt tccgtaggaa agcaggctgt   1500
cagtactatg ctcctcactt agaccaaact ggcaactggc cttggactag tcctaaagat   1560
ggaggattag gggatgtgcg atatagatac tgcttaacta ctctcaccgt accccaaagg   1620
tgtattggat ttgcacgaag acgggttggg cgcggtggaa gggtcttact ggacagagct   1680
cattcagact atgacagtgt gtttcaccat ctggatttgg aaatgctttc ctcaccacaa   1740
cattctccag tcaatcagtt tgccaatacc tcagaaacaa atacctcgga caaatctttc   1800
tctaaagacc tcagtcagat actagtcaat atcaaatcat gtagatggcg gcattttagg   1860
```

cctcggacac catccctaca tgacagtgac aatgatgaac tctcctgtag aaaattatat 1920

aggagtataa accgaacagg aacagcacaa cctgggaccc agacatgcag tacctctacg 1980

caaagtaaaa gtagcagtgg ttcagcacac tttgcattta cagccgaaca ataccagcaa 2040

catcaacagc aactggcact catgcagaaa cagcagcttg cacaaattca gcaacagcaa 2100

gcaaatagta attcctccac caacacatca cagaaccttg catctaacca gcagaaaagt 2160

ggctttcgcc tgaatataca gggtttagaa agaacactac agggttttgt ttctaagact 2220

ttggattctg ctagtgcaca gtttgctgct tctgctttgg tgacatcaga acaactgatg 2280

ggattcaaga tgaaggatga tgtggtgctt ggaatcgggg tgaatggcgt ccttccagcc 2340

tcaggagtat acaagggctt acacctcagt agtactacac caacagcact tgtacataca 2400

agtccatcaa cggcaggttc agctttgtta cagccttcaa atattacaca gacttcaagt 2460

tcccacagtg cactgagtca tcaagtaact gctgccaatt ctgcaacaac tcaggttctg 2520

attgggaaca acattcgatt aactgtacct tcatcagttg ccactgtaaa ctctattgcc 2580

ccaataaatg cacgacatat acctaggact ttaagtgctg ttccatcatc tgccttaaag 2640

ctggccgctg cagcaaactg tcaagtttcc aaggtcccat cttcatcctc tgtagattca 2700

gttccaaggg aaaatcatga atcagaaaag ccagcactga acaacatagc agacaacaca 2760

gtagcgatgg aggtgacgta gcttcctccg agtggaactg cagcctgggg acttgattag 2820

gtgctatgca tcagtagcat tttgaatgca agggatgatg gaaagcagca catgcgtttc 2880

tgtggcagct gtggggactt gacagcaatg ctc 2913

<210> 28
<211> 1691
<212> mRNA
<213> Homo sapiens

<400> 28
gtgactgggt ggggctgcct cacttctgcc tgatttggga agcgctgcaa ggacaaccgg 60

ctggggtcct tgcgcgccgc ggctcaggga ggagcaccga ctgcgccgca ccctgagaga 120

tggttggtgc catgtggaag gtgattgttt cgctggtcct gttgatgcct ggcccctgtg 180

atgggctgtt tcgctcccta tacagaagtg tttccatgcc acctaaggga gactcaggac 240

agccattatt tctcacccct tacattgaag ctgggaagat ccaaaaagga agagaattga 300

gtttggtcgg ccctttccca ggactgaaca tgaagagtta tgccggcttc ctcaccgtga 360

ataagactta caacagcaac ctcttcttct ggttcttccc agctcagata cagccagaag 420

atgccccagt agttctctgg ctacagggtg ggccgggagg ttcatccatg tttggactct 480

ttgtggaaca tgggccttat gttgtcacaa gtaacatgac cttgcgtgac agagacttcc 540

cctggaccac aacgctctcc atgctttaca ttgacaatcc agtgggcaca ggcttcagtt 600

ttactgatga tacccacgga tatgcagtca atgaggacga tgtagcacgg gatttataca 660

gtgcactaat tcagtttttc cagatatttc ctgaatataa aaataatgac ttttatgtca 720

ctggggagtc ttatgcaggg aaatatgtgc cagccattgc acacctcatc cattccctca 780

accctgtgag agaggtgaag atcaacctga acggaattgc tattggagat ggatattctg 840

atcccgaatc aattataggg ggctatgcag aattcctgta ccaaattggc ttgttggatg 900

agaagcaaaa aaagtacttc cagaagcagt gccatgaatg catagaacac atcaggaagc 960

agaactggtt tgaggccttt gaaatactgg ataaactact agatggcgac ttaacaagtg 1020

atccttctta cttccagaat gttacaggat gtagtaatta ctataacttt ttgcggtgca 1080

cggaacctga ggatcagctt tactatgtga aatttttgtc actcccagag gtgagacaag 1140

ccatccacgt ggggaatcag acttttaatg atggaactat agttgaaaag tacttgcgag 1200

aagatacagt acagtcagtt aagccatggt taactgaaat catgaataat tataaggttc 1260

tgatctacaa tggccaactg gacatcatcg tggcagctgc cctgacagag cgctccttga 1320

tgggcatgga ctggaaagga tcccaggaat acaagaaggc agaaaaaaaa gtttggaaga 1380

tctttaaatc tgacagtgaa gtggctggtt acatccggca agcgggtgac ttccatcagg 1440

taattattcg aggtggagga catattttac cctatgacca gcctctgaga gcttttgaca 1500

tgattaatcg attcatttat ggaaaaggat gggatcctta tgttggataa actaccttcc 1560

caaaagagaa catcagaggt tttcattgct gaaaagaaaa tcgtaaaaac agaaaatgtc 1620

ataggaataa aaaaattatc ttttcatatc tgcaagattt ttttcatcaa taaaaattat 1680

ccttgaaaca a 1691

<210> 29
<211> 2769
<212> mRNA
<213> Homo sapiens

<400> 29
gtgctctgag tttttggttt ctgtttcacc ttgtgtctga gctggtctga aggctggttg 60

ttcagactga gcttcctgcc tgcctgtacc ccgccaacag cttcagaaga aggagcagcc 120

cctgggtgcg tccactttct gggcacgtga ggttgggcct tggccgcctg agcccttgag 180

ttggtcactt gaaccttggg aatattgaga tggacttcag cagaaatctt tatgatattg 240

gggaacaact ggacagtgaa gatctggcct ccctcaagtt cctgagcctg gactacattc 300

cgcaaaggaa gcaagaaccc atcaaggatg ccttgatgtt attccagaga ctccaggaaa 360

agagaatgtt ggaggaaagc aatctgtcct tcctgaagga gctgctcttc cgaattaata 420

gactggattt gctgattacc tacctaaaca ctagaaagga ggagatggaa agggaacttc 480

agacaccagg cagggctcaa atttctgcct acagggtcat gctctatcag atttcagaag 540

aagtgagcag atcagaattg aggtctttta agtttctttt gcaagaggaa atctccaaat 600
gcaaactgga tgatgacatg aacctgctgg atattttcat agagatggag aagagggtca 660
tcctgggaga aggaaagttg gacatcctga aaagagtctg tgcccaaatc aacaagagcc 720
tgctgaagat aatcaacgac tatgaagaat tcagcaaaga gagaagcagc agccttgaag 780
gaagtcctga tgaattttca aatggggagg agttgtgtgg ggtaatgaca atctcggact 840
ctccaagaga acaggatagt gaatcacaga ctttggacaa agtttaccaa atgaaaagca 900
aacctcgggg atactgtctg atcatcaaca atcacaattt tgcaaaagca cgggagaaag 960
tgcccaaact tcacagcatt agggacagga atggaacaca cttggatgca ggggctttga 1020
ccacgacctt tgaagagctt cattttgaga tcaagcccca cgatgactgc acagtagagc 1080
aaatctatga gattttgaaa atctaccaac tcatggacca cagtaacatg gactgcttca 1140
tctgctgtat cctctcccat ggagacaagg gcatcatcta tggcactgat ggacaggagg 1200
cccccatcta tgagctgaca tctcagttca ctggtttgaa gtgcccttcc cttgctggaa 1260
aacccaaagt gtttttattt caggcttgtc aggggggataa ctaccagaaa ggtatacctg 1320
ttgagactga ttcagaggag caaccctatt tagaaatgga tttatcatca cctcaaacga 1380
gatatatccc ggatgaggct gactttctgc tggggatggc cactgtgaat aactgtgttt 1440
cctaccgaaa ccctgcagag ggaacctggt acatccagtc actttgccag agcctgagag 1500
agcgatgtcc tcgaggcgat gatattctca ccatcctgac tgaagtgaac tatgaagtaa 1560
gcaacaagga tgacaagaaa aacatgggga aacagatgcc tcagcctact ttcacactaa 1620
gaaaaaaact tgtcttccct tctgattgat ggtgctattt tgtttgtttt gttttgtttt 1680
gtttttttga gacagaatct cgctctgtcg cccaggctgg agtgcagtgg cgtgatctcg 1740
gctcaccgca agctccgcct cccgggttca ggccattctc ctgcctcagc ctcccgagta 1800
gctgggacta caggggcccg ccaccacacc tggctaattt tttaaaaata tttttagtag 1860
agacagggtt tcactgtgtt agccagggtg gtcttgatct cctgacctcg tgatccaccc 1920
acctcggcct cccaaagtgc tgggattaca ggcgtgagcc accgcgcctg gccgatggta 1980
ctatttagat ataacactat gtttatttac taattttcta gattttctac tttattaatt 2040
gttttgcact tttttataag agctaaagtt aaataggata ttaacaacaa taacactgtc 2100
tcctttctct tatgcttaag gctttgggaa tgtttttagc tggtggcaat aaataccaga 2160
cacgtacaaa atccagctat gaatatagag ggcttatgat tcagattgtt atctatcaac 2220
tataagccca ctgttaatat tctattaact ttaattctct ttcaaagcta aattccacac 2280
taccacatta aaaaaattag aaagtagcca cgtatggtgg ctcatgtcta taatcccagc 2340
actttgggag gttgaggtgg gaggattgct tgaacccaag aggtcaaggc tgcagtgagc 2400
catgttcaca ccgctgcact caagcttggg tgacagaaca agaccccgtc tcaaaaaaaa 2460

tttttttttt aataaaacaa aatttgtttg aaatctttta aaaattcaaa tgatttttac 2520

aagttttaaa taagctctcc ccaaacttgc tttatgcctt cttattgctt ttatgatata 2580

tatatgcttg gctaactata tttgcttttt gctaacaatg ctctggggtc tttttatgca 2640

tttgcatttg ctctttcatc tctgcttgga ttattttaaa tcattaggaa ttaagttatc 2700

tttaaaattt aagtatcttt tttcaaaaac atttttttaat agaataaaat ataatttgat 2760

cttattaaa 2769

<210> 30
<211> 2655
<212> mRNA
<213> Homo sapiens

<400> 30
gtagagctgg ctggctgtgc ccttccgtcc ttcattagac gtttgctctt gtcttagatg 60

ctcagatggt agtggatagg cctgtgacga aggtgctacc atcgtgagag taagattata 120

ttctcctgcc ttttaaaaag atggacttca gcagaaatct ttatgatatt ggggaacaac 180

tggacagtga agatctggcc tccctcaagt tcctgagcct ggactacatt ccgcaaagga 240

agcaagaacc catcaaggat gccttgatgt tattccagag actccaggaa aagagaatgt 300

tggaggaaag caatctgtcc ttcctgaagg agctgctctt ccgaattaat agactggatt 360

tgctgattac ctacctaaac actagaaagg aggagatgga aagggaactt cagacaccag 420

gcagggctca aatttctgcc tacagggtca tgctctatca gatttcagaa gaagtgagca 480

gatcagaatt gaggtctttt aagtttcttt tgcaagagga aatctccaaa tgcaaactgg 540

atgatgacat gaacctgctg gatattttca tagagatgga gaagagggtc atcctgggag 600

aaggaaagtt ggacatcctg aaaagagtct gtgcccaaat caacaagagc ctgctgaaga 660

taatcaacga ctatgaagaa ttcagcaaag gggaggagtt gtgtggggta atgacaatct 720

cggactctcc aagagaacag gatagtgaat cacagacttt ggacaaagtt taccaaatga 780

aaagcaaacc tcggggatac tgtctgatca tcaacaatca caattttgca aaagcacggg 840

agaaagtgcc caaacttcac agcattaggg acaggaatgg aacacacttg gatgcagggg 900

ctttgaccac gacctttgaa gagcttcatt ttgagatcaa gccccacgat gactgcacag 960

tagagcaaat ctatgagatt ttgaaaatct accaactcat ggaccacagt aacatggact 1020

gcttcatctg ctgtatcctc tcccatggag acaagggcat catctatggc actgatggac 1080

aggaggcccc catctatgag ctgacatctc agttcactgg tttgaagtgc ccttcccttg 1140

ctggaaaacc caaagtgttt tttattcagg cttgtcaggg ggataactac cagaaaggta 1200

tacctgttga gactgattca gaggagcaac cctatttaga aatggattta tcatcacctc 1260

aaacgagata tatcccggat gaggctgact ttctgctggg gatggccact gtgaataact 1320

```
gtgtttccta ccgaaaccct gcagagggaa cctggtacat ccagtcactt tgccagagcc   1380
tgagagagcg atgtcctcga ggcgatgata ttctcaccat cctgactgaa gtgaactatg   1440
aagtaagcaa caaggatgac aagaaaaaca tggggaaaca gatgcctcag cctactttca   1500
cactaagaaa aaaacttgtc ttcccttctg attgatggtg ctattttgtt tgttttgttt   1560
tgttttgttt ttttgagaca gaatctcgct ctgtcgccca ggctggagtg cagtggcgtg   1620
atctcggctc accgcaagct ccgcctcccg ggttcaggcc attctcctgc ctcagcctcc   1680
cgagtagctg ggactacagg ggcccgccac cacacctggc taattttta aaaatatttt    1740
tagtagagac agggtttcac tgtgttagcc agggtggtct tgatctcctg acctcgtgat   1800
ccacccacct cggcctccca aagtgctggg attacaggcg tgagccaccg cgcctggccg   1860
atggtactat ttagatataa cactatgttt atttactaat tttctagatt ttctacttta   1920
ttaattgttt tgcacttttt tataagagct aaagttaaat aggatattaa caacaataac   1980
actgtctcct ttctcttatg cttaaggctt tgggaatgtt tttagctggt ggcaataaat   2040
accagacacg tacaaaatcc agctatgaat atagagggct tatgattcag attgttatct   2100
atcaactata agcccactgt taatattcta ttaactttaa ttctctttca aagctaaatt   2160
ccacactacc acattaaaaa aattagaaag tagccacgta tggtggctca tgtctataat   2220
cccagcactt tgggaggttg aggtgggagg attgcttgaa cccaagaggt caaggctgca   2280
gtgagccatg ttcacaccgc tgcactcaag cttgggtgac agaacaagac cccgtctcaa   2340
aaaaaatttt ttttttaata aaacaaaatt tgtttgaaat cttttaaaaa ttcaaatgat   2400
ttttacaagt tttaaataag ctctccccaa acttgcttta tgccttctta ttgcttttat   2460
gatatatata tgcttggcta actatatttg ctttttgcta acaatgctct ggggtctttt   2520
tatgcatttg catttgctct ttcatctctg cttggattat tttaaatcat taggaattaa   2580
gttatcttta aaatttaagt atcttttttc aaaaacattt tttaatagaa taaaatataa   2640
tttgatctta ttaaa                                                    2655
```

<210> 31
<211> 1123
<212> mRNA
<213> Homo sapiens

<400> 31

```
gtgctctgag tttttggttt ctgtttcacc ttgtgtctga gctggtctga aggctggttg     60
ttcagactga gcttcctgcc tgcctgtacc ccgccaacag cttcagaaga aggtgactgg    120
tggctgcctg aggaatacca gtgggcaaga gaattagcat ttctggagca tctgctgtct    180
gagcagcccc tgggtgcgtc cactttctgg gcacgtgagg ttgggccttg gccgcctgag    240
cccttgagtt ggtcacttga accttgggaa tattgagatt atattctcct gccttttaaa    300
```

aagatggact tcagcagaaa tctttatgat attggggaac aactggacag tgaagatctg 360

gcctccctca agttcctgag cctggactac attccgcaaa ggaagcaaga acccatcaag 420

gatgccttga tgttattcca gagactccag gaaaagagaa tgttggagga aagcaatctg 480

tccttcctga aggagctgct cttccgaatt aatagactgg atttgctgat tacctaccta 540

aacactagaa aggaggagat ggaaagggaa cttcagacac caggcagggc tcaaatttct 600

gcctacaggg tcatgctcta tcagatttca gaagaagtga gcagatcaga attgaggtct 660

tttaagtttc ttttgcaaga ggaaatctcc aaatgcaaac tggatgatga catgaacctg 720

ctggatattt tcatagagat ggagaagagg gtcatcctgg gagaaggaaa gttggacatc 780

ctgaaaagag tctgtgccca aatcaacaag agcctgctga agataatcaa cgactatgaa 840

gaattcagca aagagagaag cagcagcctt gaaggaagtc ctgatgaatt ttcaaatgac 900

tttggacaaa gtttaccaaa tgaaaagcaa acctcgggga tactgtctga tcatcaacaa 960

tcacaatttt gcaaaagcac gggagaaagt gcccaaactt cacagcatta gggacaggaa 1020

tggaacacac ttggatgcag ggtttgagaa tgtttttagc tggtggcaat aaatattaga 1080

agcctgcaga atccagctac gaatatagag ggttttgctc ttg 1123

<210> 32
<211> 1157
<212> mRNA
<213> Homo sapiens

<400> 32
gctcacagtc atcaattata gaccccacaa catgcgccct gaagacagaa tgttccatat 60

cagagctgtg atcttgagag ccctctcctt ggctttcctg ctgagtctcc gaggagctgg 120

ggccatcaag gcggaccatg tgtcaactta tgccgcgttt gtacagacgc atagaccaac 180

aggggagttt atgtttgaat ttgatgaaga tgagatgttc tatgtggatc tggacaagaa 240

ggagaccgtc tggcatctgg aggagtttgg ccaagccttt tcctttgagg ctcagggcgg 300

gctggctaac attgctatat tgaacaacaa cttgaatacc ttgatccagc gttccaacca 360

cactcaggcc accaacgatc cccctgaggt gaccgtgttt cccaaggagc ctgtggagct 420

gggccagccc aacaccctca tctgccacat tgacaagttc ttcccaccag tgctcaacgt 480

cacgtggctg tgcaacgggg agctggtcac tgagggtgtc gctgagagcc tcttcctgcc 540

cagaacagat tacagcttcc acaagttcca ttacctgacc tttgtgccct cagcagagga 600

cttctatgac tgcagggtgg agcactgggg cttggaccag ccgctcctca agcactggga 660

ggcccaagag ccaatccaga tgcctgagac aacggagact gtgctctgtg ccctgggcct 720

ggtgctgggc ctagtcggca tcatcgtggg caccgtcctc atcataaagt ctctgcgttc 780

tggccatgac ccccgggccc aggggaccct gtgaaatact gtaaaggtga caaaatatct 840

gaacagaaga ggacttagga gagatctgaa ctccagctgc cctacaaact ccatctcagc 900

ttttcttctc acttcatgtg aaaactactc cagtggctga ctgaattgct gacccttcaa 960

gctctgtcct tatccattac ctcaaagcag tcattcctta gtaaagtttc caacaaatag 1020

aaattaatga cactttggta gcactaatat ggagattatc ctttcattga gccttttatc 1080

ctctgttctc ctttgaagaa cccctcactg tcaccttccc gagaataccc taagaccaat 1140

aaatacttca gtatttc 1157

<210> 33
<211> 907
<212> mRNA
<213> Homo sapiens

<400> 33
gtcggggcgc gcccggaaac cccaaggccc agacggctct cagatccggg ggctgcggat 60

aaatccccgt aggccgcggg cagcgagatg ttgcgttccg gtgtgggtgt gggtgtgcct 120

ccgacggcgt ctcggtgcca gtgtcgaggt tctttctgct tagctacccg gagccgacta 180

cggaggagga cacctgagtt tacgtctctt ccatctgctg ctcacctcag ctgcctgggt 240

ccccgacgag agccaggtga cacttaactc cgccatctgc gttttgagca ctgttctcat 300

aatggagttt cctgatttgg ggaagcattg ttcagaaaag acttgcaagc agctagattt 360

tcttccagta aaatgtgatg catgtaaaca agatttctgt aaagatcatt ttccatacgc 420

tgcacataag tgtccgtttg cattccagaa ggatgttcac gtcccagtat gcccactctg 480

taataccccc atcccagtaa aaaagggcca gataccagac gtggtggttg gtgatcacat 540

tgacagagac tgtgactctc accctgggaa gaagaaagag aagattttta cataccgttg 600

ctcaaaagag ggctgcaaga agaaagagat gctgcagatg gtatgtgccc aatgtcacgg 660

caacttctgt atccagcaca gacacccttt ggaccacagc tgcagacacg ggagtcgccc 720

caccatcaaa gctgggtgag aagagactcc gctgcgatgg ctcggagcac gcagtagcat 780

gcgtggaagc agcttacact ctagtgggaa gtggagcccc attgagcacc atcccacact 840

ggctgctgat cttgtttgtt gagggagatg ggactataat aaaattgaat gctgaagttg 900

aaaaaaa 907

<210> 34
<211> 7751
<212> mRNA
<213> Homo sapiens

<400> 34
ggggccgggg ggcggagcct tgcgggctgg agcgaaagaa tgcgggggct gagcgcagaa 60

gcggctcgag gctggaagag gatcttgggc gccgccagtc tctctctgtt gcccaagctg 120

gagtgcagtg gcacagtctt ggctcactgc aacctccacc tcctgggtgc aagcgattct 180
cgtgtctcag cctctcaagt agctgggatt acaggttctg tggacaatca caatgggaat 240
ccaaggaggg tctgtcctgt tcgggctgct gctcgtcctg gctgtcttct gccattcagg 300
tcatagcctg cagtgctaca actgtcctaa cccaactgct gactgcaaaa cagccgtcaa 360
ttgttcatct gattttgatg cgtgtctcat taccaaagct gggttacaag tgtataacaa 420
gtgttggaag tttgagcatt gcaatttcaa cgacgtcaca acccgcttga gggaaaatga 480
gctaacgtac tactgctgca agaaggacct gtgtaacttt aacgaacagc ttgaaaatgg 540
tgggacatcc ttatcagaga aaacagttct tctgctggtg actccatttc tggcagcagc 600
ctggagcctt catccctaag tcaacaccag gagagcttct cccaaactcc ccgttcctgc 660
gtagtccgct ttctcttgct gccacattct aaaggcttga tattttccaa atggatcctg 720
ttgggaaaga ataaaattag cttgagcaac ctggctaaga tagaggggct ctgggagact 780
ttgaagacca gtcctgtttg cagggaagcc ccacttgaag gaagaagtct aagagtgaag 840
taggtgtgac ttgaactaga ttgcatgctt cctcctttgc tcttgggaag accagctttg 900
cagtgacagc ttgagtgggt tctctgcagc cctcagatta tttttcctct ggctccttgg 960
atgtagtcag ttagcatcat tagtacatct ttggagggtg gggcaggagt atatgagcat 1020
cctctctcac atggaacgct ttcataaact tcagggatcc cgtgttgcca tggaggcatg 1080
ccaaatgttc catatgtggg tgtcagtcag ggacaacaag atccttaatg cagagctaga 1140
ggacttctgg cagggaagtg gggaagtgtt ccagatagca gggcatgaaa acttagagag 1200
gtacaagtgg ctgaaaatcg agtttttcct ctgtctttaa attttatatg ggctttgtta 1260
tcttccactg gaaaagtgta atagcataca tcaatggtgt gttaaagcta tttccttgcc 1320
tttttttat tggaatggta ggatatcttg gctttgccac acacagttac agagtgaaca 1380
ctctactaca tgtgactggc agtattaagt gtgcttattt taaatgttac tggtagaaag 1440
gcagttcagg tatgtgtgta tatagtatga atgcagtggg gacacccttt gtggttacag 1500
tttgagactt ccaaaggtca tccttaataa caacagatct gcaggggtat gttttaccat 1560
ctgcatccag cctcctgcta actcctagct gactcagcat agattgtata aaataccttt 1620
gtaacggctc ttagcacact cacagatgtt tgaggctttc agaagctctt ctaaaaaatg 1680
atacacacct ttcacaaggg caaacttttt ccttttccct gtgtattcta gtgaatgaat 1740
ctcaagattc agtagaccta atgacatttg tattttatga tcttggctgt atttaatggc 1800
ataggctgac ttttgcagat ggaggaattt cttgattaat gttgaaaaaa aacccttgat 1860
tatactctgt tggacaaacc gagtgcaatg aatgatgctt ttctgaaaat gaaatataac 1920
aagtgggtga atgtggttat ggccgaaaag gatatgcagt atgcttaatg gtagcaactg 1980
aaagaagaca tcctgagcag tgccagcttt cttctgttga tgccgttccc tgaacatagg 2040

aaaatagaaa cttgcttatc aaaacttagc attaccttgg tgctctgtgt tctctgttag 2100
ctcagtgtct ttccttacat caataggttt tttttttttt ttttggcctg aggaagtact 2160
gaccatgccc acagccaccg gctgagcaaa gaagctcatt tcatgtgagt tctaaggaat 2220
gagaaacaat tttgatgaat ttaagcagaa aatgaatttc tgggaacttt tttgggggcg 2280
gggggtggg gaattcagcc acactccaga aagccaggag tcgacagttt tggaagcctc 2340
tctcaggatt gagattctag gatgagattg gcttactgct atcttgtgtc atgtacccac 2400
tttttggcca gactacactg ggaagaaggt agtcctctaa agcaaaatct gagtgccact 2460
aaatggggag atggggctgt taagctgtcc aaatcaacaa gggtcatata aatggcctta 2520
aactttgggg ttgctttctg caaaaagttg ctgtgactca tgccatagac aaggttgagt 2580
gcctggaccc aaaggcaata ctgtaatgta aagacattta tagtactagg caaacagcac 2640
cccaggtact ccaggccctc ctggctggag agggctgtgg caatagaaaa ttagtgccaa 2700
ctgcagtgag tcagcctagg ttaaatagag agtgtaagag tgctggacag gaacctccac 2760
cctcatgtca catttcttca atgtgaccct tctggcccct ctcctcctga cagcggaaca 2820
atgactgccc cgataggtga ggctggagga agaatcagtc ctgtccttgg caagctcttc 2880
actatgacag taaaggctct ctgcctgctg ccaaggcctg tgactttcta acctggcctc 2940
acgctgggta agcttaaggt agaggtgcag gattagcaag cccacctggc taccaggccg 3000
acagctacat cctccaactg accctgatca acgaagaggg attcatgtgt ctgtctcagt 3060
tggttccaaa tgaaaccagg gagcagggga gttaggaatc gaacaccagt catgcctact 3120
ggctctctgc tcgagagcca ataccctgtg ccctccactc atctggattt acaggaactg 3180
tcatagtgtt cagtattggg tggtgataag cccattggat tgtccccttg gggggatgag 3240
ctaggggtgc aaggaacacc tgatgagtag ataagtggag ctcatggtat ttcctgaaag 3300
atgctaatct atttgccaaa cttggtcttg aatgtactgg gggcttcaag gtatgggtat 3360
atttttcttg tgtccttgca gttagccccc atgtcttatg tgtgtcctga aaaaataaga 3420
gcctgcccaa gactttgggc ctcttgacag aattaaccac ttttatacat ctgagttctc 3480
ttggtaagtt ctttagcagt gttcaaagtc tactagctcg cattagtttc tgttgctgcc 3540
aacagatctg aactaatgct aacagatccc cctgagggat tcttgatggg ctgagcagct 3600
ggctggagct agtactgact gacattcatt gtgatgaggg cagctttctg gtacaggatt 3660
ctaagctcta tgttttatat acattttcat ctgtacttgc acctcacttt acacaagagg 3720
aaactatgca aagttagctg gatcgctcaa ggtcacttag gtaagttggc aagtccatgc 3780
ttcccactca gctcctcagg tcagcaagtc tacttctctg cctattttgt atactctctt 3840
taatatgtgc ctagctttgg aaagtctaga atgggtccct ggtgcctttt tactttgaag 3900

aaatcagttt ctgcctcttt ttggaaaaga aaacaaagtg caattgtttt ttactggaaa 3960
gttacccaat agcatgaggt gaacaggacg tagttaggcc ttcctgtaaa cagaaaatca 4020
tatcaaaaca ctatcttccc atctgtttct caatgcctgc tacttcttgt agatatttca 4080
tttcaggaga gcagcagtta aacccgtgga ttttgtagtt aggaacctgg gttcaaaccc 4140
tcttccacta attggctatg tctctggaca agtttttttt tttttttttt tttaaaccct 4200
ttctgaactt tcactttcta tgtctacctc aaagaattgt tgtgaggctt gagataatgc 4260
atttgtaaag ggtctgccag ataggaagat gctagttatg gatttacaag gttgttaagg 4320
ctgtaagagt ctaaaaccta cagtgaatca caatgcattt acccccactg acttggacat 4380
aagtgaaaac tagccagaag tctctttttc aaattactta caggttattc aatataaaat 4440
ttttgtaatg gataatctta tttatctaaa ctaaagcttc ctgtttatac acactcctgt 4500
tattctggga taagataaat gaccacagta ccttaatttc taggtgggtg cctgtgatgg 4560
ttcattgtag gtaaggacat tttctctttt tcagcagctg tgtaggtcca gagcctctgg 4620
gagaggaggg gggtagcatg cacccagcag gggactgaac tgggaaactc aaggttcttt 4680
ttactgtggg gtagtgagct gcctttctgt gatcggtttc cctagggatg ttgctgttcc 4740
cctccttgct attcgcagct acatacaacg tggccaaccc cagtaggctg atcctatata 4800
tgatcagtgc tggtgctgac tctcaatagc cccacccaag ctggctatag gtttacagat 4860
acattaatta ggcaacctaa aatattgatg ctggtgttgg tgtgacataa tgctatggcc 4920
agaactgaaa cttagagtta taattcatgt attagggttc tccagaggga cagaattagt 4980
aggatatatg tatatatgaa agggaggtta ttagggagaa ctggctccca cagttagaag 5040
gcgaagtcgc acaataggcc gtctgcaagc tgggttagag agaagccagt agtggctcag 5100
cctgagttca aaaacctcaa aactggggaa gctgacagtg cagccagcct tcagtctgtg 5160
gccaaaggcc caagagcccc tggcaaccaa cccactggtg caagtcctag attccaaagg 5220
ctgaagaacc tggagtctga tgtccaagag caggaagagt ggaagaaagc cagaagactc 5280
agcaaacaag gtagacagtg tctaccacca tagtggccat accaaagagg ctaccgattc 5340
cttcctgcta cctggatccc tgaagttgcc ctggtctctg caccttctaa acctagttct 5400
taagagcttt ccattacatg agctgtctca aagccctcca ataaattctc agtgtaagct 5460
tctgttgctt gtggacagaa aattctgaca gacctaccct ataagtgtta ctgtcaggat 5520
aacatgagaa cgcacaacag taagtggtca ctaagtgtta gctacggtta ttttgcccaa 5580
ggtagcatgg ctagttgatg ccggttgatg gggcttaaac ccagctccct catcttccag 5640
gcctctgtac tccctattcc actaaactac ctctcaggtt tattttttta aattcttact 5700
ctgcaagtac ataggaccac atttacctgg gaaaacaaga ataaaggctg ctctgcattt 5760
tttagaaact tttttgaaag ggagatggga atgcctgcac ccccaagtcc agaccaacac 5820

aatggttaat tgagatgaat aataaaggaa agactgttct gggcttccca gaatagcttg 5880
gtccttaaat tgtggcacaa acaacctcct gtcagagcca gcctcctgcc aggaagaggg 5940
gtaggagact agaggccgtg tgtgcagcct tgccctgaag gctagggtga caatttggag 6000
gctgtccaaa caccctggcc tctagagctg gcctgtctat ttgaaatgcc ggctctgatg 6060
ctaatcggcg accctcaggc aagttactta accttacatg cctcagtttt ctcatctgga 6120
aaatgagaac cctaggttta gggttgttag aaaagttaaa tgagttaaga caagtgcctg 6180
ggacacagta gcctcttgtg tgtgtttatc attatgtcct cagcaggtcg tagaagcagc 6240
ttctcaggtg tgaggctggc gcgattatct ggagtgggtt gggttttcta ggatggaccc 6300
cctgctgcat tttcctcatt catccaccag ggcttaatgg ggaatcaagg aatccatgtg 6360
taactgtata ataactgtag ccacactcca atgaccacct actagttgtc cctggcactg 6420
cttatacata tgtccatcaa atcaatccta tgaagtagat actgtcttca ttttatagat 6480
cagagacaat tggggttcag agagctgatg tgattttccc agggtcacag agagtcccag 6540
attcaggcac aactcttgta ttccaagaca caaccactac atgtccaaag gctgcccaga 6600
gccaccgggc acggcaaatt gtgacatatc cctaaagagg ctgagcacct ggtcaggatc 6660
tgatggctga cagtgtgtcc agatgcagag ctggagtggg ggaggggaag gggggctcct 6720
tgggacagag aaggctttct gtgctttctc tgaagggagc agtctgagga ccaagggaac 6780
ccggcaaaca gcacctcagg tactccaggc cctcctggct ggagagggct gtggcaatgg 6840
aaaattagtg ccaactgcaa tgagtcagcc tcggttaaat agagagtgaa gaatgctgga 6900
caggaacctc caccctcatg tcacatttct tcagtgtgac ccttctggcc cctctcctcc 6960
tgacagcgga acaatgactg ccccgatagg tgaggctgga ggaagaatca gtcctgtcct 7020
tggcaagctc ttcactatga cagtaaaggc tctctgcctg ctgccaaggc ctgtgacttt 7080
ctaacctggc ctcacgctgg gtaagcttaa ggtagaggtg caggattagc aagcccacct 7140
ggctaccagg ccgacagcta catctttcaa ctgaccctga tcaacgaaga gggacttgtg 7200
tctctcagtt ggttccaaat gaaaccaggg agcaggggcg ttaggaagct ccaacaggat 7260
ggtacttaat ggggcatttg agtggagagg taggtgacat agtgctttgg agcccaggga 7320
gggaaaggtt ctgctgaagt tgaattcaag actgttcttt catcacaaac ttgagtttcc 7380
tggacatttg tttgcagaaa caaccgtagg gttttgcctt aacctcgtgg gtttattatt 7440
acctcatagg gactttgcct cctgacagca gtttatgggt gttcattgtg gcacttgagt 7500
tttcttgcat acttgttaga gaaaccaagt ttgtcatcaa cttcttattt aaccccctgg 7560
ctataacttc atggattatg ttataattaa gccatccaga gtaaaatctg tttagattat 7620
cttggagtaa gggggaaaaa atctgtaatt ttttctcctc aactagatat atacataaaa 7680

aatgattgta ttgcttcatt taaaaaatat aacgcaaaat ctcttttcct tctaaaaaaa 7740

aaaaaaaaaa a 7751

<210> 35
<211> 7796
<212> mRNA
<213> Homo sapiens

<400> 35
ggggccgggg ggcggagcct tgcgggctgg agcgaaagaa tgcgggggct gagcgcagaa 60

gcggctcgag gctggaagag gatcttgggc gccgccagtc tctctctgtt gcccaagctg 120

gagtgcagtg gcacagtctt ggctcactgc aacctccacc tcctgggtgc aagcgattct 180

cgtgtctcag cctctcaagt agctgggatt acagtcttta gcaccagttg gtgtaggagt 240

tgagacctac ttcacagtag ttctgtggac aatcacaatg ggaatccaag gagggtctgt 300

cctgttcggg ctgctgctcg tcctggctgt cttctgccat tcaggtcata gcctgcagtg 360

ctacaactgt cctaacccaa ctgctgactg caaaacagcc gtcaattgtt catctgattt 420

tgatgcgtgt ctcattacca aagctgggtt acaagtgtat aacaagtgtt ggaagtttga 480

gcattgcaat ttcaacgacg tcacaacccg cttgagggaa aatgagctaa cgtactactg 540

ctgcaagaag gacctgtgta actttaacga acagcttgaa aatggtggga catccttatc 600

agagaaaaca gttcttctgc tggtgactcc atttctggca gcagcctgga gccttcatcc 660

ctaagtcaac accaggagag cttctcccaa actccccgtt cctgcgtagt ccgctttctc 720

ttgctgccac attctaaagg cttgatattt tccaaatgga tcctgttggg aaagaataaa 780

attagcttga gcaacctggc taagatagag gggctctggg agactttgaa gaccagtcct 840

gtttgcaggg aagccccact tgaaggaaga agtctaagag tgaagtaggt gtgacttgaa 900

ctagattgca tgcttcctcc tttgctcttg ggaagaccag ctttgcagtg acagcttgag 960

tgggttctct gcagccctca gattattttt cctctggctc cttggatgta gtcagttagc 1020

atcattagta catctttgga gggtggggca ggagtatatg agcatcctct ctcacatgga 1080

acgctttcat aaacttcagg gatcccgtgt tgccatggag gcatgccaaa tgttccatat 1140

gtgggtgtca gtcagggaca acaagatcct taatgcagag ctagaggact tctggcaggg 1200

aagtggggaa gtgttccaga tagcagggca tgaaaactta gagaggtaca agtggctgaa 1260

aatcgagttt ttcctctgtc tttaaatttt atatgggctt tgttatcttc cactggaaaa 1320

gtgtaatagc atacatcaat ggtgtgttaa agctatttcc ttgccttttt tttattggaa 1380

tggtaggata tcttggcttt gccacacaca gttacagagt gaacactcta ctacatgtga 1440

ctggcagtat taagtgtgct tattttaaat gttactggta gaaaggcagt tcaggtatgt 1500

gtgtatatag tatgaatgca gtggggacac cctttgtggt tacagtttga gacttccaaa 1560

ggtcatcctt aataacaaca gatctgcagg ggtatgtttt accatctgca tccagcctcc 1620
tgctaactcc tagctgactc agcatagatt gtataaaata cctttgtaac ggctcttagc 1680
acactcacag atgtttgagg ctttcagaag ctcttctaaa aaatgataca cacctttcac 1740
aagggcaaac tttttccttt tccctgtgta ttctagtgaa tgaatctcaa gattcagtag 1800
acctaatgac atttgtattt tatgatcttg gctgtattta atggcatagg ctgacttttg 1860
cagatggagg aatttcttga ttaatgttga aaaaaaaccc ttgattatac tctgttggac 1920
aaaccgagtg caatgaatga tgcttttctg aaaatgaaat ataacaagtg ggtgaatgtg 1980
gttatggccg aaaaggatat gcagtatgct taatggtagc aactgaaaga agacatcctg 2040
agcagtgcca gctttcttct gttgatgccg ttccctgaac ataggaaaat agaaacttgc 2100
ttatcaaaac ttagcattac cttggtgctc tgtgttctct gttagctcag tgtctttcct 2160
tacatcaata ggtttttttt tttttttttg gcctgaggaa gtactgacca tgcccacagc 2220
caccggctga gcaaagaagc tcatttcatg tgagttctaa ggaatgagaa acaattttga 2280
tgaatttaag cagaaaatga atttctggga actttttttgg gggcgggggg gtggggaatt 2340
cagccacact ccagaaagcc aggagtcgac agttttggaa gcctctctca ggattgagat 2400
tctaggatga gattggctta ctgctatctt gtgtcatgta cccacttttt ggccagacta 2460
cactgggaag aaggtagtcc tctaaagcaa aatctgagtg ccactaaatg gggagatggg 2520
gctgttaagc tgtccaaatc aacaagggtc atataaatgg ccttaaactt tggggttgct 2580
ttctgcaaaa agttgctgtg actcatgcca tagacaaggt tgagtgcctg gacccaaagg 2640
caatactgta atgtaaagac atttatagta ctaggcaaac agcaccccag gtactccagg 2700
ccctcctggc tggagagggc tgtggcaata gaaaattagt gccaactgca gtgagtcagc 2760
ctaggttaaa tagagagtgt aagagtgctg gacaggaacc tccaccctca tgtcacattt 2820
cttcaatgtg acccttctgg cccctctcct cctgacagcg gaacaatgac tgccccgata 2880
ggtgaggctg gaggaagaat cagtcctgtc cttggcaagc tcttcactat gacagtaaag 2940
gctctctgcc tgctgccaag gcctgtgact ttctaacctg gcctcacgct gggtaagctt 3000
aaggtagagg tgcaggatta gcaagcccac ctggctacca ggccgacagc tacatcctcc 3060
aactgaccct gatcaacgaa gagggattca tgtgtctgtc tcagttggtt ccaaatgaaa 3120
ccagggagca ggggagttag gaatcgaaca ccagtcatgc ctactggctc tctgctcgag 3180
agccaatacc ctgtgccctc cactcatctg gatttacagg aactgtcata gtgttcagta 3240
ttgggtggtg ataagcccat tggattgtcc ccttgggggg atgagctagg ggtgcaagga 3300
acacctgatg agtagataag tggagctcat ggtatttcct gaaagatgct aatctatttg 3360
ccaaacttgg tcttgaatgt actgggggct tcaaggtatg ggtatatttt tcttgtgtcc 3420
ttgcagttag cccccatgtc ttatgtgtgt cctgaaaaaa taagagcctg cccaagactt 3480

tgggcctctt gacagaatta accactttta tacatctgag ttctcttggt aagttcttta 3540
gcagtgttca aagtctacta gctcgcatta gtttctgttg ctgccaacag atctgaacta 3600
atgctaacag atccccctga gggattcttg atgggctgag cagctggctg gagctagtac 3660
tgactgacat tcattgtgat gagggcagct ttctggtaca ggattctaag ctctatgttt 3720
tatatacatt ttcatctgta cttgcacctc actttacaca agaggaaact atgcaaagtt 3780
agctggatcg ctcaaggtca cttaggtaag ttggcaagtc catgcttccc actcagctcc 3840
tcaggtcagc aagtctactt ctctgcctat tttgtatact ctctttaata tgtgcctagc 3900
tttggaaagt ctagaatggg tccctggtgc ctttttactt tgaagaaatc agtttctgcc 3960
tctttttgga aaagaaaaca aagtgcaatt gtttttttact ggaaagttac ccaatagcat 4020
gaggtgaaca ggacgtagtt aggccttcct gtaaacagaa aatcatatca aaacactatc 4080
ttcccatctg tttctcaatg cctgctactt cttgtagata tttcatttca ggagagcagc 4140
agttaaaccc gtggattttg tagttaggaa cctgggttca aaccctcttc cactaattgg 4200
ctatgtctct ggacaagttt tttttttttt ttttttttaa accctttctg aactttcact 4260
ttctatgtct acctcaaaga attgttgtga ggcttgagat aatgcatttg taaagggtct 4320
gccagatagg aagatgctag ttatggattt acaaggttgt taaggctgta agagtctaaa 4380
acctacagtg aatcacaatg catttacccc cactgacttg gacataagtg aaaactagcc 4440
agaagtctct ttttcaaatt acttacaggt tattcaatat aaaatttttg taatggataa 4500
tcttatttat ctaaactaaa gcttcctgtt tatacacact cctgttattc tgggataaga 4560
taaatgacca cagtacctta atttctaggt gggtgcctgt gatggttcat tgtaggtaag 4620
gacattttct ctttttcagc agctgtgtag gtccagagcc tctgggagag gagggggggta 4680
gcatgcaccc agcaggggac tgaactggga aactcaaggt tctttttact gtggggtagt 4740
gagctgcctt tctgtgatcg gtttccctag ggatgttgct gttcccctcc ttgctattcg 4800
cagctacata caacgtggcc aaccccagta ggctgatcct atatatgatc agtgctggtg 4860
ctgactctca atagccccac ccaagctggc tataggttta cagatacatt aattaggcaa 4920
cctaaaatat tgatgctggt gttggtgtga cataatgcta tggccagaac tgaaacttag 4980
agttataatt catgtattag ggttctccag agggacagaa ttagtaggat atatgtatat 5040
atgaaaggga ggttattagg gagaactggc tcccacagtt agaaggcgaa gtcgcacaat 5100
aggccgtctg caagctgggt tagagagaag ccagtagtgg ctcagcctga gttcaaaaac 5160
ctcaaaactg gggaagctga cagtgcagcc agccttcagt ctgtggccaa aggcccaaga 5220
gcccctggca accaacccac tggtgcaagt cctagattcc aaaggctgaa gaacctggag 5280
tctgatgtcc aagagcagga agagtggaag aaagccagaa gactcagcaa acaaggtaga 5340

cagtgtctac caccatagtg gccataccaa agaggctacc gattccttcc tgctacctgg 5400
atccctgaag ttgccctggt ctctgcacct tctaaaccta gttcttaaga gctttccatt 5460
acatgagctg tctcaaagcc ctccaataaa ttctcagtgt aagcttctgt tgcttgtgga 5520
cagaaaattc tgacagacct accctataag tgttactgtc aggataacat gagaacgcac 5580
aacagtaagt ggtcactaag tgttagctac ggttattttg cccaaggtag catggctagt 5640
tgatgccggt tgatggggct taaacccagc tccctcatct tccaggcctc tgtactccct 5700
attccactaa actacctctc aggtttattt ttttaaattc ttactctgca agtacatagg 5760
accacattta cctgggaaaa caagaataaa ggctgctctg catttttag aaactttttt 5820
gaaagggaga tgggaatgcc tgcacccccca agtccagacc aacacaatgg ttaattgaga 5880
tgaataataa aggaaagact gttctgggct tcccagaata gcttggtcct taaattgtgg 5940
cacaaacaac ctcctgtcag agccagcctc ctgccaggaa gaggggtagg agactagagg 6000
ccgtgtgtgc agccttgccc tgaaggctag ggtgacaatt tggaggctgt ccaaacaccc 6060
tggcctctag agctggcctg tctatttgaa atgccggctc tgatgctaat cggcgaccct 6120
caggcaagtt acttaacctt acatgcctca gttttctcat ctggaaaatg agaaccctag 6180
gtttagggtt gttagaaaag ttaaatgagt taagacaagt gcctgggaca cagtagcctc 6240
ttgtgtgtgt ttatcattat gtcctcagca ggtcgtagaa gcagcttctc aggtgtgagg 6300
ctggcgcgat tatctggagt gggttgggtt ttctaggatg gaccccctgc tgcattttcc 6360
tcattcatcc accagggctt aatggggaat caaggaatcc atgtgtaact gtataataac 6420
tgtagccaca ctccaatgac cacctactag ttgtccctgg cactgcttat acatatgtcc 6480
atcaaatcaa tcctatgaag tagatactgt cttcatttta tagatcagag acaattgggg 6540
ttcagagagc tgatgtgatt ttcccagggt cacagagagt cccagattca ggcacaactc 6600
ttgtattcca agacacaacc actacatgtc caaaggctgc ccagagccac cgggcacggc 6660
aaattgtgac atatccctaa agaggctgag cacctggtca ggatctgatg gctgacagtg 6720
tgtccagatg cagagctgga gtgggggagg ggaaggggggg ctccttggga cagagaaggc 6780
tttctgtgct ttctctgaag ggagcagtct gaggaccaag ggaacccggc aaacagcacc 6840
tcaggtactc caggccctcc tggctggaga gggctgtggc aatggaaaat tagtgccaac 6900
tgcaatgagt cagcctcggt taaatagaga gtgaagaatg ctggacagga acctccaccc 6960
tcatgtcaca tttcttcagt gtgacccttc tggcccctct cctcctgaca gcggaacaat 7020
gactgccccg ataggtgagg ctggaggaag aatcagtcct gtccttggca agctcttcac 7080
tatgacagta aaggctctct gcctgctgcc aaggcctgtg actttctaac ctggcctcac 7140
gctgggtaag cttaaggtag aggtgcagga ttagcaagcc cacctggcta ccaggccgac 7200
agctacatct ttcaactgac cctgatcaac gaagagggac ttgtgtctct cagttggttc 7260

caaatgaaac cagggagcag gggcgttagg aagctccaac aggatggtac ttaatggggc 7320

atttgagtgg agaggtaggt gacatagtgc tttggagccc agggagggaa aggttctgct 7380

gaagttgaat tcaagactgt tctttcatca caaacttgag tttcctggac atttgtttgc 7440

agaaacaacc gtagggtttt gccttaacct cgtgggttta ttattacctc atagggactt 7500

tgcctcctga cagcagttta tgggtgttca ttgtggcact tgagttttct tgcatacttg 7560

ttagagaaac caagtttgtc atcaacttct tatttaaccc cctggctata acttcatgga 7620

ttatgttata attaagccat ccagagtaaa atctgtttag attatcttgg agtaaggggg 7680

aaaaaatctg taattttttc tcctcaacta gatatataca taaaaaatga ttgtattgct 7740

tcatttaaaa aatataacgc aaaatctctt ttccttctaa aaaaaaaaaa aaaaaa 7796

<210> 36
<211> 7680
<212> mRNA
<213> Homo sapiens

<400> 36
ggggccgggg ggcggagcct tgcgggctgg agcgaaagaa tgcgggggct gagcgcagaa 60

gcggctcgag gctggaagag gatcttgggc gccgccagtc tttagcacca gttggtgtag 120

gagttgagac ctacttcaca gtagttctgt ggacaatcac aatgggaatc caaggagggt 180

ctgtcctgtt cgggctgctg ctcgtcctgg ctgtcttctg ccattcaggt catagcctgc 240

agtgctacaa ctgtcctaac ccaactgctg actgcaaaac agccgtcaat tgttcatctg 300

attttgatgc gtgtctcatt accaaagctg ggttacaagt gtataacaag tgttggaagt 360

ttgagcattg caatttcaac gacgtcacaa cccgcttgag ggaaaatgag ctaacgtact 420

actgctgcaa gaaggacctg tgtaacttta acgaacagct tgaaaatggt gggacatcct 480

tatcagagaa aacagttctt ctgctggtga ctccatttct ggcagcagcc tggagccttc 540

atccctaagt caacaccagg agagcttctc ccaaactccc cgttcctgcg tagtccgctt 600

tctcttgctg ccacattcta aaggcttgat attttccaaa tggatcctgt tgggaaagaa 660

taaaattagc ttgagcaacc tggctaagat agaggggctc tgggagactt tgaagaccag 720

tcctgtttgc agggaagccc cacttgaagg aagaagtcta agagtgaagt aggtgtgact 780

tgaactagat tgcatgcttc ctcctttgct cttgggaaga ccagctttgc agtgacagct 840

tgagtgggtt ctctgcagcc ctcagattat ttttcctctg gctccttgga tgtagtcagt 900

tagcatcatt agtacatctt tggagggtgg ggcaggagta tatgagcatc ctctctcaca 960

tggaacgctt tcataaactt cagggatccc gtgttgccat ggaggcatgc caaatgttcc 1020

atatgtgggt gtcagtcagg gacaacaaga tccttaatgc agagctagag gacttctggc 1080

agggaagtgg ggaagtgttc cagatagcag ggcatgaaaa cttagagagg tacaagtggc 1140

tgaaaatcga gtttttcctc tgtctttaaa ttttatatgg gctttgttat cttccactgg 1200
aaaagtgtaa tagcatacat caatggtgtg ttaaagctat ttccttgcct ttttttatt 1260
ggaatggtag gatatcttgg ctttgccaca cacagttaca gagtgaacac tctactacat 1320
gtgactggca gtattaagtg tgcttatttt aaatgttact ggtagaaagg cagttcaggt 1380
atgtgtgtat atagtatgaa tgcagtgggg acaccctttg tggttacagt ttgagacttc 1440
caaaggtcat ccttaataac aacagatctg caggggtatg ttttaccatc tgcatccagc 1500
ctcctgctaa ctcctagctg actcagcata gattgtataa aatacctttg taacggctct 1560
tagcacactc acagatgttt gaggctttca gaagctcttc taaaaaatga tacacacctt 1620
tcacaagggc aaactttttc cttttccctg tgtattctag tgaatgaatc tcaagattca 1680
gtagacctaa tgacatttgt attttatgat cttggctgta tttaatggca taggctgact 1740
tttgcagatg gaggaatttc ttgattaatg ttgaaaaaaa acccttgatt atactctgtt 1800
ggacaaaccg agtgcaatga atgatgcttt tctgaaaatg aaatataaca agtgggtgaa 1860
tgtggttatg gccgaaaagg atatgcagta tgcttaatgg tagcaactga aagaagacat 1920
cctgagcagt gccagctttc ttctgttgat gccgttccct gaacatagga aaatagaaac 1980
ttgcttatca aaacttagca ttaccttggt gctctgtgtt ctctgttagc tcagtgtctt 2040
tccttacatc aataggtttt tttttttttt tttggcctga ggaagtactg accatgccca 2100
cagccaccgg ctgagcaaag aagctcattt catgtgagtt ctaaggaatg agaaacaatt 2160
ttgatgaatt taagcagaaa atgaatttct gggaactttt ttgggggcgg gggggtgggg 2220
aattcagcca cactccagaa agccaggagt cgacagtttt ggaagcctct ctcaggattg 2280
agattctagg atgagattgg cttactgcta tcttgtgtca tgtacccact ttttggccag 2340
actacactgg gaagaaggta gtcctctaaa gcaaaatctg agtgccacta aatggggaga 2400
tggggctgtt aagctgtcca aatcaacaag ggtcatataa atggccttaa actttggggt 2460
tgctttctgc aaaaagttgc tgtgactcat gccatagaca aggttgagtg cctggaccca 2520
aaggcaatac tgtaatgtaa agacatttat agtactaggc aaacagcacc ccaggtactc 2580
caggccctcc tggctggaga gggctgtggc aatagaaaat tagtgccaac tgcagtgagt 2640
cagcctaggt taaatagaga gtgtaagagt gctggacagg aacctccacc ctcatgtcac 2700
atttcttcaa tgtgaccctt ctggcccctc tcctcctgac agcggaacaa tgactgcccc 2760
gataggtgag gctggaggaa gaatcagtcc tgtccttggc aagctcttca ctatgacagt 2820
aaaggctctc tgcctgctgc caaggcctgt gactttctaa cctggcctca cgctgggtaa 2880
gcttaaggta gaggtgcagg attagcaagc ccacctggct accaggccga cagctacatc 2940
ctccaactga ccctgatcaa cgaagaggga ttcatgtgtc tgtctcagtt ggttccaaat 3000

gaaaccaggg agcaggggag ttaggaatcg aacaccagtc atgcctactg gctctctgct 3060
cgagagccaa taccctgtgc cctccactca tctggattta caggaactgt catagtgttc 3120
agtattgggt ggtgataagc ccattggatt gtccccttgg ggggatgagc taggggtgca 3180
aggaacacct gatgagtaga taagtggagc tcatggtatt tcctgaaaga tgctaatcta 3240
tttgccaaac ttggtcttga atgtactggg ggcttcaagg tatgggtata tttttcttgt 3300
gtccttgcag ttagcccccca tgtcttatgt gtgtcctgaa aaaataagag cctgcccaag 3360
actttgggcc tcttgacaga attaaccact tttatacatc tgagttctct tggtaagttc 3420
tttagcagtg ttcaaagtct actagctcgc attagtttct gttgctgcca acagatctga 3480
actaatgcta acagatcccc ctgagggatt cttgatgggc tgagcagctg gctggagcta 3540
gtactgactg acattcattg tgatgagggc agctttctgg tacaggattc taagctctat 3600
gttttatata cattttcatc tgtacttgca cctcacttta cacaagagga aactatgcaa 3660
agttagctgg atcgctcaag gtcacttagg taagttggca agtccatgct tcccactcag 3720
ctcctcaggt cagcaagtct acttctctgc ctattttgta tactctcttt aatatgtgcc 3780
tagctttgga aagtctagaa tgggtccctg gtgccttttt actttgaaga aatcagtttc 3840
tgcctctttt tggaaaagaa aacaaagtgc aattgttttt tactggaaag ttacccaata 3900
gcatgaggtg aacaggacgt agttaggcct tcctgtaaac agaaaatcat atcaaaacac 3960
tatcttccca tctgtttctc aatgcctgct acttcttgta gatatttcat ttcaggagag 4020
cagcagttaa acccgtggat tttgtagtta ggaacctggg ttcaaaccct cttccactaa 4080
ttggctatgt ctctggacaa gttttttttt tttttttttt ttaaaccctt tctgaacttt 4140
cactttctat gtctacctca aagaattgtt gtgaggcttg agataatgca tttgtaaagg 4200
gtctgccaga taggaagatg ctagttatgg atttacaagg ttgttaaggc tgtaagagtc 4260
taaaacctac agtgaatcac aatgcattta cccccactga cttggacata agtgaaaact 4320
agccagaagt ctctttttca aattacttac aggttattca atataaaatt tttgtaatgg 4380
ataatcttat ttatctaaac taaagcttcc tgtttataca cactcctgtt attctgggat 4440
aagataaatg accacagtac cttaatttct aggtgggtgc ctgtgatggt tcattgtagg 4500
taaggacatt ttctcttttt cagcagctgt gtaggtccag agcctctggg agaggagggg 4560
ggtagcatgc acccagcagg ggactgaact gggaaactca aggttctttt tactgtgggg 4620
tagtgagctg cctttctgtg atcggtttcc ctagggatgt tgctgttccc ctccttgcta 4680
ttcgcagcta catacaacgt ggccaacccc agtaggctga tcctatatat gatcagtgct 4740
ggtgctgact ctcaatagcc ccacccaagc tggctatagg tttacagata cattaattag 4800
gcaacctaaa atattgatgc tggtgttggt gtgacataat gctatggcca gaactgaaac 4860
ttagagttat aattcatgta ttagggttct ccagagggac agaattagta ggatatatgt 4920

atatatgaaa gggaggttat tagggagaac tggctcccac agttagaagg cgaagtcgca 4980
caataggccg tctgcaagct gggttagaga gaagccagta gtggctcagc ctgagttcaa 5040
aaacctcaaa actggggaag ctgacagtgc agccagcctt cagtctgtgg ccaaaggccc 5100
aagagcccct ggcaaccaac ccactggtgc aagtcctaga ttccaaaggc tgaagaacct 5160
ggagtctgat gtccaagagc aggaagagtg gaagaaagcc agaagactca gcaaacaagg 5220
tagacagtgt ctaccaccat agtggccata ccaaagaggc taccgattcc ttcctgctac 5280
ctggatccct gaagttgccc tggtctctgc accttctaaa cctagttctt aagagctttc 5340
cattacatga gctgtctcaa agccctccaa taaattctca gtgtaagctt ctgttgcttg 5400
tggacagaaa attctgacag acctacccta taagtgttac tgtcaggata acatgagaac 5460
gcacaacagt aagtggtcac taagtgttag ctacggttat tttgcccaag gtagcatggc 5520
tagttgatgc cggttgatgg ggcttaaacc cagctccctc atcttccagg cctctgtact 5580
ccctattcca ctaaactacc tctcaggttt attttttaa attcttactc tgcaagtaca 5640
taggaccaca tttacctggg aaaacaagaa taaaggctgc tctgcatttt ttagaaactt 5700
ttttgaaagg gagatgggaa tgcctgcacc cccaagtcca gaccaacaca atggttaatt 5760
gagatgaata ataaaggaaa gactgttctg ggcttcccag aatagcttgg tccttaaatt 5820
gtggcacaaa caacctcctg tcagagccag cctcctgcca ggaagagggg taggagacta 5880
gaggccgtgt gtgcagcctt gccctgaagg ctagggtgac aatttggagg ctgtccaaac 5940
accctggcct ctagagctgg cctgtctatt tgaaatgccg gctctgatgc taatcggcga 6000
ccctcaggca agttacttaa ccttacatgc ctcagttttc tcatctggaa aatgagaacc 6060
ctaggtttag ggttgttaga aaagttaaat gagttaagac aagtgcctgg gacacagtag 6120
cctcttgtgt gtgtttatca ttatgtcctc agcaggtcgt agaagcagct tctcaggtgt 6180
gaggctggcg cgattatctg gagtgggttg ggttttctag gatggacccc ctgctgcatt 6240
ttcctcattc atccaccagg gcttaatggg gaatcaagga atccatgtgt aactgtataa 6300
taactgtagc cacactccaa tgaccaccta ctagttgtcc ctggcactgc ttatacatat 6360
gtccatcaaa tcaatcctat gaagtagata ctgtcttcat tttatagatc agagacaatt 6420
ggggttcaga gagctgatgt gattttccca gggtcacaga gagtcccaga ttcaggcaca 6480
actcttgtat tccaagacac aaccactaca tgtccaaagg ctgcccagag ccaccgggca 6540
cggcaaattg tgacatatcc ctaaagaggc tgagcacctg gtcaggatct gatggctgac 6600
agtgtgtcca gatgcagagc tggagtgggg gaggggaagg ggggctcctt gggacagaga 6660
aggctttctg tgctttctct gaagggagca gtctgaggac caagggaacc cggcaaacag 6720
cacctcaggt actccaggcc ctcctggctg gagagggctg tggcaatgga aaattagtgc 6780

caactgcaat gagtcagcct cggttaaata gagagtgaag aatgctggac aggaacctcc 6840

accctcatgt cacatttctt cagtgtgacc cttctggccc ctctcctcct gacagcggaa 6900

caatgactgc cccgataggt gaggctggag gaagaatcag tcctgtcctt ggcaagctct 6960

tcactatgac agtaaaggct ctctgcctgc tgccaaggcc tgtgactttc taacctggcc 7020

tcacgctggg taagcttaag gtagaggtgc aggattagca agcccacctg gctaccaggc 7080

cgacagctac atctttcaac tgaccctgat caacgaagag ggacttgtgt ctctcagttg 7140

gttccaaatg aaaccaggga gcaggggcgt taggaagctc caacaggatg gtacttaatg 7200

gggcatttga gtggagaggt aggtgacata gtgctttgga gcccagggag ggaaaggttc 7260

tgctgaagtt gaattcaaga ctgttctttc atcacaaact tgagtttcct ggacatttgt 7320

ttgcagaaac aaccgtaggg ttttgcctta acctcgtggg tttattatta cctcataggg 7380

actttgcctc ctgacagcag tttatgggtg ttcattgtgg cacttgagtt ttcttgcata 7440

cttgttagag aaaccaagtt tgtcatcaac ttcttattta accccctggc tataacttca 7500

tggattatgt tataattaag ccatccagag taaaatctgt ttagattatc ttggagtaag 7560

ggggaaaaaa tctgtaattt tttctcctca actagatata tacataaaaa atgattgtat 7620

tgcttcattt aaaaaatata acgcaaaatc tcttttcctt ctaaaaaaaa aaaaaaaaaa 7680

<210> 37
<211> 2979
<212> mRNA
<213> Homo sapiens

<400> 37
gggcagcctg ctgtcggctt agaggggatg ggcagtgtgg agggcctggc agagcaagag 60

gactcatcct tccaaaggga ctttctctgg gaagcctgct cctcgggcca ctgcgaaccc 120

tctctactct ccgaagggaa ttgtccttcc tggcttccac tacttccacc cctgaatgca 180

caggcagccc ggcccaagtc tcccactagg gatgcagatg gattcggtgt gaagggctgg 240

ctgctgttgc ctccggctct tgaaagtcaa gttcagaggc gtgcaaagac tccagaattg 300

gaggcatgat gaagactctg ctgctgtttg tggggctgct gctgacctgg gagagtgggc 360

aggtcctggg ggaccagacg gtctcagaca atgagctcca ggaaatgtcc aatcagggaa 420

gtaagtacgt caataaggaa attcaaaatg ctgtcaacgg ggtgaaacag ataaagactc 480

tcatagaaaa aacaaacgaa gagcgcaaga cactgctcag caacctagaa gaagccaaga 540

agaagaaaga ggatgcccta aatgagacca gggaatcaga gacaaagctg aaggagctcc 600

caggagtgtg caatgagacc atgatggccc tctgggaaga gtgtaagccc tgcctgaaac 660

agacctgcat gaagttctac gcacgcgtct gcagaagtgg ctcaggcctg gttggccgcc 720

agcttgagga gttcctgaac cagagctcgc ccttctactt ctggatgaat ggtgaccgca 780

tcgactccct gctggagaac gaccggcagc agacgcacat gctggatgtc atgcaggacc 840
acttcagccg cgcgtccagc atcatagacg agctcttcca ggacaggttc ttcacccggg 900
agccccagga tacctaccac tacctgccct tcagcctgcc ccaccggagg cctcacttct 960
tctttcccaa gtcccgcatc gtccgcagct tgatgccctt ctctccgtac gagcccctga 1020
acttccacgc catgttccag cccttccttg agatgataca cgaggctcag caggccatgg 1080
acatccactt ccatagcccg gccttccagc acccgccaac agaattcata cgagaaggcg 1140
acgatgaccg gactgtgtgc cgggagatcc gccacaactc cacgggctgc ctgcggatga 1200
aggaccagtg tgacaagtgc cgggagatct tgtctgtgga ctgttccacc aacaacccct 1260
cccaggctaa gctgcggcgg gagctcgacg aatccctcca ggtcgctgag aggttgacca 1320
ggaaatacaa cgagctgcta aagtcctacc agtggaagat gctcaacacc tcctccttgc 1380
tggagcagct gaacgagcag tttaactggg tgtcccggct ggcaaacctc acgcaaggcg 1440
aagaccagta ctatctgcgg gtcaccacgg tggcttccca cacttctgac tcggacgttc 1500
cttccggtgt cactgaggtg gtcgtgaagc tctttgactc tgatcccatc actgtgacgg 1560
tccctgtaga agtctccagg aagaacccta aatttatgga gaccgtggcg gagaaagcgc 1620
tgcaggaata ccgcaaaaag caccgggagg agtgagatgt ggatgttgct tttgcaccta 1680
cgggggcatc tgagtccagc tccccccaag atgagctgca gccccccaga gagagctctg 1740
cacgtcacca agtaaccagg ccccagcctc caggccccca actccgccca gcctctcccc 1800
gctctggatc ctgcactcta acactcgact ctgctgctca tgggaagaac agaattgctc 1860
ctgcatgcaa ctaattcaat aaaactgtct tgtgagctga tcgcttggag ggtcctcttt 1920
ttatgttgag ttgctgcttc ccggcatgcc ttcattttgc tatggggggc aggcaggggg 1980
gatggaaaat aagtagaaac aaaaaagcag tggctaagat ggtataggga ctgtcatacc 2040
agtgaagaat aaaagggtga agaataaaag ggatatgatg acaaggttga tccacttcaa 2100
gaattgcttg ctttcaggaa gagagatgtg tttcaacaag ccaactaaaa tatattgctg 2160
caaatggaag cttttctgtt ctattataaa actgtcgatg tattctgacc aaggtgcgac 2220
aatctcctaa aggaatacac tgaaagttaa ggagaagaat cagtaagtgt aaggtgtact 2280
tggtattata atgcataatt gatgttttcg ttatgaaaac atttggtgcc cagaagtcca 2340
aattatcagt tttatttgta agagctattg cttttgcagc ggttttattt gtaaaagctg 2400
ttgatttcga gttgtaagag ctcagcatcc caggggcatc ttcttgactg tggcatttcc 2460
tgtccaccgc cggtttatat gatcttcata cctttccctg gaccacaggc gtttctcggc 2520
ttttagtctg aaccatagct gggctgcagt accctacgct gccagcaggt ggccatgact 2580
acccgtggta ccaatctcag tcttaaagct caggcttttc gttcattaac attctctgat 2640
agaattctgg tcatcagatg tactgcaatg gaacaaaact catctggctg catcccaggt 2700

gtgtagcaaa gtccacatgt aaatttatag cttagaatat tcttaagtca ctgtcccttg 2760

tctctctttg aagttataaa caacaaactt aaagcttagc ttatgtccaa ggtaagtatt 2820

ttagcatggc tgtcaaggaa attcagagta aagtcagtgt gattcactta atgatataca 2880

ttaattagaa ttatggggtc agaggtattt gcttaagtga tcataattgt aaagtatatg 2940

tcacattgtc acattaatgt caaaaaaaaa aaaaaaaaa 2979

<210> 38
<211> 20
<212> DNA
<213> Homo sapiens

<400> 38
cccttacaca tcctactcct 20

<210> 39
<211> 21
<212> DNA
<213> Homo sapiens

<400> 39
cagcaataac agacgtaacc g 21

<210> 40
<211> 133
<212> DNA
<213> Homo sapiens

<400> 40
cccttacaca tcctactcct tatttaaaag ttctatttgt gacttttcat ttcctgaaag 60

tttaaaaata caatttgaga atgtttataa tacattctct cctgtctttt cacggttacg 120

tctgttattg ctg 133

<210> 41
<211> 20
<212> DNA
<213> Homo sapiens

<400> 41
cacacctttc acaagggcaa 20

<210> 42
<211> 20
<212> DNA
<213> Homo sapiens

<400> 42
ctccatctgc aaaagtcagc 20

<210> 43
<211> 141

<212> DNA
<213> Homo sapiens

<400> 43
cacacctttc acaagggcaa actttttcct tttccctgtg tattctagtg aatgaatctc 60

aagattcagt agacctaatg acatttgtat tttatgatct tggctgtatt taatggcata 120

ggctgacttt tgcagatgga g 141

<210> 44
<211> 20
<212> DNA
<213> Homo sapiens

<400> 44
acaacgagct gctaaagtcc 20

<210> 45
<211> 20
<212> DNA
<213> Homo sapiens

<400> 45
gatagtactg gtcttcgcct 20

<210> 46
<211> 129
<212> DNA
<213> Homo sapiens

<400> 46
acaacgagct gctaaagtcc taccagtgga agatgctcaa cacctcctcc ttgctggagc 60

agctgaacga gcagtttaac tgggtgtccc ggctggcaaa cctcacgcaa ggcgaagacc 120

agtactatc 129

<210> 47
<211> 20
<212> DNA
<213> Homo sapiens

<400> 47
gtggaaggtg attgtttcgc 20

<210> 48
<211> 20
<212> DNA
<213> Homo sapiens

<400> 48
ggatcttccc agcttcaatg 20

<210> 49
<211> 149
<212> DNA

<213> Homo sapiens

<400> 49
gtggaaggtg attgtttcgc tggtcctgtt gatgcctggc ccctgtgatg ggctgtttcg 60

ctccctatac agaagtgttt ccatgccacc taagggagac tcaggacagc cattatttct 120

caccccttac attgaagctg ggaagatcc 149

<210> 50
<211> 20
<212> DNA
<213> Homo sapiens

<400> 50
ccgtaatggg attttctggg 20

<210> 51
<211> 19
<212> DNA
<213> Homo sapiens

<400> 51
gcttaaagtg cttgggtct 19

<210> 52
<211> 113
<212> DNA
<213> Homo sapiens

<400> 52
ccgtaatggg attttctggg gtacctggcc tggtgtaagt gaggcacacc ctggtggcta 60

caagtcctcc ttcaaagagg ctaagatgat gatcagaccc aagcacttta agc 113

<210> 53
<211> 24
<212> DNA
<213> Homo sapiens

<400> 53
cttctcactt catgtgaaaa ctac 24

<210> 54
<211> 23
<212> DNA
<213> Homo sapiens

<400> 54
ccatattagt gctaccaaag tgt 23

<210> 55
<211> 148
<212> DNA
<213> Homo sapiens

<400> 55

cttctcactt catgtgaaaa ctactccagt ggctgactga attgctgacc cttcaagctc 60

tgtccttatc cattacctca aagcagtcat tccttagtaa agtttccaac aaatagaaat 120

taatgacact ttggtagcac taatatgg 148

<210> 56
<211> 20
<212> DNA
<213> Homo sapiens

<400> 56
ctgtaaggca gatgatcgtc 20

<210> 57
<211> 21
<212> DNA
<213> Homo sapiens

<400> 57
tgttcagtgc tttctgacaa g 21

<210> 58
<211> 113
<212> DNA
<213> Homo sapiens

<400> 58
ctgtaaggca gatgatcgtc cccgtatgat gattgtcaga agacaggact aagtagcaga 60

gaatagctaa gagataaatt gggctgggga aacttgtcag aaagcactga aca 113

<210> 59
<211> 20
<212> DNA
<213> Homo sapiens

<400> 59
ctcagggctg tgtgtactaa 20

<210> 60
<211> 20
<212> DNA
<213> Homo sapiens

<400> 60
gtcaaagtct ctacccacag 20

<210> 61
<211> 98
<212> DNA
<213> Homo sapiens

<400> 61
ctcagggctg tgtgtactaa tacagactat gtcacccaat gcatatccaa catgtgctca 60

gggaataatc cagaaaaact gtgggtagag actttgac 98

<210> 62
<211> 20
<212> DNA
<213> Homo sapiens

<400> 62
ggtttagttg gactggatgg 20

<210> 63
<211> 20
<212> DNA
<213> Homo sapiens

<400> 63
aatagccgta agcctgtctc 20

<210> 64
<211> 114
<212> DNA
<213> Homo sapiens

<400> 64
ggtttagttg gactggatgg gatctggaaa taaatatttc ccaaaagttg tccatgggat 60

cctagcagtc agggttgaga attgctaagt tatagagaca ggcttacggc tatt 114

<210> 65
<211> 19
<212> DNA
<213> Homo sapiens

<400> 65
ggagtctaat tgcagttcc 19

<210> 66
<211> 20
<212> DNA
<213> Homo sapiens

<400> 66
ctgttactag tgacgtttgg 20

<210> 67
<211> 88
<212> DNA
<213> Homo sapiens

<400> 67
ggagtctaat tgcagttccc tgagccatgt gcctttctct tcactgagga ctgccccatt 60

cttgagtgcc aaacgtcact agtaacag 88

<210> 68
<211> 20
<212> DNA

<213>  Homo sapiens

<400>  68
gagtggtctg cagctatact                                                   20

<210>  69
<211>  20
<212>  DNA
<213>  Homo sapiens

<400>  69
aagcatgagt attgggaagg                                                   20

<210>  70
<211>  143
<212>  DNA
<213>  Homo sapiens

<400>  70
gagtggtctg cagctatact tccagtatct ccaagatatg actgtacttt ttcttgtatt      60

tatttactgg tgacagcttt tctttttata gttatatcac ttagaataat aattttgtta     120

tttccttccc aatactcatg ctt                                              143

<210>  71
<211>  18
<212>  DNA
<213>  Homo sapiens

<400>  71
gtgtaccctg actcgttc                                                     18

<210>  72
<211>  21
<212>  DNA
<213>  Homo sapiens

<400>  72
agagtgacag tgcatacaat g                                                 21

<210>  73
<211>  119
<212>  DNA
<213>  Homo sapiens

<400>  73
gtgtaccctg actcgttctc tggggtttcc ctcttctctc tttccacgga aggttgtgat      60

gaagaggaag tcttaaagtc cctaaagttc tccctttca ttgtatgcac tgtcactct     119

<210>  74
<211>  21
<212>  DNA
<213>  Homo sapiens

<400>  74

actttctgtat ccagcacaga c 21

<210> 75
<211> 21
<212> DNA
<213> Homo sapiens

<400> 75
ccctcaacaa acaagatcag c 21

<210> 76
<211> 203
<212> DNA
<213> Homo sapiens

<400> 76
acttctgtat ccagcacaga caccctttgg accacagctg cagacacggg agtcgcccca 60

ccatcaaagc tgggtgagaa gagactccgc tgcgatggct cggagcacgc agtagcatgc 120

gtggaagcag cttacactct agtgggaagt ggagccccat tgagcaccat cccacactgg 180

ctgctgatct tgtttgttga ggg 203

<210> 77
<211> 20
<212> DNA
<213> Homo sapiens

<400> 77
gctaaattcc acactaccac 20

<210> 78
<211> 20
<212> DNA
<213> Homo sapiens

<400> 78
ttgagtgcag cggtgtgaac 20

<210> 79
<211> 157
<212> DNA
<213> Homo sapiens

<400> 79
gctaaattcc acactaccac attaaaaaaa ttagaaagta gccacgtatg gtggctcatg 60

tctataatcc cagcactttg ggaggttgag gtgggaggat tgcttgaacc caagaggtca 120

aggctgcagt gagccatgtt cacaccgctg cactcaa 157

<210> 80
<211> 19
<212> DNA
<213> Homo sapiens

<400> 80
tgacagagcc agtgggaag 19

<210> 81
<211> 19
<212> DNA
<213> Homo sapiens

<400> 81
caattctcct acctcaacc 19

<210> 82
<211> 224
<212> DNA
<213> Homo sapiens

<400> 82
tgacagagcc agtgggaaga tacagaaaaa gaggggctgg gctgggcgcg gtggttcacg 60

cctgtaatcc cagcactttg ggaggccaag gagggtggat cacctgaggt caggagttag 120

aggccagcct ggcgaaaccc catctctact aaaaatacaa aatccaggcg tggtggcaca 180

cacctgtagt cccagctact caggaggttg aggtaggaga attg 224

<210> 83
<211> 19
<212> DNA
<213> Homo sapiens

<400> 83
aggtgtgagc tactgtacc 19

<210> 84
<211> 18
<212> DNA
<213> Homo sapiens

<400> 84
ggattacagg catgcaac 18

<210> 85
<211> 115
<212> DNA
<213> Homo sapiens

<400> 85
aggtgtgagc tactgtaccc agccttaacc tgtttcacag ttgattatac ttcatgctgt 60

tttccagcat ggtattatta agggatttaa agtttgggtt gcatgcctgt aatcc 115